(19) Europäisches Patentamt
European Patent Office
Office européen des brevets

(11) EP 1 048 652 A1

(12) **EUROPEAN PATENT APPLICATION**
published in accordance with Art. 158(3) EPC

(43) Date of publication:
**02.11.2000 Bulletin 2000/44**

(21) Application number: **98961642.0**

(22) Date of filing: **28.12.1998**

(51) Int. Cl.7: **C07D 211/26**, C07D 401/04, C07D 401/06, C07D 401/14, A61K 31/495, A61K 31/505

(86) International application number:
**PCT/JP98/06002**

(87) International publication number:
**WO 99/33805 (08.07.1999 Gazette 1999/27)**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.12.1997 JP 36753897**
**30.10.1998 JP 31149198**

(71) Applicant:
**MOCHIDA PHARMACEUTICAL CO., LTD.**
**Shinjuku-ku Tokyo 160-8515 (JP)**

(72) Inventors:
- **NISHIDA, Hidemitsu**
  **Mochida Pharmaceutical Co., Ltd**
  **Tokyo 160-8515 (JP)**
- **HOSAKA, Yoshitaka**
  **Mochida Pharmaceutical Co., Ltd.**
  **Tokyo 160-8515 (JP)**
- **MIYAZAKI, Yutaka**
  **Mochida Pharmaceutical Co., Ltd.**
  **Tokyo 160-8515 (JP)**
- **MATSUSUE, Tomokazu**
  **Mochida Pharmaceutical Co., Ltd**
  **Tokyo 160-8515 (JP)**
- **MUKAIHIRA, Takafumi,**
  **Mochida Pharma. Co., Ltd**
  **Tokyo 160-8515 -8515 (JP)**

(74) Representative:
**Grünecker, Kinkeldey,**
**Stockmair & Schwanhäusser**
**Anwaltssozietät**
**Maximilianstrasse 58**
**80538 München (DE)**

(54) **AROMATIC COMPOUNDS HAVING CYCLIC AMINO OR SALTS THEREOF**

(57) Compounds represented by general formula (I) or salts thereof which specifically inhibit FXa, exert a potent anticoagulant effect and thus are useful as medicinal compositions, wherein $G_1$ to $G_4$, X and Y represent each CH or N; $Z_1$ represents $-SO_2-$ or $-CH_2-$; Q represents aryl or heteroaryl; and $R_1$ to $R_9$ represent each hydrogen or a substituent.

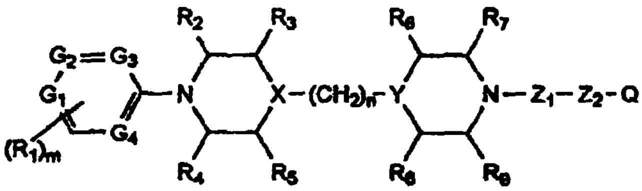

(I)

EP 1 048 652 A1

## Description

Technical Field

This invention relates to orally administrable aromatic compounds having cyclic amino groups or salts thereof that are useful as pharmaceuticals, particularly as an inhibitor of activated blood coagulation factor X (hereunder referred to as FXa), and which show potent anticoagulation action.

Background Art

With the recent shift to western life style and the increasing number of aged people, the incidence of thromboembolic diseases including ischemic heart diseases and many other cardiovascular lesions, in particular, myocardial infarction, cerebral thrombosis, pulmonary embolism and peripheral arteriovenous obstruction is increasing each year and the social importance of treating those diseases is ever increasing. In the treatment and prevention of these thrombotic cases, anticoagulation therapy as well as antiplatelet therapy and fibrinolytic therapy are important medical therapeutic methods. For the treatment and prevention of thrombosis, safety that permits long-term drug administration and the development of a positive and appropriate anticoagulant activity are essential.

Heretofore, anticoagulants such as warfarin and heparin have been used in order to prevent and treat thrombosis due to hypercoagulability but, at the same time, many defects of them have been pointed out, including the risk of bleeding and interactions with other drugs. Warfarin is extensively used in the world as the sole peroral anticoagulant. However, due to its characteristics based on the mechanism of action, the concentration range for the development of efficacy is narrow and yet it takes long to develop efficacy and the half-life in blood is as long as 36 hours; what is more, for several reasons such as the great individual difference of effective dose, it is difficult to control the anticoagulability of warfarin (N. Eng. J. Med. 324 (26) 1865-1875, 1991) and frequent monitoring is necessary to prevent bleeding as a side effect; in addition, warfarin has many other side effects such as nausea, vomiting, diarrhea and alopecia; thus, warfarin is a drug that involves considerable difficulty in clinical use. On the other hand, heparin is extensively used in the world as an intravenously administrable anticoagulant. However, since it is a direct inhibitor of thrombin, heparin has a high risk of bleeding and needs as frequent monitoring as warfarin; what is more, due to its characteristics based on the mechanism of action, adequate coagulation inhibiting effect is not expected at a lowered antithrombin III level; thus, heparin is a drug that involves considerable difficulty in clinical use. Under these circumstances, the advent of an improved anticoagulant has been desired that has none of the defects inherent in warfarin and heparin.

The blood coagulation cascade is a chain reaction involving restricted protein decomposition that starts upon activation of an extrinsic or intrinsic coagulation cascade and, once activated, the reaction amplifies like an avalanche. Since the final stage of the blood coagulation cascade is thrombin-mediated conversion of fibrinogen to fibrin, efforts have recently been made to develop thrombin inhibitors; however, drugs that directly inhibit thrombin are known to increase the risk of bleeding. In addition, they have low bioavailability in oral administration and no commercial thrombin inhibitor has ever been proposed that can be administrated perorally.

FXa which is located upstream of thrombin in the coagulation cascade is a key enzyme found at the point of convergence between the extrinsic and intrinsic coagulation cascades and one molecule of FXa is known to produce about 100 molecules of thrombin per minute. Hence, an FXa inhibitor can potentially inhibit the coagulation cascade more efficiently than a thrombin inhibitor (Thrombosis Research, Vol. 19, 339-349, 1980; Mebio, Vol. 14, No. 8, 1997).

Compounds that exhibit FXa inhibiting actions have been disclosed in several patents, among which Japanese Patent Application Laid-Opened No. 208946/1993 and WO96/16940 disclose aromatic amidine derivatives, in particular, amidinonaphthyl derivatives, and WO97/38984 discloses cyclic urea compounds having an amidinophenyl group. However, these compounds are still in the process of development and none have been commercialized to date. In addition to low bioavailability, they have slight dissociations to be improved between the thrombin or trypsin inhibiting action and the FXa inhibiting action and there is also a concern about the possible occurrence of side effects such as hypotension and dyspnea due to the amidino group.

Referring to the compounds it discloses, Japanese Patent Application Laid-Opened No. 208946/1993 teaches using them as an agent for preventing and treating infections with influenza virus by means of their activity in inhibiting the growth of the influenza virus based on the FXa inhibiting action.

Compounds having an aminoheterocyclic group typified by 1-(4-pyridyl)piperidin-4-yl group can be used as an FXa inhibitor; for example, WO96/10022 discloses

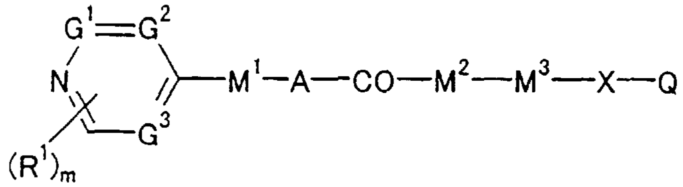

(the definitions of the substituents in the formula are omitted), WO97/29104 discloses

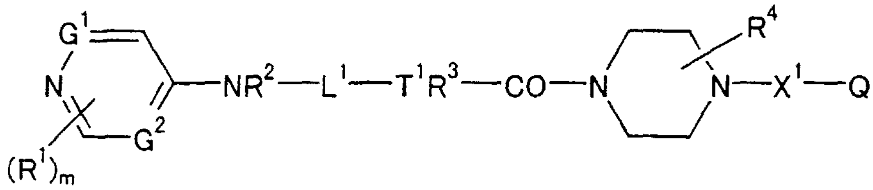

(the definitions of the substituents in the formula are omitted), and WO97/28129 discloses

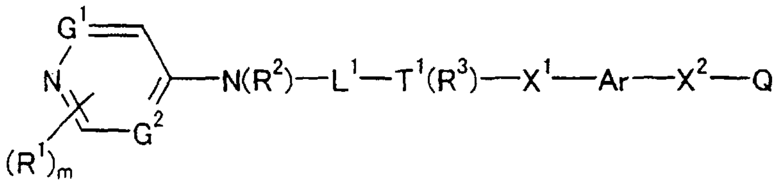

(wherein ... Ar is phenylene or a single 5- or 6-membered aromatic heterocyclic ring containing up to three hetero atoms selected from among a nitrogen atom, an oxygen atom and a sulfur atom, ...).

It has been reported that some of the compounds disclosed in those patents have the activity of inhibiting oxidosqualene cyclase (WO97/06802 and WO97/28128).

However, as of today, none of these compounds have been commercialized as pharmaceuticals. The five patents mentioned above claim an extremely broad scope of compounds but the bridge group linking two rings comprising combinations of piperazine or piperidine rings requires the presence of a carbonyl group as an essential component and there are no derivatives in which the two rings are bridged by an alkylene group alone or they are directly linked by a single bond.

With respect to an oxidosqualene cyclase inhibitor, two patents have been published that disclose the following two structures. WO98/35956 discloses

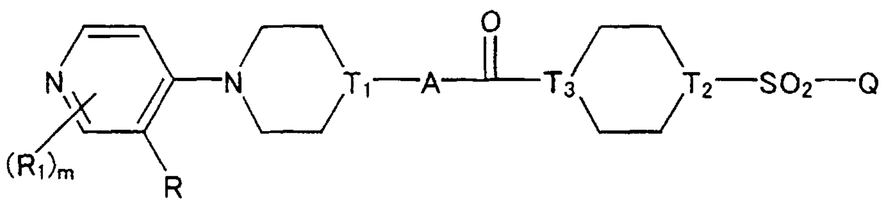

(the definitions of the substituents in the formula are omitted) and WO98/35959 discloses

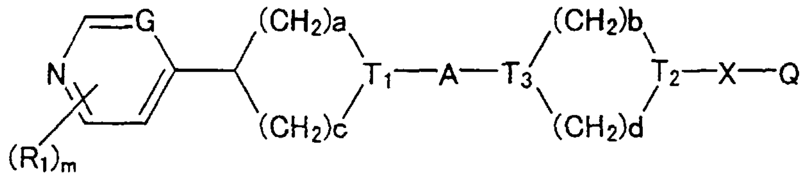

(the definitions of the substituents in the formula are omitted).

Like the compounds of other prior art techniques, the former compound requires the presence of a carbonyl group in the bride group as an essential component. The latter compound mainly has 4-(4-pyridyl)piperidin-1-yl group as the basic skeleton and because of this basic structural feature, it differs from the compounds of the present invention. According to the description, A is preferably $C_{1-4}$ alkylenecarbonyl or carbonyl. The specification of neither patent suggests the FXa inhibiting activity.

Compounds having an aminoheterocyclic group typified by 1-(4-pyridyl)piperidin-4-yl group can also be used as a platelet agglutination inhibitor and they have been disclosed in many patent applications including, for example, WO94/22834, WO94/22835, WO96/38416, EP718287, WO96/24581 and WO96/19223. However, intending to inhibit GPIIb/IIIa, the compounds disclosed in these patents have a characteristic structure in that an aliphatic carboxyl group, an aliphatic alkoxycarbonyl group or the like is positioned in a terminal side chain of the molecule remote from the aminoheterocyclic ring. FXa inhibiting action has not been reported for these compounds.

Disclosure of Invention

In the development of pharmaceutical products, the desired pharmacological activity is not the sole requirement but long-term safety is also needed. Another requirement is that strict criteria be met in various aspects including absorption, distribution, metabolism and excretion. To mention a few examples, drug interactions, desensitization or tolerance, gastrointestinal absorption upon oral administration, rate of transfer into the small intestine, absorption rate and the first pass effect, organ barrier, protein binding, induction of drug metabolizing enzymes, route of excretion and in vivo clearance, dose regimen (site of application, its method and object) and various other considerations need be satisfied but only a limited number of compounds have been found to meet these criteria.

Anticoagulants are not an exception and they are at all times required to satisfy the above-mentioned considerations in the development of pharmaceuticals. In addition, an FXa inhibitor must avoid the aforementioned problem of potential side effects in the oral administration of warfarin, as well as the risk of bleeding due to the thrombin inhibiting activity of heparin which can be administered only by intravenous injection.

Under these circumstances, an anticoagulant drug is needed that has high safety, exhibits high efficacy and provides greater ease of use. To be more specific, an anticoagulant drug is pressingly needed that has solved at least one of the aforementioned problems by, for example, eliminating interactions with other drugs, reducing side effects such as the risk of bleeding or improving dose response and which is orally administrable to mammals including man, with the particular advantage of being very convenient to use in clinical settings.

With a view to meeting this demand, the present inventors made intensive studies to provide compounds having an enhanced FXa inhibiting action. As a result, they found that among the aromatic compounds having cyclic amino groups, those in which two rings comprising combinations of piperazine or piperidine rings are bridged together by an alkylene or linked directly by a single bond, particularly those in which the nitrogen atom on either piperazine or piperidine ring is substituted with a group represented by the formula $-Z_1-Z_2-Q$, had an outstanding FXa inhibiting action. The present invention has been accomplished on the basis of this finding.

On the pages that follow, we describe the present invention in detail. The present invention relates to aromatic compounds having cyclic amino groups as represented by the formula (I) to be set forth below or pharmaceutically acceptable salts thereof.

To be specific, a first aspect of the present invention is to provide compounds represented by the formula (I) to be set forth below.

A second aspect of the present invention is to provide a pharmaceutical composition characterized by containing a compound represented by the formula (I) or a pharmaceutically acceptable salt thereof as an active ingredient.

A third aspect of the present invention is to provide an activated blood coagulation factor X (FXa) inhibitor characterized by containing a compound represented by the formula (I) or a pharmaceutically acceptable salt thereof as an active ingredient. More particularly, the inhibitor is a specific FXa inhibitor, or an orally administrable FXa inhibitor, or an orally administrable specific FXa inhibitor.

A fourth aspect of the present invention is to provide an anticoagulant characterized by containing a compound represented by the formula (I) or a pharmaceutically acceptable salt thereof as an active ingredient.

A fifth aspect of the present invention is to provide a preventive and/or a therapeutic agent for diseases caused by thrombus or embolus that is characterized by containing a compound represented by the formula (I) or a pharmaceutically acceptable salt thereof as an active ingredient.

A sixth aspect of the present invention is to provide a preventive and/or a therapeutic agent for diseases against which an anticoagulant is effective, characterized by containing a compound represented by the formula (I) or a pharmaceutically acceptable salt thereof as an active ingredient.

A seventh aspect of the present invention is to provide a preventive and/or a therapeutic agent for diseases against which an FXa inhibitor is effective, characterized by containing a compound represented by the formula (I) or a

pharmaceutically acceptable salt thereof as an active ingredient.

An eighth aspect of the present invention is to provide a preventive and/or therapeutic agent for embolus that accompanies atrial fibrillation, heart valve replacement or valvular heart disease, characterized by containing a compound represented by the formula (I) or a pharmaceutically acceptable salt thereof as an active ingredient. Preferably, it relates to a preventive agent for the onset of cerebral embolism that accompanies atrial fibrillation, heart valve replacement or valvular heart disease.

A ninth aspect of the present invention is to provide a preventive and/or therapeutic agent for transient cerebral ischemic attacks characterized by containing a compound represented by the formula (I) or a pharmaceutically acceptable salt thereof as an active ingredient. It particularly relates to a preventive agent for the recurrence of transient cerebral ischemic attacks.

A tenth aspect of the present invention is to provide a preventive and/or therapeutic agent for DIC characterized by containing a compound represented by the formula (I) or a pharmaceutically acceptable salt thereof as an active ingredient.

An eleventh aspect of the present invention is to provide a preventive and/or therapeutic agent for influenza viral infections characterized by containing a compound represented by the formula (I) or a pharmaceutically acceptable salt thereof as an active ingredient.

A twelfth aspect of the present invention is to provide a preventive and/or therapeutic agent for deep venous thrombosis characterized by containing a compound represented by the formula (I) or a pharmaceutically acceptable salt thereof as an active ingredient.

The first to twelfth aspects of the present invention encompass therapeutic and preventive methods comprising administering the stated compounds, pharmaceutical composition, FXa inhibitors, anticoagulant and preventives and/or therapeutics. Preferably, these aspects relate to compounds represented by the formula (II) to be set forth below or pharmaceutically acceptable salts thereof, more preferably to compounds represented by the formula (II'), particularly preferably to compounds represented by the formula (II''), or pharmaceutically acceptable salts thereof.

A thirteenth aspect of the present invention is to provide compounds represented by the formula (IV)-b to be set forth below which may be protected with suitable protective groups or salts thereof. In particular, it relates to compounds represented by the formula (IV')-b which may be protected with suitable protective groups, especially compounds represented by the formula (IV'')-b which may be protected with suitable protective groups, or salts thereof. These compounds (IV)-b, (IV')-b and (IV'')-b or salts thereof are at least useful as intermediates for the production of the compounds of the formulae (I), (II') and (II''), respectively, or salts thereof.

A fourteenth aspect of the present invention is to provide compounds represented by the formula (VI) to be set forth below which may be protected with suitable protective groups or salts thereof. In particular, it relates to compounds represented by the formula (VI') which may be protected with suitable protective groups, especially compounds represented by the formula (VI'') which may be protected with suitable protective groups, or salts thereof. These compounds (VI), (VI') and (VI'') or salts thereof are at least useful as intermediates for the production of the compounds of the formulae (I), (II') and (II''), respectively, or salts thereof.

The compounds of the present invention are aromatic compounds having cyclic amino groups as represented by the following formula (I) or pharmaceutically acceptable salts thereof:

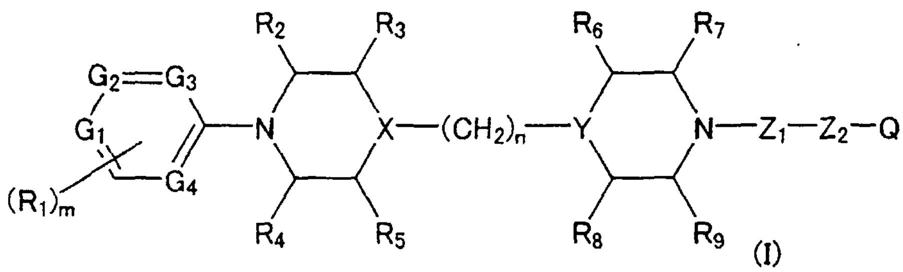

(I)

(wherein the respective symbols have the following meanings)

$G_1$, $G_2$, $G_3$ and $G_4$ are independently CH or N;
X and Y are independently CH or N;
$Z_1$ is a group represented by the formula $-SO_2-$ or $-CH_2-$;
$Z_2$ is a single bond, a lower alkylene group, a lower alkenylene group or a lower alkynylene group;
Q is an optionally substituted aryl or an optionally substituted heteroaryl group;
$R_1$ is either any substituent selected from group A (a hydrogen atom, a halogen atom, a trifluoromethyl group, a trifluoromethoxy group, a carboxyl group, a carbamoyl group, an amino group, a cyano group, a nitro group, a lower alkanoyl group, a lower alkoxy group, a lower alkoxycarbonyl group, a mono- or di-substituted lower alkylamino group, a cyclic amino group, a lower alkanoylamino group, a phenyl group, a phenoxy group, a benzyloxy group, a benzoyl group, a mercapto group, a lower alkylthio group, a lower alkylthiocarbonyl group, a hydroxyl group or a mono- or di-substituted lower alkylaminocarbonyl group), or an oxygen atom that forms a N oxido group with N in any one of $G_1$ - $G_4$, or a lower alkyl group, a lower alkoxy group or a lower alkenyl group that may be substituted with a desired number of substituents of group A;

Each of $R_2$, $R_3$, $R_4$, $R_5$, $R_6$, $R_7$, $R_8$ and $R_9$ forms a carbonyl group when combined with the carbon atom on the ring to which they are bound, or they are each a hydrogen atom, a carboxyl group, a lower alkylcarbonyl group, a lower alkoxycarbonyl group, a lower alkoxycarbonylalkylcarbonyl group, an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperazin-1-ylcarbonyl group that may be substituted by a methyl group in 4-position, a piperidin-1-ylcarbonyl group that may be substituted by a methyl group or a hydroxyl group in 4-position, an N-phenylcarbamoyl group or a group represented by the formula - $CONH(CH_2)_pS(O)_qR_{10}$ or - $CONH(CH_2)_rNR_{11}R_{12}$, or a lower alkyl group that may be substituted by $R_{15}$;

Each of $R_{10}$, $R_{11}$, and $R_{12}$ independently represents a hydrogen atom, a lower alkyl group, a phenyl group or a lower alkylphenyl group;

$R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group;

provided that $R_6$, when combined with the carbon atom to which it is bound, may represent $R_{6a}$-C-$R_{6b}$, wherein either $R_{6a}$ or $R_{6b}$ is a hydrogen atom and the other is the same as defined above for $R_6$ or, alternatively, each of $R_{6a}$ and $R_{6b}$ represents a lower alkyl group;

also provided that if any one of the substituents $R_2$ - $R_9$ includes cyclic group, such cyclic group may be substituted by one or two lower alkyl groups;

m and n are independently an integer of 0 - 3, p is an integer of 0 - 4, q is an integer of 0 - 2, and r is an integer of 1 - 4;

substituents in Q include a group selected from among substituents of either group B [a halogen atom, a trifluoromethyl group, a trifluoromethoxy group, a trifluoromethanesulfonyl group, a carboxyl group, a carbamoyl group, an amino group, a cyano group, a nitro group, a lower alkanoyl group, a lower alkoxyl group, a lower alkoxycarbonyl group, a mono- or di-substituted lower alkylamino group, a lower alkanoylamino group, a cyclic amino group, a mercapto group, a lower alkylthio group, a lower alkylthiocarbonyl group, a lower alkylsulfonyl group, a lower alkylsulfinyl group, a hydroxyl group or a mono- or di-substituted lower alkylaminocarbonyl group, an amidino group which is optionally substituted with sulfamoyl or carbamoyl group, the formula -$NHCR_{13}$-$NHR_{14}$ (wherein $R_{13}$ is an optionally cyano-substituted imino group or a group =$CHNO_2$; $R_{14}$ is a hydrogen atom or a methyl group), a phenyl group, a phenoxy group, a heteroaryl group, a heteroaryloxy group, or a group represented by phenyl-S(O)t or heteroaryl-S(O)t (wherein t is an integer of 0 - 2), the heteroaryl group of group B is a 5- or 6-membered aromatic monocyclic group containing not more than four oxygen atoms, sulfur atoms or nitrogen atoms, provided that all aromatic rings of group B may be mono-, di- or tri-substituted by any substituent of group C (a halogen atom, a trifluoromethyl group, a cyano group, a hydroxyl group, an amino group, a mono- or di-substituted lower alkylamino group, a cyclic amino group, a nitro group, a carboxyl group, a mono-or di-substituted lower alkylaminocarbonyl group, a lower alkyl group, a lower alkoxyl group)] or a lower alkoxycarbonyl group), or a lower alkyl group that may be substituted by a desired number of substituents of group B; Q may be substituted by 1 - 4 groups in desired combinations of the above-mentioned substituents, with the proviso that when X and Y are both N, n is 2 or 3 and $Z_1$ is -$CH_2$- those compounds of formula (I) in which $R_6$ and $R_8$ in pair or $R_7$ and $R_9$ in pair are both carbonyl groups are excluded from the present invention.

It should also be noted that the compounds of the present invention are clearly different from the compounds described in connection with the prior art in that they have a potent FXa inhibiting activity, that they have two rings comprising combinations of piperazine or piperidine rings, with no carbonyl group being present in the bridge group between the two rings, and that the molecule has no terminal alkyl side chain that is substituted by a carboxyl group, an alkoxycarbonyl group or the like. In addition, the limitation introduced by the provisio also distinguishes the compounds of the present invention from the compounds of Japanese Patent Application Laid-Opened No. 23874/1988.

Further referring to the compounds of the present invention, those in which two piperazine or piperidine rings are bridged by methylene, particularly one that is substituted by a pyridin-4-yl group, have not been synthesized to date since intermediates for them (compounds of the formula (IV) to be set forth below, particularly those of the formula (IV)-

b in which $G_1$ is N and $G_2$ - $G_4$ are CH) have been difficult to obtain in a consistent manner. Therefore, although a multitude of compounds were disclosed or contemplated in the aforementioned prior art patents, the compounds of the present invention were not obtained or contemplated as the final compounds. As a result of many considerations on the reaction process and the intensive studies that followed, the present inventors captured the above-mentioned intermediates as reactive ones by the reaction methods to be described below and successfully produced the final compounds in good yield. It should be noted that those intermediates are also applicable to the synthesis of compounds other than the final compounds of the present invention.

In the definitions of the groups in the structural formulae of the present invention,

the "halogen atom" is exemplified by a fluorine atom, a chlorine atom, a bromine atom and an iodine atom;
the term "lower", unless otherwise noted, refers to a straight or branched carbon chain having 1 - 6 carbon atoms; therefore, the "lower alkyl group" may be exemplified by a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group and a 1-ethyl-2-methylpropyl group; among these, alkyl groups having 1 - 3 carbon atoms are preferred, and a methyl group and an ethyl group are particularly preferred;
the "lower alkoxy group" may be exemplified by a methoxy group, an ethoxy group, a propoxy group, an isopropoxy group, a butoxy group, an isobutoxy group, a sec-butoxy group, a tert-butoxy group, a pentyloxy(amyloxy) group, an isopentyloxy group, a tert-pentyloxy group, a neopentyloxy group, a 2-methylbutoxy group, a 1,2-dimethylpropoxy group, a 1-ethylpropoxy group and a hexyloxy group; preferred lower alkoxy groups are those having 1 - 3 carbon atoms, and a methoxy group and an ethoxy group are particularly preferred;
the "lower alkoxycarbonyl group" may be exemplified by a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a neopentyloxycarbonyl group, a tert-pentyloxycarbonyl group, a hexyloxycarbonyl group and other groups which form an ester between a straight-chained or branched alcohol having 1 - 6 carbon atoms and a carboxyl group; preferred lower alkoxycarbonyl groups are those having 1 - 3 carbon atoms, and a methoxycarbonyl group and an ethoxycarbonyl group are given as examples;
the "mono- or di-substituted lower alkylamino group" is meant an amino group in which one or two hydrogen atoms are replaced by the above-defined "lower alkyl group"; specific examples include a methylamino group, an ethylamino group, a propylamino group, an isopropylamino group, a butylamino group, an isobutylamino group, a pentylamino group, an isopentylamino group, a hexylamino group and an isohexylamino group. The dialkylamino group may be of a symmetric type which is di-substituted by a straight-chained or branched alkyl group having 1 - 6 carbon atoms, as exemplified by a dimethylamino group, a diethylamino group, a dipropylamino group, a diisopropylamino group, a dibutylamino group and a dipentylamino group, or it may be of a type that is asymmetrically substituted by a straight-chained or branched alkyl group having 1 - 6 carbon atoms, as exemplified by an ethylmethylamino group, a methylpropylamino group, an ethylpropylamino group, a butylmethylamino group, a butylethylamino group and a butylpropylamino group;
the "cyclic amino group" may be a cyclic cycloalklylamino group that may have a branched chain of 2 - 6 carbon atoms, as exemplified by a pyrrolidinyl group, a piperidinyl group or a methylpiperidinyl group, or it may be a saturated cyclic amino group as exemplified by a morpholino group or a piperazinyl group; these cyclic amino groups include those which are substituted by a lower alkyl group or a hydroxyl group and a preferred example is a 4-hydroxy-1-piperidinyl group;
the "lower alkylene group" is an alkylene group having 1 - 6 carbon atoms, as exemplified by a methylene group, an ethylene group, a methylmethylene group, a trimethylene group, a dimethylmethylene group, a tetramethylene group, a methyltrimethylene group, an ethylethylene group, a dimethylethylene group, an ethylmethylmethylene group, a pentamethylene group, a methyltetramethylene group, a dimethyltrimethylene group, a trimethylethylene group, a dimethylmethylene group, a hexamethylene group, a methylpentamethylene group and a dimethyltetramethylene group; preferred are alkylene groups having 1 - 3 carbon atoms as exemplfied by a methylene group, an ethylene group, a methylmethylene group, a trimethylene group and a dimethylmethylene group, with a methylene group and an ethylene group being more preferred;
the "lower alkenylene group" is an alkenylene group having 1 - 6 carbon atoms, as exemplified by a vinylene group, a propenylene group, an isopropenylene group, a 2-butenylene group and a 1,3-butadienylene group, and a vinylene group is preferred;
the "lower alkynylene group" may be exemplified by an ethynylene group and a propynylene group;

the "lower alkenyl group" may be exemplified by a vinyl group, a propenyl group, an isopropenyl group, a 2-butenyl group and a 1,3-butadienyl group;

the "lower alkanoyl group' may be exemplified by a formyl group, an acetyl group, a propionyl group, a butyryl group, an isobutyryl group, a valeryl group, an isovaleryl group, a pivaloyl group and a hexanoyl group, and an acetyl group, a propionyl group and a butyryl group are preferred;

the "lower alkanoylamino group" is a group in which the hydrogen atom in an amino group is substituted by the above-defined lower alkanoyl group and examples include a formylamino group, an acetylamino group, a propionylamino group, a butyrylamino group, an isobutyrylamino group, a valerylamino group, an isovalerylamino group, a pivaloylamino group and a hexanoylamino group, with an acetylamino group, a propionylamino group and a butyrylamino group being preferred;

the "lower alkanoyloxy group" is a group in which the hydrogen atom in a hydroxyl group is substituted by the above-defined lower alkanoyl group and examples include an acetyloxy group, a propionyloxy group, a butyryloxy group, an isobutyryloxy group, a valeryloxy group, an isovaleryloxy group, a pivaloyloxy group and a hexanoyloxy group, with an acetyloxy group, a propionyloxy group and a butyryloxy group being preferred;

the "lower alkylthio group" may be a group in which the hydrogen atom in a mercapto group is substituted by the above-defined "lower alkyl group" and examples include a methylthio group, an ethylthio group, a propylthio group, an isopropylthio group, a butylthio group, an isobutylthio group, a pentylthio group and a hexylthio group;

the "lower alkylthiocarbonyl group" is the same as the above-defined "lower alkanoyl group" except that the carbonyl group is replaced by a thiocarbonyl group and examples include a methylthiocarbonyl group, an ethylthiocarbonyl group and a propylthiocarbonyl group;

the "lower alkylsulfonyl group" is the same as the above-defined "lower alkylthio group" except that the sulfur atom is replaced by a sulfonyl group and examples include a methanesulfonyl group, an ethanesulfonyl group and a propanesulfonyl group;

the "lower alkylsulfinyl group" is the same as the above-defined "lower alkylthio group" except that the sulfur atom is replaced by a sulfinyl group and examples include a methanesulfinyl group, an ethanesulfinyl group and a propanesulfinyl group;

the "lower alkylsulfonylamino group" is an amino group substituted by the above-defined lower alkylsulfonylamino group and examples include a methanesulfonylamino group and an ethanesulfonylamino group;

the "optionally mono- or di-lower alkyl substituted carbamoyl group" may be exemplified by a carbamoyl group, an N-methylcarbamoyl group, an N-ethylcarbamoyl group, an N,N-dimethylcarbamoyl group, an N,N-diethylcarbamoyl group and an N-ethyl-N-methylcarbamoyl group;

by the "lower alkoxycarbonylalkyl group" is meant a lower alkyl group substituted by the above-defined "lower alkoxycarbonyl group" and examples include a methoxycarbonylmethyl group and an ethoxycarbonylethyl group;

the "aryl group", unless otherwise noted, is an aryl group in the form of a monocyclic or fused hydrocarbon ring having 6 - 14 carbon atoms and may specifically be exemplified by a phenyl group, a naphthyl group, a biphenyl group and an anthryl group, with a phenyl group, a naphthyl group and a p-biphenylyl group being preferred;

the "heteroaryl group", unless otherwise noted, may be a monocyclic or fused cyclic heteroaryl group having 1 - 4 hetero atoms comprising oxygen, sulfur or nitrogen atoms, as exemplified by a furyl group, a thienyl group, a pyrrolyl group, a pyrazolyl group, an imidazolyl group, an isothiazolyl group, an oxazolyl group, an isoxazolyl group, a thiazolyl group, a pyridyl group, a pyridazinyl group, a pyrimidinyl group, a pyrazinyl group, a triazinyl group, a benzimidazolyl group, a benzofuranyl group, a 1,2-benzoisoxazolyl group, a benzoxazolyl group, a benzothiazolyl group, an indolyl group, an imidazopyridyl group, an oxazolopyridyl group, an isothiazolopyridyl group, a benzothienyl group, a naphthyridinyl group, a quinolyl group, an isoquinolyl group, a quinazolinyl group, a quinolizinyl group, a quinoxalinyl group, a cinnolinyl group, a benzoxazinyl group, a benzothiazinyl group, a 1,2,3-triazolyl group, a 1,2,4-triazolyl group, an oxadiazolyl group, a furazanyl group, a thiadiazolyl group, a tetrazolyl group, a dibenzofuranyl group, a dibenzothienyl group, a 1,2,3,4-tetrahydroquinolyl group and a 1,2,3,4-tetrahydroisoquinolyl group;

the "heteroaryloxy group" is a hydroxyl group having the hydrogen atom replaced by the above-defined "heteroaryl group".

The following are the preferred definitions of the substituents in the compounds of the present invention.

As for $G_1$ - $G_4$ in the formula (I), it is preferred that at least one of $G_1$, $G_2$, $G_3$ and $G_4$ is N; more preferred cases are as follows: $G_1$ is N and $G_2$, $G_3$ and $G_4$ are CH; $G_2$ is N and $G_1$, $G_3$ and $G_4$ are CH; $G_3$ is N and $G_1$, $G_2$ and $G_4$ are CH; $G_1$ and $G_2$ are N and $G_3$ and $G_4$ are CH; $G_1$ and $G_3$ are N and $G_2$ and $G_4$ are CH; $G_1$, $G_2$ and $G_4$ are N and $G_3$ is CH; and $G_1$, $G_3$, and $G_4$ are N and $G_2$ is CH; further preferred cases are as follows: $G_1$ is N and $G_2$, $G_3$ and $G_4$ are CH; $G_1$ and $G_3$ are N and $G_2$ and $G_4$ are CH; $G_1$, $G_3$ and $G_4$ are N and $G_2$ is CH. While N in any one of $G_1$ - $G_4$ mentioned above may combine with $R_1$ to form N-oxide, it is preferred for $G_1$ to make N-oxide.

Preferably, $R_1$ is a hydrogen atom, a fluorine atom, a chlorine atom, a bromine atom, an iodine atom, an amino group, a methyl group, an ethyl group, a methoxy group, a trifluoromethyl group, a trifluoromethoxy group, a

cyano group, an aminomethyl group, a hydroxyl group, a hydroxymethyl group, a carbamoyl group, or an N-oxide group formed in combination with any one of $G_1$ - $G_4$.

Preferably, m is 0, 1, 2 or 3, more preferably 0, 1 or 2, and further preferably 0.

Referring to $R_2$, $R_3$, $R_4$, $R_5$, $R_6$, $R_7$, $R_8$ and $R_9$, it is preferred that $R_2$, $R_3$, $R_4$ and $R_5$ are each a hydrogen atom, a methyl group or an ethyl group whereas $R_6$, $R_7$, $R_8$ and $R_9$ form a carbonyl group when combined with the carbon atom on the ring to which they are bound, or they are each a hydrogen atom, a carboxyl group, a lower alkylcarbonyl group, a lower alkoxycarbonyl group, a lower alkoxycarbonylalkylcarbonyl group, an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperazin-1-ylcarbonyl group that may be substituted by a methyl group in 4-position, a piperidin-1-ylcarbonyl group that may be substituted by a methyl group or a hydroxyl group in 4-position, an N-phenylcarbamoyl group or a group represented by the formula - $CONH(CH_2)_pS(O)_qR_{10}$ or $CONH(CH_2)_rNR_{11}R_{12}$, or a lower alkyl group that may be substituted by $R_{15}$; $R_{10}$, $R_{11}$ and $R_{12}$ are independently a hydrogen atom, a lower alkyl group, a phenyl group or a lower alkylphenyl group;

$R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group;

provided that $R_6$, when combined with the carbon atom to which it is bound, may represent $R_{6a}$-C-$R_{6b}$, wherein either $R_{6a}$ or $R_{6b}$ is a hydrogen atom and the other is the same as defined above for $R_6$ or, alternatively, each of $R_{6a}$ and $R_{6b}$ represents a lower alkyl group;

also provided that if any one of the substituents $R_6$ - $R_9$ include cyclic group, such cyclic group may be substituted by one or two lower alkyl groups;

more preferably, each of $R_2$, $R_3$, $R_4$ and $R_5$ independently represents a hydrogen atom whereas $R_6$, $R_7$, $R_8$ and $R_9$ form a carbonyl group when combined with the carbon atom on the ring to which they are bound, or they are each a hydrogen atom, a carboxyl group, a lower alkoxycarbonyl group, a lower alkoxycarbonylalkylcarbonyl group, or a lower alkyl group that may be substituted by $R_{15}$; $R_{15}$ is a carboxyl group, a lower alkoxycarbonyl group, a hydroxyl group, a lower alkoxy group, a lower acyloxy group, an amino group, a mono- or di-substituted lower alkylamino group, a lower alkanoylamino group, a lower alkylsulfonylamino group, a cyclic amino group or an N-hydroxyimino group;

or preferably either one of $R_{6a}$ and $R_{6b}$ is a hydrogen atom and the other is a carboxyl group, a lower alkoxycarbonyl group, a lower alkoxycarbonylalkylcarbonyl group or a lower alkyl group that may be substituted by $R_{15}$; $R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group (an aldoxime group); or each of $R_{6a}$ and $R_{6b}$ represents a lower alkyl group;

each of X and Y independently represents CH or N; the preferred cases are where X is CH and Y is CH or N and where X is N and Y is CH; the more preferred case is where X is CH and Y is N;

n is preferably 0, 1, 2 or 3, preferably 0 or 1, more preferably 1;

$Z_1$ is preferably the formula $-SO_2-$ or $-CH_2-$, more preferably $-SO_2-$;

$Z_2$ is preferably a single bond, a lower alkylene group or a lower alkenylene group;

the aryl group Q is an aryl group in the form of a monocylic or fused hydrocarbon ring having 6 - 14 carbon atoms and is preferably a phenyl group, a biphenylyl group, a 1-naphthyl group or a 2-naphthyl group; the heteroaryl group is a monocyclic or fused cyclic heteroaryl group having 1 - 4 hetero atoms comprising oxygen, sulfur or nitrogen atoms and is preferably a thienyl group, a benzofuranyl group, a benzothienyl group, a benzothiazolyl group, a 1,2,3,4-tetrahydroquinolyl group, a 1,2,3,4-tetrahydroisoquinolyl group, a pyridylphenyl group or a pyridylthienyl group; these groups may preferably be substituted by any one of the above-listed substituents of group B;

the group represented by the formula $-Z_2-Q-$ is preferably a phenyl group, a 1-naphthyl group, a 2-naphthyl group, a biphenylyl group, a benzyl group, a phenethyl group, a styryl group, a 2-phenylethynyl group, a benzofuranyl group, a benzothienyl group or a benzothiazolyl group;

these groups are either unsubstituted or preferably mono-, di- or tri-substituted by any substituent selected from among a hydroxyl group, an amino group, an amidino group, a sulfamoylamidino group, an N'-cyano-guanidino group, an N'-methyl-2-nitro-1,1-ethenediamino group, a fluorine atom, a chlorine atom, a bromine atom, an iodine atom, a cyano group, a trifluoromethyl group, a nitro group, a carboxyl group, a carbamoyl group, a methoxycarbonyl group, an ethoxycarbonyl group, a methyl group, an ethyl group, a methoxy group and an ethoxy group, and more preferably mono-, di- or tri-substituted by a chlorine or bromine atom.

The compounds of the present invention are those of the formula (I) or salts thereof. The following are specific examples of the compounds having the preferred combinations of substituents.

(1) Compounds of the formula (I) where at least one of $G_1$, $G_2$, $G_3$ and $G_4$ is N or salts thereof are preferred.

More preferred are the compounds where at least $G_1$ is N, m is 0 - 2, n is 1 and $Z_1$ is $-SO_2-$ or salts thereof. In this case, the formula (I) may be rewritten as the following formula (II), wherein the respective substituents have the same meanings as in the formula (I):

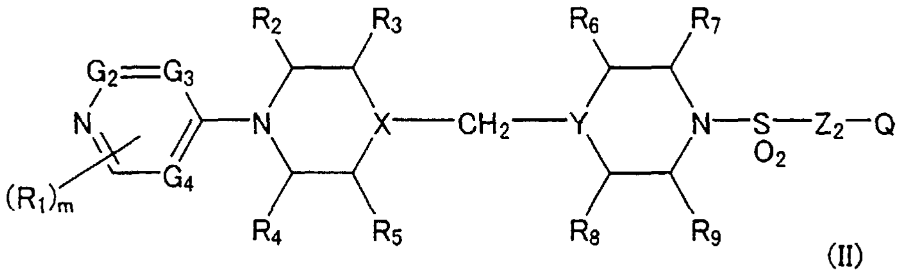

(II)

Further preferred are the following compounds or salts thereof: in the combination of $G_2$ - $G_4$, $G_2$, $G_3$ and $G_4$ are CH, or $G_3$ is N and $G_2$ and $G_4$ are CH, or $G_3$ and $G_4$ are N and $G_2$ is CH; $R_1$ is a hydrogen atom, a fluorine atom, a chlorine atom, a bromine atom, an iodine atom, an amino group, a methyl group, an ethyl group, a methoxy group, a trifluoromethyl group, a trifluoromethoxy group, a cyano group, an aminomethyl group, a hydroxyl group, a hydroxymethyl group, a carbamoyl group, or an N-oxide group formed together with any one of $G_1$ - $G_4$; each of $R_2$, $R_3$, $R_4$ and $R_5$ independently represents a hydrogen atom, a methyl group or an ethyl group; each of $R_6$, $R_7$, $R_8$ and $R_9$ forms a carbonyl group when combined with the carbon atom on the ring to which they are bound, or they are each a hydrogen atom, a carboxyl group, a methoxycarbonyl group, an ethoxycarbonyl group, a carbamoyl group, an N-methylcarbamoyl group, an N,N-dimethylcarbamoyl group, a carboxymethyl group, a methoxycarbonylmethyl group, an ethoxycarbonylmethyl group, a hydroxymethyl group, a methoxymethyl group, a methyl group, an ethyl group, a pyrrolidin-1-ylcarbonyl group, a piperidinocarbonyl group, a morpholinocarbonyl group, a piperazin-1-ylcarbonyl group, a 4-methylpiperazin-1-ylcarbonyl group or a benzyl group (if any one of the substituents $R_6$ - $R_9$ includes cyclic groups, such cyclic group may be substituted by one or two methyl or ethyl groups);

X is CH and Y is CH or N, or X is N and Y is CH;
$Z_2$ is a single bond, a lower alkylene group or a lower alkenylene group;
Q is a phenyl group, a biphenylyl group, a l-naphthyl group or 2-naphthyl group, a thienyl group, a benzofuranyl group, a benzothienyl group, a benzothiazolyl group, a 1,2,3,4-tetrahydroquinolyl group, a 1,2,3,4-tetrahydroisoquinolyl group, a pyridylphenyl group or a pyridylthienyl group, provided that these groups may be mono, di- or tri-substituted by a substituent of the above-defined group B or by a lower alkyl group which may be mono-, di- or tri-substituted by a substituent of group B.

Particularly preferred are the following compounds or salts thereof: $G_1$ is N, $G_2$, $G_3$ and $G_4$ are CH; $R_1$ is a hydrogen atom, a fluorine atom, a chlorine atom, a bromine atom, an iodine atom, an amino group, a methyl group, an ethyl group, a methoxy group, a trifluoromethyl group, a trifluoromethoxy group, a cyano group, an aminomethyl group, a hydroxyl group, a hydroxymethyl group, a carbamoyl group, or an N-oxide group formed together with $G_1$; m is 0, 1 or 2; $R_2$, $R_3$, $R_4$ and $R_5$ are each a hydrogen atom; each of $R_6$, $R_7$, $R_8$ and $R_9$ forms a carbonyl group when combined with the carbon atom on the ring to which they are bound, or they are each a hydrogen atom, a carboxyl group, a methoxycarbonyl group, an ethoxycarbonyl group, a carbamoyl group, an N-methylcarbamoyl group, a carboxymethyl group, a methoxycarbonylmethyl group, an N-(2-ethylthioethyl)carbamoyl group, a hydroxymethyl group, a methyl group, an ethyl group, a piperidinocarbonyl group or a benzyl group; X is CH and Y is N; the group represented by the formula $-Z_2$-Q is a phenyl group, a 1-naphthyl group, a 2-naphthyl group, a biphenylyl group, a benzyl group, a phenethyl group, a styryl group, a 2-phenylethynyl group, a benzofuranyl group, a benzothienyl group, a benzothiazolyl group, a dibenzofuranyl group, a 1,2,3,4-tetrahydroquinolyl group or a 1,2,3,4-tetrahydroisoquinolyl group; these aromatic rings are either unsubstituted or mono-, di- or tri-substituted by any substituent selected from among a hydroxyl group, an amino group, a fluorine atom, a chlorine atom, a bromine atom, an iodine atom, a cyano group, a trifluoromethyl group, a nitro group, a carboxyl group, a carbamoyl group, a methoxycarbonyl group, an ethoxycarbonyl group, a methyl group, an ethyl group, a methoxy group, an ethoxy group, an amidino group, a sulfamoylamidino group, an N'-cyano-guanidino group and an N'-methyl-2-nitro-1,1-ethenediamino group.

Certain compounds of the formula (I) in which $G_4$ is CH, $R_1$, $R_2$ - $R_5$, $R_7$ and $R_9$ are all a hydrogn atom, and $R_6$, when taken together with the carbon atom to which it is bound, represents $R_{6a}$-C-$R_{6b}$ (one of $R_{6a}$ and $R_{6b}$ is a

hydrogen atom and the other is the same as already defined for $R_6$ or, alternatively, $R_{6a}$ and $R_{6b}$ are both a lower alkyl group) and $R_8$ forms a carbonyl when combined with the carbon atom to which it is bound, are represented by the following structure (in which Q' represents $-Z_2$-Q and the respective substituents have the same definitions as given in the formula (I)):

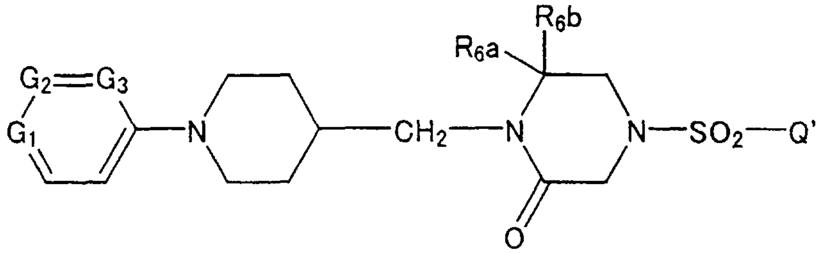

If the respective substituents are limited to the preferred cases, the resulting compounds are represented by the following formula (II'):

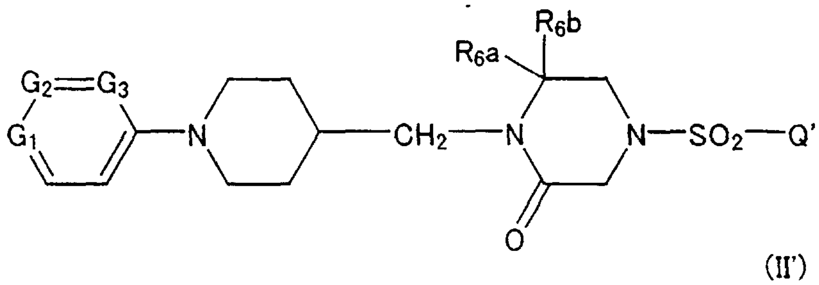

(II')

(where $G_1$, $G_2$ and $G_3$ are independently CH or N, provided that at least one of them is N;
one of $R_{6a}$ and $R_{6b}$ is a hydrogen atom and the other is

1) a group selected from among a carboxyl group, a lower alkylcarbonyl group, a lower alkoxycarbonyl group and a lower alkoxycarbonylalkylcarbonyl group;
2) a group selected from among an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperidin-1-ylcarbonyl group which may be substituted by a methyl group or a hydroxyl group in 4-position, an N-phenylcarbamoyl group or a group selected from among the groups represented by the formulae $-CONH(CH_2)_pS(O)_qR_{10}$ and $-CONH(CH_2)_rNR_{11}R_{12}$ (where $R_{10}$, $R_{11}$ and $R_{12}$ are independently a hydrogen atom, a lower alkyl group, a phenyl group or a lower alkylphenyl group; p is an integer of 0 - 4, g is an integer of 0 - 2, and r is an integer of 1 - 4), or
3) a lower alkyl group optionally substituted by $R_{15}$; $R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group;

or $R_{6a}$ and $R_{6b}$ are both a lower alkyl group;
Q' is an aryl group optionally substituted by a group having any 1 - 4 halogen atoms or an aryl lower alkenylene group which may be similarly substituted).

Preferred are the compounds of the formula (II') where at least $G_1$ is N.
Further preferred is the following case: in the combination of $G_2$ and $G_3$, both $G_2$ and $G_3$ are CH or $G_3$ is N and $G_5$ is CH;

one of $R_{6a}$ and $R_{6b}$ is a hydrogen atom and the other is a carboxyl group, a lower alkylcarbonyl group, a lower alkoxycarbonyl group, a lower alkoxycarbonylalkylcarbonyl group, an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperidin-1-ylcarbonyl group which may be substituted by a methyl group or a hydroxyl group in 4-position, an N-phenylcarbamoyl group or a group represented by the formula -CONH$(CH_2)_pS(O)_qR_{10}$ or -CONH$(CH_2)_rNR_{11}R_{12}$ or a lower alkyl group optionally substituted by $R_{15}$; $R_{10}$, $R_{11}$ and $R_{12}$ are independently a hydrogen atom, a lower alkyl group, a phenyl group or a lower alkylphenyl group, and $R_{15}$ is a carboxyl group, a lower alkoxycarbonyl group, a hydroxyl group, a lower alkoxy group, a lower acyloxy group, an amino group, a mono- or di-substituted lower alkylamino group, a lower alkanoylamino group, a lower alkylsulfonylamino group, a cyclic amino group or an N-hydroxyimino group; or each of $R_{6a}$ and $R_{6b}$ represents a lower alkyl group;

Q' is a phenyl group, a biphenyl group, a 1-naphthyl group, a 2-naphthyl group or a styryl group, provided these groups may be substituted by any 1 - 4 halogen atoms.

Particularly preferred are compounds of the formula (II') or salts thereof, where $G_1$ is N, $G_2$ and $G_3$ are CH, Q' is a phenyl group, a 1-naphthyl group, a 2-naphthyl group, a biphenylyl group or a styryl group, provided these aromatic rings are either unsubstituted or mono-, di- or tri-substituted by any substituent selected from among a fluorine atom, a chlorine atom, a bromine atom and an iodine atom, particularly a chlorine atom and a bromine atom, Q' being preferably a 6-halogeno-2-naphthyl group or a p-halogenostyryl group.

Even more preferred are the compounds of the formula (II"):

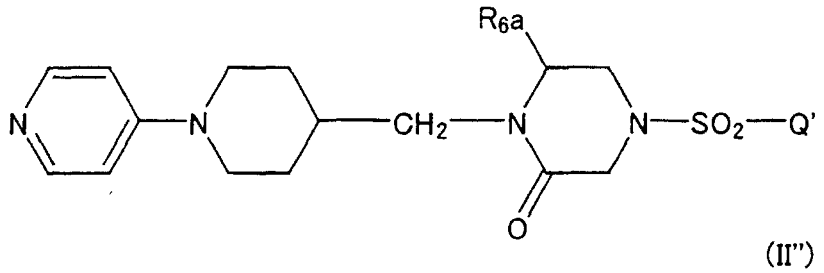

(II")

(wherein $R_{6a}$ and Q' have the same definitions as given for the substituent $R_{6a}$ and Q' in the formula (II') but more preferably, $R_{6a}$ is a carboxyl group, a lower alkoxycarbonyl group or a lower alkyl group that may be substituted by $R_{15}$; provided $R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group (an aldoxime group); more preferably, $R_{6a}$ is a carboxyl group, a lower alkoxycarbonyl group, a lower alkoxycarbonylalkyl group or a lower alkoxy lower alkyl group; and Q' is a phenyl group, a 1-naphthyl group, a 2-naphthyl group, a biphenylyl group or a styryl group, provided these aromatic rings are either unsubstituted or mono-, di- or tri-substituted by any substituent selected from among a fluorine atom, a chlorine atom, a bromine atom and an iodine atom, particularly a chlorine atom and a bromine atom, Q' being more preferably a 6-halogeno-2-naphthyl group or a p-halogenostyryl group) or salts thereof.

Preferred examples of the compounds of the present invention are specifically listed below:

1-[1-((E)-4-chlorostyrylsulfonyl)piperidin-4-yl]-4-(4-pyridyl)piperazine;
1-[1-((E)-4-chlorostyrylsulfonyl)piperidin-4-ylmethyl]-4-(4-pyridyl)piperazine;
1-[(E)-4-chlorostyrylsulfonyl]-4-[1-(4-pyridyl)piperidin-4-ylmethyl]piperazine;
4-(naphthalene-2-ylsulfonyl)-1-[1-(4-pyridyl)piperidin-4-ylmethyl]piperazine;
4-(6-chloronaphthalen-2-ylsulfonyl)-1-[1-(4-pyridyl)piperidin-4-ylmethyl]piperazine;
4-(6-bromonaphthalen-2-ylsulfonyl)-1-[1-(4-pyridyl)piperidin-4-ylmethyl]piperazine;
4-(benzo[b]thiophen-2-ylsulfonyl)-1-[1-(4-pyridyl)piperidin-4-yl-methyl]piperazine;
4-(5-fluorobenzo[b]thiophen-2-ylsulfonyl)-1-[1-(4-pyridyl)piperidin-4-ylmethyl]piperazine;
4-(6-chlorobenzo[b]thiophen-2-ylsulfonyl)-1-[1-(4-pyridyl)piperidin-4-ylmethyl]piperazine;
4-(4-methoxybenzo[b]thiophen-2-ylsulfonyl)-1-[1-(4-pyridyl)piperidin-4-ylmethyl]piperazine;
4-(6-methoxybenzo[b]thiophen-2-ylsulfonyl)-1-[1-(4-pyridyl)piperidin-4-ylmethyl]piperazine;
4-[3-(ethoxycarbonylmethyl)benzo[b]thiophen-2-ylsulfonyl]-1-[1-(4-pyridyl)piperidin-4-ylmethyl]piperazine;
1-[1-(4-pyridyl)piperidin-4-ylmethyl]-4-[3-(trifluoromethyl)benzo[b]thiophen-2-ylsulfonyl]piperazine;
4-(3-nitrobenzo[b]thiophen-2-ylsulfonyl)-1-[1-(4-pyridyl)piperidin-4-ylmethyl]piperazine;
4-(benzo[b]furan-2-ylsulfonyl)-1-[1-(4-pyridyl)piperidin-4-ylmethyl]piperazine;
4-(5-chlorobenzo[b]furan-2-ylsulfonyl)-1-[1-(4-pyridyl)piperidin-4-ylmethyl]piperazine;
4-(2-methylbenzothiazol-6-ylsulfonyl)-1-[1-(4-pyridyl)piperidin-4-ylmethyl]piperazine;

4-(4-phenylbenzenesulfonyl)-1-[1-(4-pyridyl)piperidin-4-ylmethyl]piperazine;
4-(5-carboxy-2-chlorobenzenesulfonyl)-1-[1-(4-pyridyl)piperidin-4-ylmethyl]piperazine;
4-[5-(carboxymethyl)-2-chlorobenzenesulfonyl]-1-[1-(4-pyridyl)piperidin-4-ylmethyl]piperazine;
4-(5-acetamidonaphthalen-2-ylsulfonyl)-1-[1-(4-pyridyl)piperidin-4-ylmethyl]piperazine;
4-(5-aminonaphthalen-2-ylsulfonyl)-1-[1-(4-pyridyl)piperidin-4-ylmethyl]piperazine;
4-[(E)-4-chlorostyrylsulfonyl]-1-[1-(4-pyridyl)piperidin-4-ylmethyl]piperazin-2-one;
4-(5-aminonaphthalen-2-ylsulfonyl)-1-[1-(4-pyridyl)piperidin-4-ylmethyl]piperazine;
4-[(E)-4-chlorostyrylsulfonyl]-1-[1-(4-pyridyl)piperidin-4-ylmethyl]piperazin-2-one;
4-(6-bromonaphthalen-2-ylsulfonyl)-1-[1-(4-pyridyl)piperidin-4-ylmethyl]piperazin-2-one;
4-(6-chloronaphthalen-2-ylsulfonyl)-1-[1-(4-pyridyl)piperidin-4-ylmethyl]piperazin-2-one;
4-(naphthalen-2-ylsulfonyl)-1-[1-(4-pyridyl)piperidin-4-ylmethyl]piperazin-2-one;
4-(6-methylnaphthalen-2-ylsulfonyl)-1-[1-(4-pyridyl)piperidin-4-ylmethyl]piperazin-2-one;
4-(6-cyanonaphthalen-2-ylsulfonyl)-1-[1-(4-pyridyl)piperidin-4-ylmethyl]piperazin-2-one;
4-(6-hydroxynaphthalen-2-ylsulfonyl)-1-[1-(4-pyridyl)piperidin-4-ylmethyl]piperazin-2-one;
4-(1-fluoronaphthalen-2-ylsulfonyl)-1-[1-(4-pyridyl)piperidin-4-ylmethyl]piperazin-2-one;
4-(6-chloronaphthalen-2-ylsulfonyl)-2-ethoxycarbonyl-1-[1-(4-pyridyl)piperidn-4-ylmethyl]piperazine;
4-(6-chloronaphthalen-2-ylsulfonyl)-2-hydroxymethyl-1-[1-(4-pyridyl)piperidin-4-ylmethyl]piperazine;
2-carboxy-4-(6-chloronaphthalen-2-ylsulfonyl)-1-[1-(4-pyridyl)piperidin-4-ylmehyl]piperazine;
4-(6-chloronaphthalen-2-ylsulfonyl)-2-[(2-ethoxycarbonyl)acetyl]-1-[1-(4-pyridyl)piperidin-4-ylmethyl]piperazine;
2-aminocarbonyl-4-(6-chloronaphthalen-2-ylsulfonyl)-1-[1-(4-pyridyl)piperidin-4-ylmethyl]piperazine;
4-(6-chloronaphthalen-2-ylsulfonyl)-2-[N-(ethylthioethyl)aminocarbonyl]-1-[1-(4-pyridyl)piperidin-4-ylmethyl]piperazine;
2-acetyl-4-(6-chloronaphthalen-2-ylsulfonyl)-1-[1-(4-pyridyl)piperidin-4-ylmethyl]piperazine; and
4-(6-chloronaphthalen-2-ylsulfonyl)-2-(N,N-dimethylaminocarbonyl)-1-[1-(4-pyridyl)piperidin-4-ylmethyl]piperazine.

These compounds are capable of forming the aforementioned salts. Preferred salts are exemplified by methanesulfonates and hydrochlorides.

Particularly preferred examples of the compounds of the present invention are specifically listed below:

4-(6-chloronaphthalen-2-ylsulfonyl)-6-ethoxycarbonyl-1-[1-(4-pyridyl)piperidin-4-ylmethyl]piperazin-2-one;
6-carboxy-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-hydroxymethyl-1-[1-(4-pyridyl)piperidin-4-ylmethyl]piperazin-2-one;
4-(6-chloronaphthalen-2-ylsulfonyl)-6-methoxymethyl-1-[1-(4-pyridyl)piperidin-4-ylmethyl]piperazin-2-one;
6-acetoxymethyl-4-(6-chloronaphthalen-2-ylsulfonyl)-1-[1-(4-pyridyl)piperidin-4-ylmethyl]piperazin-2-one;
4-[(E)-4-chlorostyrylsulfonyl]-6-ethoxycarbonyl-1-[1-(4-pyridyl)piperidin-4-ylmethyl]piperazin-2-one;
6-carboxy-4-[(E)-4-chlorostyrylsulfonyl]-1-[1-(4-pyridyl)piperidin-4-ylmethyl]piperazin-2-one;
6-aminocarbonyl-4-(6-chloronaphthalen-2-ylsulfonyl)-1-[1-(4-pyridyl)piperidin-4-ylmethyl]piperazin-2-one;
6-aldoximyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-morpholinocarbonyl-1-[1-(4-pyridyl)piperidin-4-ylmethyl]piperazin-2-one;
4-(6-chloronaphthalen-2-ylsulfonyl)-6-dimethylaminocarbonyl-1-[1-(4-pyridyl)piperidin-4-ylmethyl]piperazin-2-one;
4-(6-chloronaphthalen-2-ylsulfonyl)-6-methoxyaminocarbonyl-1-[1-(4-pyridyl)piperidin-4-ylmethyl]piperazin-2-one;
4-(6-chloronaphthalen-2-ylsulfonyl)-6-(4-hydroxypiperidinecarbonyl)-1-[1-(4-pyridyl)piperidin-4-ylmethyl]piperazin-2-one;
6-aminomethyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-morpholinomethyl-1-[1-(4-pyridyl)piperidin-4-ylmethyl]piperazin-2-one;
4-(6-chloronaphthalen-2-ylsulfonyl)-6-dimethylaminomethyl-1-[1-(4-pyridyl)piperidin-4-ylmethyl]piperazin-2-one;
6-acetamidomethyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-methanesulfonylamidomethyl-1-[1-(4-pyridyl)piperidin-4- ylmethyl]piperazin-2-one;
4-(6-chloronaphthalen-2-ylsulfonyl)-6-(4-hydroxypiperidinemethyl)-1-[1-(4-pyridyl)piperidin-4-ylmethyl]piperazin-2-one;
4-(6-chloronaphthalen-2-ylsulfonyl)-6-dimethyl-1-[1-(4-pyridyl)piperidin-4-ylmethyl]piperazin-2-one;
4-(2-naphthylsulfonyl)-6-hydroxymethyl-1-[1-(4-pyridyl)piperidin-4-ylmethyl]piperazin-2-one;
6-acetoxymethyl-4-(2-naphthylsulfonyl)-1-[1-(4-pyridyl)piperidin-4-ylmethyl]piperazin-2-one;
(R)-4-(6-chloronaphthalen-2-ylsulfonyl)-6-ethoxycarbonyl-1-[1-(4-pyridyl)piperidin-4-ylmethyl]piperazin-2-one;
(S)-4-(6-chloronaphthalen-2-ylsulfonyl)-6-ethoxycarbonyl-1-[1-(4-pyridyl)piperidin-4-ylmethyl]piperazin-2-one;
(R)-4-(6-chloronaphthalen-2-ylsulfonyl)-6-methoxymethyl-1-[1-(4-pyridyl)piperidin-4-ylmethyl]piperazin-2-one;

(S)-4-(6-chloronaphthalen-2-ylsulfonyl)-6-methoxymethyl-1-[1-(4-pyridyl)piperidin-4-ylmethyl]piperazin-2-one;
(R)-6-carboxy-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-n-propoxycarbonyl-1-[1-(4-pyridyl)piperidin-4-ylmethyl]piperazin-2-one;
(R)-4-(6-chloronaphthalen-2-ylsulfonyl)-6-n-propoxycarbonyl-1-[1-(4-pyridyl)piperidin-4-ylmethyl]piperazin-2-one;
4-(6-chloronaphthalen-2-ylsulfonyl)-6-isopropoxycarbonyl-1-[1-(4-pyridyl)piperidin-4-ylmethyl]piperazin-2-one;
(R)-4-(6-chloronaphthalen-2-ylsulfonyl)-6-isopropoxycarbonyl-1-[1-(4-pyridyl)piperidin-4-ylmethyl]piperazin-2-one;
6-t-butoxycarbonyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6,6-dimethyl-1-[1-(4-pyridyl)piperidin-4-ylmethyl]piperazin-2-one; and
(R)-4-[(E)-4-chlorostyrylsulfonyl]-6-methoxymethyl-1-[1-(4-pyridyl)piperidin-4-ylmethyl]piperazin-2-one.

These compounds can also form the above-mentioned salts. Preferred salts are exemplified by methanesulfonates and hydrochlorides.

Also preferred are the compounds of the formula (I) where each of $G_1$, $G_2$, $G_3$ and $G_4$ is CH, or salts thereof.

More preferred are those compounds or salts thereof, in which $R_1$ is a hydrogen atom, a fluorine atom, a chlorine atom, a bromine atom, an iodine atom, an amino group, a methyl group, an ethyl group, a methoxy group, a trifluoromethyl group, a trifluoromethoxy group, a cyano group, an aminomethyl group, a hydroxyl group, a hydroxymethyl group or a carbamoyl group; m is 0, 1 or 2; each of $R_2$, $R_3$, $R_4$ and $R_5$ independently represents a hydrogen atom; each of $R_6$, $R_7$, $R_8$ and $R_9$ forms a carbonyl group when combined with the carbon atom on the ring to which they are bound, or they are each a hydrogen atom, a carboxyl group, a methoxycarbonyl group, an ethoxycarbonyl group, a carbamoyl group, an N-methylcarbamoyl group, a carboxymethyl group, a methoxycarbonylmethyl group, an N-(2-ethylthioethyl)carbamoyl group, a hydroxymethyl group, a methyl group, an ethyl group, a piperidinocarbonyl group or a benzyl group; X is CH and Y is N; the group represented by the formula $-Z_2-Q$ is a phenyl group, a 1-naphthyl group, a 2-naphthyl group, a biphenylyl group, a benzyl group, a phenethyl group, a styryl group, a 2-phenylethynyl group, a benzofuranyl group, a benzothienyl group, a benzothiazolyl group, a dibenzofuranyl group, a 1,2,3,4-tetrahydroquinolyl group or a 1,2,3,4-tetrahydroisoquinolyl group; these aromatic rings are either unsubstituted or mono-, di- or tri-substituted by any substituent selected from among a hydroxyl group, an amino group, a fluorine atom, a chlorine atom, a bromine atom, an iodine atom, a cyano group, a trifluoromethyl group, a nitro group, a carboxyl group, a carbamoyl group, a methoxycarbonyl group, an ethoxycarbonyl group, a methyl group, an ethyl group, a methoxy group, an ethoxy group, an amidino group, a sulfamoylamidino group, an N'-cyano-guanidino group and an N'-methyl-2-nitro-1,1-ethenediamino group.

The compounds of the present invention sometimes have asymmetric carbon atoms and hence include various stereoisomers such as geometrical isomers, tautomers and optical isomers, either in admixture or isolated form. To isolate and purify these stereoisomers, the skilled artisan may employ any ordinary techniques including optical resolution by preferential crystallization or column chromatography or asymmetric synthesis.

The compounds (I) of the present invention occasionally form acid addition salts. Depending on the type of substituents, they also form salts with bases. While there are no particular limitations on the salts that can be formed as long as they are pharmaceutically acceptable, specific examples include: acid addition salts as with mineral acids such as hydrochloric acid, hydrobromic acid, hydroiodic acid, sulfuric acid, nitric acid and phosphoric acid, organocarboxylic acids such as acetic acid, propionic acid, oxalic acid, malonic acid, succinic acid, fumaric acid, maleic acid, lactic acid, formic acid, malic acid, tartaric acid, citric acid and mandelic acid, organosulfonic acids such as methanesulfonic acid, ethanesulfonic acid, p-toluenesulfonic acid and 2-hydroxyethanesulfonic acid, and acidic amino acids such as aspartic acid and glutamic acid; salts with alkali metals or alkaline earth metals (e.g. sodium, potassium, magnesium, calcium and aluminum), and organic bases such as methylamine, ethylamine, ethanolamine, pyridine, lysine, arginine and ornithine; and ammonium salts.

The salts of the compounds of the present invention include mono-, di- and tri-salts. Alternatively, depending on the substituents in side chains, the compounds of the present invention can form both an acid addition salt and a base salt at the same time.

Further, the present invention encompasses hydrates of the compounds (I), pharmaceutically feasible various solvates of the compounds, their polymorphs and the like. Needless to say, the present invention is not limited to the compounds described in the Examples to be set forth later but encompasses all aromatic compounds having cyclic amino groups as represented by the formula (I), and all pharmaceutically acceptable salts thereof.

The present invention also includes compounds of the formula (IV)-b that may be protected by suitable protective groups or salts thereof:

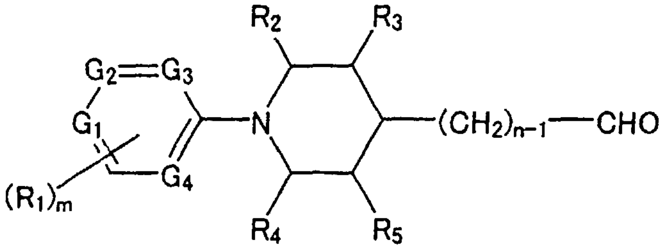

(IV) - b

(wherein $G_1$, $G_2$, $G_3$, $G_4$, $R_1$, $R_2$, $R_3$, $R_4$, $R_5$, n and m have the same meanings as respectively defined in the formula (I) and so do the preferred combinations of their definitions). In particular, compounds of the formula (IV')-b are useful:

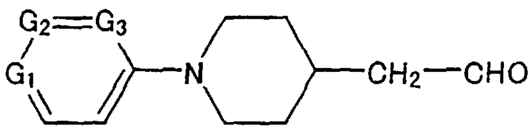

(IV') - b

(wherein $G_1$, $G_2$ and $G_3$ have the same meanings as respectively defined in the formula (II') and so do the preferred combinations of their definitions). Notably, the compound of the formula (IV")-b is useful:

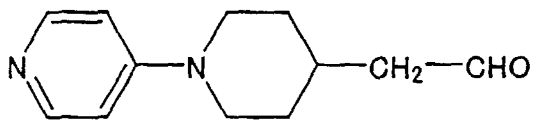

(IV") - b

The compounds mentioned above are at least useful as intermediates for the manufacture of the compounds of the formulae (I), (II') and (II"), respectively, or salts thereof.

The present invention also includes compounds of the formula (VI) that may be protected by suitable protective groups or salts thereof:

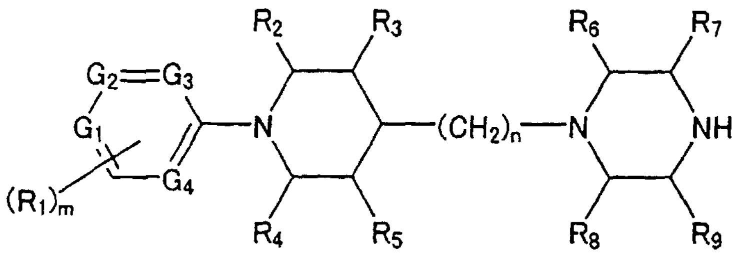

(VI)

(wherein $G_1$ - $G_4$, $R_1$ - $R_9$, m and n have the same meanings as defined in the formula (I) and so do the preferred combinations of their definitions). In particular, compounds of the formula (VI') or salts thereof are useful:

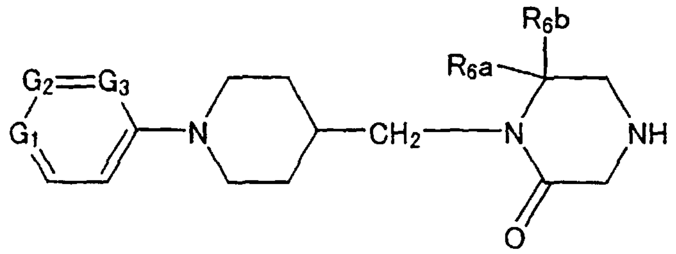

(VI')

(wherein $G_1$, $G_2$ and $G_3$ have the same meanings as respectively defined in the formula (II') and so do the preferred combinations of their definitions). Notably, compounds of the formula (VI") are useful:

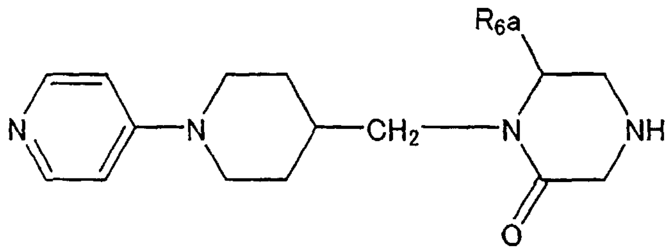

(VI")

(wherein $R_{6a}$ has the same meaning as defined in the formula (II')). The compounds mentioned above are at least useful as intermediates for the manufacture of the compounds of the formulae (I), (II') and (II"), respectively, or salts thereof.

Salts of the intermediates such as the compounds of the formulae (IV)-b and (VI) set forth above include both acid addition salts and salts with bases that have already been described in connection with the salts of the compounds of the formula (I) and it will be readily understood by the person skilled in the art that they are not necessarily subject to pharmaceutical limitations. The skilled artisan can select suitable protective groups as appropriate by referring to the definition of P in the formula (VII) to be set forth later and relevant textbooks such as "Protective Groups in Organic Synthesis", 2nd Edition, 1991.

(Methods of Production)

The compounds of the present invention which are represented by the formula (I) can be produced by the methods described below. In the following 〈Production Method 1〉, 〈Production Method 2〉and 〈Production Method 3〉and the explanation, unless otherwise stated, the definitions of $R_1$, $R_2$, $R_3$, $R_4$, $R_5$, $R_6$, $R_7$, $R_8$, $R_9$, $G_1$, $G_2$, $G_3$, $G_4$, Q, X, Y, $Z_1$, $Z_2$, m and n in the formulae (I), (I)-a, (I)-b, (III), (IV)-a, (IV)-b, (V), (VI), (VII), (VIII), (IX), (X), (XI), (XII), (XIII), (XIV), (XV), (XVI), (XVII), (XVIII), (XIX), (XX), (XXI)-a, (XXI)-b, (XXII), (XXIII), (XXIV), (XXV), (XXVI), (XXVII), (XXVIII), (XXIX), (XXX), (XXXI), (XXXII)-a, (XXXII)-b, (XXXIII), (XXXIV) and (XXXV) are the same as the former definitions described as to each in the formula (I). If the respective definitions are restricted to the preferred cases, the following explanation should be understood as describing the methods of producing the compounds of the formulae (II), (II') and (II").

The compounds of the present invention which are represented by the formula (I) and salts thereof can be synthesized by 〈Production Method 1〉, 〈Production Method 2〉and 〈Production Method 3〉starting from compounds represented by the formulae (III), (IX), (XIV), (XIX) and (XXIV) or salts thereof which can be readily prepared from documented or commercial compounds.

On the pages that follow, the methods of production of the compounds of the present invention are described in detail.

〈Production Method 1〉

A class of compounds of the formula (I) wherein X = CH and Y = N are represented by the formula (1)-a and the methods of producing them are described below.

Compounds represented by the formula (1)-a:

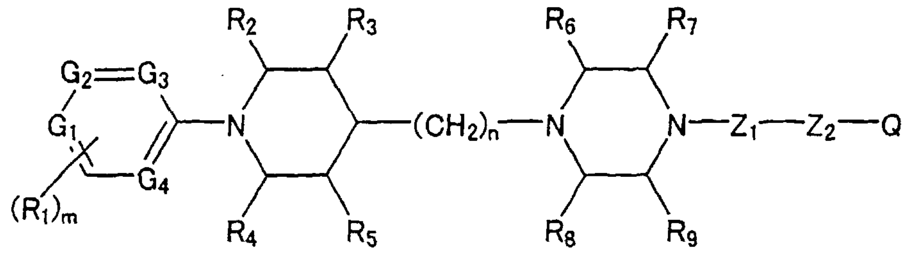

(I) - a

(wherein $G_1$, $G_2$, $G_3$, $G_4$, Q, $R_1$, $R_2$, $R_3$, $R_4$, $R_5$, $R_6$, $R_7$, $R_8$, $R_9$, $Z_1$, $Z_2$, m and n have the same meanings as defined above) or salts thereof are produced by the following methods designated 〈Production Method 1-1〉, 〈Production Method 1-2〉and 〈Production Method 1-3〉.

(Production Method 1-1)

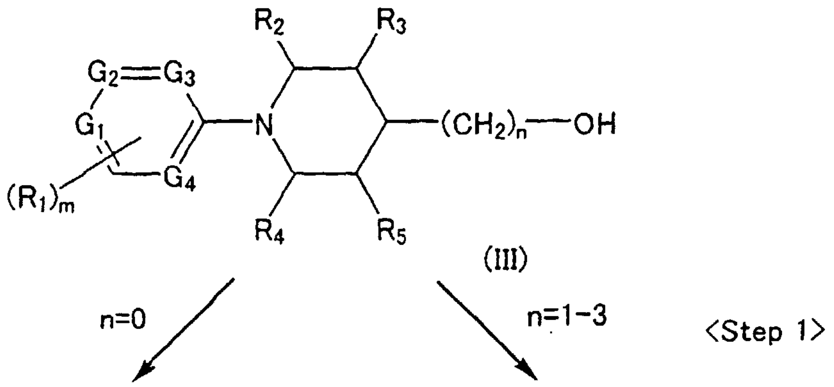


(III)

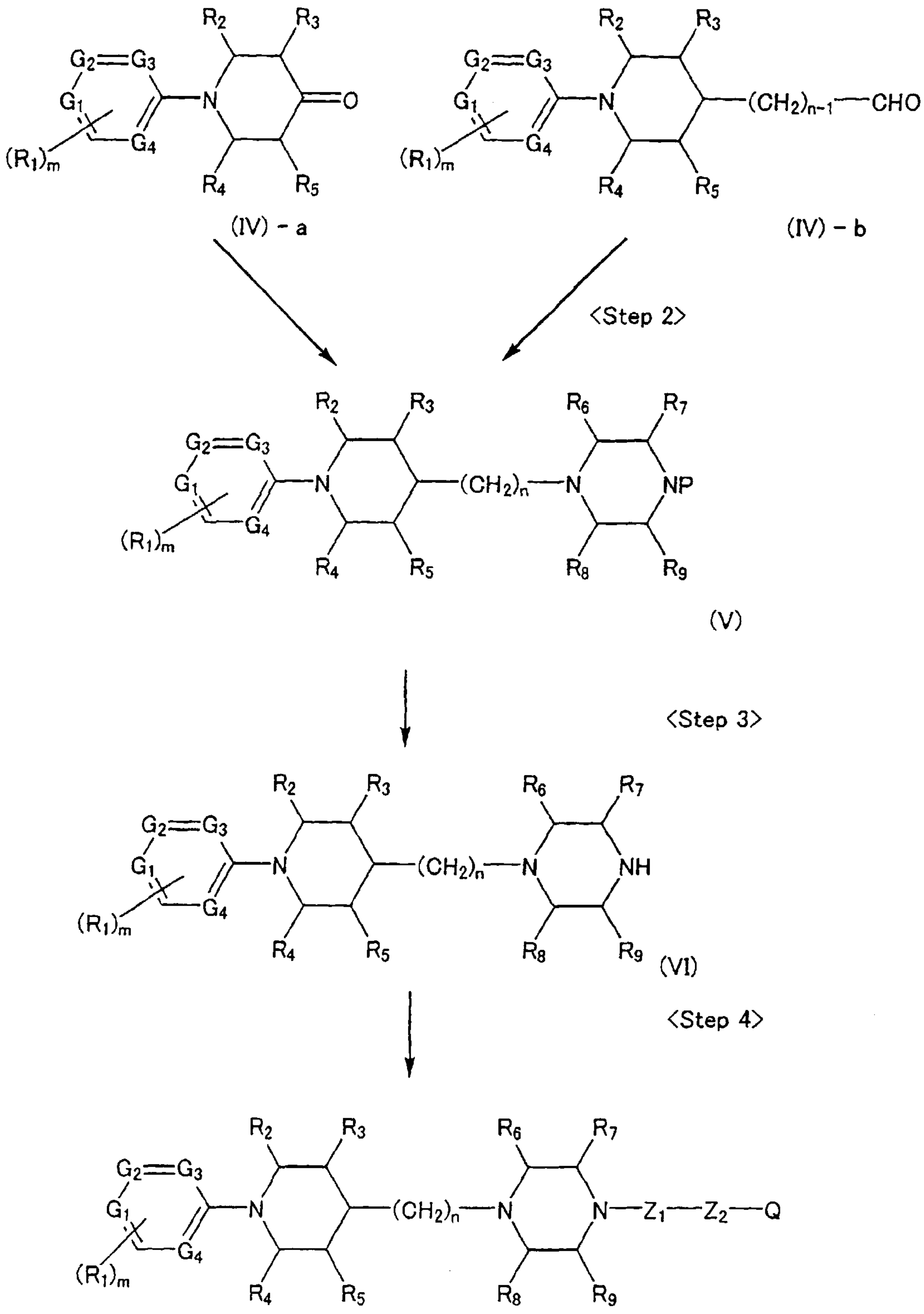


(IV) - a

(IV) - b

(V)

(VI)

( I ) - a

(Production Method 1-1)

〈Step 1〉

A compound of the formula (III) which can be readily derived from a commercial product or a salt thereof:

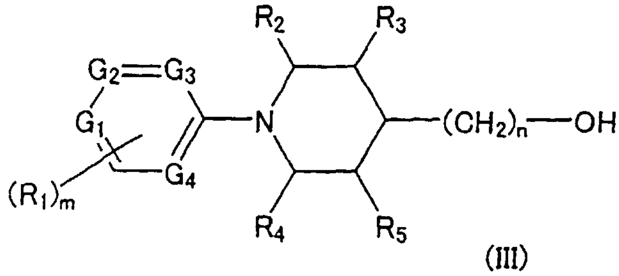

(III)

(wherein $G_1$ - $G_4$, $R_1$ - $R_5$, m and n have the same meanings as defined above) is subjected to an oxidation reaction. While the production method is described below in detail, it should be understood that the present invention is in no way limited to this method. The compound of the formula (III) or a salt thereof is subjected to an oxidation reaction such as the Swern oxidation (dimethyl sulfoxide (DMSO)/oxalyl chloride), oxidation with tetrapropylammonium perruthenate (TPAP)/N-methylmorpholine-N-oxide, the Corey-Kim oxidation (N-chlorosuccinimide (NCS)-dimethyl sulfide (DMS) complex), oxidation with pyridinium dichromate (PDC), oxidation with pyridinium chlorochromate (PCC) or the Jones oxidation ($Na_2Cr_2O_7$/Cr(VI)/sulfuric acid), preferably the Swern oxidation using DMSO/oxalyl chloride, in a halogenated hydrocarbon solvent typified by chloroform, methylene chloride and dichloroethane, preferably methylene chloride, at between -78°C and 0°C, preferably at between -78°C and -65°C, for a sufficient time to proceed the reaction to an adequate extent, specifically for 15 minutes to 1 hour, thereby producing a compound of the following formula (IV)-a or a salt thereof if n = 0 and a compound of the following formula (IV)-b or a salt thereof if n = 1 - 3:

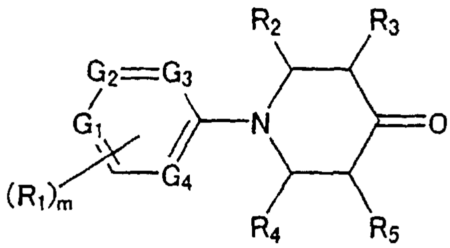

(IV) - a

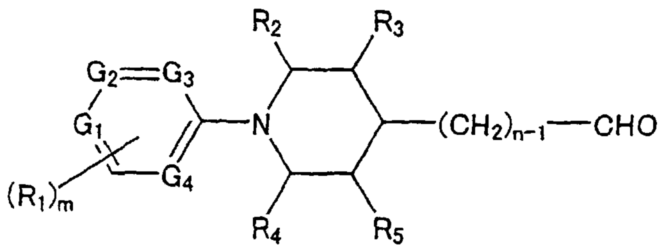

(IV) - b

(wherein $G_1$ - $G_4$, $R_1$ - $R_5$, m and n have the same meanings as defined above).

〈Step 2〉

The compound of the formula (IV)-a or (IV)-b or salt thereof that was obtained in 〈step 1〉 and a compound of the formula (VII) or a salt thereof:

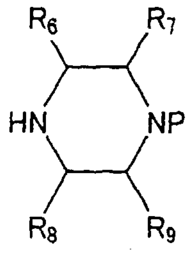

(VII)

(wherein $R_6$ - $R_9$ have the same meanings as defined above; P is a secondary amino protecting group such as a carbamate typified by t-butoxycarbonyl and benzyloxycarbonyl, an amide typified by formyl, acetyl and benzoyl, or an alkyl typified by benzyl, allyl, trityl and methoxymetyl) are subjected to reaction with a reducing agent within an aromatic hydrocarbon solvent such as toluene or benzene or a halogenated hydrocarbon solvent typified by chloroform, methylene chloride and dichlroethane, preferably methylene chloride, in the presence or absence of acetic acid, preferably in its presence, under an argon atmosphere. Generally, all reducing agents can be used as long as they can reduce the imino group to the amino group. Preferably, using a reducing agent such as sodium triacetoxyborohydride, sodium borohydride, lithium borohydride, diisobutylaluminum hydride or sodium cyanoborohydride, a reductive amination reaction is performed at between -78°C and room temperature, preferably under cooling with ice, for a sufficient time to proceed the reaction to an adequate extent, specifically for 3 - 12 hours, thereby producing a compound of the formula (V) or a salt thereof:

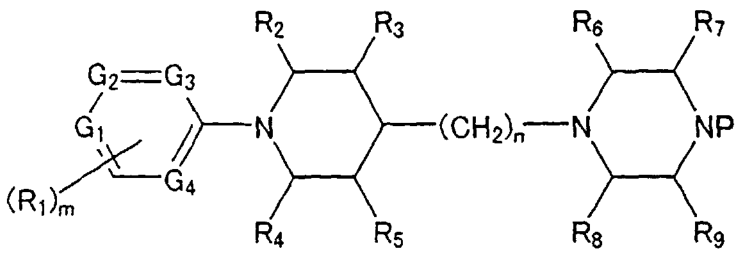

(V)

(wherein $G_1$ - $G_4$, P, $R_1$ - $R_9$, m and n have the same meanings as defined above).

〈Step 3〉

The compound of the formula (V) or salt thereof that was obtained in 〈step 2〉is subjected to a deprotective reaction in the presence or absence of anisole, preferably in its presence, using an acid such as trifluoroacetic acid, hydrochloric acid, sulfuric acid, p-toluenesulfonic acid or methanesulfonic acid, preferably trifluoroacetic acid, or a methanol solution containing about 5% - 20% hydrogen chloride gas, or an ethyl acetate solution containing about 5% - 20% hydrogen chloride gas, under an argon atmosphere at a temperature between cooling with ice and room temperature, preferably at room temperature, for a sufficient time to proceed the reaction to an adequate extent, specifically for 3 - 12 hours, thereby producing a compound of the formula (VI) or a salt thereof:

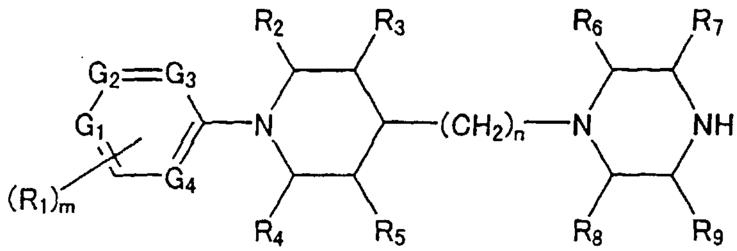

(VI)

(wherein $G_1$ - $G_4$, $R_1$ - $R_9$, m and n have the same meanings as defined above).

〈Step 4〉

The compound of the formula (VI) or a salt thereof that was obtained in 〈step 3〉and a reactive derivative of the formula (VIII) or a salt thereof:

$$W\text{-}Z_1\text{-}Z_2\text{-}Q \qquad \text{(VIII)}$$

(wherein W is a halogen atom, a leaving group such as a methanesulfonyloxy group or a p-toluenesulfonyloxy group or an exchangeable substituent such as an alcohol or an alkoxy group; $Z_1$, $Z_2$ and Q have the same meanings as defined above) are subjected to a reaction using an inorganic base such as potassium carbonate, cesium carbonate, calcium carbonate or sodium hydride or an organic base such as triethylamine, pyridine or N,N-dialkylaniline, preferably triethylamine, within a polar solvent such as acetonitrile or N,N-dimethylformamide (DMF), a halogenated hydrocarbon solvent typified by chloroform and methylene chloride or an ether-based solvent typified by diethyl ether and tetrahydrofuran (THF), preferably methylene chloride, under an argon atmosphere at a temperature between room temperature and an elevated temperature where the reaction mixture refluxes, preferably at room temperature, for a sufficient time to proceed the reaction to an adequate extent, specifically for 1 - 12 hours; alternatively, the compound of the formula (VIII) is first activated with a phosphorus compound (e.g. triphenylphosphine or tributylphosphine) and diethyl azodicarboxylate (DEAD) and then it is heated in an inert solvent (e.g. tetrahydrofuran, benzene, toluene, dichloromethane or chloroform) at a temperature between room temperature and the boiling point of the solvent used, thereby producing a compound of the formula (I)-a or a salt thereof:

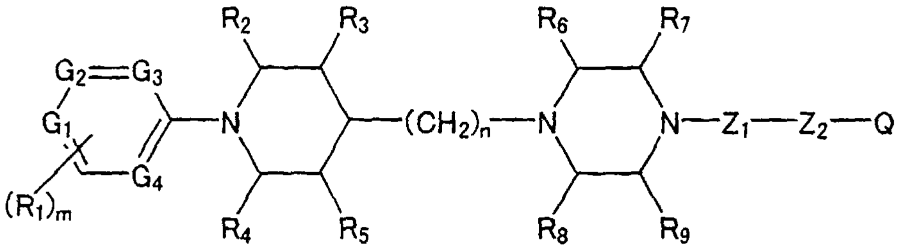

(I) - a

(wherein $G_1$ - $G_4$, Q, $R_1$ - $R_9$, $Z_1$, $Z_2$, m and n have the same meanings as defined above).

Alternatively, the same compound can be produced by the procedure described in Production Method 1-2.

The following are details of Production Method 1-2.

(Production Method 1-2)

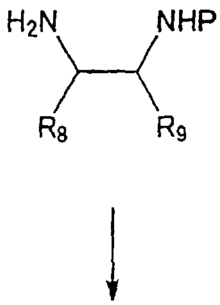

(IX)

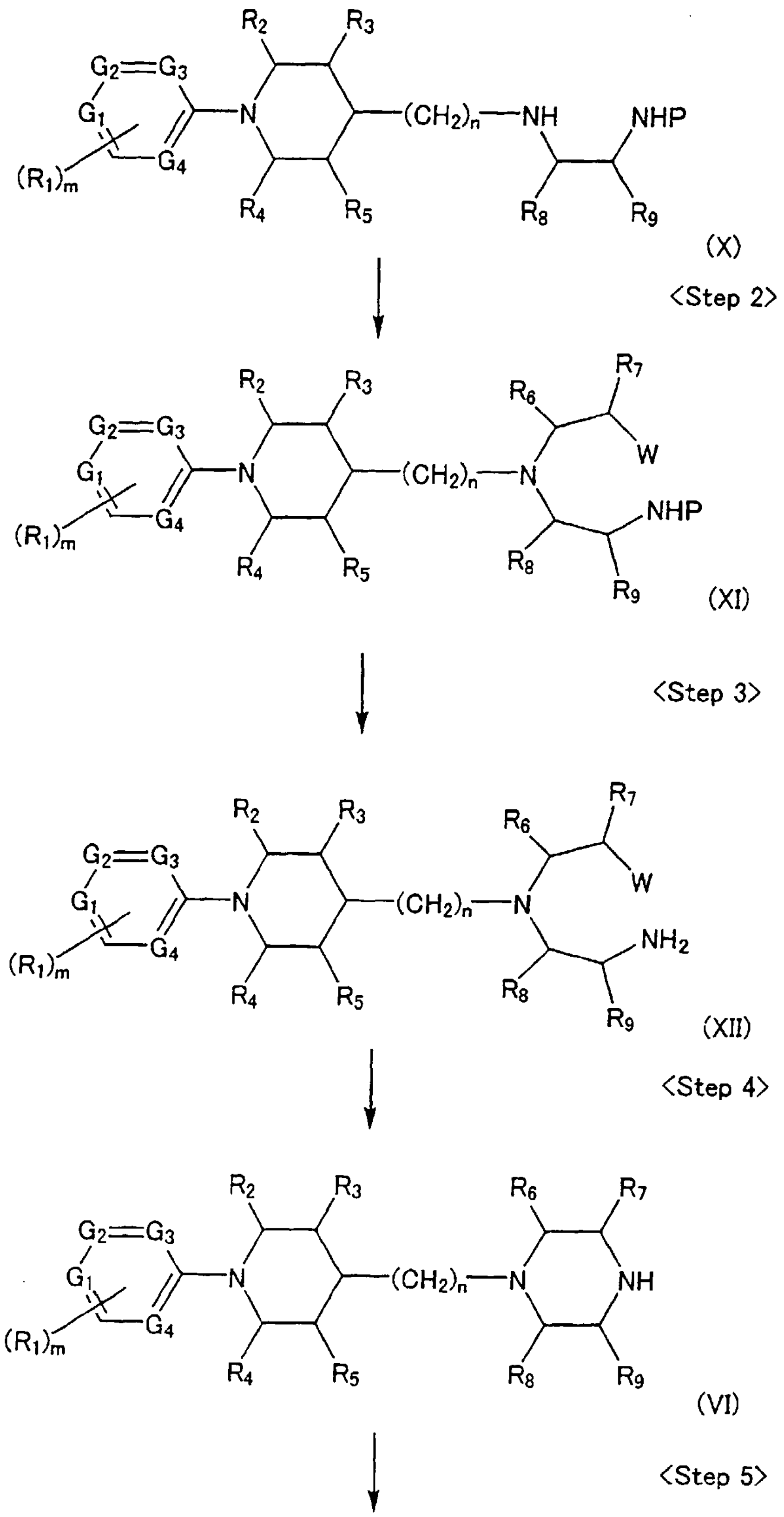
(X)
<Step 2>
(XI)
<Step 3>
(XII)
<Step 4>
(VI)
<Step 5>

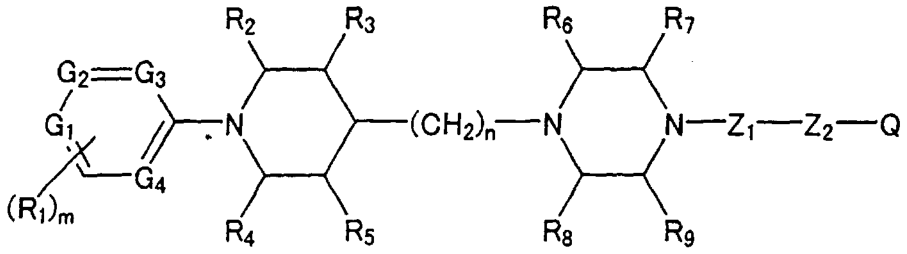

(I)-a

(Production Method 1-2)

〈Step 1〉

A compound of the formula (IV)-a or (IV)-b or a salt thereof that was derived from commercial products by the same procedure as in 〈Step 1〉of (Production Method 1-1) and a compound of the formula (IX) or a salt thereof:

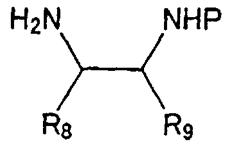

(IX)

(wherein P, $R_8$ and $R_9$ have the same meanings as defined above) are reacted by the same procedure as in 〈Step 2〉of (Production Method 1-1) to produce a compound of the formula (X) or a salt thereof:

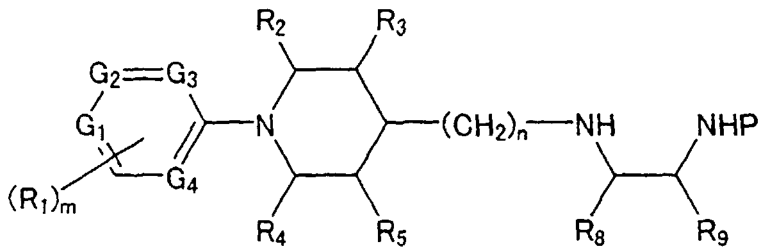

(X)

(wherein $G_1$ - $G_4$, P, $R_1$ - $R_5$, $R_8$, $R_9$, m and n have the same meanings as defined above).

〈Step 2〉

The compound of the formula (X) or a salt thereof that was obtained in 〈step 1〉and a compound of the formula (XIII) or a salt thereof:

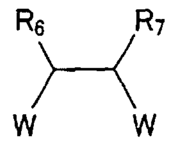

(XIII)

(wherein $R_6$, $R_7$ and W have the same meanings as defined above) are subjected to a reaction using an inorganic base such as potassium carbonate, cesium carbonate, calcium carbonate or sodium hydride or an organic base such as triethylamine, pyridine or N,N-dialkylaniline, preferably triethylamine, within a polar solvent such as acetonitrile or N,N-dimethylformamide (DMF), a halogenated hydrocarbon solvent typified by chloroform and methylene chloride or an ether-based solvent typified by ether or tetrahydrofuran (THF), preferably methylene chloride, under an argon atmosphere at a temperature between room temperature and an elevated temperature where the reaction mixture refluxes, preferably at room temperature, for a sufficient time to proceed the reaction to an adequate extent, specifically for 1 - 12 hours; alternatively, the compound of the formula (XIII) is first activated with a phosphorus compound (e.g. triphenylphosphine or tributylphosphine) and diethyl azodicarboxylate (DEAD) and then it is heated in an inert solvent (e.g. tetrahydrofuran, benzene, toluene, dichloromethane or chloroform) at a temperature between room temperature and the boiling point of the solvent used, thereby producing a compound of the formula (XI) or a salt thereof:

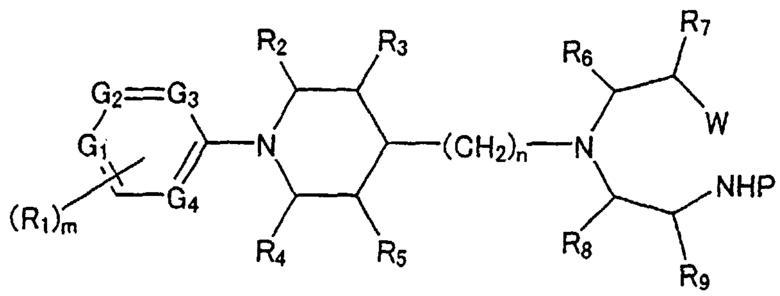

(XI)

(wherein $G_1$ - $G_4$, P, $R_1$ - $R_9$, W, m and n have the same meanings as defined above).

〈Step 3〉

The compound of the formula (XI) or a salt thereof that was obtained in 〈step 2〉is subjected to the same reaction as in 〈step 3〉of (Production Method 1-1) to produce a compound of the formula (XII) or a salt thereof:

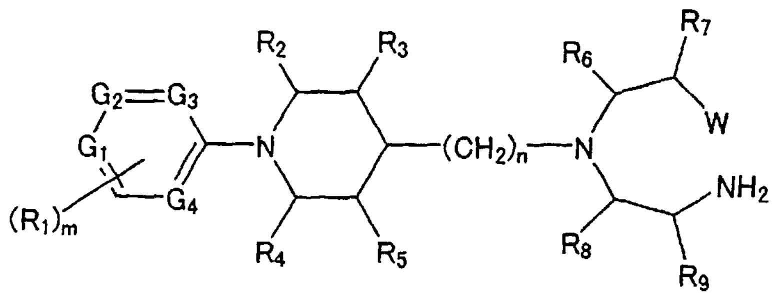

(XII)

(wherein $G_1$ - $G_4$, $R_1$ - $R_9$, W, m and n have the same meanings as defined above).

〈Step 4〉

The compound of the formula (XII) or a salt thereof that was obtained in 〈step 3〉is subjected to the same reaction as in 〈step 2〉of (Production Method 1-2) to produce a compound of the formula (VI) or a salt thereof:

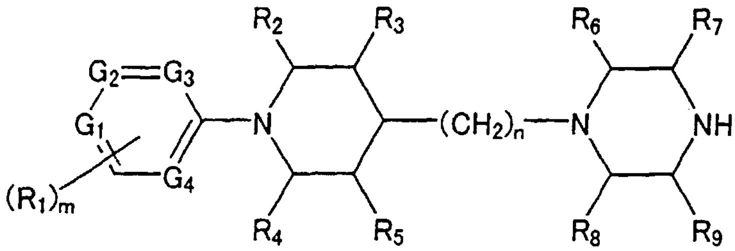

(VI)

(wherein $G_1$ - $G_4$, $R_1$ - $R_9$, m and n have the same meanings as defined above).

〈Step 5〉

The compound of the formula (VI) or a salt thereof that was obtained in 〈step 4〉is subjected to the same reaction as in 〈step 4〉of (Production Method 1-1) with a compound of the formula (VIII) or a salt thereof to produce a compound of the formula (I)-a or a salt thereof:

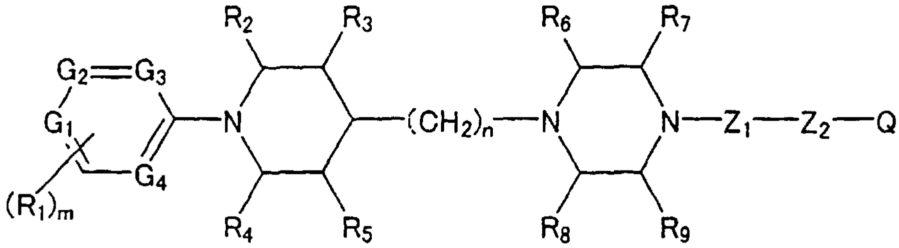

(I) - a

(wherein $G_1$ - $G_4$, Q, $R_1$ - $R_9$, $Z_1$, $Z_2$, m and n have the same meanings as defined above).

Alternatively, the same compound can be produced by the procedure described in Production Method 1-3.

The following are details of Production Method 1-3.

(Production Method 1-3)

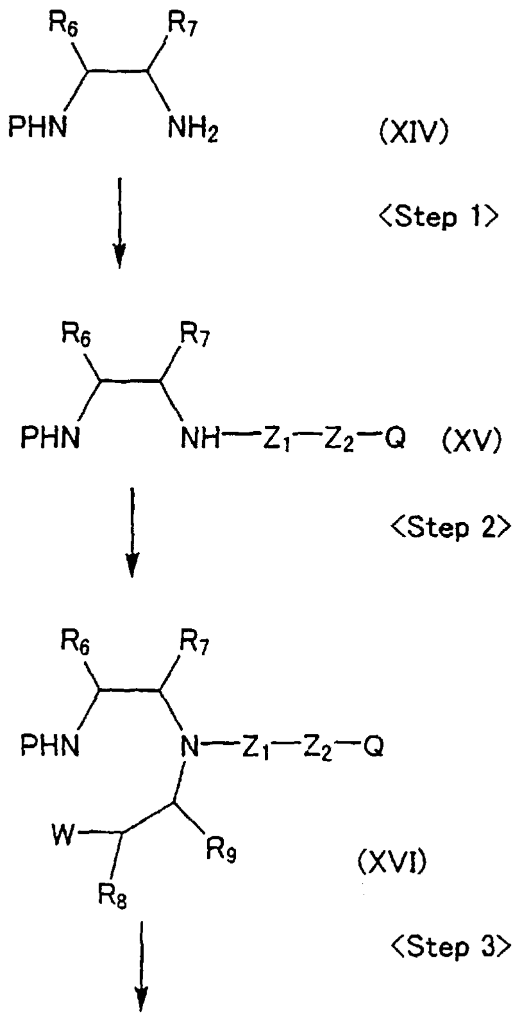

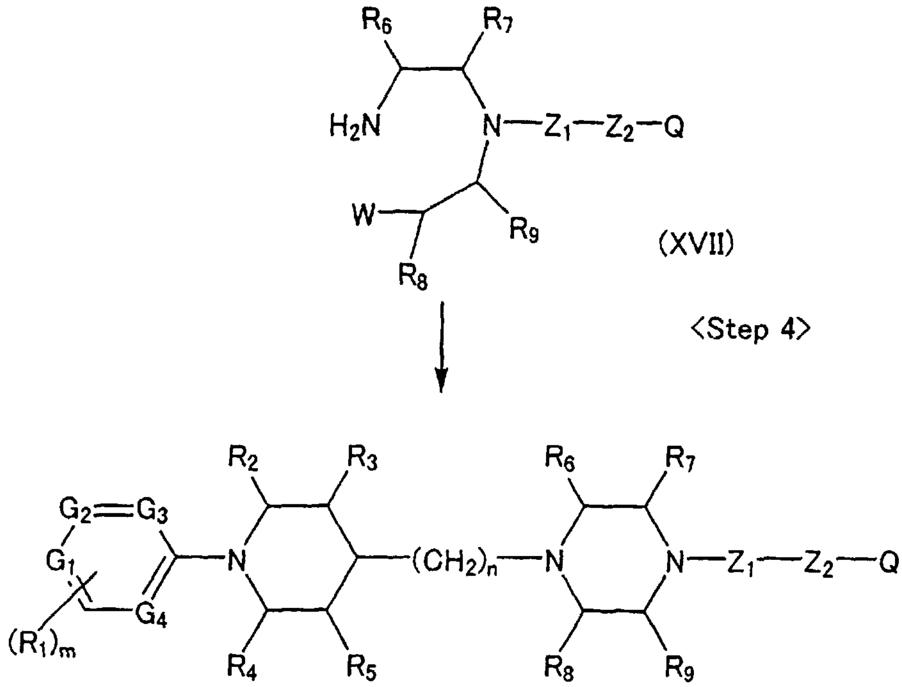


(XVII)

( I ) - a

(Production Method 1-3)

〈Step 1〉

A compound of the formula (XIV) that can be readily derived from a commercial product or a salt thereof:

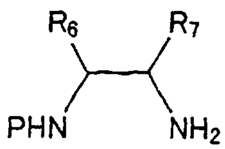

(XIV)

(wherein P, $R_6$ and $R_7$ have the same meanings as defined above) and a compound of the formula (VIII) or a salt thereof are subjected to the same reaction as in 〈Step 4〉 of (Production Method 1-1) to produce a compound of the formula (XV) or a salt thereof:

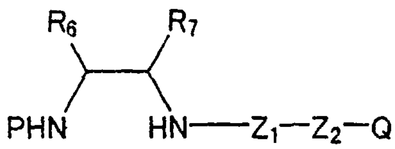

(XV)

(wherein P, Q, $R_6$, $R_7$, $Z_1$ and $Z_2$ have the same meanings as defined above).

〈Step 2〉

The compound of the formula (XV) or a salt thereof that was obtained in 〈Step 1〉 and a compound of the formula (XVIII), which is either commercially available or readily derived from a commercial product, or a salt thereof:

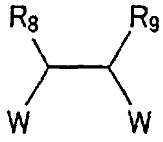

(XVIII)

(wherein $R_8$, $R_9$ and W have the same meanings as defined above) are subjected to the same reaction as in 〈step 2〉of (Production Method 1-2) to produce a compound of the formula (XVI) or a salt thereof:

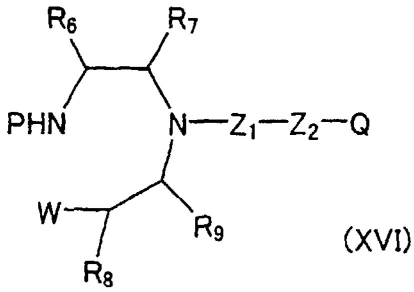

(XVI)

(wherein P, Q, $R_6$ - $R_9$, W, $Z_1$ and $Z_2$ have the same meanings as defined above).

〈Step 3〉

The compound of the formula (XVI) or a salt thereof that was obtained in 〈step 2〉is subjected to the same reaction as in 〈step 3〉of (Production Method 1-1) to produce a compound of the formula (XVII) or a salt thereof:

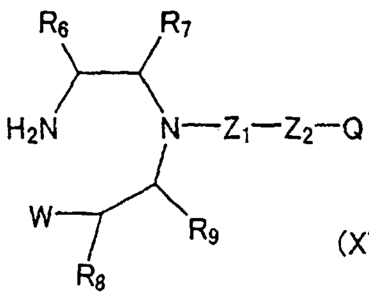

(XVII)

(where Q, $R_6$ - $R_9$, W, $Z_1$ and $Z_2$ have the same meanings as defined above).

〈Step 4〉

The compound of the formula (XVII) or a salt thereof that was obtained in 〈step 3〉is reacted as in 〈step 2〉of (Production Method 1-1) with a compound of the formula (IV)-a or (IV)-b or a salt thereof derived from a commercial product by the same procedure as in 〈step 1〉of (Production 1-1), thereby producing a compound of the formula (I)-a or a salt thereof:

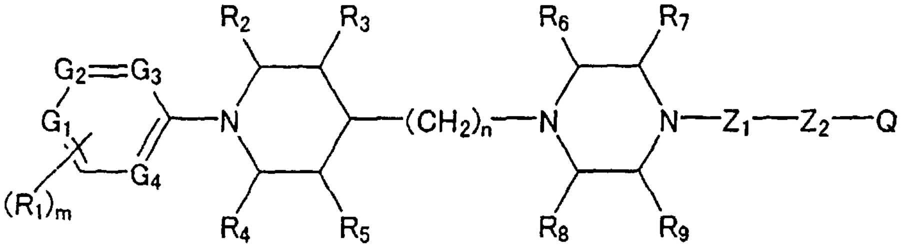

(I) - a

(wherein $G_1$ - $G_4$, Q, $R_1$ - $R_9$, $Z_1$, $Z_2$, m and n have the same meanings as defined above).

〈Production Method 2〉

A class of compounds of the formula (I) wherein X = N and Y = CH are represented by the formula (I)-b and the methods of producing them are described below.

Compounds represented by the formula (I)-b:

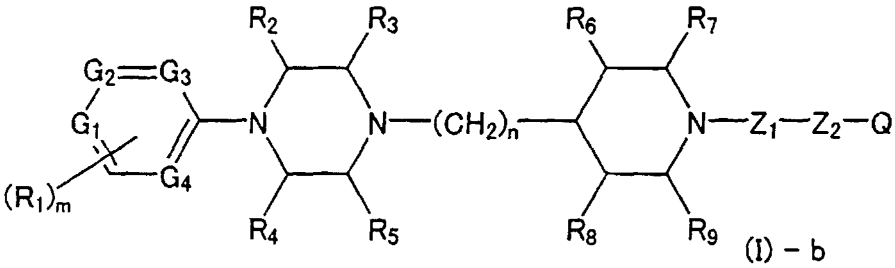

(I) - b

(wherein $G_1$ - $G_4$, Q, $R_1$ - $R_9$, $Z_1$, $Z_2$, m and n have the same meanings as defined above) or salts thereof are produced as described in 〈Production Method 2-1〉and 〈Production Method 2-2〉

(Production Method 2-1)

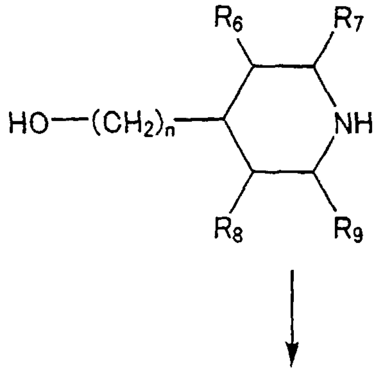

(XIX)

<Step 1>

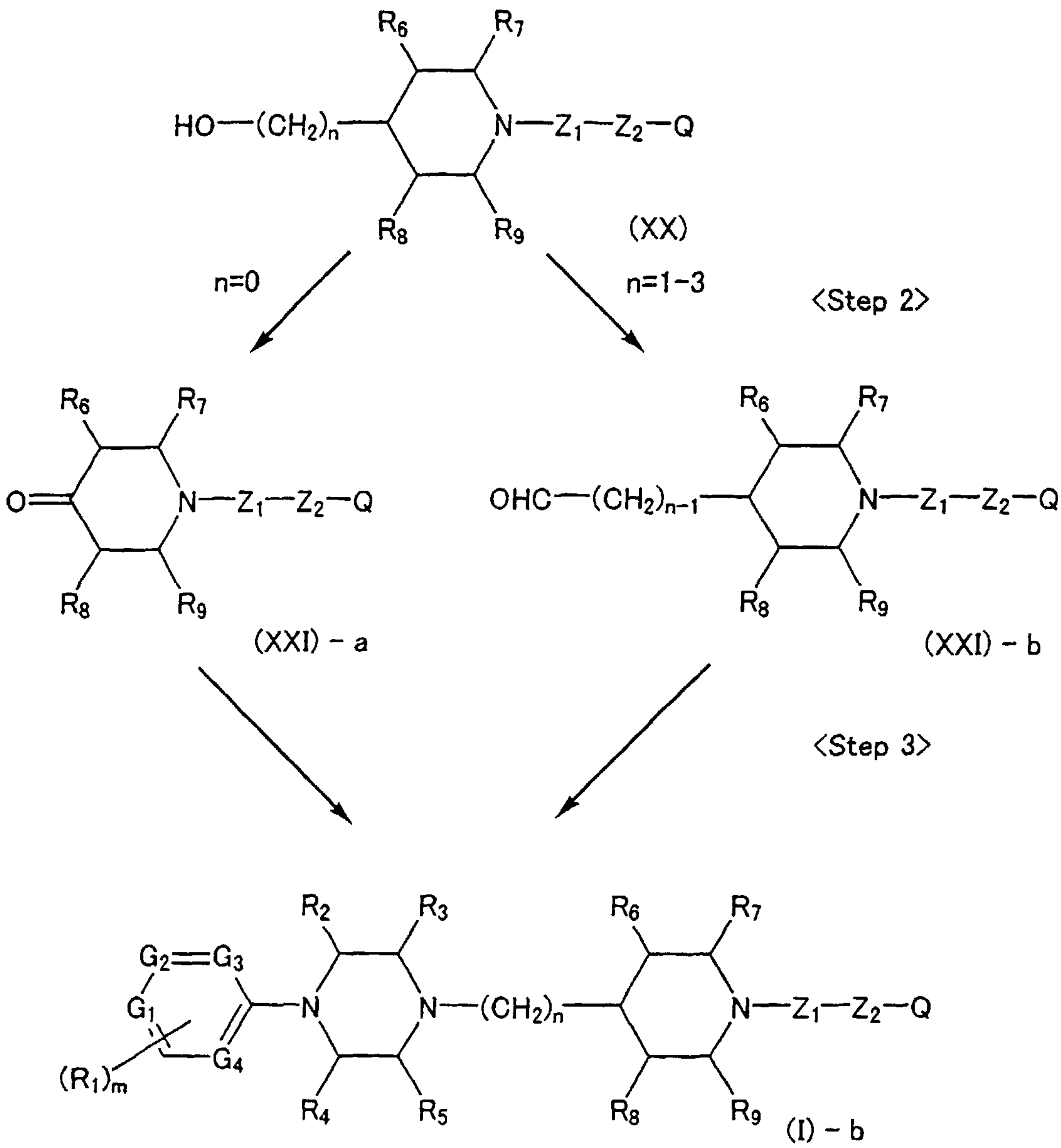


(Production Method 2-1)

〈Step 1〉

A compound of the formula (XIX), which is either commercially available or derived from commercial products by documented procedures, or a salt thereof:

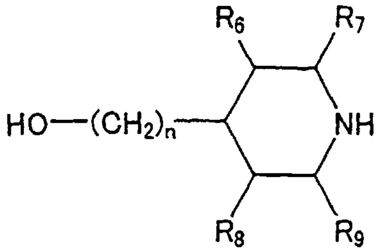

(XIX)

(wherein $R_6$ - $R_9$ and n have the same meanings as defined above) is subjected to the same reaction as in 〈step 4〉 of (Production Method 1-1) with a reactive derivative of the formula (VIII) or a salt thereof:

$$W\text{-}Z_1\text{-}Z_2\text{-}Q \qquad \text{(VIII)}$$

(wherein W is a halogen atom, a leaving group such as a methanesulfonyloxy group or a p-toluenesulfonyloxy group or an exchangeable substituent such as an alcohol or an alkoxy group; $Z_1$, $Z_2$ and Q have the same meanings as defined above), thereby producing a compound of the formula (XX) or a salt thereof:

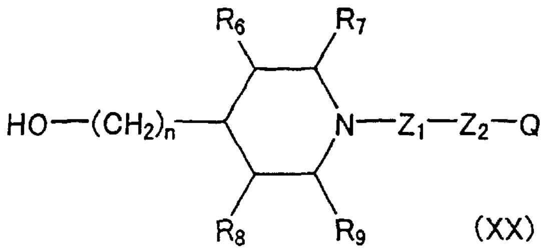

(XX)

(wherein Q, $R_6$ - $R_9$, $Z_1$, $Z_2$ and n have the same meanings as defined above).

〈step 2〉

The compound of the formula (XX) or a salt thereof that was obtained in 〈step 1〉 is subjected to the same reaction as in 〈step 1〉 of (Production Method 1-1) to produce a compound of the following formula (XXI)-a or a salt thereof if n = 0 and a compound of the following formula (XXI)-b or a salt thereof if n = 1 - 3:

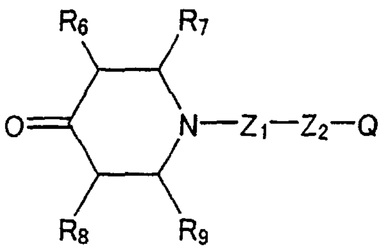

(XXI) - a

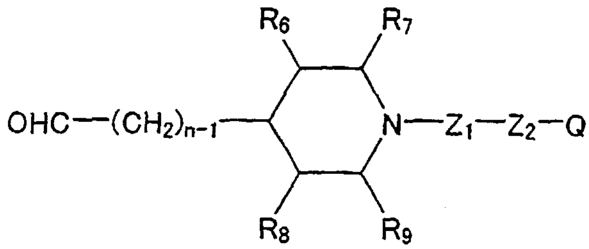

(XXI) - b

(wherein Q, $R_6$ - $R_9$, $Z_1$, $Z_2$ and n have the same meanings as defined above).

〈step 3〉

The compound of the formula (XXI)-a or (XXI)-b or a salt thereof that was obtained in 〈step 2〉 is subjected to the same reaction as in 〈step 2〉 of (Production Method 1-1) with a compound of the formula (XXII) or a salt thereof:

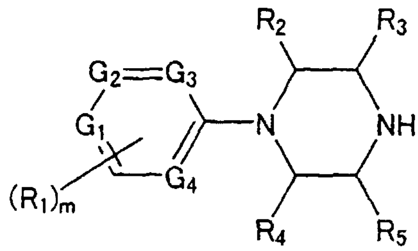

(XXII)

(wherein $G_1$ - $G_4$, $R_1$ - $R_5$ and m have the same meanings as defined above) to produce a compound of the formula (I)-b or a salt thereof:

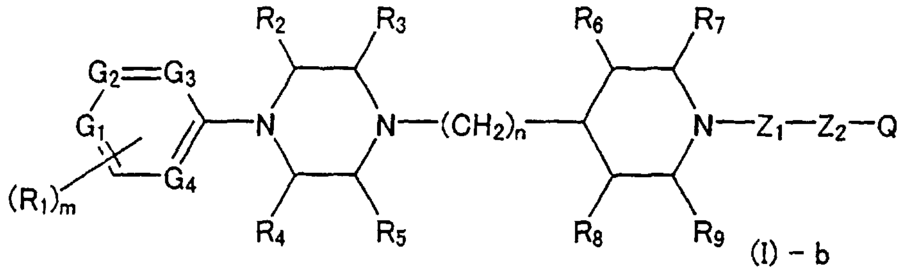

(I) - b

(wherein $G_1$ - $G_4$, Q, $R_1$ - $R_9$, $Z_1$, $Z_2$, m and n have the same meanings as defined above).

Alternatively, the same compound can be produced by the procedure described in Production 2-2.

The following are details of Production Method 2-2.

(Production Method 2-2)

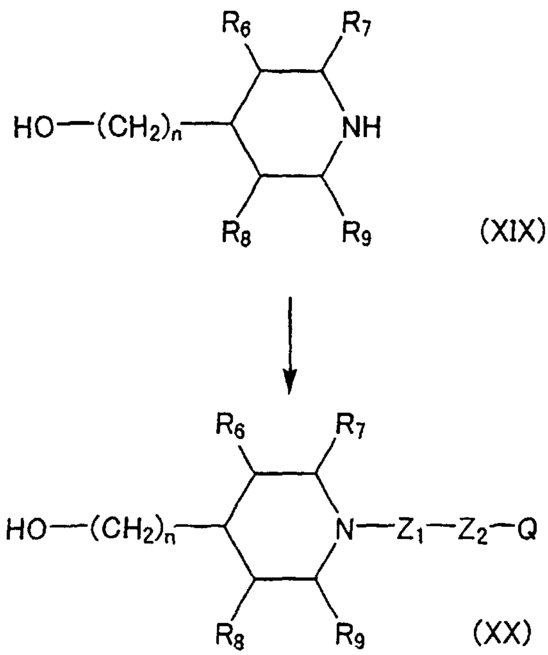

(XIX)

<Step 1>

(XX)

<Step 2>

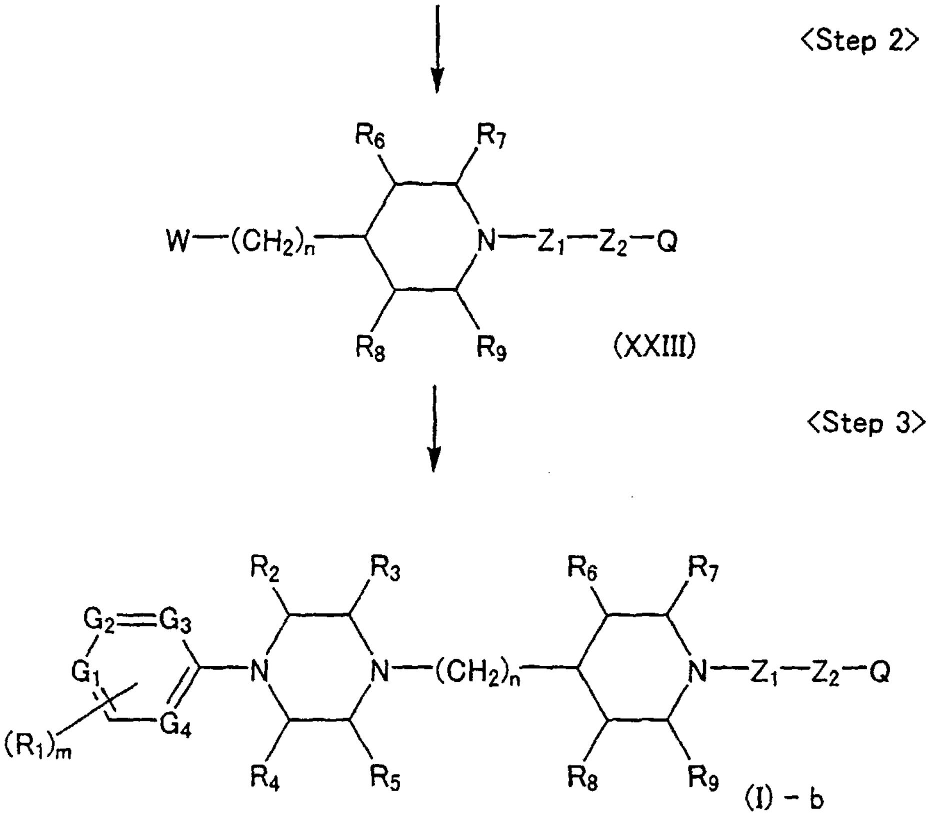

(XXIII)

<Step 3>

(I) - b

(Production Method 2-2)

〈Step 1〉

A compound of the formula (XIX), which is either commercially available or obtainable from a commercial product by a documented method, or a salt thereof:

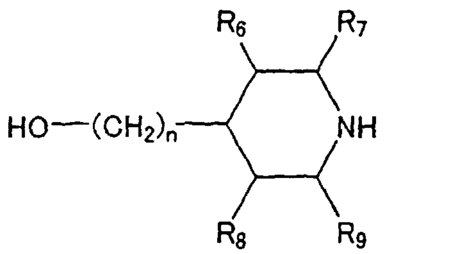

(XIX)

(wherein $R_6$ - $R_9$ and n have the same meanings as defined above) is subjected to the same reaction as in 〈step 4〉of (Production Method 1-1) with a reactive derivative of the formula (VIII) or a salt thereof:

$$W\text{-}Z_1\text{-}Z_2\text{-}Q \qquad \text{(VIII)}$$

(wherein W is a halogen atom, a leaving group such as a methanesulfonyloxy group or a p-toluenesulfonyloxy group or an exchangeable substituent such as an alcohol or an alkoxy group; $Z_1$, $Z_2$ and Q have the same meanings as defined above), thereby producing a compound of the formula (XX) or a salt thereof:

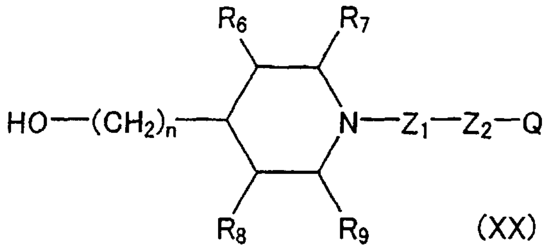

(XX)

(wherein Q, $R_6$ - $R_9$, $Z_1$, $Z_2$ and n have the same meanings as defined above).

〈Step 2〉

The compound of the formula (XX) or a salt thereof that was produced in 〈step 1〉is subjected to reaction in a halogenated hydrocarbon solvent typified by chloroform, methylene chloride and dichloroethane, preferably methylene chloride, using thionyl chloride, phosphorus pentachloride, phosphorus oxychloride, thionyl bromide, phosphorus pentabromide or phosphorus oxybromide, preferably thionyl chloride or thionyl bromide, at between -20°C and 50°C, preferably at a temperature between the temperature created by cooling with ice and room temperature; alternatively, the same compound or a salt thereof is subjected to reaction in a solvent of carbon tetrachloride or carbon tetrabromide, using triphenylphosphine at a temperature between room temperature and the temperature where the solvent refluxes; or alternatively, the same compound or a salt thereof is subjected to reaction in an ether-based solvent such as ether or THF, preferably ether, using phosphorus trichloride or phosphorus tribromide, preferably phosphorus trichloride, at between -20°C and 50°C, preferably under cooling with ice; either scheme produces a compound of the formula (XXIII) or a salt thereof:

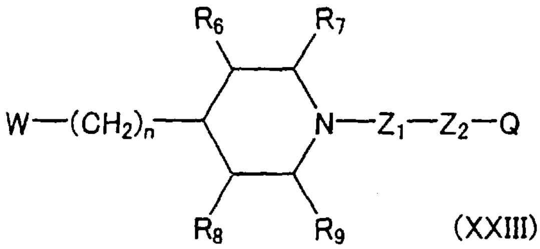

(XXIII)

(wherein Q, $R_6$ - $R_9$, W, $Z_1$, $Z_2$ and n have the same meanings as defined above).

〈Step 3〉

The compound of the formula (XXIII) or salt thereof that was obtained in 〈step 2〉and a compound of the formula (XXII) or a salt thereof:

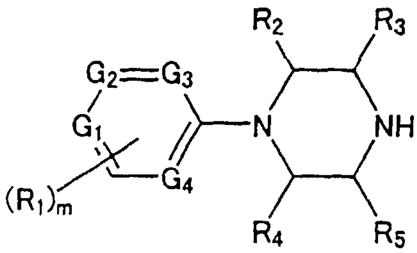

(XXII)

(wherein $G_1$ - $G_4$, $R_1$ - $R_5$ and m have the same meanings as defined above) are subjected to reaction using an inorganic base such as potassium carbonate, cesium carbonate, calcium carbonate or sodium hydride or an organic base

such as triethylamine, pyridine or N,N-dialkylaniline, preferably triethylamine, within a polar solvent such as acetonitrile or DMF, a halogenated hydrocarbon solvent typified by chloroform and methylene chloride or an ether-based solvent typified by ether and THF, preferably methylene chloride, under an argon atmosphere at a temperature between room temperature and an elevated temperature where the reaction mixture refluxes, preferably at room temperature, for a sufficient time to proceed the reaction to an adequate extent, specifically for 1 - 12 hours, thereby producing a compound of the formula (I)-b or a salt thereof:

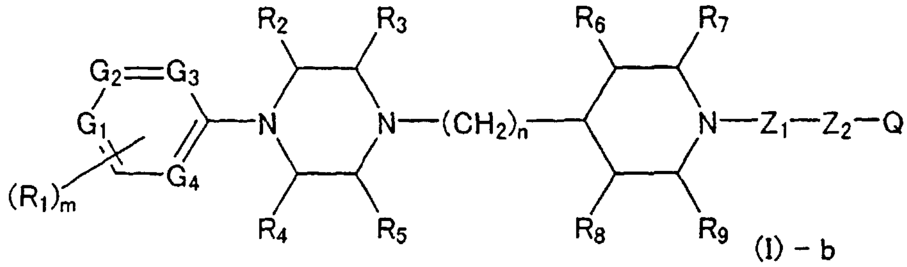

(I) - b

(wherein $G_1$ - $G_4$, Q, $R_1$ - $R_9$, $Z_1$, $Z_2$, m and n have the same meanings as defined above).

⟨Production Method 3⟩

The compound of the formula (I) or a salt thereof can also be produced by the following (Production Method 3-1):

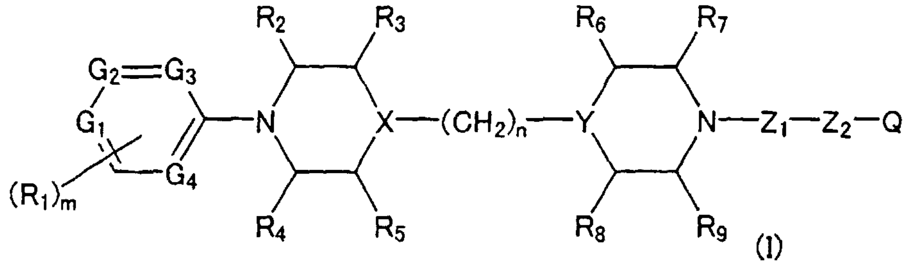

(I)

(wherein $G_1$ - $G_4$, Q, $R_1$ - $R_9$, X, Y, $Z_1$, $Z_2$, m and n have the same meanings as defined above).

(Production Method 3-1)

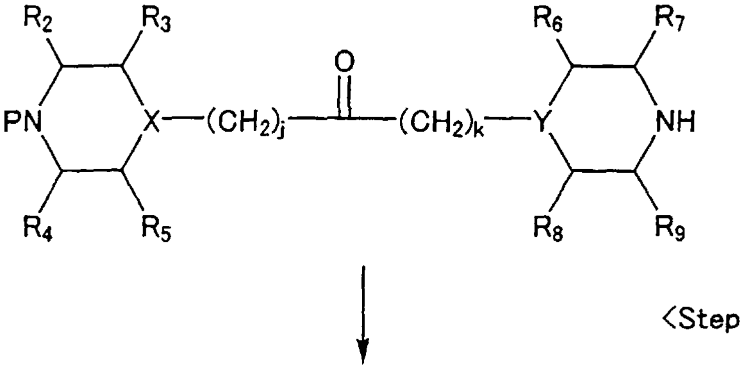

(XXIV)

⟨Step 1⟩

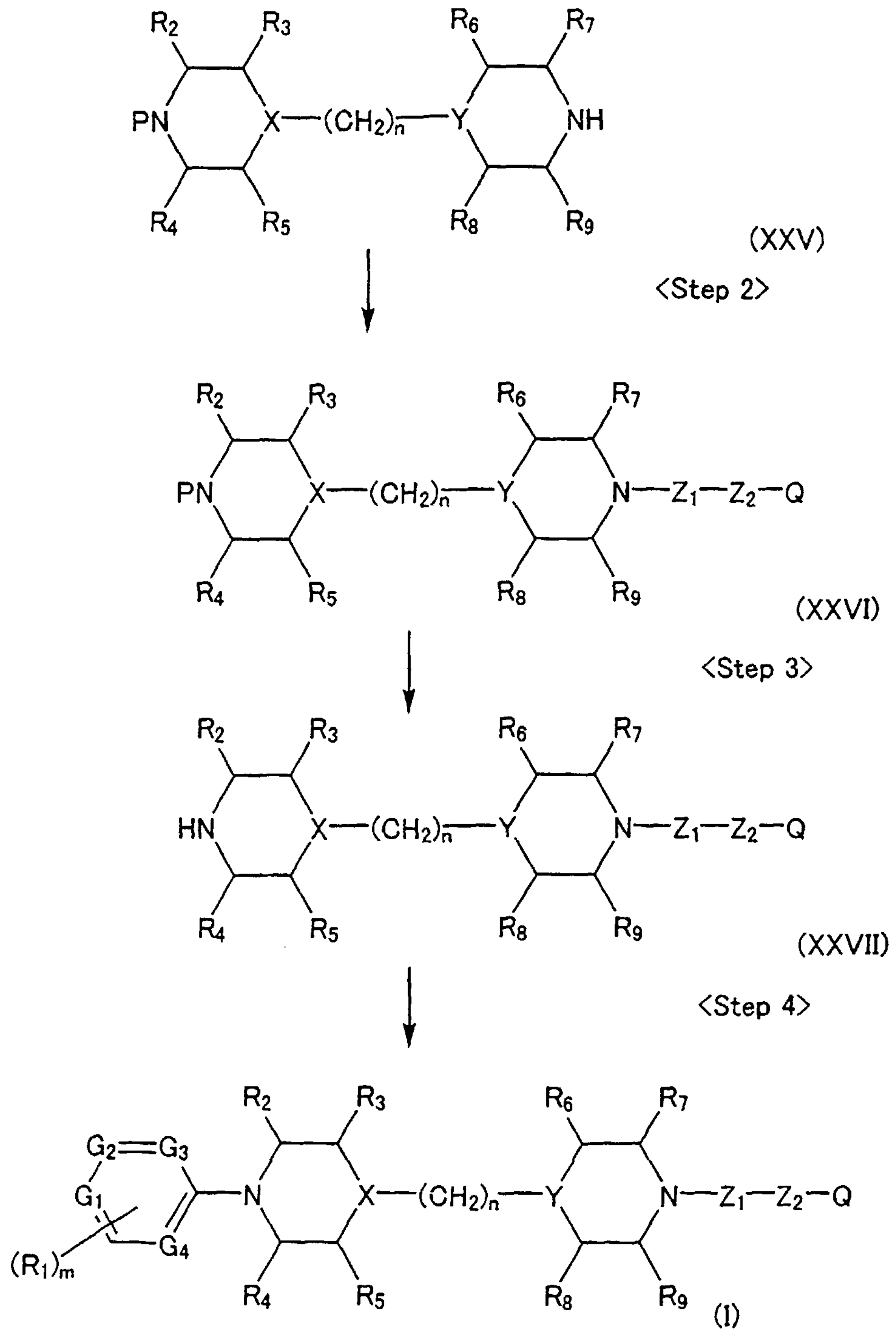


(Production Method 3-1)

〈Step 1〉

A compound of the formula (XXIV) prepared by a documented (WO96/10022) method or a salt thereof:

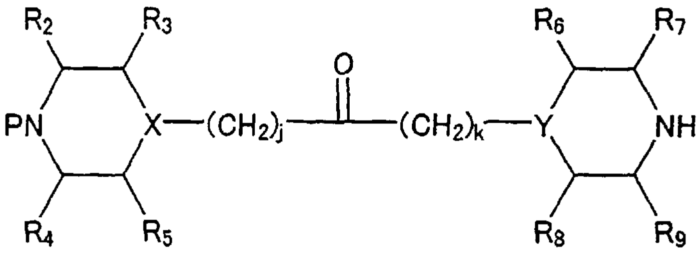

(XXIV)

(wherein P, $R_2$ - $R_9$, X and Y have the same meanings as defined above; j and k are each an integer of 0 - 2; the total number of carbon atoms in the bridge between the two rings is 1 - 3) is subjected to a reduction reaction in an ether-based solvent typified by ether, THF, DME and diglyme (diethylene glycol dimethyl ether), preferably THF, under an argon atmosphere using a reducing agent such as lithium aluminum hydride, diisobutyl aluminum hydride, borane dimethyl sulfide complex, borane THF complex, borane trimethylamine complex or alane at a temperature between -78°C and the temperature where the solvent refluxes, for a sufficient time to proceed the reaction to an adequate extent, specifically for 1 - 12 hours, thereby producing a compound of the formula (XXV) or a salt thereof:

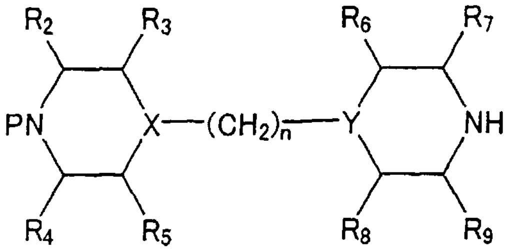

(XXV)

(wherein P, $R_2$ - $R_9$, X, Y and n have the same meanings as defined above).

〈Step 2〉

The compound of the formula (XXV) or a salt thereof that was obtained in 〈step 1〉 is subjected to the same reaction as in 〈step 4〉 of (Production Method 1-1) with a reactive derivative of the formula (VIII):

$$W\text{-}Z_1\text{-}Z_2\text{-}Q \qquad \text{(VIII)}$$

(wherein W is a halogen atom, a leaving group such as a methanesulfonyloxy group or a p-toluenesulfonyloxy group or an exchangeable substituent such as an alcohol or an alkoxy group; $Z_1$, $Z_2$ and Q have the same meanings as defined above), thereby producing a compound of the formula (XXVI) or a salt thereof:

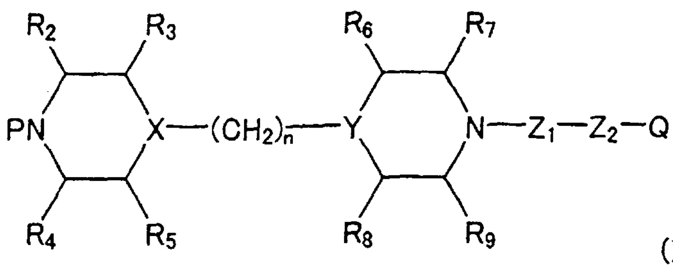

(XXVI)

(wherein P, Q, $R_2$ - $R_9$, $Z_1$, $Z_2$ , X, Y and n have the same meanings as defined above).

〈Step 3〉

The compound of the formula (XXVI) or a salt thereof that was obtained in 〈step 2〉 is subjected to the same reaction as in 〈step 3〉 of (Production Method 1-1) to produce a compound of the formula (XXVII) or a salt thereof:

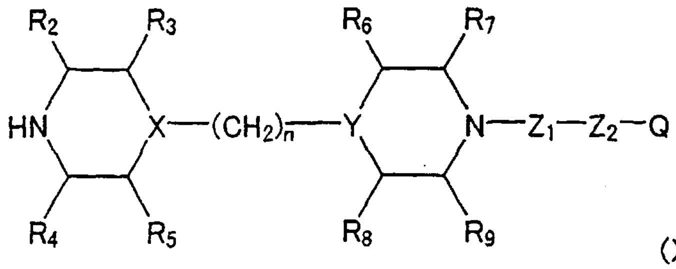

(XXVII)

(wherein Q, $R_2$ - $R_9$, X, Y, $Z_1$, $Z_2$ and n have the same meanings as defined above).

〈Step 4〉

The compound of the formula (XXVII) or a salt thereof that was obtained in 〈step 3〉 and a compound of the following formula (XXVIII) that is either commercially available or readily synthesizable from a commercial product by a documented method:

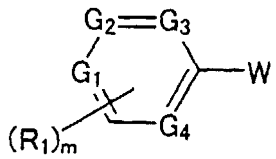

(XXX)

(wherein $G_1$ - $G_4$, $R_1$, W and m have the same meanings as defined above) are subjected to reaction in the presence or absence of a copper powder, copper oxide or an iron powder using an inorganic base such as potassium hydroxide, sodium hydroxide or potassium carbonate or an organic base such as triethylamine or diisopropylethylamine, preferably diisopropylethylamine, either in the absence of a solvent or in the presence of a suitable high-boiling point solvent such as DMF, DMSO, dimethoxyethane (DME), dibutyl ether, xylene, decalin, 1,2-dimethyl-2-imidazolidone (DMI) or ethoxyethanol, preferably using ethoxyethanol, at a temperature in the range between room temperature and the boiling point of the solvent used, preferably under reflux, for a sufficient time to proceed the reaction to an adequate extent, specifically for 1 - 12 hours.

Depending on the case, a metal such as copper, palladium, chromium or bismuth may be used to form a complex with the above-mentioned compound (XXVIII) so that the activity of the compound is enhanced before it is subjected to the above reaction.

By the above-described method, one can produce the compound of the formula (I) or a salt thereof: Formula (I)

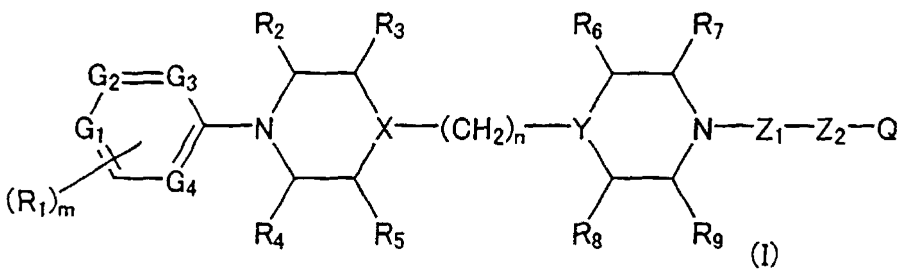

(I)

(wherein $G_1$ - $G_4$, Q, $R_1$ - $R_9$, X, Y, $Z_1$, $Z_2$, m and n have the same meanings as defined above).

(Production Method 3-2)

The class of compounds represented by the formula (I)-a or a salt thereof (X = C and Y = N in formula (I)) can also be produced by the following (Production Method 3-2):

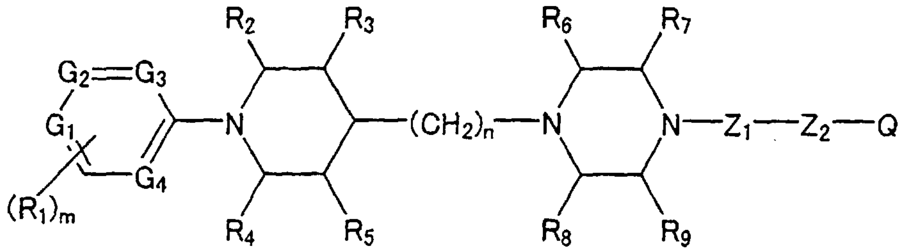

(I) - a

(wherein $G_1$ - $G_4$, Q, $R_1$ - $R_9$, $Z_1$, $Z_2$, m and n have the same meanings as defined above).

(Production Method 3-2)

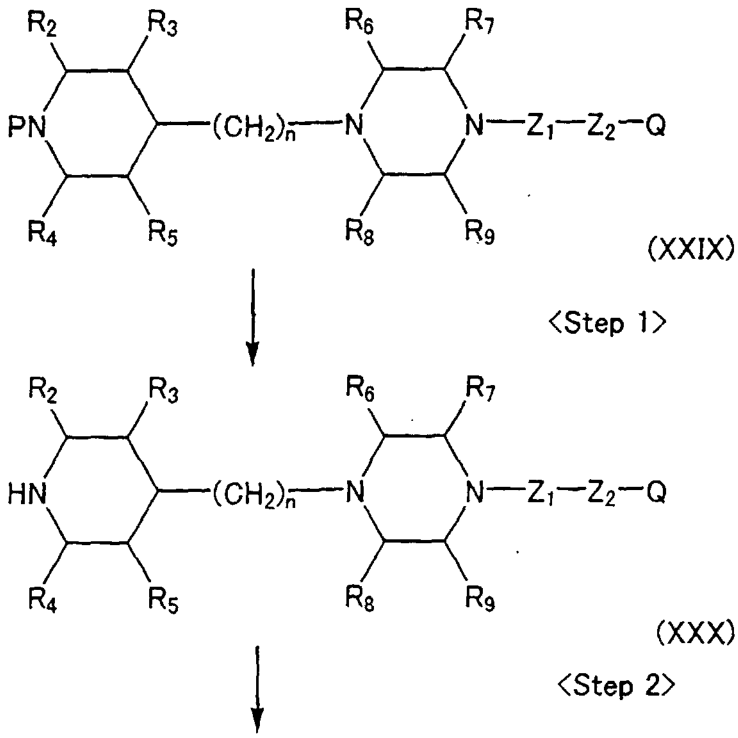

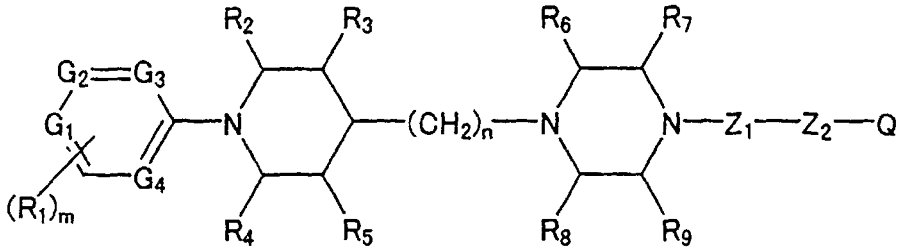

(I)-a

〈Step 1〉

The starting material, which is a compound of the formula (XXIX) or a salt thereof, can primarily be prepared by the methods described below. It should, however, be noted that the actual production methods vary with the types of substituents on the rings and the protective groups used and are by no means limited to the schemes set forth below.

In place of the compound of the formula (III) or a salt thereof that was used as the starting material in 〈Step 1〉of (Production Method 1-1), a compound of the formula (XXXI), either commercially available or readily derived from a commercial product, or a salt thereof:

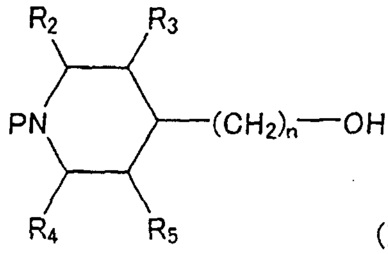

(XXXI)

(wherein P, $R_2$ - $R_5$ and n have the same meanings as defined above) may be used as a starting material and subjected to the same reactions as in 〈Step 1〉, 〈Step 2〉, 〈Step 3〉and 〈Step 4〉of (Production Method 1-1), thereby producing a compound of the formula (XXIX) or a salt thereof:

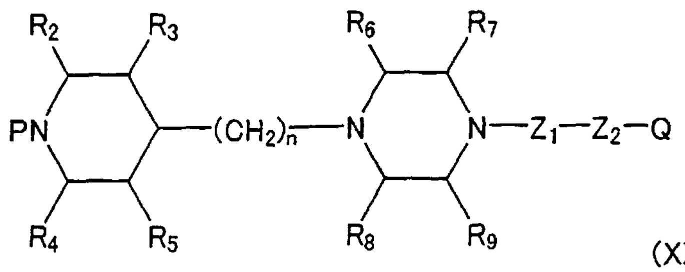

(XXIX)

(wherein P, Q, $R_2$ - $R_9$, $Z_1$, $Z_2$ and n have the same meanings as defined above).

Alternatively, in place of the compound of the formula (IV)-a or (IV)-b or a salt thereof that was used as the starting material in 〈Step 1〉of (Production Method 1-2), a compound of the formula (XXXII)-a or (XXXII)-b or a salt thereof:

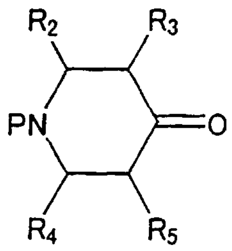

(XXXII) - a

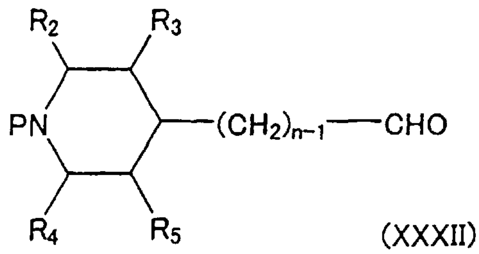

(XXXII) - b

(wherein P, $R_2$ - $R_5$ and n have the same meanings as defined above) that can readily be synthesized from a compound of the formula (XXXI) or a salt thereof by the same reaction as in 〈Step 1〉of (Production Method 1-1) may be used as a starting material and subjected to the same reactions as in 〈Step 1〉, 〈Step 2〉, 〈Step 3〉, 〈Step 4〉and 〈Step 5〉of (Production Method 1-2), thereby producing a compound of the formula (XXIX) or a salt thereof.

Alternatively, in place of the compound of the formula (IV)-a or (IV)-b or a salt thereof that was used as the starting material in 〈Step 4〉of (Production Method 1-3), a compound of the formula (XXXII)-a or (XXXII)-b set forth above or a salt thereof may be used and subjected to the same reaction as in 〈Step 4〉of (Production Method 1-3) to produce a compound of the formula (XXIX) or a salt thereof.

The compound of the formula (XXIX) or a salt thereof that has been prepared by one of the methods described above is subjected to the same reaction as in 〈Step 3〉of (Production Method 1-1) to produce a compound of the formula (XXX) or a salt thereof:

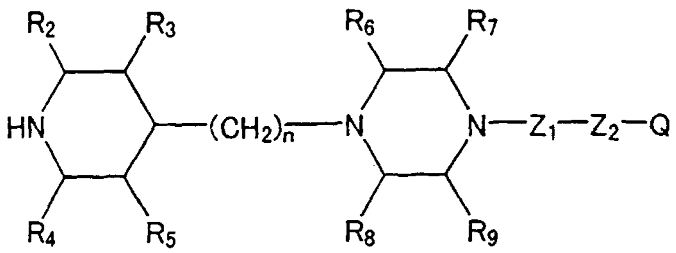

(XXX)

(wherein Q, $R_2$ - $R_9$ $Z_1$, $Z_2$ and n have the same meanings as defined above).

〈Step 2〉

The compound of the formula (XXX) or a salt thereof that was obtained in 〈Step 1〉is subjected to the same reaction as in 〈Step 4〉of (Production Method 3-1) to produce a compound of the formula (I)-a or a salt thereof:

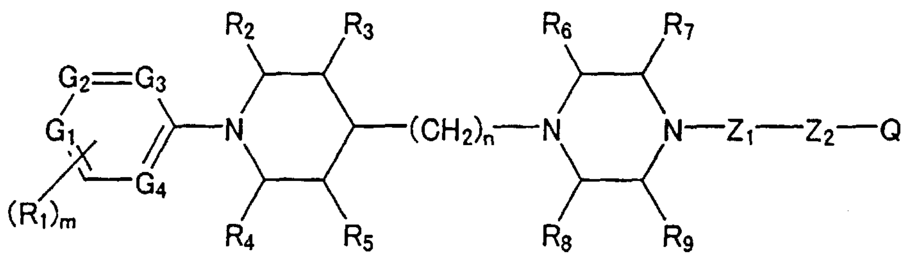

( I ) - a

(wherein $G_1$, $G_2$, $G_3$, $G_4$, Q, $R_1$, $R_2$, $R_3$, $R_4$, $R_5$, $R_6$, $R_7$, $R_8$, $R_9$, $Z_1$, $Z_2$, m and n have the same meanings as defined above).

(Production Method 3-3)

A compound of the formula (I)-b or a salt thereof ( X = N and Y = C in formula (I)) can also be produced by (Production Method 3-3):

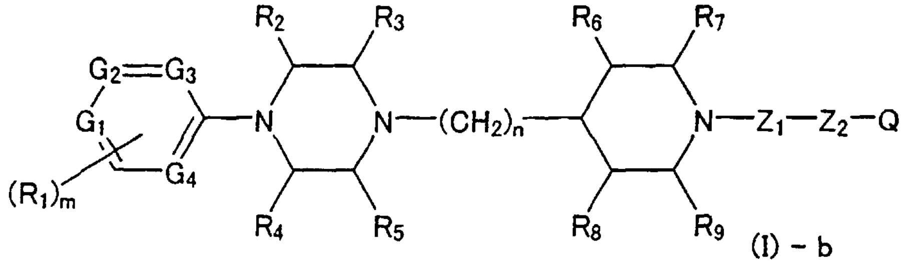

(I) - b

(wherein $G_1$ - $G_4$, Q, $R_1$ - $R_9$, $Z_1$, $Z_2$, m and n have the same meanings as defined above).

(Production Method 3-3)

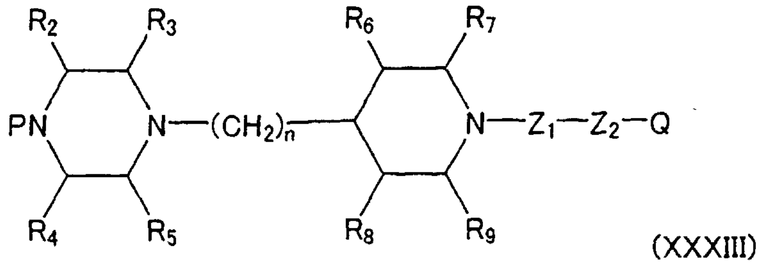

(XXXIII)

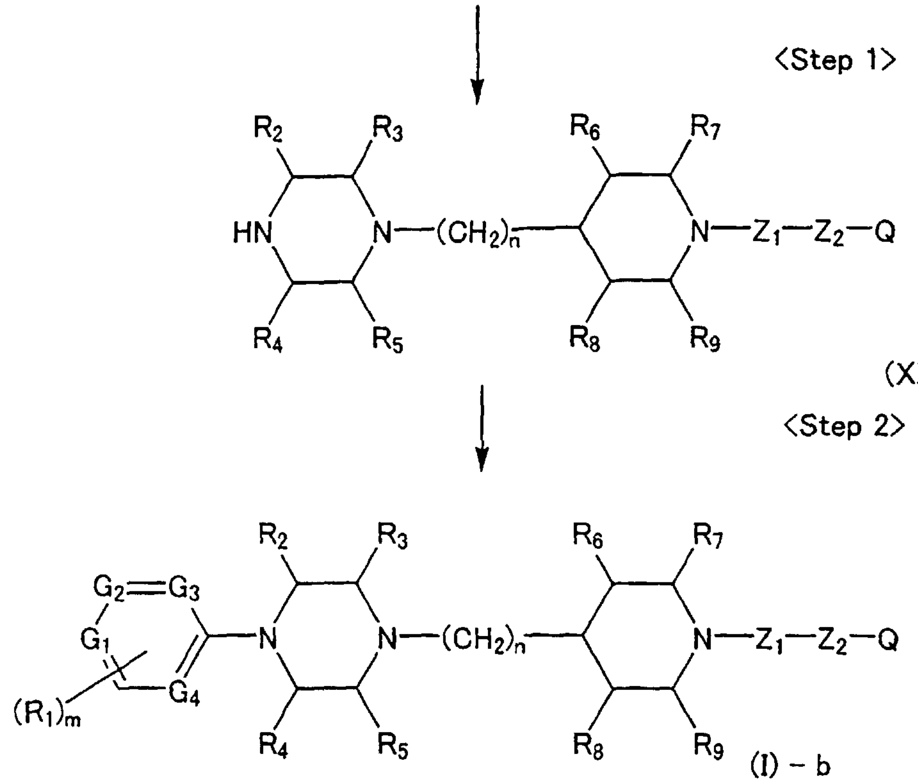


(XXXIV)

(I) - b

〈Step 1〉

The starting material, which is a compound of the formula (XXXIII) or a salt thereof, can primarily be prepared by the methods described below. It should, however, be noted that the actual production methods vary with the types of substituents on the rings and the protective groups used and are by no means limited to the schemes set forth below.

In place of the compound of the formula (XXII) or a salt thereof that was used as the starting material in 〈Step 3〉 of (Production Method 2-1), a compound of the formula (XXXV), either commercially available or readily derivable from a commercial product, or a salt thereof:

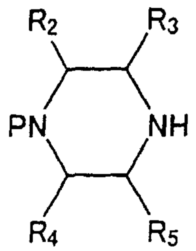

(XXXV)

(wherein P and $R_2$ - $R_5$ have the same meanings as defined above) may be used as a starting material and subjected to the same reaction as in 〈Step 3〉 of (Production Method 2-1), thereby producing a compound of the formula (XXXIII) or a salt thereof:

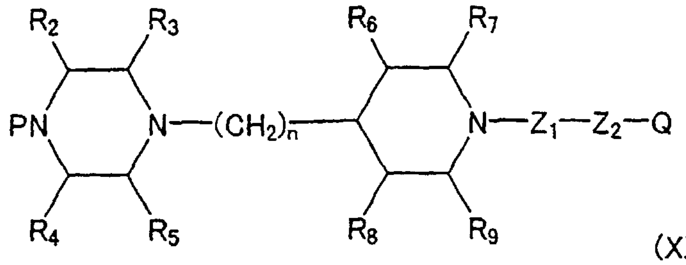

(XXXIII)

(wherein P, Q, $R_2$ - $R_9$, $Z_1$, $Z_2$ and n have the same meanings as defined above).

Alternatively, in place of the compound of the formula (XXII) or a salt thereof that was also used as the starting material in ⟨step 3⟩ of (Production Method 2-2), a compound of the formula (XXXV) or a salt that are either commercially available or readily derivable from a commercial product may be used and subjected to the same reaction as in ⟨step 3⟩ of (Production Method 2-2), thereby producing a compound of the formula (XXXIII) or a salt thereof.

The compound of the formula (XXXIII) or a salt thereof that has been prepared by one of the methods described above is subjected to the same reaction as in ⟨step 3⟩ of (Production Method 1-1) to produce a compound of the formula (XXXIV) or a salt thereof:

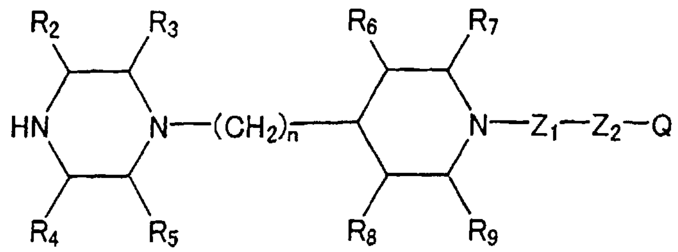

(XXXIV)

(wherein Q, $R_2$ - $R_9$, $Z_1$, $Z_2$ and n have the same meanings as defined above).

⟨Step 2⟩

The compound of the formula (XXXIV) or a salt thereof that was obtained in ⟨step 1⟩ is subjected to the same reaction as in ⟨step 4⟩ of (Production Method 3-1) to produce a compound of the formula (I)-b or a salt thereof:

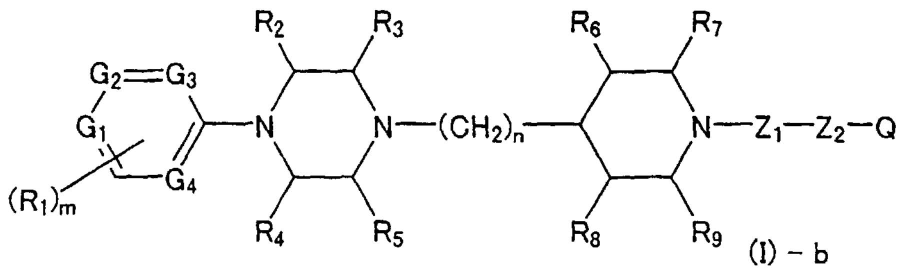

(I) - b

(wherein $G_1$ - $G_4$, Q, $R_1$ - $R_9$, $Z_1$, $Z_2$, m and n have the same meanings as defined above).

If some of the substituents in the compounds to be synthesized in the above-described ⟨Production Method 1⟩, ⟨Production Method 2⟩ and ⟨Production Method 3⟩ are reactive groups such as a hydroxyl group, an amino group, a carboxyl group and a thiol group, these groups may be protected as appropriate in each reaction step, with the protective groups being removed at suitable stages. Protective groups may be introduced and removed as appropriate for the types of the groups to be protected and the protective groups used; reference may be had to the Review section of "Protective Groups in Organic Synthesis", 2nd Edition, 1991.

We now describe the therapeutics, preventives and pharmaceutical compositions of the present invention.

The pharmaceutical compositions of the present invention may contain at least one compound of the general formula (I) (as already defined above) as an active ingredient and they may also contain a pharmaceutically acceptable carrier. The preferred examples of the compounds of the general formula (I) have been described above.

The compounds of the present invention possess a potent FXa inhibitory activity. Hence, the compositions of the present invention are a potent FXa inhibitor, more particularly a specific FXa inhibitor that does not inhibit other enzymes. The compositions are also an orally administrable FXa inhibitor, as well as an orally administrable specific FXa inhibitor. While there are many serine proteases, the activity of FXa is specifically inhibited by the compounds of the present invention and potently. They do not inhibit trypsin or chymotrypsin at all, nor do they inhibit thrombin which is another serine protease in the blood coagulation cascade. Hence, the compounds of the present invention solve the aforementioned problems with the conventional thrombin inhibitors, for example, the tendency to cause bleeding. As further advantages, the compounds of the present invention can be rapidly absorbed by the digestive tract after oral administration, their activity is not attenuated upon absorption, and they exhibit satisfactory characteristics in such aspects as absorption, distribution, metabolism and excretion. They therefore have high value of use as an oral drug.

The compositions containing the compounds of the present invention can be used as preventives and/or therapeutics of diseases for which the FXa inhibitor is indicated. The compositions containing the compounds of the present invention can also be used as an anticoagulant, and as preventives and/or therapeutics of diseases for which the anticoagulant is indicated.

In short, these drugs are effective in the prevention and/or treatment of diseases caused by thrombus or embolus. To mention specific examples of such diseases, they include: diseases from ischemic cerebrovascular disorders such as cerebral thrombosis, cerebral infarction, cerebral embolism, transient cerebral ischemic attacks (TIA) and cerebrovascular contractions after subarachnoid hemorrhage; diseases associated with ischemic heart diseases such as acute or chronic myocardial infarction, unstable angina pectoris and coronary thrombolysis; diseases from pulmonary infarction, pulmonary embolism and pulmonary angiopathy; and diseases from various cases of angiopathy including renal embolism, atherosclerotic plaque formation, peripheral arterial obstruction, peripheral venous obstruction, deep venous thrombosis, disseminated intravascular coagulation (DIC), thrombosis after artificial blood vessel or heart valve replacement, reocclusion and restenosis following coronary artery bypass surgery, reocclusion and restenosis on or after PTCA or after stent installation, and thrombosis on extracorporeal circulation of blood. Also to be mentioned are: the prevention of disorders in vascular endothelial cells of diabetic patients, hypercoagulation accompanied by transplantation, or resistance of activated protein C (APC); excessive coagulation accompanying vascular diseases, postoperative trauma, obesity, pregnancy, use of oral contraceptives, prolonged depression of movement or cancer; and gestosis. The drugs find particular use in the prevention of embolism, preferably the onset of cerebral embolism, that accompanies atrial fibrillation, heart valve replacement or valvular heart disease, the prevention of transient cerebral ischemic attacks, especially their recurrence, and in the prevention and treatment of deep venous thrombosis or DIC.

If the drugs of the present invention are to be used as pharmaceuticals, administering them for the purpose of preventing the above-mentioned diseases is recommended and particularly important. The drugs of the present invention are not direct acting thrombolytic agents nor are they direct platelet agglutination inhibiting agents. Hence, they are preferably administered for preventive purposes to patients predisposed to thrombus formation or patients having the risk factor of thrombosis and embolism. In particular, patients who have atrial fibrillation, patients who underwent heart valve replacement and patients suffering from valvular heart disease have the high risk of thrombus formation in the lesions or the area of transplantation, which often triggers the development of cerebral infarction and the occurrence of fatal attacks is by no means rare. The drugs of the present invention are expected to prove extremely useful in preventing the induced thrombus or embolus formation in such patients, most preferably for preventing the onset of cerebral infarction.

Therapy on the above-mentioned conditions is performed over a prolonged period. The drugs of the present invention can be administered perorally, have less side effects such as bleeding, need no frequent monitoring and hence can be used safely for a prolonged time.

In other words, the drugs of the present invention are preventives and/or therapeutics of embolus that accompanies atrial fibrillation, heart valve replacement or valvular heart disease. Preferably, they are preventives of the onset of cerebral embolism that accompanies these events. They are also preventives and/or therapeutics of transient cerebral ischemic attacks. In particular, they are preventives of the recurrence of the disease. They are also preventives and/or therapeutics of deep venous thrombosis or DIC.

Depending on the substituent $R_6$ ($R_{6a}$ or $R_{6b}$), some of the compounds of the present invention will easily undergo metabolism during drug absorption and distribution. Some of the resulting metabolites are also included in the formula (I) of the compounds of the present invention and possess potent FXa inhibitory activity, thus giving very interesting findings from pharmacological/pharmacokinetic viewpoints.

The compositions containing the compounds of the present invention as an active ingredient are also effective as veterinary drugs and have high value of use. They are also useful in the measurement of various functional

parameters in blood coagulation or as reagents in laboratories.

Since the compounds of the present invention have FXa inhibitory action, the compositions containing them can also be used as a preventive or therapeutic of the infection with influenza virus due to their activity in inhibiting the growth of the virus.

Brief Description of the Drawings

Figs. 1 - 39 tabulate the structural formulae of 446 compounds of the present invention; shown in the column $S_1$ are exemplary 6-membered rings together with the substituent $R_1$; shown in the column $S_2$ are exemplary structures of the bridge which, either directly or via an alkylene group, connects two rings comprising combinations of piperazine or piperidine rings; shown in the column $S_3$ are examples of the formula $-Z_1-Z_2-Q$.

Figs. 40 - 50 are tables showing NMR data on the compounds synthesized in the Examples of the present invention.

Best Mode for Carrying Out the Invention

While experimental runs and examples of the present invention are described below, it should be understood that they are by no means intended to limit the scope of the present invention.

The outstanding FXa inhibitory activity of the compounds of the present invention can be verified by the following tests.

1) Measurement of Enzyme Inhibitory Action

a) Measuring human FXa inhibitory action

*In vitro* FXa inhibitory activity is measured in accordance with the method of Kettner et al., J. Biol. Chem., 1990, 265, 18289-18297. Human FXa (Enzyme Research Laboratories, Inc., 0.019 U/ml) is mixed with a test compound diluted with dimethyl sulfoxide (DMSO) at various concentrations and a synthesis substrate S-2222 (Chromogenix AB, 0.4 mM) and incubated in a Tris-HCl buffer (pH 7.5) at 37 °C while the absorbance at 405 nm is measured continuously. To calculate the FXa inhibitory activity of the test cmpound, the initial reaction velocity is compared with the value for a control containing no test compound. The FXa inhibitory activity of a test compound is usually expressed as $IC_{50}$.

The compounds of the present invention were measured for their FXa inhibitory activity by the above-described method and they had potencies between 1 nM and 1μm in terms of $IC_{50}$. The data for specific examples are shown in Table 1. The control in the assay system was 1-((E)-4-chlorostyrylsulfonyl)-4-[1-(4-pyridyl)piperidin-4-ylcarbonyl]piperazine (the compound synthesized in Example 39-2b of WO96/10022) and it had an $IC_{50}$ of 0.15 μM.

Table 1

| Test Compound | $IC_{50}$ (μM) |
|---|---|
| Example 39 | 0.019 |
| Example 40 | 0.0074 |
| Example 46 | 0.017 |
| Example 47 | 0.013 |
| Example 52 | 0.012 |
| Example 54 | 0.0085 |
| Example 55 | 0.0074 |
| Example 60 | 0.016 |
| Example 61 | 0.070 |
| Control | 0.15 |

The compounds synthesized in the Examples of the present invention had FXa inhibitory activities at least equal to that of the control compound.

2) Measurement of Anticoagulation Activity (*in vitro*)

a) Measuring extrinsic coagulation time

Thromboplastin time (PT) is measured in the presence of test compounds diluted at various concentrations. A test compound diluted with DMSO at various concentrations is mixed with rat plasma and incubated at 37°C for 3 minutes. Then, a thromboplastin reagent is added and the coagulation time is measured. The anticoagulation activity of the test compound is indicated in terms of the concentration required to double the coagulation time for the case where no test compound is added. In the actual test, the compounds of the present invention were found to be effective in extending the PT. The effectiveness of selected compounds of the present invention is shown in Table 2.

Table 2

| Test Compound | PT Doubling Concentration, μM |
|---|---|
| Example 39 | 2.8 |
| Example 40 | 2.7 |

3) Characteristics of Anticoagulation Activity (*ex vivo*)

a) *Ex vivo* measurement of coagulation time in rats (i.v.)

Male Wistar rats (200 - 300 g; SLC, Inc.) that have been starved for more than 12 hours are administered through a femoral vein with a single dose of a drug (3 - 30 mg/kg) dissolved in physiological saline (or 10% DMSO solution) and blood is collected at given time intervals in a tenth volume of 3.8% sodium citrate and centrifuged at 3000 rpm for 10 minutes to separate plasma, which is used in the following measurement of Prothrombin Time (PT).

A 50-μl portion of the plasma is incubated at 37°C for 3 minutes and a thromboplastin solution (100 μl) is added to start coagulation. The coagulation time is measured. In the actual test, the intravenously administered compounds of the present invention were found to be effective in extending the PT on account of enzyme inhibition.

b) *Ex vivo* measurement of coagulation time in rats (p.o.)

The test a) was repeated, except that the administration of a single dose through a femoral vein was replaced by forced peroral administration via an oral probe. At given time intervals, blood was collected in a tenth volume of 3.8% sodium citrate. As in the test a), extrinsic coagulation time and intrinsic coagulation time were measured.

In this test b), the compounds of the present invention were found to be effective in extending the coagulation time upon oral administration of 10 - 100 mg/kg.

In none of the *ex vivo* tests with rats was found any safety problem.

All that is required for the pharmaceutical compositions of the present invention is that they contain at least one of the compounds of the general formula (I) (as already defined above) or salts thereof as an active ingredient. They may also contain any pharmaceutically acceptable carriers. The preferred examples of the compounds of the general formula (I) have already been mentioned.

As described above, the compounds of the present invention show a potent FXa activity inhibitory action and they have high specificity since they do not inhibit trypsin, chymotrypsin or thrombin. They exhibit antithrombotic action in rats if they are administered perorally at a dose of 0.3 - 3 mg/kg or administered intravenously at a dose of 0.01 - 1 mg/kg. On the other hand, the compounds of the present invention in no way extend the bleeding time of rats even if they are administered perorally at a dose of 3 mg/kg or administered intravenously at a dose of 1 mg/kg. Therefore, unlike the known anticoagulants such as heparin and warfarin, the compounds of the present invention exhibit the intended anticoagulation action without the risk of showing bleeding tendency. As a further advantage, the compounds of the present invention have good absorbability upon oral administration, their action lasts for a reasonable time and high safety is assured.

To prevent or treat the various diseases mentioned hereinabove, the compounds of the present invention may be administered either individually or in combination with other pharmacologically active ingredients.

Exemplary pharmacologically active ingredients include: known anticoagulating agents [e.g. tissue plasminogen activator (t-PA) and its derivatives (inclusive of modified products and derivatives of the so-called "second generation"), urokinase and streptokinase]; known anticoagulants (e.g. warfarin, heparin and thrombomodulin); known platelet agglutination inhibitors (e.g. aspirin, thromboxane antagonist, thromboxane synthesis inhibitor and GPIIb/IIIa inhibitor); known therapeutics of hyperlipidemia (e.g. clofibrate and related drugs, HMG-CoA inhibitor and EPA-E); and known antihypertensives (e.g. nifedipine and diltiazem). The term "combination" as used herein covers not only the administration of a combination drug containing both the compound of the present invention and another pharmacologically active ingredient but also the case where the two are in separate dosage forms and administered either at a time or at different times. The mode of administration is in no way limited as long as the compound of the present invention and another pharmacologically active ingredient exist simultaneously in the patient's blood.

A pharmaceutical composition containing one or more of the compounds of the present invention and pharmaceutically acceptable salts thereof as an active ingredient may be formulated as capsules, pills, tablets, granules, subtilized granules and powders, drugs for internal application such as suspensions, emulsions, limonades, elixirs and syrups, as well as injections, nasal inhalants, suppositories, ointments and plasters using common pharmaceutical carriers, vehicles and other additives and thereafter applied to man and other animals either perorally or parenterally.

The clinical dose at which the compounds of the present invention are to be administrated to humans is determined as appropriate in consideration of various factors such as the symptoms of the patient to be treated, his or her body weight, age and sex; the usual daily dose for an adult ranges from 0.1 mg to 1000 mg, preferably from 1 mg to 300 mg (for oral administration), and from 0.01 mg to 300 mg, preferably from 0.1 mg to 100 mg (for parenteral administration), which is administrated either at a time or in divided portions. The dose is variable under actual conditions and smaller doses may sometimes suffice.

The solid compositions to be administered perorally according to the present invention may be formulated as capsules, pills, tablets, powders, granules, etc. In these solid compositions, one or more active ingredients are combined with at least one inert carrier. Specific examples of inert carriers include vehicles (e.g. lactose, sucrose, mannitol, glucose, hyroxypropyl cellulose, microcrystalline cellulose and metasilicates), binders (e.g. crystalline cellulose, saccharides, dextrin, hydroxypropyl cellulose, hydroxypropylmethyl cellulose, polyvinyl pyrrolidone and macrogol), lubricants (e.g. magnesium stearate, calcium stearate and talc), disintegrators (e.g. corn starch, carboxymethyl cellulose and cellulosic calcium glycolate), stabilizers (e.g. sugar alcohols and saccharides such as lactose), solubilizers or solvent promoters (e.g. cholesterol, triethanolamine, glutamic acid and aspartic acid), coloring agents, flavoring agents, antiseptics, isotonization agents, dispersing agents, antioxidants (e.g. ascorbic acid and butyl hydroxyanisole), buffers, and preservatives (e.g. paraben and benzyl alcohol). If necessary, tablets, pills, granules and the like may have gastric or enteric film coatings formed of sucrose, gelatin, hydroxyproopylmethyl cellulose phthalate, etc.

Injections for parenteral administration include sterilized aqueous or non-aqueous solutions, suspensions and emulsions. Carriers for aqueous solutions and suspensions include, for example, distilled water for injection and physiological saline. Carriers for non-aqueous solutions and suspensions include, for example, propylene glycol, polyethylene glycol, vegetable oils such as olive oil, alcohols such as ethyl alcohol, and polysorbate 80 (TM). These compositions may further contain the additives exemplified above, for example, isotonization agents, antiseptics, moistening agents, emulsifiers, dispersing agents, stabilizers, solubilizers and solvent promoters. These compositions are sterilized by suitable techniques such as passage through a membrane filter, incorporation of sterilizers and irradiation with uv light. Sterile solid compositions may also be prepared and formulated as an injection that is dissolved, emulsified or suspended just prior to use. If the compounds of the present invention have low solubility, they may be subjected to a solubilizing treatment. Several solubilizing methods are known to be applicable to pharmaceutical preparations and they include the addition of surfactants (e.g. polyoxyethylene hydrogenated castor oils, higher aliphatic acid esters of polyoxyethylene sorbitan and aliphatic acid esters of sucrose), and the formation of solid dispersions from the drug and solubilizers such as high-molecular weight compounds (e.g. water-soluble polymers such as polyethylene glycol (PEG), hydroxypropylmethyl cellulose (HPMC) and polyvinyl pyrrolidone (PVP), and enteric polymers such as hydroxypropylmethyl cellulose phthalate (HPMCP) and methyl methacrylate-methacrylic acid copolymer (Eudragit L, S (TM); Rohm and Haas). If necessary, inclusion compounds may be formed using α-, β- or γ-cyclodextrin or hydroxypropyl cyclodextrin. These solubilizing methods may be modified as appropriate for the intended drug by having reference to literature such as "Yakugaku Monogurafu No. 1, Seibutsukagaku Riyono (Series of Monographs on Pharmacy, No. 1, Biochemical Availability)", Koji Nagai et al., Soft Science, 78-82 (1988) and "Saikin no Seizaigijutsu to Sono Oyo (Recent Advances in Pharmaceutical Formulation Technology and Its Applications)", Isamu Utsumi, Iyaku Journal, 157-159 (1983). Among the methods mentioned above, the formation of a solid dispersion from the drug and a solubilizer to enhance its solubility is recommended Japanese

Patent Application Laid-Opened No. 49314/1981 and FR 2460667].

⟨Pharmaceutical Preparations⟩

The following are representative examples of the pharmaceutical compositions of the present invention. In the description, Compound M means the compound of the present invention which is represented by the formula (I) or a pharmaceutically acceptable salt thereof; specifically, it is any one of the compounds synthesized in the Examples that follow.

(a) Capsule (50 mg)

| | |
|---|---|
| Compound M | 100 g |
| Lactose | 398.5 g |
| Magnesium stearate | 1.5 g |

Weighed amounts of the above ingredients were mixed uniformly and the resulting powder mixture was filled in 250-mg portions into hard capsules of JP No. 1.

(b) Tablet (1 mg)

| | |
|---|---|
| Compound M | 1.0 g |
| Lactose | 92.2 g |
| Carboxymethylcellulose sodium | 5.0 g |
| Corn starch paste (5% W/V paste) | 0.8 g |
| Magnesium stearate | 1.0 g |

Weighed amounts of the above ingredients were compressed into tablets in the usual manner, each weighing 100 mg.

(c) Tablet (10 mg)

| | |
|---|---|
| Compound M | 10 g |
| Lactose | 160 g |
| Croscarmellose sodium | 4.0 g |
| Corn starch | 20.7 g |
| Polyvinyl pyrrolidone | 2.3 g |
| Magnesium stearate | 3 g |

Weighed amounts of the above ingredients were compressed into tablets in the usual manner, each weighing 200 mg. The tablets were then enteric-coated with cellulose acetate phthalate.

(d) Tablet (100 mg)

| | |
|---|---|
| Compound M | 100 g |
| Lactose | 181.5 g |
| Croscarmellose sodium | 12 g |
| Corn starch (5% W/V paste) | 3.5 g |
| Magnesium stearate | 3 g |

Weighed amounts of the above ingredients were compressed into tablets in the usual manner, each weighing 300 mg.

(e) Injection (0.1 mg/ml)

| | |
|---|---|
| Compound M | 0.1 W/V |
| Sodium phosphate buffered solution | 2.3 % W/V |
| Citric acid | 0.4% |
| Macrogol 400 | 3.5% |
| Water for injection | q.s. to make 100% |

The above ingredients were mixed into solution and 1-ml portions of the solution were filled into injection ampules to make injections.

(f) Injection (1.0 mg/ml)

| | |
|---|---|
| Compound M | 1.0% W/V |
| Sodium phosphate buffered solution | 3.6% W/V |
| 1 M Sodium hydroxide solution | 15% W/V |
| Water for injection | q.s. to make 100% |

The above ingredients were mixed into solution and 1-ml portions of the solution were filled into injection ampules to make injections.

Examples

The following examples are provided for the purpose of further illustrating the present invention but are in no way to be taken as limiting.

Nuclear magnetic resonance (NMR) spectra were taken with JEOL JNM-EX270 FT-NMR (JEOL LTD.) or JEOL JNM-LA300 FT-NMR (JEOL LTD.; data marked with an asterisk); high-resolution mass spectra (HRMS) were taken with JEOL JMS-GCMATE (JEOL LTD.); and high-performance liquid chromatography (HPLC) was performed with SHIMADZU LC-10A (Shimadzu Corp.)

Example 1 Synthesis of 1-[1-((E)-4-chlorostyrylsulfonyl)-piperidin-4-yl]-4-(4-pyridyl)piperazine

〈Step 1〉Synthesis of 4-[1-((E)-4-chlorostyrylsulfonyl)]piperidone

Triethylamine (0.7 ml) and a solution in anhydrous methylene chloride (10 ml) of (E)-4-chlorostyrylsulfonyl chloride (1.1 g) prepared by a documented (WO96/10022) method were added to a solution of 4-piperidone monohydrate hydrochloride (0.5 g) in anhydrous methylene chloride (50 ml) and the mixture was stirred overnight in an argon atmosphere at room temperature. Water was added to the reaction mixture and subjected to extraction with methylene chloride. The methylene chloride layer was washed with water and saturated sodium chloride, dried over anhydrous sodium sulfate and the solvent was evaporated under reduced pressure. The residue was purified by silica gel column chromatography (eluent; methylene chloride) to obtain the titled compound (500 mg).

NMR spectrum (*DMSO-$d_6$) δ ppm:7. 83~7. 77 (2 H, m) , 7. 55~7. 38 (4H, m) , 3. 49 (4H, t, J=6 Hz) , 2. 47 (4H, t, J=6Hz)

〈Step 2〉Synthesis of 1-[1-((E)-4-chlorostyrylsulfonyl)piperidin-4-yl]-4-(4-pyridyl)piperazine

Acetic acid (0.18 ml) was added to a solution in anhydrous methylene chloride (12 ml) of a portion (490 mg) of the compound obtained in step 1 and 1-(4-pyridyl)piperazine (254 mg) prepared by a documented Japanese Patent Application Laid-Opened No. 192225/1994) method and the mixture was stirred for 30 minutes in an argon atmosphere at room temperature. To the stirred mixture, sodium triacetoxyborohydride (660 mg) was added and the mixture was stirred overnight at room temperature. Water was added to the reaction mixture, which was adjusted to alkaline with 1 N sodium hydroxide solution and extracted with methylene chloride. The methylene chloride layer was washed with water and saturated sodium chloride, dried over anhydrous sodium sulfate and the solvent was evaporated under reduced pressure. The residue was purified by silica gel column chromatography (eluting solvents; methylene chloride:methanol = 95:5 - 90:10) to give the titled compound (290 mg).

HRMS: $C_{22}H_{27}ClN_4O_2S(M^+)$ Cal'd 446.1543 Found 446.1555

Example 2 Synthesis of 1-[1-((E)-4-chlorostyrylsulfonyl)piperidin-4-ylmethyl]-4-(4-pyridyl)piperazine

〈Step 1〉Synthesis of 1-[(E)-4-chlorostyrylsulfonyl)]piperidin-4-yl methanol

Triethylamine (0.88 ml) and (E)-4-chlorostyrylsulfonyl chloride (1.03 g) were added to a solution in anhydrous methylene chloride (10 ml) of piperidin-4-yl methanol (0.5 g) prepared by a documented (J. Med. Chem., 34, 1073, 1991) method and the mixture was stirred for 4 hours in an argon atmosphere at room temperature. Water was added to the reaction mixture, which was then subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1 - 97:3) to give the titled compound (180 mg).

NMR spectrum (*DMSO-$d_6$ ) δ ppm: 7. 80 (2H, d, J=9Hz), 7. 52 (2H, d, J=9Hz) , 7. 38 (1H, d, J=16Hz) , 7. 33 (1H, d, J=16Hz) , 4. 54 (1H, t, J=5Hz) , 3. 63~3. 50 (2H, m) , 3. 28 ~3. 21 (2H, m) , 2. 70~2. 56 (2H, m) , 1. 80~1. 35 (3H, m), 1. 23 ~1. 07 (2H, m)

〈Step 2〉Synthesis of 1-[1-((E)-4-chlorostyrylsulfonyl)piperidin-4-ylmethyl]-4-(4- pyridyl)piperazine

A solution of oxalyl chloride (0.05 ml) in anhydrous methylene chloride (1.33 ml) was cooled to -78°C in an argon atmosphere. To the cooled solution, a solution of anhydrous dimethyl sulfoxide (0.09 ml) in anhydrous methylene chloride (1.33 ml) was added dropwise over 20 minutes. Then, a solution in anhydrous methylene chloride (1.33 ml) of a portion (150 mg) of the compound obtained in step 1 was added dropwise over 20 minutes. After stirring for 1 hour at between -65°C and -60°C, the mixture was cooled to -78°C and triethylamine (0.25 ml) was added. The reaction mixture was stood at room temperature, water was added and was extracted with methylene chloride was conducted. The methylene chloride layer was washed with water and saturated sodium chloride, dried with anhydrous sodium sulfate and the solvent was distilled off under reduced pressure. The resulting residue was suspended in anhydrous methylene chloride (2.0 ml) and 1-(4-pyridyl)piperazine (42 mg) and acetic acid (0.02 ml) were added in that order. The resulting mixture was stirred at room temperature for 30 minutes in an argon atmosphere, sodium triacetoxyborohydride (0.10 g) was added and the mixture was stirred overnight at room temperature. Water was added to the reaction mixture, which

was adjusted to pH 9 with 1 N sodium hydroxide solution and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 95:5) to yield the titled compound (60 mg).

Example 3 Synthesis of 1-[(E)-4-chlorostyrylsulfonyl]-4-[1-(4-pyridyl)piperidin-4-ylmethyl]piperazine

〈Step 1〉Synthesis of 4-[1-(4-pyridyl)piperidine]carbaldehyde

A solution of oxalyl chloride (1.77 ml) in anhydrous methylene chloride (85 ml) was cooled to -78°C in an argon atmosphere. To the cooled solution, a solution of anhydrous dimethyl sulfoxide (3.25 ml) in anhydrous methylene chloride (85 ml) was added dropwise over 20 minutes. Then, a solution in anhydrous methylene chloride (48 ml) and anhydrous dimethyl sulfoxide (48 ml) of 1-(4-pyridyl)piperidin-4-yl methanol (3.0 g) prepared by a documented (EP 0359389) method was added dropwise over 20 minutes. After stirring at between -65°C and -60°C for 1 hour, the mixture was cooled to -78°C and triethylamine (8.31 ml) was added. The reaction mixture was stood at room temperature, water was added and was extracted with methylene chloride was conducted. The methylene chloride layer was washed with water and saturated sodium chloride, dried with anhydrous sodium sulfate and the solvent was distilled off under reduced pressure. The resulting aldehyde was rather labile and should preferably be used in the next reaction without purification. It can, however, be analyzed with reasonable accuracy. The above-described treatment was done quickly and the concentrated residue was dissolved in $CDCl_3$ and subjected to NMR measurement; a signal for the proton of aldehyde was identified but disappeared with time.

EIMS: 190 ($M^+$)

NMR spectrum (*$CDCl_3$ ) δ ppm:9 . 56 (1H, s), 8. 16~7. 99 (2H, m) , 6. 82~6. 69 (2H, m) , 3. 83~3. 71 (2H, m) , 3. 02~ 2. 90 (2H, m), 2. 61~2. 45 (1H, m) , 1. 90~1. 78 (2H, m), 1. 52~1. 36 (2H, m)

〈Step 2〉Synthesis of 1-t-butoxycarbonyl-4-[1-(4-pyridyl)piperidin-4-ylmethyl]piperazine

The product of step 1 was suspended in anhydrous methylene chloride (65 ml); to the suspension, 1-t-butoxycarbonyl piperazine (3.19 g) and acetic acid (1.55 ml) were added in that order. After stirring the mixture at room temperature for 30 minutes in an argon atmosphere, sodium triacetoxyborohydride (6.61 g) was added and the mixture was stirred overnight at room temperature. Water was added to the reaction mixture, which was adjusted to pH 9 with 1 N sodium hydroxide solution and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1) to give the titled compound (0.59 g).

HRMS: $C_{20}H_{32}N_4O_2$ ($M^+$) Cal'd 360.2525 Found 360.2545
NMR spectrum (*$CDCl_3$ ) δ ppm: 8. 26~8. 20 (2H, m), 6. 68 ~6. 62 (2H, m) . 3. 92~3. 82 (2H, m) , 3. 46~3. 37 (4H, m). 2. 90~2. 77 (2H, m), 2. 40~2. 31 (4H, m) , 2. 20 (2H, d, J=7 Hz), 1. 90~1. 68 (3H, m) , 1. 46 (9H, s) , 1. 30~1. 18 (2H, m)

〈Step 3〉Synthesis of 1-[1-(4-pyridyl)piperidin-4-ylmethyl]piperazine trifluoroacetate

Anisole (2.23 ml) and trifluoroacetic acid (17.2 ml) were added to the compound (3.21 g) obtained in step 2 under cooling with ice bath and the mixture was stirred overnight at room temperature in an argon atmosphere. Ether (200 ml) was added to the reaction mixture and stirred vigorously. After standing, the supernatant was removed by decantation and another 200 ml portion of ether was added; this procedure was repeated. Ether (200 ml) was added to the residue and the mixture was divided into adequately fine particles, which were filtered to give the titled compound (3.24 g).

HRMS: $C_{15}H_{24}N_4$ ($M^+$) Cal'd 260.2001 Found 260.2003
NMR spectrum (*DMSO-$d_6$ ) δ ppm: 8. 30~8. 18 (2H, m) , 7. 25~7. 17 (2H, m) , 4. 29~4. 18 (2H, m) , 4. 00~3. 35 (4H, m) , 3. 35~3. 09 (6H, m) , 2. 50~2. 40 (2H, m) , 2. 12~1. 98 (1H, m) , 1. 93~1. 82 (2H, m) , 1. 23~1. 06 (2H, m)

〈Step 4〉Synthesis of 1-[(E)-4-chlorostyrylsulfonyl]-4-[1-(4-pyridyl)piperidin-4-ylmethyl]piperazine

Triethylamine (0.091 ml) and (E)-4-chlorostyrylsulfonyl chloride (15.6 mg) were added to a suspension in anhydrous methylene chloride (1.0 ml) of a portion (47 mg) of the compound obtained in step 3. The resulting mixture was stirred overnight at room temperature in an argon atmosphere. Water was added to the reaction mixture, rendered alkaline with 1 N sodium hydroxide and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 95:5 - 90:10) to yield the titled compound (13 mg).

HRMS: $C_{23}H_{29}ClN_4O_2S$ ($M^+$) Cal'd 460.1699 Found 460.1709

In subsequent Examples 4 - 21, a commercial grade of sulfonyl chloride or sulfonyl chloride obtained by chlorinating a commercial grade of sulfonic acid was used and the method of 〈step 4〉in Example 3 was repeated to synthesize the end compounds.

Example 22 Synthesis of 4-(5-aminonaphthalen-2-ylsulfonyl)-1-[1-(4-pyridyl piperidin-4-ylmethyl]piperazine

A portion (50 mg) of the compound obtained in Example 21 was suspended in 1 N HCl (2 ml) and heated under reflux for 1.5 hours. The reaction mixture was subjected to extraction with methylene chloride and the methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 90:10) to yield the titled compound (26 mg).

Example 23 Synthesis of 4-[(E)-4-chlorostyrylsulfonyl]-1-[1-(4-pyridyl)piperidin-4-ylmethyl]piperazin-2-one

〈Step 1〉Synthesis of 4-[N-(2-(t-butoxycarbonylamino)-ethyl)aminomethyl]-1-(4-pyridyl)piperidine borane complex

A crude product obtained from 1-(4-pyridyl)piperidin-4-yl methanol (3.0 g) by the method of 〈step 1〉in Example 3 was suspended in anhydrous methylene chloride (13 ml). To the suspension, N-t-butoxycarbonyl-1,2-ethylenediamine (0.54 g) and acetic acid (0.31 ml) were added in that order. After stirring the resulting mixture at room temperature for 30 minutes in an argon atmosphere, sodium triacetoxyborohydride (1.33 g) was added and the mixture was stirred overnight at room temperature. Water was added to the reaction mixture, adjusted to pH 9 with 1 N sodium hydroxide solution, and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1) to give the titled compound (0.62 g).

NMR spectrum ($CDCl_3$ ) δ ppm: 8. 22~8. 14 (2H, m) , 6. 73~ 6. 64 (2H, m) , 4. 93~4. 78 (1H, br) , 4. 20~3. 92 (2H, m). 3. 29~3. 12 (2H, m) , 3. 12~2. 98 (2H, m) , 2. 73 (2H, t, J=6 Hz) , 2. 54 (2H, d, 1=7Hz) , 2. 03 (6H, s) 1. 99~1. 72 (3H, m) , 1. 45 (9H, s) 1. 33~1. 15 (2H, m)

〈Step 2〉Synthesis of 4-[N-bromoacetyl-N-[2-(t-butoxycarbonylamino)ethyl]aminomethyl]-1-(4- pyridyl)piperidine borane complex

Triethylamine (0.28 ml) was added to a solution in anhydrous methylene chloride (5 ml) of a portion (0.6 g) of the compound obtained in step 1. To the mixture, a solution of bromoacetyl chloride (0.31 g) in anhydrous methylene chloride (5 ml) was added dropwise under cooling with ice water and the mixture was stirred at room temperature for 2 hours in an argon atmosphere. Ice water was added to the reaction mixture and extraction with methylene chloride was conducted. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1) to give the titled compound (0.61 g).

NMR spectrum (*$CDCl_3$ ) δ ppm: 8. 26~8. 15 (2H, m) , 6. 76 ~6. 65 (2H, m) , 4. 95~4. 73 (1H, m) , 4. 18~3. 34 (4H, m), 3. 56~3. 45 (2H, m), 3. 37~3. 21 (4H, m) , 3. 13~2. 99 (2H, m) , 2. 25~2. 10 (1H, m) , 2. 04 (6H, s) , 1. 92~1. 77 (2H, m) , 1. 44 (9H, s) , 1. 39~1. 19 (2H, m)

〈Step 3〉Synthesis of 4-[N-(2-aminoethyl)-N-bromoacetylaminomethyl]-1-(4-pyridyl)piperidine trifluoroacetate

To a portion (0.54 g) of the compound obtained in step 2, anisole (0.29 g) and trifluoroacetic acid (2.3 ml) were added under cooling with ice bath and the mixture was stirred at room temperature for 1.5 hours in an argon atmosphere. Ether (50 ml) was added to the reaction mixture and stirred vigorously. After standing, the supernatant was removed by decantation and another 50 ml of ether was added; this procedure was repeated. Ether (50 ml) was added to the residue and the mixture was divided into adequately fine particles, which were filtered to give the titled compound (0.58 g).

NMR spectrum (*DMSO-$d_6$ ) δ ppm: 8. 26~8. 16 (2H, m) , 7. 23-7. 13 (2H, m) , 4. 55-4. 35 (2H, m) , 4. 34~4. 18 (2H, m) , 3. 60~3. 45 (2H, m) , 3. 29~2. 87 (6H, m) , 2. 15~1. 96 (1H, m) , 1. 78~1. 63 (2H, m) , 1. 32~1. 04 (2H, m)

〈Step 4〉Synthesis of 1-[1-(4-pyridyl)piperidin-4-ylmethyl]piperazin-2-one

To a solution in anhydrous dimethylformamide (20 ml) of a portion (0.46 g) of the compound obtained in step 3, triethylamine (1.43 ml) was added under cooling with ice water; the mixture was stirred at the same temperature for 1 hour, then at room temperature for another one hour. The solvent was distilled off under reduced pressure and the residue was used as such in the next reaction.

HRMS: $C_{15}H_{22}N_4O$ ($M^+$) Cal'd 274.1793 Found 274.1767
NMR spectrum (*$CDCl_3$ ) δ ppm: 8. 24~8. 20 (2H, m) , 6. 67 ~6. 63 (2H, m) , 3. 96~3. 86 (2H, m) , 3. 55 (2H, s) , 3. 40~ 3. 26 (4H, m) , 3. 14~3. 06 (2H, m) , 2. 92~2. 81 (2H, m) , 2. 10~1. 94 (1H, m) , 1. 90~1. 72 (2H, m) , 1. 42~1. 24 (2H, m)

〈Step 5〉Synthesis of 4-[(E)-4-chlorostyrylsulfonyl]-1-[1-(4-pyridyl)piperidin-4-ylmethyl]piperazin-2-one

The crude product of step 4 was suspended in anhydrous methylene chloride (34 ml); to the suspension, triethylamine (0.72 ml) and a solution of (E)-4-chlorostyrylsulfonyl chloride (245 mg) in anhydrous methylene chloride (5 ml) were added in that order, and the mixture was stirred overnight at room temperature in an argon atmosphere. Water was added to the reaction mixture, which was adjusted to pH 9 with 1 N sodium hydroxide solution and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1 - 95:5) to yield the titled compound (130 mg).

HRMS: $C_{23}H_{27}ClN_4O_3S$ ($M^+$) Cal'd 474.1492 Found 474.1503

Example 24 Synthesis of 4-(6-bromonaphthalen-2-ylsulfonyl)-1-[1-(4-pyridyl)piperidin-4-ylmethyl]piperazin-2-one

Using 6-bromonaphthalen-2-ylsulfonyl chloride (22.3 mg), synthesis was performed by the method of 〈step 5〉in Example 23 to yield the titled compound (12 mg). HRMS: $C_{25}H_{27}BrN_4O_3S$ ($M^+$) Cal'd 542.0987 Found 542.1022

Example 25 Synthesis of 4-(6-chloronaphthalen-2-ylsulfonyl)-1-[1-(4-pyridyl)piperidin-4-ylmethyl]piperazin-2-one

Using 6-chloronaphthalen-2-ylsulfonyl chloride (94.8 mg), synthesis was performed by the method of 〈step 5〉in Example 23 to yield the titled compound (73 mg).

Example 26 Synthesis of 4-(naphthalen-2-ylsulfonyl)-1-[1-(4-pyridyl)piperidin-4-ylmethyl]piperazin-2-one

Using naphthalen-2-ylsulfonyl chloride (29 mg), synthesis was performed by the method of 〈step 5〉in Example 23 to yield the titled compound (5 mg).

Example 27 Synthesis of 4-(6-methylnaphthalen-2-ylsulfonyl)-1-[1-(4-pyridyl)piperidin-4-ylmethyl]piperazin-2-one

Using 6-methylnaphthalen-2-ylsulfonyl chloride (41 mg), synthesis was performed by the method of 〈step 5〉 in Example 23 to yield the titled compound (14 mg).

Example 28 Synthesis of 4-(6-cyanonaphthalen-2-ylsulfonyl)-1-[1-(4-pyridyl)piperidin-4-ylmethyl]piperazin-2-one

Using 6-cyanonaphthalen-2-ylsulfonyl chloride (43 mg), synthesis was performed by the method of 〈step 5〉 in Example 23 to yield the titled compound (11 mg).

Example 29 Synthesis of 4-(6-hydroxynaphthalen-2-ylsulfonyl)-1-[1-(4-pyridyl)piperidin-4-ylmethyl]piperazin-2-one

Using 6-hydroxynaphthalen-2-ylsulfonyl chloride (20 mg), synthesis was performed by the method of 〈step 5〉in Example 23 to yield the titled compound (7.4 mg).

Example 30 Synthesis of 4-(1-fluoronaphthalen-2-ylsulfonyl)-1-[1-(4-pyridyl)piperidin-4-ylmethyl]piperazin-2-one

Using 1-fluoronaphthalen-2-ylsulfonyl chloride (20.5 mg), synthesis was performed by the method of 〈step 5〉 in Example 23 to yield the titled compound (6 mg).

Example 31 Synthesis of 4-(6-chloronaphthalen-2-ylsulfonyl)-2-ethoxycarbonyl-1-[1-(4-pyridyl)piperidin-4-ylmethyl]piperazine

〈Step 1〉Synthesis of 1-benzyl-4-(6-chloronaphthalen-2-ylsulfonyl)-2-(ethoxycarbonyl)piperazine

Triethylamine (6.2 ml) was added to a solution in methylene chloride (70 ml) of 1-benzyl-2-(ethoxycarbonyl)piperazine (3.7 g) prepared by a documented (Synthesis, 318, 1991) method. To the solution, a solution of 6-chloronaphthalen-2-ylsulfonyl chloride (4.3 g) in methylene chloride (40 ml) was added dropwise under cooling with ice. After stirring the reaction mixture overnight at room temperature, water was added and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; hexane:ethyl acetate = 90:10 - 80:20) to give the titled compound (6.4 g).

NMR spectrum (*$CDCl_3$ ) δ ppm:8. 29 (1H, s) , 7. 94~7. 87 (3H, m), 7. 78~7. 73 (1H, m) , 7. 61~7. 55 (1H, m) , 7. 33~ 7. 18 (5H, m), 4. 27~4. 11 (2H, m) , 3. 88 (1H, d, J=13Hz), 3. 61 (1H, d, J=13Hz) , 3. 57~3. 47 (1H, m), 3. 42~3. 36 (1 H, m) , 3. 26~3. 08 (3H, m) , 2. 98~2. 88 (1H, m) , 2. 52~2. 4 3 (1H, m) , 1. 30 (3H, t, J=7Hz)

〈Step 2〉Synthesis of 4-(6-chloronaphthalen-2-ylsulfonyl)-2-(ethoxycarbonyl)piperazine

To a solution in dichloroethane (70 ml) of the compound (6.4 g) obtained in step 1, 1-chloroethyl chloroformate (3.7 ml) was added and the mixture was heated under reflux for 24 hours and concentrated under reduced pressure. Methanol (60 ml) was added to the residue, which was then heated under reflux for 2 hours. The reaction mixture was concentrated under reduced pressure and the residue was neutralized with an aqueous solution of sodium hydroxide and subjected to extraction with ethyl acetate. The ethyl acetate layer was washed with water and saturated sodium chloride, dried with anhydrous sodium sulfate and the solvent was distilled off under reduced pressure. The residue was purified by silica gel column chromatography (eluting solvents; hexane:ethyl acetate = 67:33 and ethyl acetate:triethylamine = 99.9:0.1) to give the titled compound (4.1 g).

NMR spectrum ($CDCl_3$) δ ppm:8. 32 (1H, s) , 7. 96~7. 88 (3 H, m) , 7. 80~7. 75 (1H, m) , 7. 58 (1H, dd, J=2, 9Hz), 4. 25 ~4. 15 (2H, m) , 3. 79~3. 72 (1H, m) , 3. 58 (1H, dd, J=3, 9H z) , 3. 47~3. 39 (1H, m) , 3. 16~3. 06 (1H, m) , 2. 96~2. 63 (3H, m) , 1. 32~1. 25 (3H, m)

〈Step 3〉Synthesis of 4-(6-chloronaphthalen-2-ylsulfonyl) -2-ethoxycarbonyl-1-[1-(4-pyridyl)piperidin-4-ylmethyl]piperazine

A crude product obtained by the method of 〈step 1〉in Example 3 using 1-(4-pyridyl)piperidin-4-yl methanol (2.0 g) was suspended in anhydrous methylene chloride (20 ml). To the suspension, a solution in anhydrous methylene chloride (30 ml) of the compound (2.0 g) obtained in step 2 and acetic acid (1.0 ml) were added in that order. After stirring the reaction mixture at room temperature for 30 minutes in an argon atmosphere, sodium triacetoxyborohydride (2.2 g) was added and the mixture was stirred overnight at room temperature. The reaction mixture was poured into water, adjusted to pH 9 with 1 N sodium hydroxide solution and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography

(eluting solvents; methylene chloride:methanol = 98:2 - 90:10) to yield the titled compound (2.6 g).

Example 32 Synthesis of 4-(6-chloronaphthalen-2-ylsulfonyl)-2-hydroxymethyl-1-[1-(4-pyridyl)piperidin-4-ylmethyl]piperazine

A solution in methylene chloride (3 ml) of the compound (9 mg) obtained in ⟨step 3⟩ of Example 31 was cooled to -78°C. To the cooled solution, 24 μl of diisobutyl aluminum hydride (as 1.5 M toluene solution) was added dropwise. The reaction mixture was heated to room temperature, saturated ammonium chloride solution was added and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 95:5) to yield the titled compound (3 mg).

Example 33 Synthesis of 2-carboxy-4-(6-chloronaphthalen-2-ylsulfonyl)-1-[1-(4-pyridyl)piperidin-4-ylmethyl]piperazine

To a solution in methanol (4 ml) of the compound (240 mg) obtained in ⟨step 3⟩ of Example 31, 2 N sodium hydroxide solution (0.86 ml) was added and the mixture was stirred at 40°C for 2 hours. The reaction mixture was concentrated under reduced pressure and the residue was dissolved in water and rendered acidic with acetic acid. The supernatant was removed by decantation and the residue was dissolved in methanol, dried with anhydrous sodium sulfate and the solvent was distilled off under reduced pressure. The residue was crystallized with ether to yield the titled compound (219 mg).

Example 34 Synthesis of 4-(6-chloronaphthalen-2-ylsulfonyl)-2-[(2-ethoxycarbonyl)acetyl]-1-[1-(4-pyridyl)piperidin-4-ylmethyl]piperazine

To a solution in anhydrous dimethylformamide (0.5 ml) of the compound (50 mg) obtained in Example 33, dimethyl aminopyridine (in catalytic amount) and carbonyl diimidazole (17 mg) were added in that order and the mixture was stirred at room temperature for 2 hours. To the stirred mixture, a solution prepared by stirring methyl malonate potassium (40 mg), magnesium chloride (27 mg) and triethylamine (33 μl) in anhydrous acetonitrile (0.5 ml) for 2 hours was added and the resulting mixture was stirred first at room temperature for 2 hours, then at 40 - 60°C for 3 hours. To the reaction mixture, 1 N HCl was added until a pH of 2 was reached; after stirring for 10 minutes, the mixture was neutralized with saturated sodium hydrogencarbonate and subjected to extraction with ethyl acetate. The ethyl acetate layer was washed with water and saturated sodium chloride, dried with anhydrous sodium sulfate and the solvent was distilled off under reduced pressure to yield the titled compound (40 mg).

Example 35 Synthesis of 2-aminocarbonyl-4-(6-chloronaphthalen-2-ylsulfonyl)-1-[1-(4-pyridyl)piperidin-4-ylmethyl]piperazine

To a solution in anhydrous dimethylformamide (0.5 ml) of the compound (50 mg) obtained in Example 33, dimethyl aminopyridine (in catalytic amount) and carbonyl diimidazole (17 mg) were added in that order and the mixture was stirred at room temperature for 1 hour. The reaction mixture was poured into 28% aqueous ammonia (1 ml) and stirred at room temperature for 2 hours. The reaction mixture was subjected to extraction with ethyl acetate and the ethyl acetate layer was washed with water and saturated sodium chloride, dried with anhydrous sodium sulfate and the solvent was distilled off under reduced pressure. The residue was crystallized with ether to yield the titled compound (21 mg).

Example 36 Synthesis of 4-(6-chloronaphthalen-2-ylsulfonyl)-2-[N-(ethylthioethyl)aminocarbonyl]-1-[1-(4-pyridyl)piperidin-4-ylmethyl]piperazine

To a solution in anhydrous dimethylformamide (2 ml) of the compound (100 mg) obtained in Example 33, 1-(3-dimethylaminopropyl)-3-ethyl carbodiimide hydrochloride (73 mg) and 1-hydroxybenzotriazole (31 mg) were added in that order. To the resulting mixture, ethylthioethylamine hydrochloride (99 mg) was added and stirred overnight at room temperature. The reaction mixture was poured into water, rendered alkaline with saturated sodium hydrogencarbonate and subjected to extraction with methylene chloride. The methylene chloride layer was washed with water and saturated sodium chloride, dried with anhydrous sodium sulfate and distilled off under reduced pressure. The residue was purified by silica gel column chromatography (eluting solvents; methylene chloride:methanol = 95:5 - 90:10) to yield the titled compound (34 mg).

Example 37 Synthesis of 2-acetyl-4-(6-chloronaphthalen-2-ylsulfonyl)-1-[1-(4-pyridyl)piperidin-4-ylmethyl]piperazine

A solution in 1 N HCl (1 ml) of the compound (35 mg) obtained in Example 34 was stirred at 60 - 70°C for 2 hours. The reaction mixture was rendered alkaline with saturated sodium hydrogencarbonate and subjected to extraction with ethyl acetate. The ethyl acetate layer was washed with water and saturated sodium chloride, dried with anhydrous sodium sulfate and the solvent was distilled off under reduced pressure to yield the titled compound (31 mg).

Example 38 Synthesis of 4-(6-chloronaphthalen-2-ylsulfonyl)-2-(N,N-dimethylaminocarbonyl)-1-[1-(4-pyridyl)piperidin-4-ylmethyl]piperazine

Using the compound (50 mg) obtained in Example 33 and 50% dimethylamine solution, synthesis was performed as in Example 35 to yield the titled compound (21 mg).

Example 39 Synthesis of 4-(6-chloronaphthalen-2-ylsulfonyl)-6-ethoxycarbonyl-1-[1-(4-pyridyl)piperidin-4-ylmethyl]piperazin-2-one

〈Step 1〉Synthesis of 4-[1-(4-pyridyl)piperidine]carbaldehyde

A solution of oxalyl chloride (0.43 ml) in anhydrous methylene chloride (20 ml) was cooled to -78°C in a nitrogen atmosphere. To the cooled solution, a solution of anhydrous dimethyl sulfoxide (0.78 ml) in anhydrous methylene chloride (20 ml) was added dropwise over 1 hour. Then, a solution in anhydrous methylene chloride (11 ml) and anhydrous dimethyl sulfoxide (11 ml) of 1-(4-pyridyl)piperidin-4-yl methanol (0.72 g) prepared by a documented (EP 0359389A) method was added dropwise over 1 hour. After stirring at between -65°C and -60°C for 1 hour, the mixture was cooled to -78°C and triethylamine (2.0 ml) was added. The reaction mixture was heated to room temperature, water was added and extraction with methylene chloride was conducted. The methylene chloride layer was washed with water and saturated sodium chloride, dried with anhydrous sodium sulfate and the solvent was distilled off under reduced pressure. The resulting aldehyde was rather labile and should preferably be used in the next reaction without being purified. It can, however, be analyzed with reasonable accuracy. The above-described treatment was done quickly and the concentrated residue was dissolved in $CDCl_3$ and subjected to NMR measurement; a signal for the proton of aldehyde was identified but disappeared with time.

EIMS: 190 ($M^+$)

NMR spectrum (*$CDCl_3$ ) δ ppm:9. 56 (1H, s) , 8. 16~7. 99 (2H, m) , 6. 82~6. 69 (2H, m), 3. 83~3. 71 (2H, m) , 3. 02~ 2. 90 (2H, m) , 2. 61~2. 45 (1H, m) , 1. 90~1. 78 (2H, m) , 1. 52~1. 36 (2M, m)

〈Step 2〉Synthesis of 4-[N-[2-(t-butoxycarbonylamino)-1-(ethoxycarbonyl)ethyl]aminomethyl]-1-(4-pyridyl)piperidine borane complex

To a solution in anhydrous methylene chloride (16 ml) of the compound obtained in step 1, β-(t-butoxycarbonylamino)-alanine ethyl ester (0.70 g) prepared by a documented (WO95/11228) method and acetic acid (0.37 ml) were added in that order. After stirring the mixture at room temperature for 30 minutes in a nitrogen atmosphere, sodium triacetoxyborohydride (1.6 g) was added and the mixture was stirred overnight at room temperature. Water was added to the reaction mixture, which was rendered alkaline with 1 N sodium hydroxide solution and subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 95:5) to give the titled compound (0.36 g).

NMR spectrum (*$CDCl_3$ ) δ ppm: 8. 18 (2H, d, J=8Hz) , 6. 6 9 (2H, d, J=8Hz) , 4. 95~4. 86 (1H, brs) , 4. 25~4. 15 (2H, m) , 4. 06~3. 96 (2H, m) , 3. 50~3. 38 (1H, m) , 3. 35~3. 18 (2H, m), 3. 11~2. 98 (2H, m), 2. 60 (1H, dd, J=7, 12Hz), 2. 43~2. 34 (1H, m) , 2. 04 (6H, s) , 2. 03~1. 66 (3H, m) , 1. 44 (9H, s) , 1. 30 (3H, t, J=7Hz) , 1. 30~1. 16 (2H, m)

〈Step 3〉Synthesis of 4-[N-bromoacetyl-N-[2-(t-butoxycarbonylamino)-1-(ethoxycarbonyl)-ethyl]aminomethyl]-1-(4-pyridyl)piperidine borane complex

A solution in anhydrous methylene chloride (2 ml) of the compound (0.34 g) obtained in step 2 was cooled with ice. To the cooled solution, triethylamine (94 μl) and a solution of bromoacetyl chloride (56 μl) in anhydrous meth-

ylene chloride (2 ml) were added in that order and the mixture was stirred at room temperature for 1 hour. After cooling the reaction mixture with ice, water was added and extraction with methylene chloride was conducted. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1 - 95:5) to give the titled compound (0.23 g).

NMR spectrum (*$CDCl_3$ ) δ ppm: 8. 22 (2H, d, J=8Hz) , 6. 7 2 (2H, d. J=8Hz), 5. 20~5. 00 (1H, m) , 4. 26~4. 16 (2H, m) , 4. 15~3. 95 (3H, m) , 3. 89~3. 73 (3H, m) , 3. 55~3. 23 (3H, m) , 3. 18~2. 94 (2H, m) , 2. 20~1. 92 (3H, m) , 2. 04 (6 H, s) , 1. 44 (9H, s) , 1. 33~1. 20 (5H, m)

〈Step 4〉Synthesis of 4-[N-[2-amino-1-(ethoxycarbonyl)ethyl]-N-bromoacetylaminomethyl]-1-(4-pyridyl)piperidine hydrochloride

To the compound (0.21 g) obtained in step 3, 3 N HCl-ethyl acetate (20 ml) was added and the mixture was stirred at room temperature for 2 hours. Ether was added to the reaction mixture and the supernatant was removed by decantation. The same procedure was repeated; ether was added and the supernatant was removed by decantation to give the titled compound (0.17 g).

NMR spectrum (DMSO-$d_6$ ) δ ppm: 13. 68~13. 47 (1H, br s) , 8. 58~8. 32 (3H, brs) , 8. 28~8. 15 (2H, m) , 7. 35~7. 1 5 (2H, m), 4. 59~4. 42 (3H, m), 4. 42~4. 24 (2H, m), 4. 18~ 4. 00 (2H, m) , 3. 52~3. 37 (2H, m), 3. 37~3. 23 (2H, m) , 3. 23~3. 02 (2H, m), 2. 17~1. 94 (2H, m) , 1. 92~1. 78 (1H, m), 1. 38~1. 13 (5H, m)

〈Step 5〉Synthesis of 6-ethoxycarbonyl-1-[1-(4-pyridyl)piperidin-4-ylmethyl]piperazin-2-one

A solution in anhydrous dimethylformamide (5 ml) of the compound (0.16 g) obtained in step 4 was cooled with ice. To the cooled solution, triethylamine (0.41 ml) was added and the mixture was stirred at room temperature for 1 hour. The reaction mixture was concentrated under reduced pressure to give a crude form of the titled compound, which was used in the next reaction without being purified. A half of the crude product was purified by silica gel column chromatography (eluting solvents; methylene chloride:methanol = 50:50) and NMR spectrum data was taken.

NMR spectrum (*$CDCl_3$ ) δ ppm: 8. 13 (2H, d. J=7Hz) , 6. 9 7 (2H, d, J=7Hz), 4. 27 (2H, q, J=7Hz), 4. 23~4. 17 (3H, m), 3. 94 (1H, dd, J=8, 14Hz), 3. 57 (2H, s) , 3. 43~3. 11 (4H, m), 2. 66 (1H, dd, J=7, 14Hz) , 2. 20~2. 03 (1H, m), 1. 98~1. 81 (2H, m) , 1. 44~1. 24 (2H, m) , 1. 32 (3H, t, J=7 Hz)

〈Step 6〉Synthesis of 4-(6-chloronaphthalen-2-ylsulfonyl)-6-ethoxycarbonyl-1-[1-(4-pyridyl)piperidin-4-ylmethyl]piperazin-2-one

The remaining half of the crude product obtained in step 5 was dissolved in anhydrous methylene chloride (5 ml). To the solution, triethylamine (0.1 ml) and 6-chloronaphthalen-2-ylsuhfonyl chloride (39.1 mg) were added in that order and the mixture was stirred overnight at room temperature. Water was added to the reaction mixture, which was subjected to extraction with methylene chloride. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 99:1 - 90:10) to yield the titled compound (48 mg).

HRMS: $C_{28}H_{31}ClN_4O_5S$ ($M^+$): Cal'd 570.1703 Found 570.1681

Example 40 Synthesis of 6-carboxy-4-(6-chloronaphthalen-2-ylsulfonyl)-1-[1-(4-pyridyl)piperidin-4-ylmethyl]piperazin-2-one

To a solution in methanol (0.5 ml) of the compound (18 mg) obtained in 〈step 6〉of Example 39, 2 N sodium hydroxide solution (63 µl) was added and the mixture was stirred at 40°C for 30 minutes. The reaction mixture was concentrated under reduced pressure and the resulting residue was dissolved in water and rendered weakly acidic with 0.1 N HCl. The supernatant was removed by decantation and the residue was dissolved in methanol, dried with anhydrous sodium sulfate and the solvent was distilled off under reduced pressure to yield the titled compound (9 mg).

Example 41 Synthesis of 4-(6-chloronaphthalen-2-ylsulfonyl)-6-hydroxymethyl-1-[1-(4-pyridyl)piperidin-4-ylmethyl]piperazin-2-one

The compound (0.15 g) obtained in ⟨step 6⟩ of Example 39 was dissolved in methanol (10 ml). To the solution, lithium borohydride (0.60 g) was added in three portions at 30 minute intervals under cooling with ice bath. To the reaction mixture, 10% HCl-methanol solution was added under cooling with ice to make it acidic and the mixture was then concentrated to dryness. Water was added to the residue, saturated sodium hydrogencarbonate was then added to make the residue alkaline, and extraction with methylene chloride was conducted. The methylene chloride layer was washed with saturated sodium chloride, dried with anhydrous sodium sulfate and the solvent was distilled off under reduced pressure. The residue was purified by silica gel column chromatography [Chromatorex NH™] (eluting solvent; ethyl acetate) to yield the titled compound (60 mg).

Example 42 Synthesis of 4-(6-chloronaphthalen-2-ylsulfonyl)-6-methoxymethyl-1-[1-(4-pyridyl)piperidin-4-ylmethyl]piperazin-2-one

The compound (43 mg) obtained in Example 41 was suspended in methylene chloride (1 ml). To the stirred suspension, 50% sodium hydroxide solution (0.3 ml) was added under cooling with ice. Then, benzyltriethylammonium chloride (3 mg) and dimethyl sulfate (9 μl) were added and the mixture was stirred for 2 hours under cooling with ice. Ice water was added to the reaction mixture to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 90:10) to yield the titled compound (16 mg).

HRMS: $C_{27}H_{31}ClN_4O_4S$ ($M^+$) Cal'd 542.1754 Found 542.1739

Example 43 Synthesis of 6-acetoxymethyl-4-(6-chloronaphthalen-2-ylsulfonyl)-1-[1-(4-pyridyl)piperidin-4-ylmethyl]piperazin-2-one

The compound obtained in Example 41 was used as a starting material and acetylated in the usual manner to yield the titled compound.

Example 44 Synthesis of 4-[(E)-4-chlorostyrylsuhfonyl]-6-ethoxycarbonyl-1-[1-(4-pyridyl)piperidin-4-ylmethyl]piperazin-2-one

In accordance with the synthesis method of ⟨step 6⟩ in Example 39, the compound obtained in ⟨step 5⟩ of Example 39 was used as a starting material and reacted with (E)-4-chlorostyrylsulfonyl chloride to yield the titled compound.

Example 45 Synthesis of 6-carboxy-4-[(E)-4-chlorostyrylsulfonyl]-1-[1-(4-pyridyl)piperidin-4-ylmethyl]piperazin-2-one

The compound obtained in Example 44 was treated in accordance with the method of synthesis in Example 40 to yield the titled compound.

Example 46 Synthesis of 6-aminocarbonyl-4-(6-chloronaphthalen-2-ylsulfonylY-1-[1-(4-pyridyl)piperidin-4-ylmethyl]piperazin-2-one

Ammonia gas was blown into a solution in 2 N ammonia-methanol (5 ml) of the compound (0.20 g) obtained in ⟨step 6⟩ of Example 39 and the solution was stirred in a sealed tube at 80 - 90°C for 8 hours. The reaction mixture was concentrated under reduced pressure and the residue was purified by silica gel column chromatography (eluting solvents; methylene chloride:methanol = 90:10) to yield the titled compound (0.14 g).

Example 47 Synthesis of 6-aldoximyl-4-(6-chloronaphthalen-2-ylsulfonyl)-1-[1-(4-pyridyl)piperidin-4-ylmethyl]piperazin-2-one

⟨Step 1⟩ Synthesis of 4-(6-chloronaphthalen-2-ylsulfonyl)-6-formyl-1-[1-(4-pyridyl)piperidin-4-ylmethyl]piperazin-2-one

A solution of oxalyl chloride (33 μl) in anhydrous methylene chloride (1 ml) was cooled to -78°C in a nitrogen atmosphere. To the cooled solution, a solution of anhydrous dimethyl sulfoxide (60 μl) in anhydrous methylene chloride

(1 ml) was added dropwise. Then, a solution in anhydrous methylene chloride (1 ml) of the compound (10 mg) obtained in Example 41 was added dropwise. The resulting mixture was stirred at between -65°C and -60°C for 2 hours, then cooled to -78°C and triethylamine (0.16 ml) was added. The reaction mixture was heated to room temperature, water was added and extraction with methylene chloride was conducted. The methylene chloride layer was washed with saturated sodium chloride, dried with anhydrous sodium sulfate and the solvent was distilled off under reduced pressure to give a crude form of the titled compound. The compound was used in the next reaction without being purified.

〈Step 2〉Synthesis of 6-aldoximyl-4-(6-chloronaphthalen-2-ylsulfonyl)-1-[1-(4-pyridyl)piperidin-4-ylmethyl]piperazin-2-one

The compound obtained in step 1 was dissolved in ethanol (1 ml). Hydroxylamine hydrochloride (2.5 mg) and sodium acetate (3 mg) were added to the solution. Acetic acid was added to the reaction mixture to adjust its pH to about 4 and the mixture was stirred overnight at room temperature. The reaction mixture was rendered alkaline by addition of saturated sodium hydrogencarbonate and subjected to extraction with methylene chloride. The methylene chloride layer was washed with saturated sodium chloride, dried with anhydrous sodium sulfate and the solvent was distilled off under reduced pressure. The residue was purified by silica gel column chromatography [Chromatorex NH™] (eluting solvents; methylene chloride:methanol = 97:3) to yield the titled compound (1.9 mg).

Example 48 Synthesis of 4-(6-chloronaphthalen-2-ylsulfonyl)-6-morpholinocarbonyl-1-[1-(4-pyridyl)piperidin-4-ylmethyl]piperazin-2-one

The compound (0.20 g) obtained in 〈step 6〉of Example 39 was dissolved in ethanol (5 ml). A solution of lithium hydroxide monohydrate (15 mg) in water (1 ml) was added and the resulting mixture was heated under reflux for 15 minutes. After concentrating the reaction mixture, thionyl chloride (1 ml) and a small amount of dimethylformamide were added to the residue in that order and the mixture was stirred at room temperature for 40 minutes. After concentrating the reaction mixture, anhydrous methylene chloride (5 ml) was added to the residue and then morpholine (1.5 ml) was added dropwise under cooling with ice. Water was added to the reaction mixture, which was subjected to extraction with methylene chloride. The methylene chloride layer was washed with saturated sodium hydrogencarbonate, water and saturated sodium chloride in that order, dried with anhydrous sodium sulfate, and the solvent was distilled off under reduced pressure. The residue was purified by silica gel column chromatography [Chromatorex NH™] (eluting solvents; methylene chloride:methanol = 99:1) to yield the titled compound (0.13 g).

Example 49 Synthesis of 4-(6-chloronaphthalen-2-ylsulfonyl)-6-dimethylaminocarbonyl-1-[1-(4-pyridyl)piperidin-4-ylmethyl]piperazin-2-one

Using diethylamine hydrochloride, the method of synthesis in Example 48 was repeated to yield the titled compound.

Example 50 Synthesis of 4-(6-chloronaphthalen-2-ylsulfonyl)-6-methoxyaminocarbonyl-1-[1-(4-pyridyl)piperidin-4-ylmethyl]piperazin-2-one

To the compound (10 mg) obtained in Example 40, thionyl chloride (0.5 ml) and a small amount of dimethylformamide were added in that order and the mixture was stirred at room temperature for 2 hours. After concentrating the reaction mixture, anhydrous methylene chloride (1 ml) was added to the residue and then a solution of methoxyamine hydrochloride (30 mg) and triethylamine (50 μl) in methylene chloride (1 ml) was added dropwise under cooling with ice. Saturated sodium hydrogencarbonate was added to the reaction mixture, which was then subjected to extraction with methylene chloride. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 90:10) to yield the titled compound (2.4 mg).

Example 51 Synthesis of 4-(6-chloronaphthalen-2-ylsulfonyl)-6-(4-hydroxypiperidinecarbonyl)-1-[1-(4-pyridyl)piperidin-4-ylmethyl]piperazin-2-one

Using 4-hydroxypiperidine, the method of synthesis in Example 48 was repeated to yield the titled compound.

Example 52 Synthesis of 6-aminomethyl-4-(6-chloronaphthalen-2-ylsulfonyl)-1-[1-(4-pyridyl)piperidin-4-ylmethyl]piperazin-2-one

〈Step 1〉Synthesis of 4-(6-chloronaphthalen-2-ylsulfonyl)-6-phthaliminomethyl-1-[1-(4-pyridyl)piperidin-4-ylmethyl]piperazin-2-one

Diethyl azodicarboxylate (as 40% toluene solution) (1.71 ml) was added to a solution of triphenyl phosphine (0.99 g) and phthalimide (0.56 g) in anhydrous methylene chloride (30 ml) under cooling with ice. After stirring the mixture at the same temperature for 10 minutes, the compound (0.50 g) obtained in Example 41 was added and the mixture was stirred at room temperature for 30 minutes. Saturated sodium hydrogencarbonate was added to the reaction mixture under cooling with ice, followed by extraction with methylene chloride, washing with saturated sodium chloride, drying with anhydrous sodium sulfate and distilling off the solvent under reduced pressure. The residue was purified by silica gel column chromatography (eluting solvents; methylene chloride:methanol = 90:10) to give the titled compound (0.61 g).

NMR spectrum ($CDCl_3$ ) δ ppm: 8. 42 (1H, s) , 8. 24~8. 1 9 (2H, m) , 7. 97~7. 87 (6H, m) , 7. 83~7. 75 (2H, m) , 7. 63~7. 57 (1H, m) , 6. 62~6. 56 (2H, m), 4. 33~4. 20 (1H ,m), 4. 11 (1H, d, J=17Hz), 4. 04~3. 93 (2H, m), 3. 87 ~3. 68 (4H, m), 3. 43 (1H, d, J=17Hz), 2. 96~2. 68 (4H , m), 2. 08~1. 87 (1H, m) , 1. 73~1. 57 (2H, m) , 1. 42~1 .17 (2H, m)

〈Step 2〉Synthesis of 6-aminomethyl-4-(6-chloronaphthalen-2 ylsulfonyl)-1-[1-(4-pyridyl)piperidin-4-ylmethyl]piperazin-2-one

A portion (0.61 g) of the compound obtained in step 1 was suspended in ethanol (15 ml). Hydrazine monohydrate (54 µl) was added to the suspension at room temperature, followed by stirring at room temperature for 20 hours. The suspension was then allowed to reflux for 4 hours. After leaving the suspension to cool, the insolubles were filtered off and the filtrate was concentrated. The residue was purified by silica gel column chromatography (eluting solvents; methylene chloride:methanol = 90:10) to yield the titled compound (0.36 g).

Example 53 Synthesis of 4-(6-chloronaphthalen-2-ylsulfonyl)-6-morpholinomethyl-1-[1-(4-pyridyl)piperidin-4-ylmethyl]piperazin-2-one

〈Step 1〉Synthesis of 4-(6-chloronaphthalen-2-ylsulfonyl)-6-(4-methylbenzenesulfonyl)oxymethyl-1-[1-(4-pyridyl)piperidin-4-ylmethyl]piperazin-2-one

A portion (50 mg) of the compound obtained in Example 41 was suspended in methylene chloride (1 ml). To the stirred suspension, triethylamine (0.3 ml) and 4-methylbenzenesulfonyl chloride (20 mg) were added in that order under cooling with ice and the mixture was stirred overnight at room temperature. Ice water was added to the reaction mixture to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 95:5) to give the titled compound (52 mg).

NMR spectrum (*$CDCl_3$ ) δ ppm:8. 34 (1H, s) , 8. 25~8. 20 (2H, m) , 7. 98~7. 92 (3H, m) , 7. 83 (2H, d, J=9Hz) , 7. 76 (1H, dd, J=2, 9Hz) , 7. 65~7. 60 (1H, m) , 7. 41 (2H, d, J=9 Hz), 6. 62~6. 57 (2H, m) , 4. 23~4. 05 (4H, m) , 3. 90~3. 67 (4H, m) , 3. 31 (1H, d. J=17Hz) , 2. 80~2. 62 (4H, m), 2. 48 (3H, s) , 1. 98~1. 84 (1H, m) , 1. 76~1. 45 (2H, m) , 1. 36~ 1. 16 (2H, m)

〈Step 2〉Synthesis of 4-(6-chloronaphthalen-2-ylsulfonyl)-6-morpholinomethyl-1-[1-(4-pyridyl)piperidin-4-ylmethyl]piperazin-2-one

The compound (100 mg) obtained in step 1 was dissolved in anhydrous dimethylformamide (2 ml). Potassium carbonate (100 mg) and morpholine (128 µl) were successively added to the solution, which was then stirred at 80°C for 4 hours. Ice water was added to the reaction solution to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 95:5) to yield the titled compound (36 mg).

Example 54 Synthesis of 4-(6-chloronaphthalen-2-ylsulfonyl)-6-dimethylaminomethyl-1-[1-(4-pyridyl)piperidin-4-ylmethyl]piperazin-2-one

A portion (31.3 mg) of the compound obtained in ⟨step 2⟩ of Example 52 and paraformaldehyde (4.9 mg) were suspended in anhydrous methylene chloride (1 ml). To the suspension, acetic acid (13.6 μl) and sodium triacetoxyborohydride (50.2 mg) were added at room temperature and the mixture was stirred at room temperature for 20 hours. Saturated sodium hydrogencarbonate was added to the reaction mixture under cooling with ice and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 90:10) to yield the titled compound (11.6 mg).

Example 55 Synthesis of 6-acetamidomethyl-4-(6-chloronaphthalen-2-ylsulfonyl)-1-[1-(4-pyridyl)piperidin-4-ylmethyl]piperazin-2-one

The compound obtained in Example 52 was used as a starting material and acetylated in the usual manner to yield the titled compound.

Example 56 Synthesis of 4-(6-chloronaphthalen-2-ylsulfonyl)-6-methanesulfonylamidomethyl-1-[1-(4-pyridyl)piperidin-4-ylmethyl]piperazin-2-one

The compound obtained in Example 52 was used as a starting material and methanesulfonylated in the usual manner to yield the titled compound.

Example 57 Synthesis of 4-(6-chloronaphthalen-2-ylsulfonyl)-6-(4-hydroxypiperidin-1-ylmethyl)-1-[1-(4-pyridyl)piperidin-4-ylmethyl]piperazin-2-one

⟨Step 1⟩ Synthesis of 4-(t-butyldimethylsiloxy)piperidine

To a stirred solution of 4-hydroxypiperidine (2.0 g) in anhydrous methylene chloride (20 ml), pyridine (1.9 ml) and t-butyldimethylsilyl trifluoromethanesulfonate (5.0 ml) were successively added under cooling with ice and the resulting mixture was stirred for 3 hours. Ice water was added to the reaction mixture to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 95:5) to give the titled compound (3.8 g).

NMR spectrum (∗$CDCl_3$ ) δ ppm:4. 13~4. 05 (1H, m), 3. 44 ~3. 20 (4H, m), 2. 10~1. 96 (2H, m), 1. 82~1. 70 (2H, m), 0. 89 (9H, s) , 0. 07 (6H, s)

⟨Step 2⟩ Synthesis of 6-[4-(t-butyldimethylsiloxy)piperidin-1-ylmethyl]-4-(6-chloronaphthalen-2-ylsulfonyl)-1-[1-(4-pyridyl)piperidin-4-ylmethyl]piperazin-2-one

The compound (0.30 g) obtained in ⟨step 1⟩ of Example 53 was dissolved in anhydrous dimethylformamide (2 ml). Potassium carbonate (0.60 g) and a portion (0.47 g) of the compound obtained in ⟨step 1⟩ of Example 57 were added in that order and the mixture was stirred at 80°C for 2.5 hours. Potassium carbonate (0.60 g) and the compound (0.47 g) obtained in ⟨step 1⟩ of Example 57 were further added and the mixture was stirred at 80°C for 6 hours. Ice water was added to the reaction mixture to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography (eluting solvents; methylene chloride:methanol = 95:5) and on [Chromatorex NH™] (eluting solvent; ethyl acetate) to give the titled compound (0.15 g).

NMR spectrum (∗$CDCl_3$ ) δ ppm: 8. 36 (1H, s) , 8. 23~8 .18 (2H, m) , 7. 98~7. 92 (3H m) , 7. 83~7. 78 (1H, m), 7. 61 (1 H, dd, J=2, 9Hz), 6. 60~6. 55 (2H, m) , 4. 23~4. 08 (2H, m), 3. 91~3. 64 (4H, m) , 3. 31 (1H, d, J=17Hz), 3. 29~3. 2 2 (1H, m) , 2. 80~2. 55 (7H, m) , 2. 42~2. 21 (3H, m), 2. 02~ 1. 12 (9H, m) , 0. 89 (9H, s) , 0. 05 (6H, s)

〈Step 3〉Synthesis of 4-(6-chloronaphthalen-2-ylsulfonyl)-6-(4-hydroxypiperidin-1-ylmethyl)-1-[1-(4-pyridyl)piperidin-4-ylmethyl]piperazin-2-one

The compound (0. 15 g) obtained in step 2 was dissolved in tetrahydrofuran (2 ml). Tetrabutylammonium fluoride (as 1.0 M tetrahydrofuran solution) (0.25 ml) was added and the mixture was stirred overnight at room temperature. Ice water was added to the reaction mixture to quench the reaction and extraction with methylene chloride was conducted. The methylene chloride layer was washed with saturated sodium chloride, dried with anhydrous sodium sulfate and the solvent was distilled off under reduced pressure. The residue was purified by silica gel column chromatography [Chromatorex NH™] (eluting solvent; ethyl acetate) to yield the titled compound (58 mg).

Example 58 Synthesis of 6-hydroxymethyl-4-(2-naphthylsulfonyl)-1-[1-(4-pyridyl)piperidin-4-ylmethyl]piperazin-2-one

Using 2-naphthalenesulfonyl chloride, the method of synthesis in Example 41 was repeated to yield the titled compound.

Example 59 synthesis of 6-acetoxymethyl-4-(2-naphthylsulfonyl)-1-[1-(4-pyridyl)piperidin-4-ylmethyl]piperazin-2-one

The compound obtained in Example 58 was used as a starting material and acetylated in the usual manner to yield the titled compound.

Example 60 Synthesis of (R)-4-(6-chloronaphthalen-2-ylsulfonyl)-6-ethoxycarbonyl-1-[1-(4-pyridyl)piperidin-4-ylmethyl]piperazin-2-one

〈Step 1〉Synthesis of 1-(4-pyridyl)piperidine-4-carbaldehyde

A solution of oxalyl chloride (2.45 ml) in anhydrous methylene chloride (100 ml) was cooled to -78°C in a nitrogen atmosphere. To the cooled solution, a solution of anhydrous dimethyl sulfoxide (4.44 ml) in anhydrous methylene chloride (100 ml) was added dropwise over 1 hour. Then, a solution in anhydrous methylene chloride (50 ml) and anhydrous dimethyl sulfoxide (50 ml) of 1-(4-pyridyl)piperidin-4-yl methanol (4.14 g) prepared by a documented (EP 0359389) method was added dropwise over 1 hour. After stirring at between -65°C and -60°C for 1 hour, the mixture was cooled to -78°C and triethylamine (11.4 ml) was added. The reaction mixture was heated to room temperature, water was added and extraction with methylene chloride was conducted. The methylene chloride layer was washed with water and saturated sodium chloride, dried with anhydrous sodium sulfate and the solvent was distilled off under reduced pressure. The resulting aldehyde was rather labile, so it was used in the next reaction without being purified.

EIMS: 190 ($M^+$)
NMR spectrum (*$CDCl_3$ ) δ ppm:9. 56 (1H, s) , 8. 16~7. 99 (2H, m), 6. 82~6. 69 (2H, m), 3. 83~3. 71 (2H, m), 3. 02~ 2. 90 (2H, m), 2. 61~2. 45 (1H, m), 1. 90~1. 78 (2H, m), 1. 52~1. 36 (2H, m)

〈Step 2〉Synthesis of (R)-4-[N-[2-(t-butoxycarbonylamino)-1-(ethoxycarbonyl)ethyl]aminomethyl]-1-(4-pyridyl)-piperidine borane complex

To a solution in anhydrous methylene chloride (90 ml) of the compound obtained in step 1, (R)-β-(t-butoxycarbonylamino)alanine ethyl ester (4.00 g) prepared by a documented (WO95/11228) method and acetic acid (2.11 ml) were added in that order. After stirring the mixture at room temperature for 30 minutes, sodium triacetoxyborohydride (9.12 g) was added and the mixture was stirred at room temperature for 2 days. The reaction mixture was cooled with ice and, after adding water, it was extracted with methylene chloride. The methylene chloride layer was washed with water and saturated sodium chloride, dried with anhydrous sodium sulfate and the solvent was distilled off under reduced pressure to give a crude form of the titled compound (5.73 g).

NMR spectrum (*$CDCl_3$ ) δ ppm:8. 18 (2H, d, J=8Hz) , 6. 6 9 (2H, d, J=8Kz) , 4. 95~4. 86 (1H, brs) , 4. 25~4. 15 (2H, m) , 4. 06~3. 96 (2H, m) , 3. 50~3. 38 (1H, m) , 3. 35~3. 18 (2H, m) , 3. 11~2. 98 (2H, m) , 2. 60 (1H, dd, J=7, 12Hz), 2. 43~2. 34 (1H, m) , 2. 04 (6H, s) , 2. 03~1. 66 (3H, m) , 1. 44 (9H, s) , 1. 30 (3H, t, J=7Hz), 1. 30~1. 16 (2H, m)

〈Step 3〉Synthesis of (R)-4-[N-bromoacetyl-N-[2-(t-butoxycarbonylamino)-1-(ethoxycarbonyl)ethyl]-aminomethyl]-1-(4-pyridyl)piperidine borane complex

A solution in anhydrous mnethylene chloride (100 ml) of the compound (5.41 g) obtained in step 2 was cooled with ice. To the cooled solution, triethylamine (3.00 ml) and a solution of bromoacetyl chloride (1.77 ml) in anhydrous methylene chloride (20 ml) were added in that order and the mixture was stirred at room temperature for 1 hour. After cooling the reaction mixture with ice, water was added and extraction with methylene chloride was conducted. The methylene chloride layer was washed with water and saturated sodium chloride, dried with anhydrous sodium sulfate and the solvent was distilled off under reduced pressure. The residue was purified by silica gel column chromatography (eluting solvents; methylene chloride:methanol = 97:3) to give the titled compound (2.94 g).

NMR spectrum (*$CDCl_3$ ) δ ppm: 8. 22 (2H, d, J=8Hz), 6. 7 2 (2H, d, J=8Hz), 5. 20~5. 00 (1H, m) , 4. 26~4. 16 (2H, m), 4. 15~3. 95 (3H, m), 3. 89~3. 73 (3H, m) , 3. 55~3. 23 (3H, m), 3. 18~2. 94 (2H, m), 2. 20~1. 92 (3H, m), 2. 04 (6 H, s) , 1. 44 (9H, s) , 1. 33~1. 20 (5H, m)

〈Step 4〉Synthesis of (R)-4-(6-chloronaphthalen-2-ylsulfonyl)-6-ethoxycarbonyl-1-[1-(4-pyridyl)piperidin-4-ylmethyl]piperazin-2-one

A solution in dry methanol (30 ml) of a portion (2.90 g) of the compound obtained in step 3 was cooled with ice and 10% HCl-methanol (30 ml) was added dropwise. The resulting mixture was stirred at room temperature for 30 minutes. The reaction mixture was concentrated, the residue was dissolved in anhydrous dimethylformamide (30 ml) and triethylamine (6.0 ml) was added dropwise under cooling with ice. The reaction mixture was stirred overnight at room temperature; under cooling with ice, a solution of triethylamine (3.0 ml) in anhydrous methylene chloride (10 ml) and then a solution of 6-chloronaphthalen-2-ylsulfonyl chloride (1.13 g) in anhydrous methylene chloride (10 ml) were added dropwise, and the mixture was stirred overnight at room temperature. The reaction mixture was cooled with ice and, after adding water, it was subjected to extraction with methylene chloride. The methylene chloride layer was washed with water and saturated sodium chloride, dried with anhydrous sodium sulfate, and the solvent was distilled off under reduced pressure. The residue was purified by silica gel column chromatography (eluting solvents; methylene chloride:methanol = 90:10) to yield the titled compound (0.92 g). The optical purity of this compound was measured by HPLC [CHIRALPAK™ AD (DAICEL CHEMICAL INDUSTRIES, LTD.); hexane:isopropanol:diethylamine = 60:40:0.4] and it was found to be 98.3% e.e.

HRMS: $C_{28}H_{31}ClN_4O_5S$ ($M^+$): Cal'd 570.1703 Found 570.1664

Example 61 Synthesis of (S)-4-(6-chloronaphthalen-2-ylsulfonyl)-6-ethoxycarbonyl-1-[1-(4-pyridyl)piperidin-4-ylmethyl]piperazin-2-one

Using (S)-β-(t-butoxycarbonylamino)alanine ethyl ester, the method of synthesis in Example 60 was repeated to yield the titled compound. The optical purity of this compound was measured by HPLC [CHIRALPACK™ AD (DAICEL CHEMICAL INDUSTRIES, LTD.); hexane:isopropanol:diethylamine = 60:40:0.4] and it was found to be 97.7% e.e. HRMS: $C_{28}H_{31}ClN_4O_5S$ ($M^+$) Cal'd 570.1703 Found 570.1689

Example 62 Synthesis of (R)-4-(6-chloronaphthalen-2-ylsulfony1)-6-methoxymethyl-1-[1-(4-pyridyl)piperidin-4-ylmethyl]piperazin-2-one

Using (R)-N-[2-amino-2-(methoxymethyl)ethyl]carbamic acid t-butyl ester, the method of synthesis in Example 60 was repeated to yield the titled compound. The optical purity of this compound was measured by HPLC [CHIRALPACK™ AD (DAICEL CHEMICAL INDUSTRIES, LTD.); hexane:isopropanol:diethylamine = 50:50:0.5] and it was found to be 89.4% e.e.

HRMS: $C_{27}H_{31}ClN_4O_4S$ ($M^+$) Cal'd 542.1754 Found 542.1794

Example 63 Synthesis of (S)-4-(6-chloronaphthalen-2-ylsulfonyl)-6-methoxymethyl-1-[1-(pyridyl)piperidin-4-ylmethyl]piperazin-2-one

Using (S)-N-[2-amino-2-(methoxymethyl)ethyl]carbamic acid t-butyl ester, the method of synthesis in Example 60 was repeated to yield the titled compound. The optical purity of this compound was measured by HPLC [CHIRALPACK™ AD (DAICEL CHEMICAL INDUSTRIES, LTD.); hexane:isopropanol:diethylamine = 50:50:0.5] and it was

found to be 90.3% e.e.

Example 64 Synthesis of (R)-6-carboxy-4-(6-chloronaphthalen-2-ylsulfonyl)-1-[1-(4-pyridyl)piperidin-4-ylmethyl]piperazin-2-one hydrochloride

To a solution in acetic acid (2.2 ml) of a portion (215 mg) of the compound obtained in ⟨step 4⟩ of Example 60, conc. hydrochloric acid (2.2 ml) was added and the mixture was heated under reflux for 3 hours. The reaction mixture was concentrated under reduced pressure. Water was added to the resulting residue and further concentrating was done under reduced pressure. Ether was added to the residue and crystallization was effected to yield the titled compound. The optical purity of this compound was measured by HPLC [CHIRALPACK™ AD (DAICEL CHEMICAL INDUSTRIES, LTD.); hexane:isopropanol:diethylamine = 60:40:0.4] and it was found to be 95.8% e.e.

Example 65 Synthesis of 4-(6-chloronaphthalen-2-ylsulfonyl)-6-n-propoxycarbonyl-1-[1-(4-pyridyl)piperidin-4-ylmethyl]piperazin-2-one

The compound (20 mg) obtained in ⟨step 6⟩ of Example 39 was dissolved in ethanol (0.4 ml). A solution of lithium hydroxide monohydrate (1.5 mg) in water (0.1 ml) was added and the resulting mixture was heated under reflux for 45 minutes. After concentrating the reaction mixture, thionyl chloride (0.2 ml) and a small amount of dimethylformamide were added to the residue in that order and the mixture was stirred at room temperature for 1 hour. The reaction mixture was concentrated under reduced pressure and the resulting residue was suspended in anhydrous methylene chloride (0.5 ml). Under cooling with ice, n-propanol (52 μl) and then triethylamine (49 μl) were added and the mixture was stirred for 2 hours at room temperature. Saturated sodium hydrogencarbonate was added to the reaction mixture, which was subjected to extraction with methylene chloride. The methylene chloride layer was washed with saturated sodium chloride, dried with anhydrous sodium sulfate, and the solvent was distilled off under reduced pressure. The residue was purified by silica gel column chromatography [Chromatorex NH™] (eluting solvents; hexane:ethyl acetate 50:50 - 20:80) to yield the titled compound (11 mg).

HRSM: $C_{29}H_{33}ClN_4O_5S$ ($M^+$) Cal'd 584.1860 Found 584.1900

Example 66 Synthesis of (R)-4-(6-chloronaphthalen-2-ylsulfonyl)-6-n-propoxycarbonyl-1-[1-(4-pyridyl)piperidin-4-ylmethyl]piperazin-2-one

Conc. hydrochloric acid (2 ml) was added to a solution in acetic acid (2 ml) of the compound (200 mg) obtained in ⟨step 4⟩ of Example 60 and the solution was heated under reflux for 3 hours. The reaction mixture was concentrated under reduced pressure and water was added to the resulting residue, followed by further concentrating under reduced pressure. To the residue, thionyl chloride (1 ml) and a small amount of dimethylformamide were added in that order and the mixture was stirred at room temperature for 1 hour. The reaction mixture was concentrated under reduced pressure and the resulting residue was suspended in anhydrous methylene chloride (3 ml). Under cooling with ice, n-propanol (0.52 ml) and then triethylamine (0.49 ml) were added and the mixture was stirred for 1 hour at room temperature. Saturated sodium hydrogencarbonate was added to the reaction mixture, which was subjected to extraction with methylene chloride. The methylene chloride layer was washed with saturated sodium chloride, dried with anhydrous sodium sulfate, and the solvent was distilled off under reduced pressure. The residue was purified by silica gel column chromatography [Chromatorex NH™] (eluting solvents; methylene chloride:methanol = 99:1) to yield the titled compound (134 mg). The optical purity of this compound was measured by HPLC [CHIRALFACK™ AD (DAICEL CHEMICAL INDUSTRIES, LTD.); hexane:isopropanol:diethylamine = 60:40:0.4] and it was found to be 95.2% e.e.

HRMS: $C_{29}H_{33}ClN_4O_5S$ ($M^+$) Cal'd 584.1860 Found 584.1873

Example 67 Synthesis of 4-(6-chloronaphthalen-2-ylsulfonyl)-6-isopropoxycarbonyl-1-[1-(4-pyridyl)piperidin-4-ylmethyl]piperazin-2-one

Using a portion (16 mg) of the compound obtained in ⟨step 6⟩ of Example 39, synthesis was performed as in Example 65 to yield the titled compound (9.5 mg). HRMS: $C_{29}H_{33}ClN_4O_5S$ ($M^+$) Cal'd 584.1860 Found 584.1883

Example 68 Synthesis of (R)-4-(6-chloronaphthalen-2-ylsulfonyl)-6-isopropoxycarbonyl-1-[1-(4-pyridyl)piperidin-4-ylmethyl]piperazin-2-one

Using a portion (200 mg) of the compound obtained in ⟨step 4⟩ of Example 60, synthesis was performed as

in Example 66 to yield the titled compound (147 mg). The optical purity of this compound was measured by HPLC [CHIRALPACK™ AD (DAICEL CHEMICAL INDUSTRIES, LTD.); hexane:isopropanol:diethylamine = 60:40:0.4] and it was found to be 96.4% e.e.

HRMS: $C_{29}H_{33}ClN_4O_5S$ ($M^+$) Cal'd 584.1860 Found 584.1865

Example 69 Synthesis of 6-t-butoxycarbonyl-4-(6-chloronaphthalen-2-ylsulfonyl)-1-[1-(4-pyridyl)piperidin-4-ylmethyl]piperazin-2-one

Conc. hydrochloric acid (0.2 ml) was added to a solution in acetic acid (0.2 ml) of the compound (20 mg) obtained in 〈step 6〉of Example 39 and the mixture was heated under reflux for 4 hours. The reaction mixture was concentrated under reduced pressure and water was added to the resulting residue, followed by further concentrating under reduced pressure. The residue was dissolved in pyridine (0.2 ml) and 4-methylbenzenesulfonyl chloride (34 mg) and then t-butanol (34 μl) were added to the solution, which was stirred at 60°C for 1 hour. Water was added to the reaction mixture and extraction with methylene chloride was conducted. The methylene chloride layer was washed with saturated sodium chloride, dried with anhydrous sodium sulfate, and the solvent was distilled off under reduced pressure. The residue was purified by silica gel column chromatography [Chromatorex NH™] (eluting solvents; methylene chloride:methanol = 98:2) to yield the titled compound (14 mg).

Example 70 Synthesis of 4-(6-chloronaphthalen-2-ylsulfonyl)-6,6-dimethyl-1-[1-(4-pyridyl)piperidin-4-ylmethyl]piperazin-2-one

〈Step 1〉Synthesis of 2-amino-1-[N-(6-chloronaphthalen-2-ylsulfonyl)]amino-2-methylpropane

To a solution in anhydrous methylene chloride (40 ml) of 1,2-diamino-2-methylpropane (0.44 ml) and triethylamine (0.80 ml), a solution of 6-chloronaphthalen-2-ylsulfonyl chloride (1.0 g) in anhydrous methylene chloride (20 ml) was added dropwise under cooling with ice. The mixture was stirred for 45 minutes under cooling with ice, then for 1 hour at room temperature. Thereafter, the reaction mixture was cooled with ice, water was added, and extraction with methylene chloride was conducted. The methylene chloride layer was washed with saturated sodium chloride, dried with anhydrous sodium sulfate, and the solvent was distilled off under reduced pressure. The residue was purified by silica gel column chromatography (eluting solvents; methylene chloride:methanol = 90:10) to give the titled compound (1.09 g).

NMR spectrum (*$CDCl_3$ ) δ ppm: 8. 39 (1H, s) , 7. 96 (1H, d d, J=2, 9Hz), 7. 89~7. 79 (3H, m), 7. 51 (1H, dd, J=2, 9H z) , 2. 93 (2H, s) , 1. 27 (6H, s)

〈Step 2〉Synthesis of 2-(t-butoxycarbonyl)amino-1-[N-(6-chloronaphthalen-2-ylsulfonyl)]amino-2-methylpropane

To a solution in dioxane (7 ml) and water (3.5 ml) of the compound (1.09 g) obtained in step 1, 1 N sodium hydroxide solution (3.5 ml) and then di-t-butyl dicarbonate (0.84 g) were added and the resulting mixture was stirred at 60°C for 1.5 hours. The reaction mixture was extracted with ethyl acetate. The ethyl acetate layer was washed with saturated sodium chloride, dried with anhydrous sodium sulfate, and the solvent was distilled off under reduced pressure. The residue was purified by silica gel column chromatography (eluting solvents; hexane:ethyl acetate = 67:33) to give the titled compound (1.32 g).

NMR spectrum (*$CDCl_3$ ) δ ppm: 8. 39 (1H, s) , 7. 92~7. 84 (4H, m) , 7. 55 (1H, dd, J=2, 9Hz) , 6. 04~5. 74 (1H, br), 4. 56~4. 45 (1H, brs), 3. 11 (2H, d, J=6Hz) , 1. 3 8 (9H, s) , 1. 25 (6H, s)

〈Step 3〉Synthesis of 2-(t-butoxycarbonyl)amino-1-[N-(6-chloronaphthalen-2-ylsulfonyl)-N-ethoxycarbonylmethyl]amino-2-methylpropane

Potassium carbonate (0.60 g) was added to a solution in dimethylformamide (25 ml) of a portion (1.2 g) of the compound obtained in step 2. Ethyl bromoacetate (0.5 ml) was also added under cooling with ice. After stirring at room temperature for 4 hours, water was added to the reaction mixture, which was then subjected to extraction with ethyl acetate. The ethyl acetate layer was washed with saturated sodium chloride, dried with anhydrous sodium sulfate, and the solvent was distilled off under reduced pressure. The residue was purified by silica gel column chromatography (eluting solvents; hexane:ethyl acetate = 80:20) to give the titled compound (1.24 g).

NMR spectrum (*$CDCl_3$ ) δ ppm: 8. 34 (1H, s) , 7. 94~7. 86 (3H, m) , 7. 80 (1H, dd, J=2, 9Hz), 7. 56 (1H, dd, J=2, 9H z) , 4. 74~4. 60 (1H, br) , 4. 12~4. 01 (2H, m) , 3. 97 (2H, s) , 3. 57 (2H, s) , 1. 38 (6H, s) , 1. 37 (9H, s) , 1. 24~1. 15 (3H, m)

〈Step 4〉Synthesis of 2-amino-1-[N-(6-chloronaphthalen-2-ylsulfonyl)-N-ethoxycarbonylmethyl]amino-2-methylpropane hydrochloride

A portion (0.10 g) of the compound obtained in step 3 was suspended in 20% HCl-ethanol (2 ml) and the suspension was stirred at room temperature for 2 hours. The reaction mixture was concentrated under reduced pressure and the residue was crystallized with ether to give the titled compound (76 mg).

NMR spectrum (*DMSO-$d_6$ ) δ ppm: 8. 59 (1H, s) , 8. 28~8. 2 1 (2H, m), 8. 18~8. 04 (4H, m) , 7. 91 (1H, dd, J=2, 9Hz), 7. 77~7. 70 (1H, m) , 4. 23 (2H, s) , 3. 77 (2H, q, J=7Hz). 3. 52 (2H, s) , 1. 31(6H, s), 0. 94(3H, t, J=7Hz)

〈Step 5〉Synthesis of 4-(6-chloronaphthalen-2-ylsulfonyl)-6,6-dimethyl-1-[1-(4-pyridyl)piperidin-4-ylmethyl]piperazin-2-one

Using 1-(4-pyridyl)piperidin-4-yl methanol (39 mg), the method of 〈step 1〉in Example 3 was repeated to yield a crude product. The product was suspended in anhydrous methylene chloride (3 ml). To the suspension, a portion (70 mg) of the compound obtained in step 4 and then acetic acid (0.02 ml) were added. The reaction mixture was stirred at room temperature for 1.5 hours in an argon atmosphere; then, sodium triacetoxyborohydride (68 mg) was added and the mixture was stirred overnight at room temperature. Saturated sodium hydrogencarbonate was added to the reaction mixture, which was adjusted to pH 9 with 1 N sodium hydroxide solution and subjected to extraction with methylene chloride. The methylene chloride layer was washed with water and saturated sodium chloride, dried with anhydrous sodium sulfate, and the solvent was distilled off under reduced pressure. The residue was purified by silica gel column chromatography (eluting solvents: methylene chloride:methanol = 98:2 - 90:10) to yield the titled compound (8.2 mg).

HRMS: $C_{27}H_{31}ClN_4O_3S$ ($M^+$) Cal'd 526.1805 Found 526.1754

Example 71 Synthesis of (R)-4-[(E)-4-chlorostyrylsulfonyl]-6-methoxymethyl-1-[1-(4-pyridyl)piperidin-4-ylmethyl]piperazin-2-one

The titled compound was obtained by repeating the method of Example 60, except that 6-chloronaphthalen-2-ylsulfonyl chloride used in 〈step 4〉of Example 60 was replaced by (E)-4-chlorostyrylsulfonyl chloride.

HRMS: $C_{25}H_{31}ClN_4O_4S$ ($M^+$) Cal'd 518.1754 Found 518.1785

Example 72 Synthesis of 1-[(E)-4-chlorostyrylsulfonyl]-4-[1-(4-pyridyl)piperidin-4-ylmethyl]piperazine methanesulfonate

Methanesulfonic acid (10.4 mg) was added to a solution in methanol (1 ml) of the compound (50 mg) obtained in 〈step 4〉of Example 3. The solvent was distilled off under reduced pressure to yield the titled compound (60 mg).

Example 73 Synthesis of 4-[(E)-4-chlorostyrylsulfonyl)-1-[1-(4-pyridyl)piperidin-4-ylmethyl]piperazin-2-one methanesulfonate

Using a portion (42 mg) of the compound obtained in 〈step 5〉of Example 23, the method of synthesis in Example 72 was repeated to yield the titled compound (50 mg).

Example 74 Synthesis of 4-(6-chloronaphthalen-2-ylsulfonyl)-1-[1-(4-pyridyl)piperidin-4-ylmethyl]piperazin-2-one methanesulfonate

Using a portion (57 mg) of the compound obtained in Example 25, the method of synthesis in Example 72 was repeated to yield the titled compound (66 mg).

Example 75 Synthesis of 4-(6-chloronaphthalen-2-ylsulfonyl)-6-ethoxycarbonyl-1-[1-(4-pyridyl)piperidin-4-ylmethyl]piperazin-2-one methanesulfonate

Using a portion (31 mg) of the compound obtained in ⟨step 6⟩ of Example 39, the method of synthesis in Example 72 was repeated to yield the titled compound (37 mg).

Example 76 Synthesis of 6-carboxy-4-(6-chloronaphthalen-2-ylsulfonyl)-1-[1-(4-pyridyl)piperidin-4-ylmethyl]piperazin-2-one methanesulfonate

Using the compound (81 mg) obtained in Example 40, the method of synthesis in Example 72 was repeated to yield the titled compound (86 mg).

Example 77 Synthesis of 4-(6-chloronaphthalen-2-ylsulfonyl)-6-hydroxymethyl-1-[1-(4-pyridyl)piperidin-4-ylmethyl]piperazin-2-one methanesulfonate

Using a portion (15.7 mg) of the compound obtained in Example 41, the method of synthesis in Example 72 was repeated to yield the titled compound (17.4 mg).

Example 78 Synthesis of 4-(6-chloronaphthalen-2-ylsulfonyl)-6-methoxymethyl-1-[1-(4-pyridyl)piperidin-4-ylmethyl]piperazin-2-one methanesulfonate

Using the compound (22 mg) obtained in Example 42, the method of synthesis in Example 72 was repeated to yield the titled compound (22 mg).

Example 79 Synthesis of 6-acetoxymethyl-4-(6-chloronaphthalen-2-ylsulfonyl)-1-[1-(4-pyridyl)piperidin-4-ylmethyl]piperazin-2-one methanesulfonate

Using the compound (53 mg) obtained in Example 43, the method of synthesis in Example 71 was repeated to yield the titled compound (53 mg).

Example 80 Synthesis of 4-[(E)-4-chlorostyrylsulfonyl]-6-ethoxycarbonyl-1-[1-(4-pyridyl)piperidin-4-ylmethyl]piperazin-2-one methanesulfonate

Using the compound (50 mg) obtained in Example 44, the method of synthesis in Example 71 was repeated to yield the titled compound (66.2 mg).

Example 81 Synthesis of 6-carboxy-4-[(E)-4-chlorostyrylsulfonyl]-1-[1-(4-pyridyl)piperidin-4-ylmethyl]piperazin-2-one methanesulfonate

Using the compound (6.5 mg) obtained in Example 45, the method of synthesis in Example 72 was repeated to yield the titled compound (9.1 mg).

Example 82 Synthesis of 4-(6-chloronaphthalen-2-ylsulfonyl)-6-morpholinocarbonyl-1-[1-(4-pyridyl)piperidin-4-ylmethyl]piperazin-2-one methanesulfonate

Using a portion (87.5 mg) of the compound obtained in Example 48, the method of synthesis in Example 72 was repeated to yield the titled compound (101 mg).

Example 83 Synthesis of 6-aminomethyl-4-[6-chloronaphthalen-2-ylsulfonyl]-1-[1-(4-pyridyl)piperidin-4-ylmethyl]piperazin-2-one methanesulfonate

Using a portion (31.2 mg) of the compound obtained in ⟨step 2⟩ of Example 52, the method of synthesis in Example 72 was repeated to yield the titled compound (34.5 mg).

Example 84 Synthesis of 4-(6-chloronaphthalen-2-ylsulfonyl)-6-morpholinomethyl-1-[1-(4-pyridyl)piperidin-4-ylmethyl]piperazin-2-one methanesulfonate

Using a portion (35 mg) of the compound obtained in ⟨step 2⟩ of Example 53, the method of synthesis in

Example 72 was repeated to yield the titled compound (35 mg).

Example 85 Synthesis of 4-(6-chloronaphthalen-2-ylsulfonyl)-6-dimethyaminomethyl-1-[1-(4-pyridyl)piperidin-4-ylmethyl]piperazin-2-one methanesulfonate

Using the compound (39 mg) obtained in Example 54, the method of synthesis in Example 72 was repeated to yield the titled compound (48 mg).

Example 86 Synthesis of 6-acetamidomethyl-4-[6-chloronaphthalen-2-ylsulfonyl]-1-[1-(4-pyridyl)piperidin-4-ylmethyl]piperazin-2-one methanesulfonate

Using the compound (29.2 mg) obtained in Example 55, the method of synthesis in Example 72 was repeated to yield the titled compound (29.7 mg).

Example 87 Synthesis of 4-(6-chloronaphthalen-2-ylsulfonyl)-6-methanesulfonylamidomethyl-1-[1-(4-pyridyl)piperidin-4-ylmethyl]piperazin-2-one methanesulfonate

Using the compound (27.2 mg) obtained in Example 56, the method of synthesis in Example 72 was repeated to yield the titled compound (27.1 mg).

Example 88 Synthesis of 4-(6-chloronaphthalen-2-ylsulfonyl)-6-(4-hydroxypiperidin-1-ylmethyl)-1-[1-(4-pyridyl)piperidin-4-ylmethyl]piperazin-2-one methanesulfonate

Using a portion (55.6 mg) of the compound obtained in 〈step 3〉of Example 57, the method of synthesis in Example 72 was repeated to yield the titled compound (66. 8 mg).

Example 89 Synthesis of (R)-4-(6-chloronaphthalen-2-ylsulfonyl)-6-ethoxycarbonyl-1-[1-(4-pyridyl)piperidin-4-ylmethyl]piperazin-2-one methanesulfonate

Using a portion (53.2 mg) of the compound obtained in 〈step 4〉of Example 60, the method of synthesis in Example 72 was repeated to yield the titled compound (58.5 mg).

Example 90 Synthesis of (S)-4-(6-chloronaphthalen-2-ylsulfonyl)-6-ethoxycarbonyl-1-[1-(4-pyridyl)piperidin-4-ylmethyl]piperazin-2-one methanesulfonate

Using the compound (50.8 mg) obtained in Example 61, the method of synthesis in Example 72 was repeated to yield the titled compound (55.5 mg).

Example 91 Synthesis of (R)-4-(6-chloronaphthalen-2-ylsulfonyl)-6-methoxymethyl-1-[1-(4-pyridyl)piperidin-4-ylmethyl]piperazin-2-one methanesulfonate

Using the compound (0.50 mg) obtained in Example 62, the method of synthesis in Example 72 was repeated to yield the titled compound (0.55 g).

Example 92 Synthesis of (S)-4-(6-chloronaphthalen-2-ylsulfonyl)-6-methoxymethyl-1-[1-(4-pyridyl)piperidin-4-ylmethyl]piperazin-2-one methanesulfonate

Using the compound (0.50 g) obtained in Example 63, the method of synthesis in Example 72 was repeated to yield the titled compound (0.57 g).

Example 93 Synthesis of (R)-6-carboxy-4-(6-chloronaphthalen-2-ylsulfonyl)-1-[1-(4-pyridyl)piperidin-4-ylmethyl]piperazin-2-one methanesulfonate

Using the free base (2.0 mg) from the compound obtained in Example 64, the method of synthesis in Example 72 was repeated to yield the titled compound (2.4 mg).

Example 94 Synthesis of (R)-4-(6-chloronaphthalen-2-ylsulfonyl)-6-n-propoxycarbonyl-1-[1-(4 -pyridyl)piperidin-4-ylmethyl]piperazin-2-one methanesulfonate

Using a portion (50 mg) of the compound obtained in Example 66, the method of synthesis in Example 71 was repeated to yield the titled compound (58 mg).

Example 95 Synthesis of (R)-4-(6-chloronaphthalen-2-ylsulfonyl)-6-isopropoxycarbonyl-1-[1-(4-pyridyl)piperidin-4-ylmethyl]piperazin-2-one methanesulfonate

Using a portion (50 mg) of the compound obtained in Example 68, the method of synthesis in Example 71 was repeated to yield the titled compound (58 mg).

The structural formulae of the compounds of the present invention are shown in accompanying Figs. 1 - 39. The NMR data for the compounds synthesized in the Examples are given in accompanying Figs. 40 - 50.

Advantages of the Invention

The compounds of the present invention are a specific FXa inhibitor and have a potent anticoagulation effect. They are, therefore, useful as anticoagulants or as preventives or therapeutics of diseases caused by thrombus or embolus. Examples of such diseases for which the compounds of the present invention are indicated include diseases from ischemic cerebrovascular disorders such as cerebral thrombosis, cerebral infarction, cerebral embolism and transient cerebral ischemic attacks (TIA), diseases associated with ischemic heart diseases such as acute or chronic myocardial infarction, unstable angina pectoris and coronary thrombolysis, diseases from pulmonary infarction, pulmonary embolism and other conditions of pulmonary angiopathy, and diseases from various cases of angiopathy including peripheral arterial obstruction, deep venous thrombosis, disseminated intravascular coagulation (DIC), thrombosis after artificial blood vessel or heart valve replacement, reocclusion and restenosis following coronary artery bypass surgery, reocclusion and restenosis on or after PTCA, and thrombosis on extracorporeal circulation of blood. The compounds of the present invention are also useful as preventives or therapeutics of infections with influenza virus.

**Claims**

**1.** A compound represented by the following formula (I) or a salt thereof:

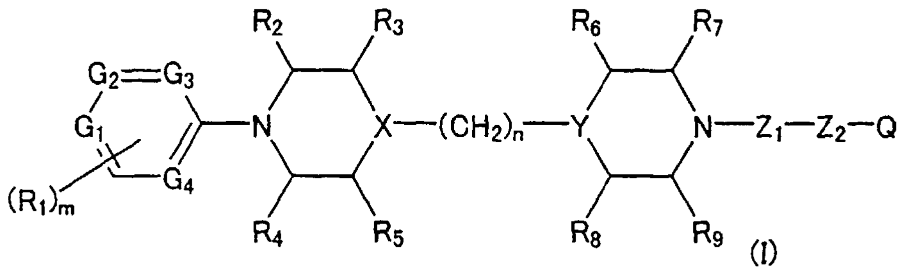

(I)

(wherein $G_1$, $G_2$, $G_3$ and $G_4$ are independently CH or N;

X and Y are independently CH or N;

$Z_1$ is a group represented by the formula $-SO_2-$ or $-CH_2-$;

$Z_2$ is a single bond, a lower alkylene group, a lower alkenylene group or a lower alkynylene group;

Q is an optionally substituted aryl or an optionally substituted heteroaryl group;

$R_1$ is either any substituentselected from group A (a hydrogen atom, a halogen atom, a trifluoromethyl group, a trifluoromethoxy group, a carboxyl group, a carbamoyl group, an amino group, a cyano group, a nitro group, a lower alkanoyl group, a lower alkoxy group, a lower alkoxycarbonyl group, a mono- or di-substituted lower alkylamino group, a cyclic amino group, a lower alkanoylamino group, a phenyl group, a phenoxy group, a benzyloxy group, a benzoyl group, a mercapto group, a lower alkylthio group, a lower alkylthiocarbonyl group, a hydroxyl group or a mono- or di-substituted lower alkylaminocarbonyl group), or an oxygen atom that forms a N-oxide group with N in any one of $G_1$ - $G_4$, or a lower alkyl group, a lower alkoxy group or a lower alkenyl group that may be substituted with a desired number of substituents of group A;

each of $R_2$, $R_3$, $R_4$, $R_5$, $R_6$, $R_7$, $R_8$ and $R_9$ forms a carbonyl group when combined with the carbon atom on the

ring to which they are bound, or they are each a hydrogen atom, a carboxyl group, a lower alkylcarbonyl group, a lower alkoxycarbonyl group, a lower alkoxycarbonylalkylcarbonyl group, an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperazin-1-ylcarbonyl group that may be substituted by a methyl group in 4-position, a piperidin-1-ylcarbonyl group that may be substituted by a methyl group or a hydroxyl group in 4-position, an N-phenylcarbamoyl group or a group represented by the formula $-CONH(CH_2)_pS(O)_qR_{10}$ or $-CONH(CH_2)_rNR_{11}R_{12}$, or a lower alkyl group that may be substituted by $R_{15}$;

each of $R_{10}$, $R_{11}$ and $R_{12}$ independently represents a hydrogen atom, a lower alkyl group, a phenyl group or a lower alkylphenyl group;

$R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group;

provided that $R_6$, when combined with the carbon atom to which it is bound, may represent $R_{6a}$-C-$R_{6b}$, wherein either $R_{6a}$ or $R_{6b}$ is a hydrogen atom and the other is the same as defined above for $R_6$ or, alternatively, each of $R_{6a}$ and $R_{6b}$ independently represents a lower alkyl group;

also provided that if any one of the substituents $R_2$ - $R_9$ includes cyclic group, such cyclic group may be substituted by one or two lower alkyl groups;

m and n are independently an integer of 0 - 3, p is an integer of 0 - 4, q is an integer of 0 - 2, and r is an integer of 1 - 4;

with the proviso that when X and Y are both N, n is 2 or 3 and $Z_1$ is $-CH_2-$ those compounds of formula (I) in which $R_6$ and $R_8$ in pair or $R_7$ and $R_9$ in pair are both carbonyl groups are excluded).

2. The compound or salt thereof according to claim 1, wherein the optionally substituted aryl or heteroaryl group as Q is an aryl or heteroaryl group that may be substituted by 1 - 4 groups in any combinations that are selected from among substituents of either group B [a halogen atom, a trifluoromethyl group, a trifluoromethoxy group, a trifluoromethanesulfonyl group, a carboxyl group, a carbamoyl group, an amino group, a cyano group, a nitro group, a lower alkanoyl group, a lower alkoxyl group, a lower alkoxycarbonyl group, a mono- or di-substituted lower alkylamino group, a lower alkanoylamino group, a cyclic amino group, a mercapto group, a lower alkylthio group, a lower alkylthiocarbonyl group, a lower alkylsulfonyl group, a lower alkylsulfinyl group, a hydroxyl group or a mono- or di-substituted lower alkylaminocarbonyl group, an amidino group which is optionally substituted with sulfamoyl or carbamoyl group, the formula $-NHCR_{13}-NHR_{14}$ (wherein $R_{13}$ is an optionally cyano-substituted imino group or a group $=CHNO_2$; $R_{14}$ is a hydrogen atom or a methyl group), a phenyl group, a phenoxy group, a heteroaryl group, a heteroaryloxy group, or a group represented by phenyl-S(O)t or heteroaryl-S(O)t (wherein t is an integer of 0 - 2), the heteroaryl group of group B is a 5- or 6-membered aromatic monocyclic group containing not more than four oxygen atoms, sulfur atoms or nitrogen atoms, provided that all aromatic rings of group B may be mono-, di-, or tri-substituted by any substituent of group C (a halogen atom, a trifluoromethyl group, a cyano group, a hydroxyl group, an amino group, a mono- or di-substituted lower alkylamino group, a cyclic amino group, a nitro group, a carboxyl group, a mono-or di-substituted lower alkylaminocarbonyl group, a lower alkyl group, a lower alkoxy group or a lower alkoxycarbonyl group)] or a lower alkyl group that may be substituted by a desired number of substituents of group B.

3. The compound or salt thereof according to claim 1 or 2, wherein at least one of $G_1$, $G_2$ and $G_3$ is N and $G_4$ is CH.

4. A compound represented by the following formula (II) or a salt thereof:

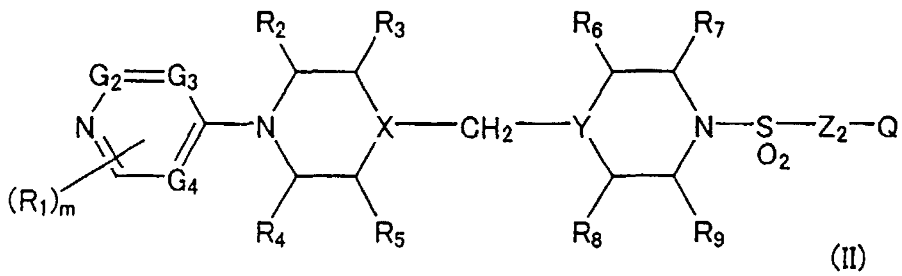

(II)

(wherein $G_2$, $G_3$, $G_4$, $R_2$ - $R_9$, $Z_2$ and Q have the same meanings as respectively defined for the formula (I) in claim 1, and m is 0 - 2).

**5.** A compound represented by the following general formula (II') or a salt thereof:

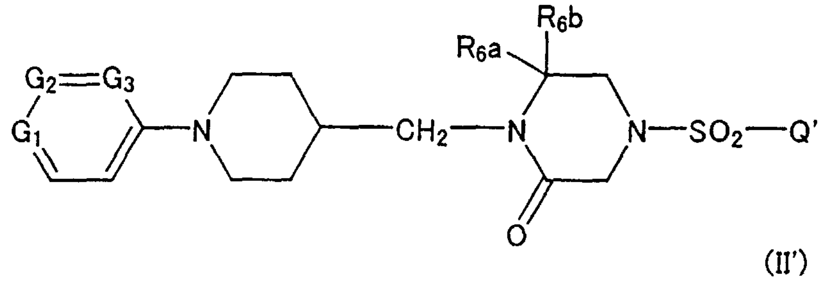

(II')

(wherein $G_1$, $G_2$ and $G_3$ are independently CH or N, provided that at least one of them is N;

one of $R_{6a}$ and $R_{6b}$ is a hydrogen atom and the other is

1) a group selected from among a carboxyl group, a lower alkylcarbonyl group, a lower alkoxycarbonyl group and a lower alkoxycarbonylalkylcarbonyl group;
2) a group selected from among an optionally mono- or di-lower alkyl substituted carbamoyl group, a lower alkoxycarbamoyl group, a lower alkoxycarbonylalkylcarbamoyl group, a pyrrolidin-1-ylcarbonyl group, a morpholinocarbonyl group, a piperidin-1-ylcarbonyl group which may be substituted by a methyl group or a hydroxyl group in 4-position, an N-phenylcarbamoyl group or a group selected from among the groups represented by the formulae $-CONH(CH_2)_pS(O)_qR_{10}$ and $-CONH(CH_2)_rNR_{11}R_{12}$ (wherein $R_{10}$, $R_{11}$ and $R_{12}$ are independently a hydrogen atom, a lower alkyl group, a phenyl group or a lower alkylphenyl group; p is an integer of 0 - 4, q is an integer of 0 - 2, and r is an integer of 1 - 4), or
3) a lower alkyl group optionally substituted by $R_{15}$; $R_{15}$ is a carboxyl group, a lower alkoxycarbonyl group, a hydroxyl group, a lower alkoxy group, a lower alkanoyloxy group, an amino group, a mono- or di-substituted lower alkylamino group, a lower alkanoylamino group, a lower alkylsulfonylamino group, a cyclic amino group or an N-hydroxyimino group;

or $R_{6a}$ and $R_{6b}$ are both a lower alkyl group;
Q' is an aryl group optionally substituted by a group having any 1 - 4 halogen atoms or an aryl lower alkenylene group which may be similarly substituted).

**6.** A compound of the formula (II"):

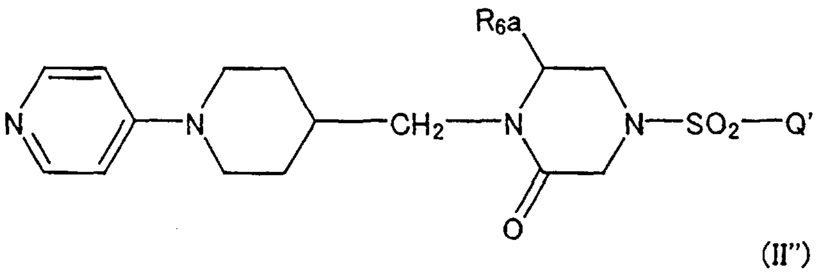

(II")

(wherein $R_{6a}$ and Q' have the same definitions as given for the substituent $R_{6a}$ and Q' in the formula (II')) or a salt thereof.

**7.** The compound or salt thereof according to claim 6, wherein $R_{6a}$ is a carboxyl group, a lower alkoxycarbonyl group or a lower alkyl group that may be substituted by $R_{15}$; $R_{15}$ is a carboxyl group, a lower alkoxycarbonyl group, a hydroxyl group, a lower alkoxy group or a lower alkanoyloxy group.

**8.** A compound selected from the following list or a salt thereof:

1-[1-((E)-4-chlorostyrylsulfonyl)piperidin-4-yl]-4-(4-pyridyl)piperazine;

1-[1-((E)-4-chlorostyrylsulfonyl)piperidin-4-ylmethyl]-4-(4-pyridyl)piperazine;
1-[(E)-4-chlorostyrylsulfonyl]-4-[1-(4-pyridyl)piperidin-4-ylmethyl]piperazine;
4-(naphthalene-2-ylsulfonyl)-1-[1-(4-pyridyl)piperidin-4-ylmethyl]piperazine;
4-(6-chloronaphthalen-2-ylsulfonyl)-1-[1-(4-pyridyl)piperidin-4-ylmethyl]piperazine;
4-(6-bromonaphthalen-2-ylsulfonyl)-1-[1-(4-pyridyl)piperidin-4-ylmethyl]piperazine;
4-(benzo[b]thiophen-2-ylsulfonyl)-1-[1-(4-pyridyl)piperidin-4-yl-methyl]piperazine;
4-(5-fluorobenzo[b]thiophen-2-ylsulfonyl)-1-[1-(4-pyridyl)piperidin-4-ylmethyl]piperazine;
4-(6-chlorobenzo[b]thiophen-2-ylsulfonyl)-1-[1-(4-pyridyl)piperidin-4-ylmethyl]piperazine;
4-(4-methoxybenzo[b]thiophen-2-ylsulfonyl)-1-[1-(4-pyridyl)piperidin-4-ylmethyl]piperazine;
4-(6-methoxybenzo[b]thiophen-2-ylsulfonyl)-1-[1-(4-pyridyl)piperidin-4-ylmethyl]piperazine;
4-[3-(ethoxycarbonylmethyl)benzo[b]thiophen-2-ylsulfonyl]-1-[1-(4-pyridyl)piperidin-4-ylmethyl]piperazine;
1-[1-(4-pyridyl)piperidin-4-ylmethyl]-4-[3-(trifluoromethyl)benzo[b]thiophen-2-ylsulfonyl]piperazine;
4-(3-nitrobenzo[b]thiophen-2-ylsulfonyl)-1-[1-(4-pyridyl)piperidin-4-ylmethyl]piperazine;
4-(benzo[b]furan-2-ylsulfonyl)-1-[1-(4-pyridyl)piperidin-4-ylmethyl]piperazine;
4-(5-chlorobenzo[b]furan-2-ylsulfonyl)-1-[1-(4-pyridyl)piperidin-4-ylmethyl]piperazine;
4-(2-methylbenzothiazol-6-ylsulfonyl)-1-[1-(4-pyridyl)piperidin-4-ylmethyl]piperazine;
4-(4-phenylbenzenesulfonyl)-1-[1-(4-pyridyl)piperidin-4-ylmethyl]piperazine;
4-(5-carboxy-2-chlorobenzenesulfonyl)-1-[1-(4-pyridyl)piperidin-4-ylmethyl]piperazine;
4-[5-(carboxymethyl)-2-chlorobenzenesulfonyl]-1-[1-(4-pyridyl)piperidin-4-ylmethyl]piperazine;
4-(5-acetamidonaphthalen-2-ylsulfonyl)-1-[1-(4-pyridyl)piperidin-4-ylmethyl]piperazine;
4-(5-aminonaphthalen-2-ylsulfonyl)-1-[1-(4-pyridyl)piperidin-4-ylmethyl]piperazine;
4-[(E)-4-chlorostyrylsulfonyl]-1-[1-(4-pyridyl)piperidin-4-ylmethyl]piperazin-2-one;
4-(5-aminonaphthalen-2-ylsulfonyl)-1-[1-(4-pyridyl)piperidin-4-ylmethyl]piperazine;
4-[(E)-4-chlorostyrylsulfonyl]-1-[1-(4-pyridyl)piperidin-4-ylmethyl]piperazin-2-one;
4-(6-bromonaphthalen-2-ylsulfonyl)-1-[1-(4-pyridyl)piperidin-4-ylmethyl]piperazin-2-one;
4-(6-chloronaphthalen-2-ylsulfonyl)-1-[1-(4-pyridyl)piperidin-4-ylmethyl]piperazin-2-one;
4-(naphthalen-2-ylsulfonyl)-1-[1-(4-pyridyl)piperidin-4-ylmethyl]piperazin-2-one;
4-(6-methylnaphthalen-2-ylsulfonyl)-1-[1-(4-pyriyl)piperidin-4-ylmethyl]piperazin-2-one;
4-(6-cyanonaphthalen-2-ylsulfonyl)-1-[1-(4-pyridyl)piperidin-4-ylmethyl]piperazin-2-one;
4-(6-hydroxynaphthalen-2-ylsulfonyl)-1-[1-(4-pyridyl)piperidin-4-ylmethyl]piperazin-2-one;
4-(1-fluoronaphthalen-2-ylsulfonyl)-1-[1-(4-pyridyl)piperidin-4-ylmethyl]piperazin-2-one;
4-(6-chloronaphthalen-2-ylsulfonyl)-2-ethoxycarbonyl-1-[1-(4-pyridyl)piperidin-4-ylmethyl]piperazine;
4-(6-chloronaphthalen-2-ylsulfonyl)-2-hydroxymethyl-1-[1-(4-pyridyl)piperidin-4-ylmethyl]piperazine;
2-carboxy-4-(6-chloronaphthalen-2-ylsulfonyl)-1-[1-(4-pyridyl)piperidin-4-ylmethyl]piperazine;
4-(6-chloronaphthalen-2-ylsulfonyl)-2-[(2-ethoxycarbonyl)acetyl]-1-[1-(4-pyridyl)piperidin-4-ylmethyl]piperazine;
2-aminocarbonyl-4-(6-chloronaphthalen-2-ylsulfonyl)-1-[1-(4-pyridyl)piperidin-4-ylmethyl]piperazine;
4-(6-chloronaphthalen-2-ylsulfonyl)-2-[N-(ethylthioethyl)aminocarbonyl]-1-[1-(4-pyridyl)piperidin-4-ylmethyl]piperazine;
2-acetyl-4-(6-chloronaphthalen-2-ylsulfonyl)-1-[1-(4-pyridyl)piperidin-4-ylmethyl]piperazine; and
4-(6-chloronaphthalen-2-ylsulfonyl)-2-(N,N-dimethylaminocarbonyl)-1-[1-(4-pyridyl)piperidin-4-ylmethyl]piperazine.

**9.** A compound selected from the following list or a salt thereof:

4-(6-chloronaphthalen-2-ylsulfonyl)-6-ethoxycarbonyl-1-[1-(4-pyridyl)piperidin-4-ylmethyl]piperazin-2-one;
6-carboxy-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-hydroxymethyl-1-[1-(4-pyridyl)piperidin-4-ylmethyl]piperazin-2-one;
4-(6-chloronaphthalen-2-ylsulfonyl)-6-methoxymethyl-1-[1-(4-pyridyl)piperidin-4-ylmethyl]piperazin-2-one;
6-acetoxymethyl-4-(6-chloronaphthalen-2-ylsulfonyl)-1-[1-(4-pyridyl)piperidin-4-ylmethyl]piperazin-2-one;
4-[(E)-4-chlorostyrylsulfonyl]-6-ethoxycarbonyl-1-[1-(4-pyridyl)piperidin-4-ylmethyl]piperazin-2-one;
6-carboxy-4-[(E)-4-chlorostyrylsulfonyl]-1-[1-(4-pyridyl)piperidin-4-ylmethyl]piperazin-2-one;
6-aminocarbonyl-4-(6-chloronaphthalen-2-ylsulfonyl)-1-[1-(4-pyridyl)piperidin-4-ylmethyl]piperazin-2-one;
6-aldoximyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-morphohinocarbonyl-1-[1-(4-pyridyl)piperidin-4-ylmethyl]piperazin-2-one;
4-(6-chloronaphthalen-2-ylsulfonyl)-6-dimethylaminocarbonyl-1-[1-(4-pyridyl)piperidin-4-ylmethyl]piperazin-2-one;

4-(6-chloronaphthalen-2-ylsulfonyl)-6-methoxyaminocarbonyl-1-[1-(4-pyridyl)piperidin-4-ylmethyl]piperazin-2-one;
4-(6-chloronaphthalen-2-ylsulfonyl)-6-(4-hydroxypiperidinecarbonyl)-1-[1-(4-pyridyl)piperidin-4-ylmethyl]piperazin-2-one;
6-aminomethyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-morpholinomethyl-1-[1-(4-pyridyl)piperidin-4-ylmethyl]piperazin-2-one;
4-(6-chloronaphthalen-2-ylsulfonyl)-6-dimethylaminomethyl-1-[1-(4-pyridyl)piperidin-4-ylmethyl]piperazin-2-one;
6-acetamidomethyl-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-methanesulfonylamidomethyl-1-[1-(4-pyridyl)piperidin-4-ylmethyl]piperazin-2-one;
4-(6-chloronaphthalen-2-ylsulfonyl)-6-(4-hydroxypiperidinemethyl)-1-[1-(4-pyridyl)piperidin-4-ylmethyl]piperazin-2-one;
4-(6-chloronaphthalen-2-ylsulfonyl)-6-dimethyl-1-[1-(4-pyridyl)piperidin-4-ylmethyl]piperazin-2-one;
4-(2-naphthylsuhfonyl)-6-hydroxymethyl-1-[1-(4-pyridyl)piperidin-4-ylmethyl]piperazin-2-one;
6-acetoxymethyl-4-(2-naphthylsulfonyl)-1-[1-(4-pyridyl)piperidin-4-ylmethyl]piperazin-2-one;
(R)-4-(6-chloronaphthalen-2-ylsulfonyl)-6-ethoxycarbonyl-1-[1-(4-pyridyl)piperidin-4-ylmethyl]piperazin-2-one;
(S)-4-(6-chloronaphthalen-2-ylsulfonyl)-6-ethoxycarbonyl-1-[1-(4-pyridyl)piperidin-4-ylmethyl]piperazin-2-one;
(R)-4-(6-chloronaphthalen-2-ylsulfonyl)-6-methoxymethyl-1-[1-(4-pyridyl)piperidin-4-ylmethyl]piperazin-2-one;
(S)-4-(6-chloronaphthalen-2-ylsulfonyl)-6-methoxymethyl-1-[1-(4-pyridyl)piperidin-4-ylmethyl]piperazin-2-one;
(R)-6-carboxy-4-(6-chloronaphthalen-2-ylsulfonyl)-1-[1-(4-pyridyl)piperidin-4-ylmethyl]piperazin-2-one;
4-(6-chloronaphthalen-2-ylsulfonyl)-6-n-propoxycarbonyl-1-[1-(4-pyridyl)piperidin-4-ylmethyl]piperazin-2-one;
(R)-4-(6-chloronaphthalen-2-ylsulfonyl)-6-n-propoxycarbonyl-1-[1-(4-pyridyl)piperidin-4-ylmethyl]piperazin-2-one;
4-(6-chloronaphthalen-2-ylsulfonyl)-6-isopropoxycarbonyl-1-[1-(4-pyridyl)piperidin-4-ylmethyl]piperazin-2-one;
(R)-4-(6-chloronaphthalen-2-ylsulfonyl)-6-isopropoxycarbonyl-1-[1-(4-pyridyl)piperidin-4-ylmethyl]piperazin-2-one;
6-t-butoxycarbonyl-4-(6-chloronaphthalen-2-ylsulfonyl)-1-[1-(4-pyridyl piperidin-4-ylmethyl]piperazin-2-one;
4-(6-chloronaphthalen-2-ylsulfonyl)-6,6-dimethyl-1-[1-(4-pyridyl)piperidin-4-ylmethyl]piperazin-2-one; and
(R)-4-[(E)-4-chlorostyrylsulfonyl]-6-methoxymethyl-1-[1-(4-pyridyl)piperidin-4-ylmethyl]piperazin-2-one.

**10.** The compound or salt thereof according to claim 1 or 2, wherein each of $G_1$, $G_2$, $G_3$ and $G_4$ is CH.

**11.** A pharmaceutical composition containing at least one compound or salt thereof according to any one of claims 1 - 10 as an active ingredient.

**12.** The pharmaceutical composition according to claim 11, which is an inhibitor of activated coagulation factor X.

**13.** The pharmaceutical composition according to claim 11, which is an anticoagulant.

**14.** The pharmaceutical composition according to claim 11, which is a preventive and/or therapeutic agent for disease caused by thrombus or embolus.

**15.** A compound of the formula (IV)-b that may be protected with a suitable protective group or a salt thereof:

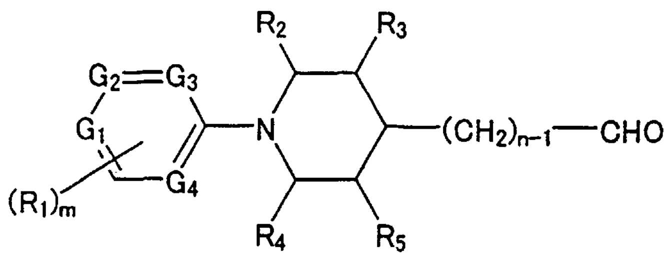

(IV) - b

(wherein $G_1$, $G_2$, $G_3$, $G_4$, $R_1$, $R_2$, $R_3$, $R_4$, $R_5$, n and m have the same meanings as respectively defined for the formula (I) in claim 1).

**16.** A compound of the formula (VI) that may be protected with a suitable protective group or a salt thereof:

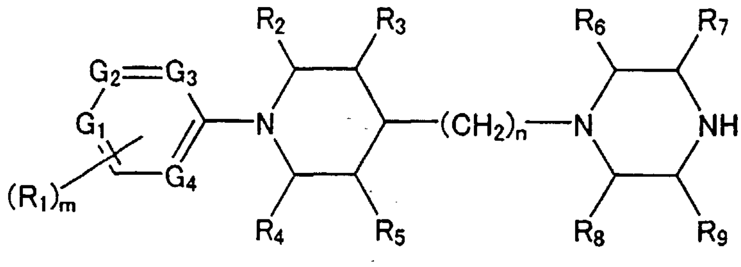

(VI)

(wherein $G_1$ - $G_4$, $R_1$ - $R_9$, m and n have the same meanings as respectively defined for the formula (I) in claim 1).

FIG.1

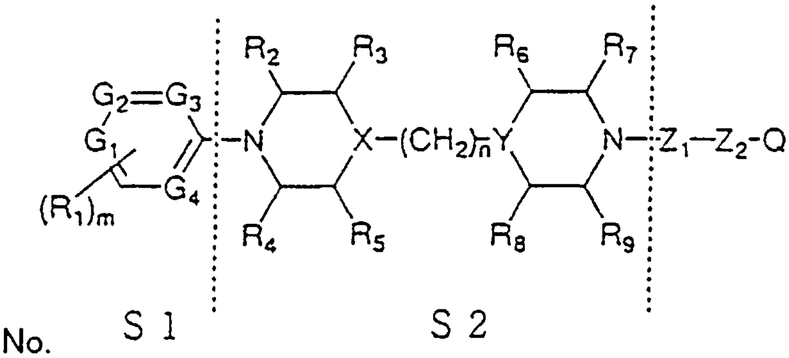

| Ex.No. | S 1 | S 2 | S 3 |
|---|---|---|---|
| 1 | 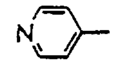 | 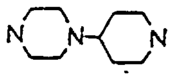 | 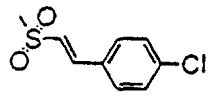 |
| 2 | 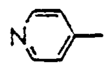 | 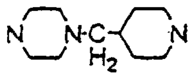 | 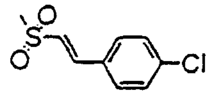 |
| 3 | 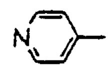 | 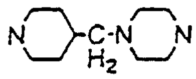 | 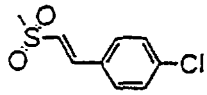 |
| 4 | 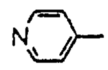 | 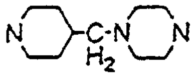 | 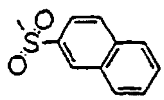 |
| 5 | 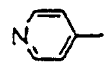 | 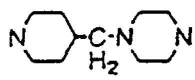 | 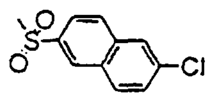 |
| 6 | 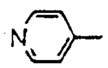 | 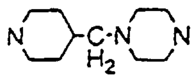 | 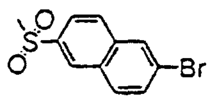 |
| 7 | 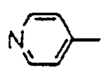 | 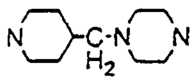 | 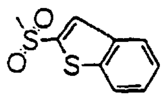 |
| 8 | 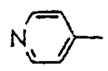 | 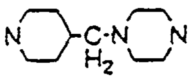 | 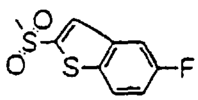 |
| 9 | 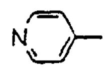 | 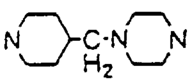 | 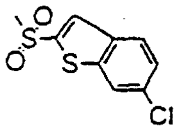 |
| 10 | 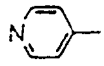 | 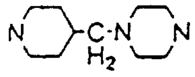 | 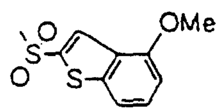 |

FIG.2

| | S 1 | S 2 | S 3 |
|---|---|---|---|
| 1 1 | 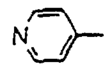 | 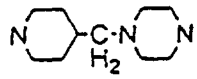 | 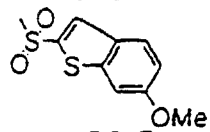 |
| 1 2 | 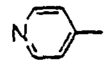 | 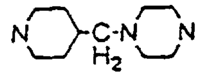 | 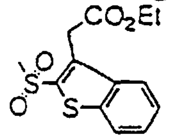 |
| 1 3 | 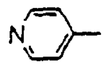 | 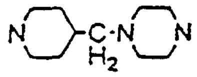 | 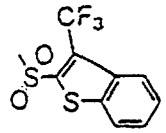 |
| 1 4 | 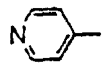 | 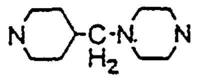 | 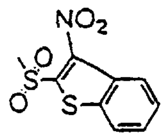 |
| 1 5 | 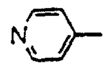 | 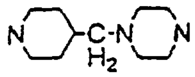 | 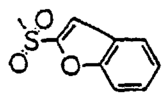 |
| 1 6 | 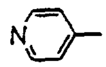 | 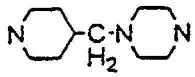 | 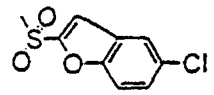 |
| 1 7 | 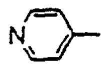 | 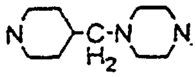 | 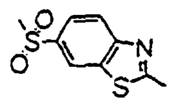 |
| 1 8 | 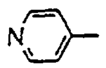 | 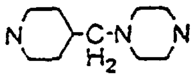 | 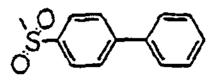 |
| 1 9 | 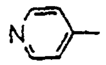 | 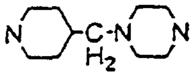 | 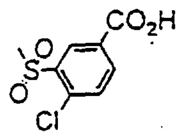 |
| 2 0 | 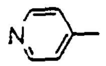 | 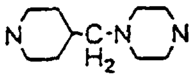 | 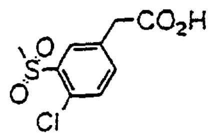 |
| 2 1 | 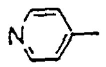 | 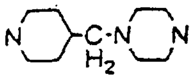 | 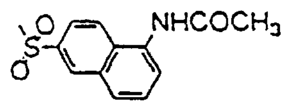 |
| 2 2 | 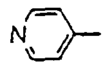 | 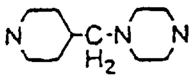 | 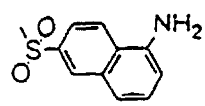 |

FIG.3

| | S 1 | S 2 | S 3 |
|---|---|---|---|
| 2 3 | 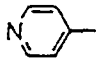 | 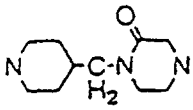 | 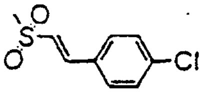 |
| 2 4 | 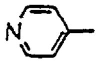 | 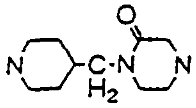 | 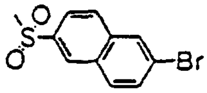 |
| 2 5 | 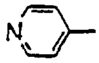 | 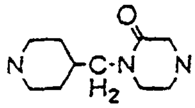 | 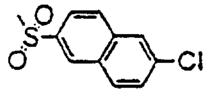 |
| 2 6 | 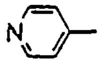 | 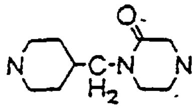 | 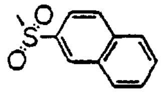 |
| 2 7 | 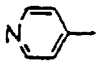 | 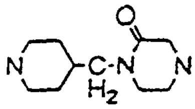 | 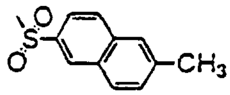 |
| 2 8 | 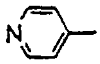 | 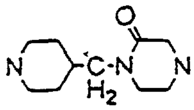 | 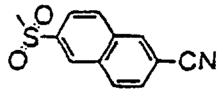 |
| 2 9 | 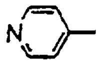 | 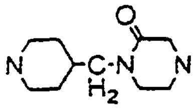 | 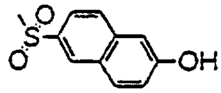 |
| 3 0 | 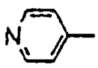 | 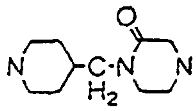 | 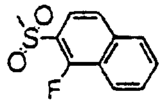 |
| 3 1 | 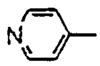 | 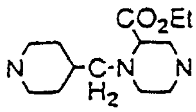 | 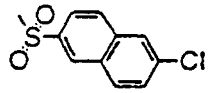 |
| 3 2 | 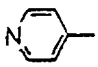 | 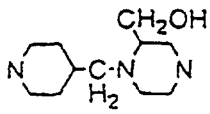 | 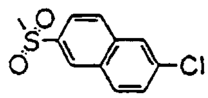 |
| 3 3 | 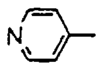 | 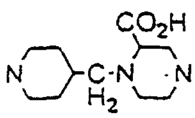 | 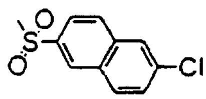 |
| 3 4 | 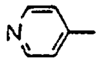 | 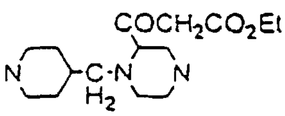 | 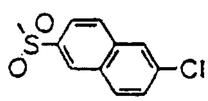 |

# FIG.4

| | S 1 | S 2 | S 3 |
|---|---|---|---|
| 3 5 | 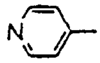 | 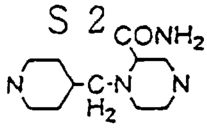 | 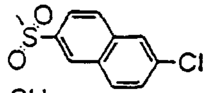 |
| 3 6 | 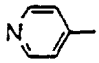 | 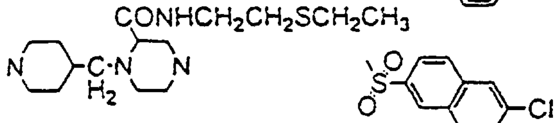 | |
| 3 7 | 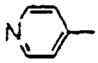 | 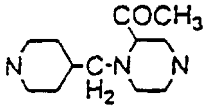 | 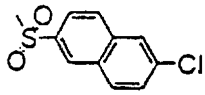 |
| 3 8 | 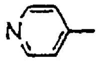 | 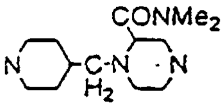 | 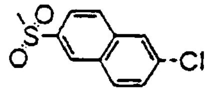 |
| 3 9 | 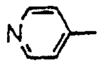 | 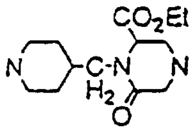 | 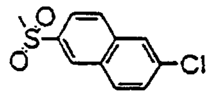 |
| 4 0 | 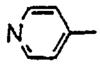 | 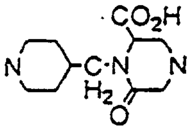 | 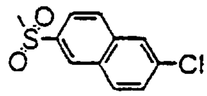 |
| 4 1 | 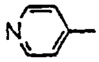 | 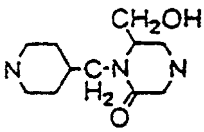 | 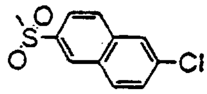 |
| 4 2 | 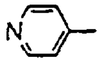 | 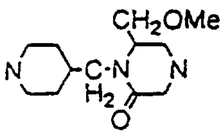 | 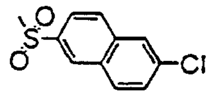 |
| 4 3 | 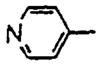 | 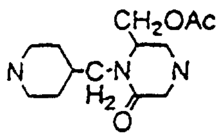 | 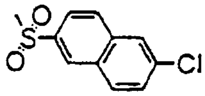 |
| 4 4 | 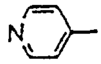 | 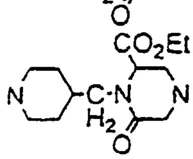 | 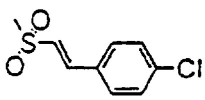 |
| 4 5 | 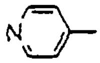 | 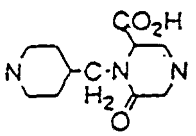 | 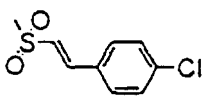 |
| 4 6 | 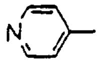 | 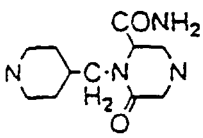 | 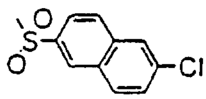 |

FIG.5

| | S 1 | S 2 | S 3 |
|---|---|---|---|
| 4 7 | 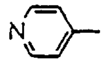 | 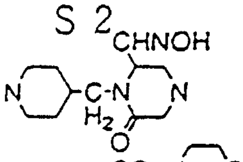 | 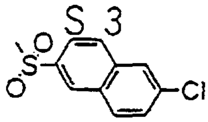 |
| 4 8 | 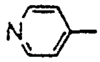 | 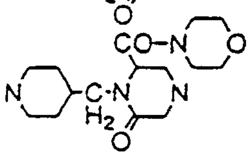 | 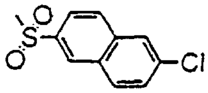 |
| 4 9 | 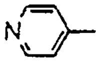 | 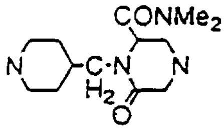 | 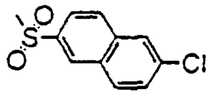 |
| 5 0 | 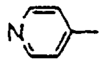 | 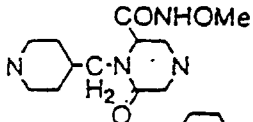 | 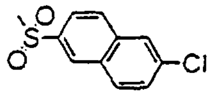 |
| 5 1 | 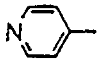 | 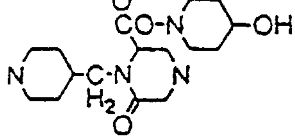 | 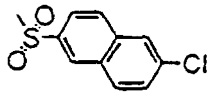 |
| 5 2 | 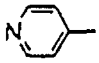 | 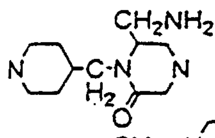 | 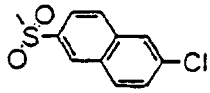 |
| 5 3 | 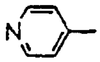 | 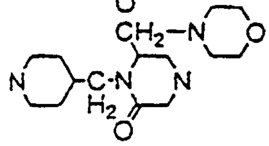 | 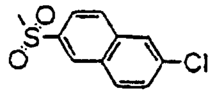 |
| 5 4 | 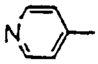 | 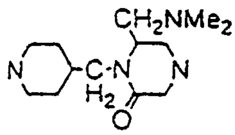 | 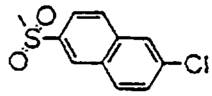 |
| 5 5 | 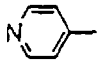 | 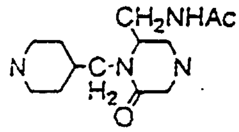 | 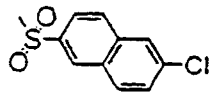 |
| 5 6 | 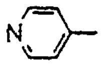 | 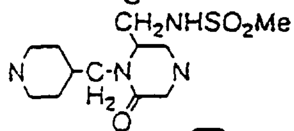 | 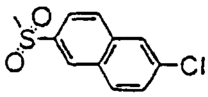 |
| 5 7 | 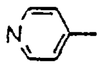 | 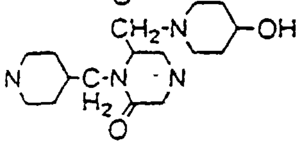 | 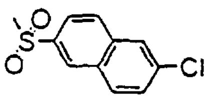 |
| 5 8 | 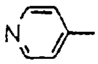 | 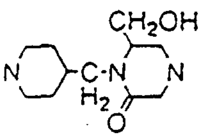 | 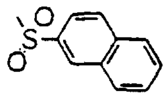 |

# FIG.6

| | S 1 | S 2 | S 3 |
|---|---|---|---|
| 59 | 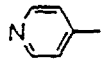 | 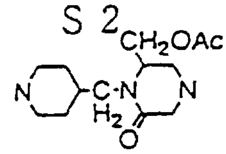 | 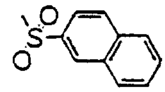 |
| 60 | 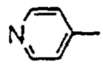 | 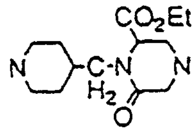 (R) | 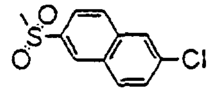 |
| 61 | 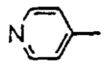 | 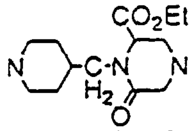 (S) | 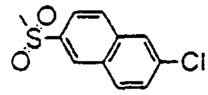 |
| 62 | 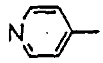 | 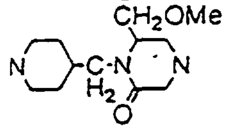 (R) | 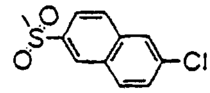 |
| 63 | 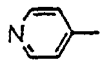 | 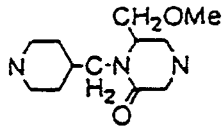 (S) | 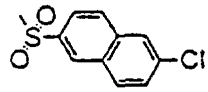 |
| 64 | 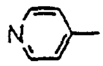 | 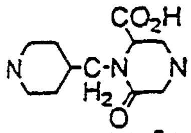 (R) | 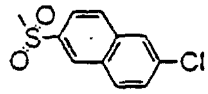 |
| 65 | 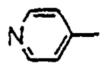 | 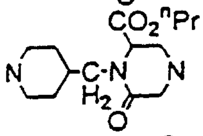 | 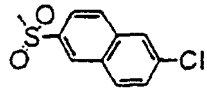 |
| 66 | 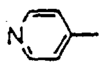 | 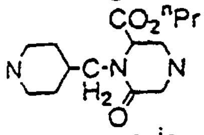 (R) | 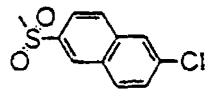 |
| 67 | 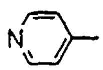 | 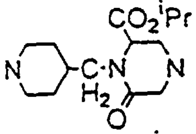 | 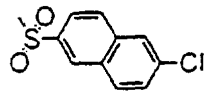 |
| 68 | 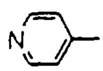 | 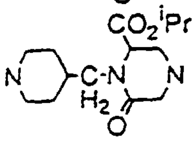 (R) | 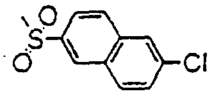 |
| 69 | 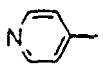 | 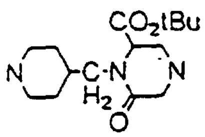 | 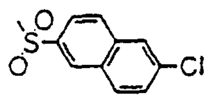 |
| 70 | 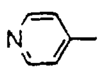 | 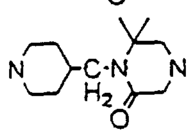 | 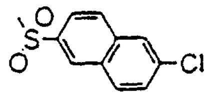 |

# FIG.7

| | S 1 | S 2 | S 3 |
|---|---|---|---|
| 71 | 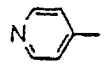 | 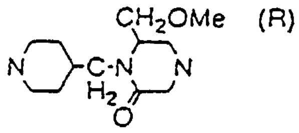 | 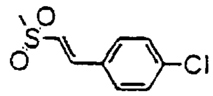 |
| 72 | Methanesulfonate of the compound of Ex.No.3 | | |
| 73 | Methanesulfonate of the compound of Ex.No.23 | | |
| 74 | Methanesulfonate of the compound of Ex.No.25 | | |
| 75 | Methanesulfonate of the compound of Ex.No.39 | | |
| 76 | Methanesulfonate of the compound of Ex.No.40 | | |
| 77 | Methanesulfonate of the compound of Ex.No.41 | | |
| 78 | Methanesulfonate of the compound of Ex.No.42 | | |
| 79 | Methanesulfonate of the compound of Ex.No.43 | | |
| 80 | Methanesulfonate of the compound of Ex.No.44 | | |
| 81 | Methanesulfonate of the compound of Ex.No.45 | | |
| 82 | Methanesulfonate of the compound of Ex.No.48 | | |

FIG.8

| S1 | S2 | S3 |
|---|---|---|
| 83 | Methanesulfonate of the compound of Ex.No.52 | |
| 84 | Methanesulfonate of the compound of Ex.No.53 | |
| 85 | Methanesulfonate of the compound of Ex.No.54 | |
| 86 | Methanesulfonate of the compound of Ex.No.55 | |
| 87 | Methanesulfonate of the compound of Ex.No.56 | |
| 88 | Methanesulfonate of the compound of Ex.No.57 | |
| 89 | Methanesulfonate of the compound of Ex.No.60 | |
| 90 | Methanesulfonate of the compound of Ex.No.61 | |
| 91 | Methanesulfonate of the compound of Ex.No.62 | |
| 92 | Methanesulfonate of the compound of Ex.No.63 | |
| 93 | Methanesulfonate of the compound of Ex.No.64 | |
| 94 | Methanesulfonate of the compound of Ex.No.66 | |

# FIG.9

| | S1 | S2 | S3 |
|---|---|---|---|
| 95 | Methanesulfonate of the compound of Ex.No.68 | | |
| 96 | 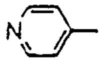 | 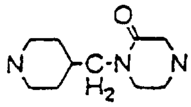 | 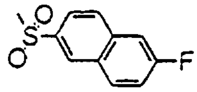 |
| 97 | 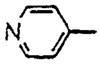 | 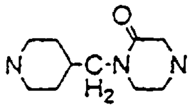 | 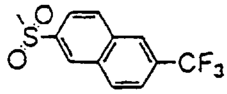 |
| 98 | 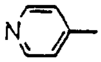 | 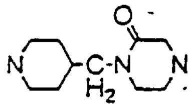 | 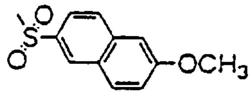 |
| 99 | 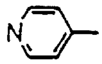 | 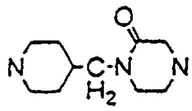 | 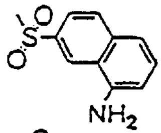 |
| 100 | 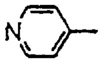 | 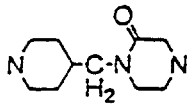 | 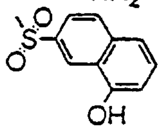 |
| 101 | 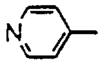 | 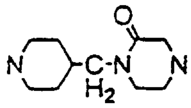 | 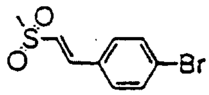 |
| 102 | 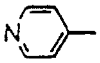 | 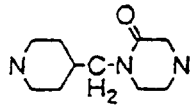 | 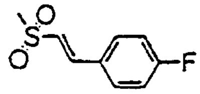 |
| 103 | 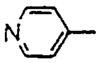 | 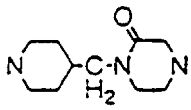 | 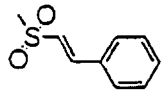 |
| 104 | 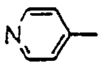 | 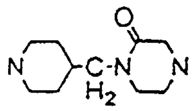 | 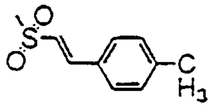 |
| 105 | 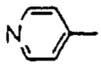 | 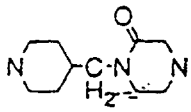 | 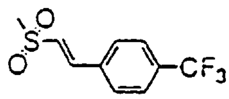 |
| 106 | 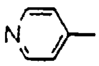 | 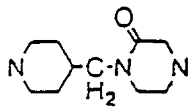 | 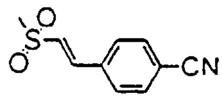 |

# FIG.10

| | S1 | S2 | S3 |
|---|---|---|---|
| 107 | 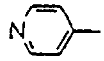 | 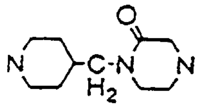 | 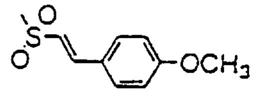 |
| 108 | 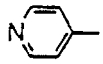 | 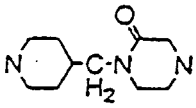 | 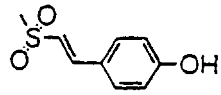 |
| 109 | 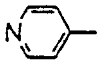 | 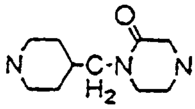 | 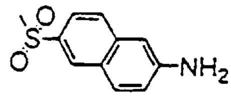 |
| 110 | 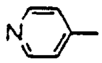 | 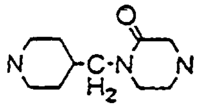 | 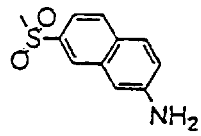 |
| 111 | 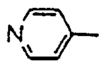 | 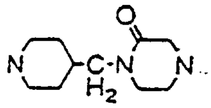 | 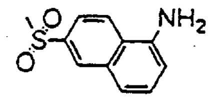 |
| 112 | 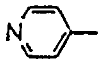 | 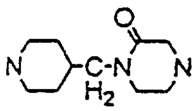 | 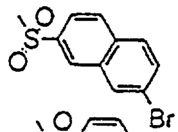 |
| 113 | 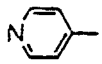 | 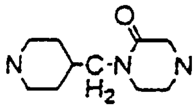 | 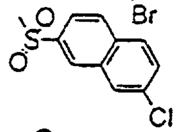 |
| 114 | 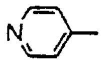 | 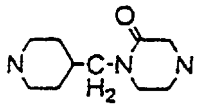 | 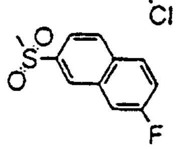 |
| 115 | 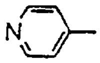 | 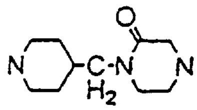 | 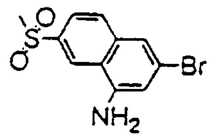 |
| 116 | 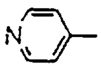 | 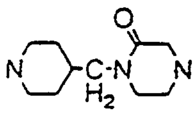 | 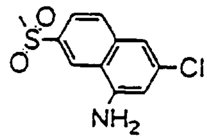 |
| 117 | 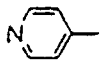 | 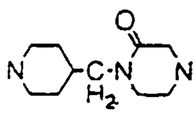 | 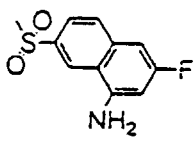 |
| 118 | 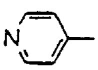 | 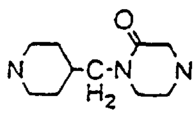 | 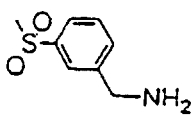 |

# FIG.11

| | S 1 | S 2 | S 3 |
|---|---|---|---|
| 1 1 9 | 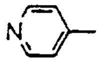 | 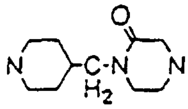 | 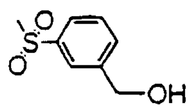 |
| 1 2 0 | 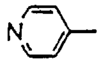 | 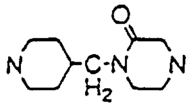 | 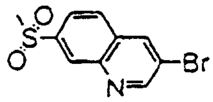 |
| 1 2 1 | 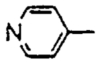 | 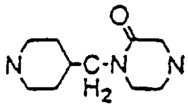 | 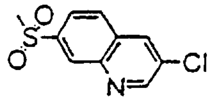 |
| 1 2 2 | 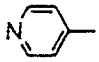 | 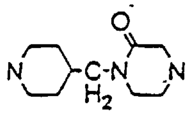 | 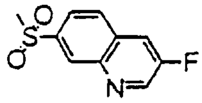 |
| 1 2 3 | 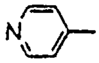 | 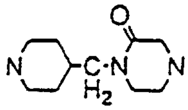 | 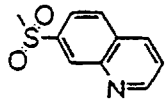 |
| 1 2 4 | 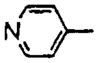 | 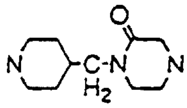 | 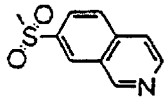 |
| 1 2 5 | 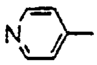 | 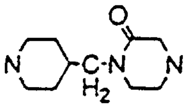 | 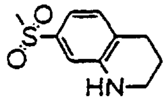 |
| 1 2 6 | 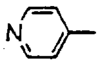 | 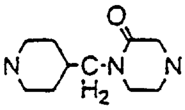 | 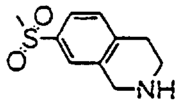 |
| 1 2 7 | 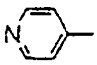 | 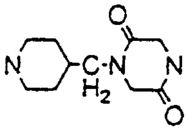 | 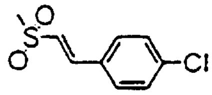 |
| 1 2 8 | 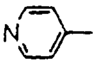 | 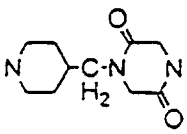 | 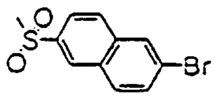 |
| 1 2 9 | 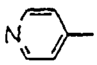 | 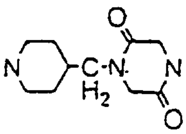 | 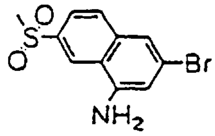 |
| 1 3 0 | 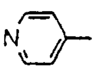 | 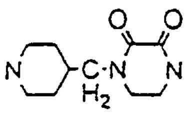 | 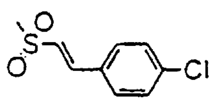 |

# FIG.12

| | S 1 | S 2 | S 3 |
|---|---|---|---|
| 131 | 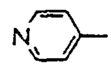 | 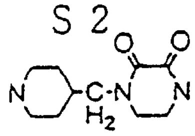 | 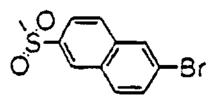 |
| 132 | 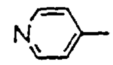 | 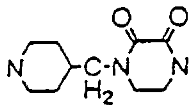 | 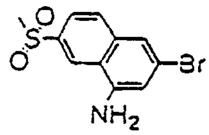 |
| 133 | 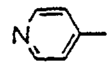 | 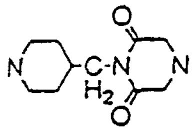 | 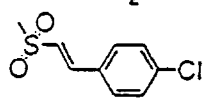 |
| 134 | 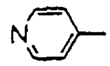 | 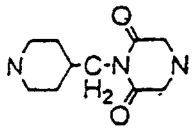 | 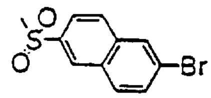 |
| 135 | 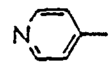 | 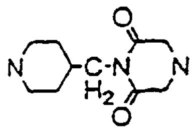 | 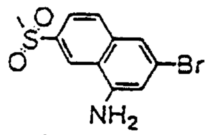 |
| 136 | 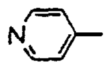 | 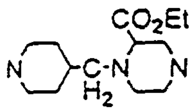 | 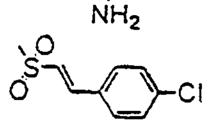 |
| 137 | 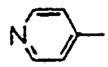 | 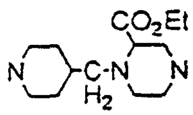 | 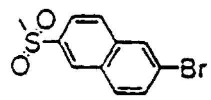 |
| 138 | 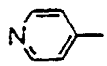 | 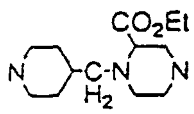 | 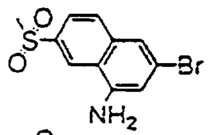 |
| 139 | 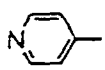 | 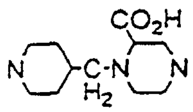 | 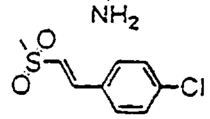 |
| 140 | 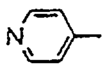 | 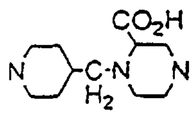 | 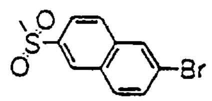 |
| 141 | 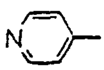 | 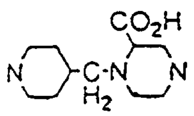 | 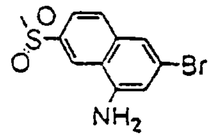 |
| 142 | 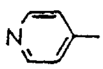 | 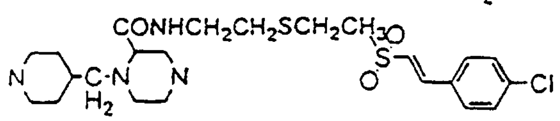 | |

FIG.13

| | S 1 | S 2 | S 3 |
|---|---|---|---|
| 143 | 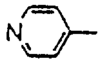 | 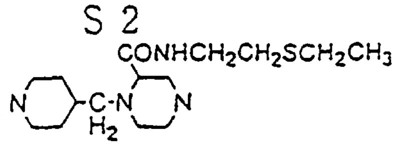 | 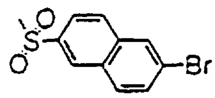 |
| 144 | 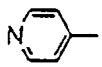 | 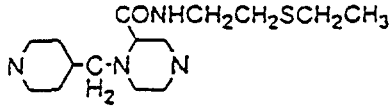 | 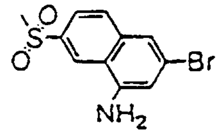 |
| 145 | 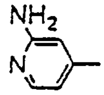 | 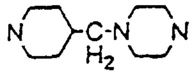 | 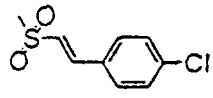 |
| 146 | 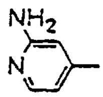 | 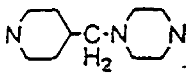 | 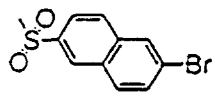 |
| 147 | 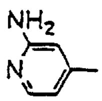 | 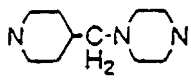 | 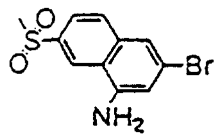 |
| 148 | 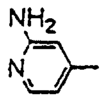 | 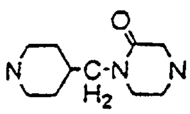 | 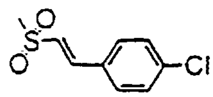 |
| 149 | 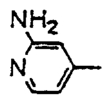 | 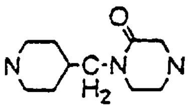 | 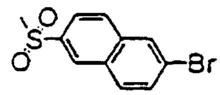 |
| 150 | 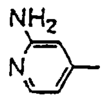 | 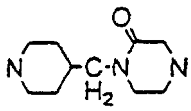 | 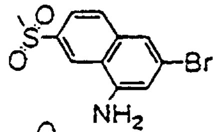 |
| 151 | 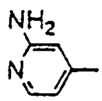 | 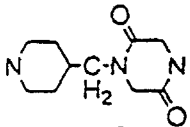 | 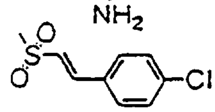 |
| 152 | 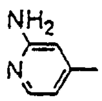 | 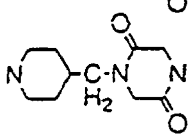 | 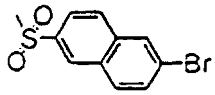 |
| 153 | 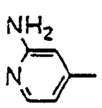 | 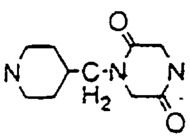 | 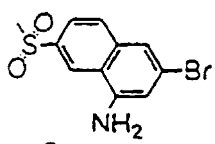 |
| 154 | 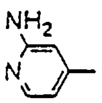 | 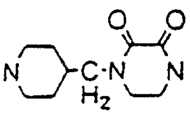 | 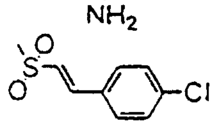 |

# FIG.14

| | S 1 | S 2 | S 3 |
|---|---|---|---|
| 155 | 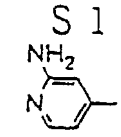 | 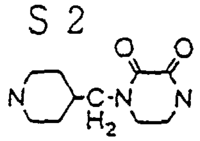 | 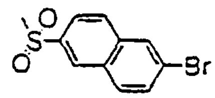 |
| 156 | 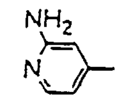 | 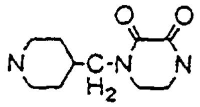 | 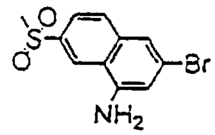 |
| 157 | 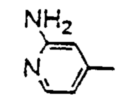 | 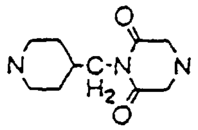 | 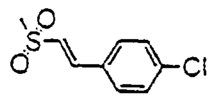 |
| 158 | 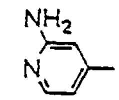 | 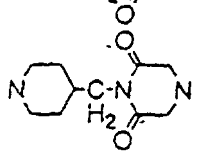 | 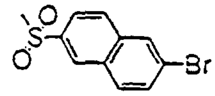 |
| 159 | 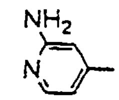 | 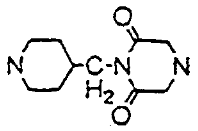 | 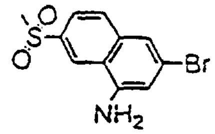 |
| 160 | 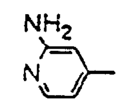 | 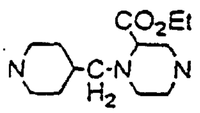 | 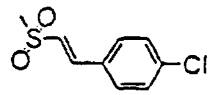 |
| 161 | 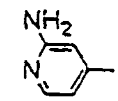 | 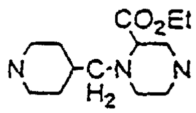 | 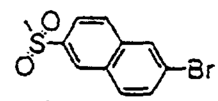 |
| 162 | 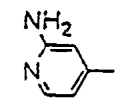 | 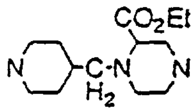 | 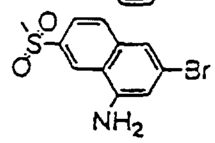 |
| 163 | 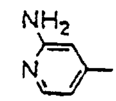 | 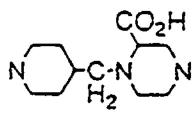 | 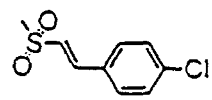 |
| 164 | 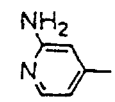 | 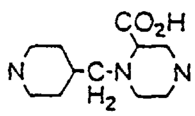 | 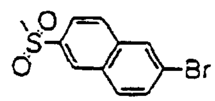 |
| 165 | 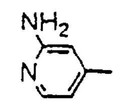 | 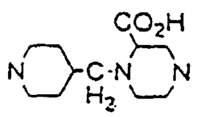 | 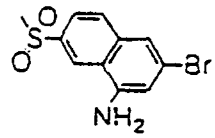 |
| 166 | 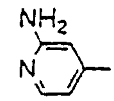 | 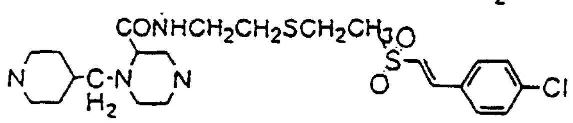 | |

FIG.15

| | S 1 | S 2 | S 3 |
|---|---|---|---|
| 167 | 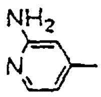 | 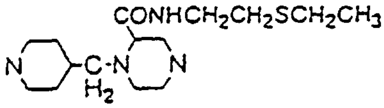 | 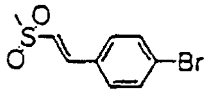 |
| 168 | 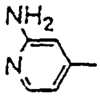 | 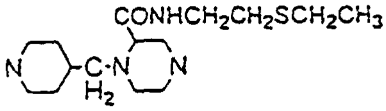 | 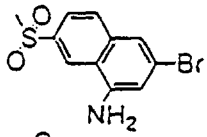 |
| 169 | 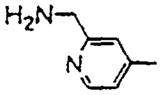 | 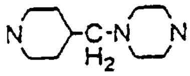 | 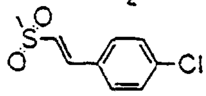 |
| 170 | 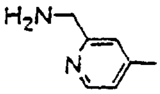 | 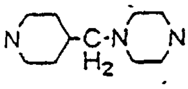 | 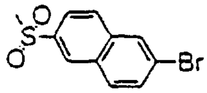 |
| 171 | 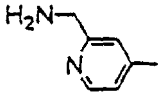 | 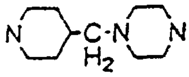 | 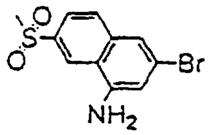 |
| 172 | 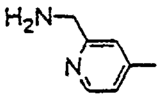 | 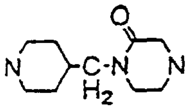 | 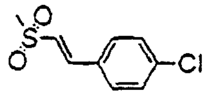 |
| 173 | 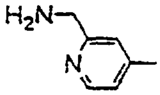 | 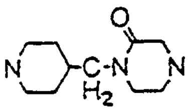 | 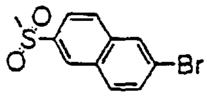 |
| 174 | 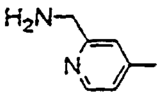 | 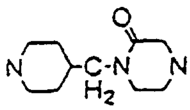 | 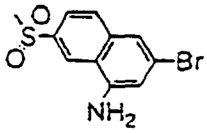 |
| 175 | 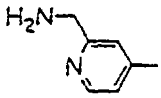 | 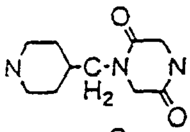 | 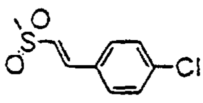 |
| 176 | 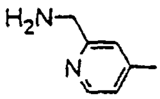 | 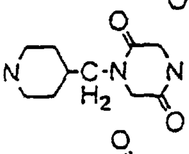 | 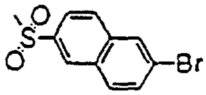 |
| 177 | 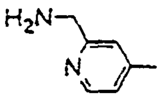 | 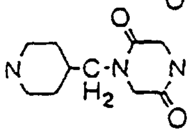 | 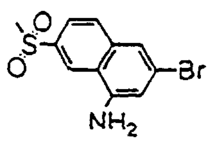 |
| 178 | 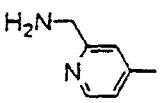 | 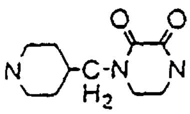 | 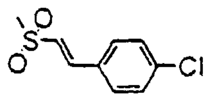 |

# FIG.16

| | S 1 | S 2 | S 3 |
|---|---|---|---|
| 1 7 9 | 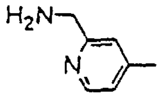 | 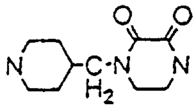 | 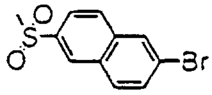 |
| 1 8 0 | 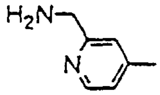 | 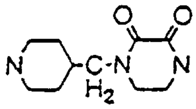 | 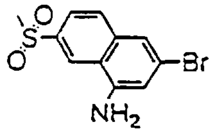 |
| 1 8 1 | 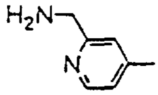 | 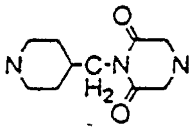 | 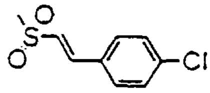 |
| 1 8 2 | 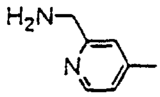 | 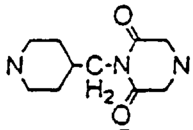 | 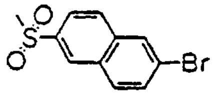 |
| 1 8 3 | 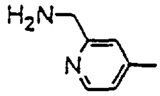 | 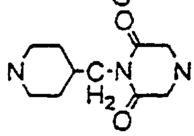 | 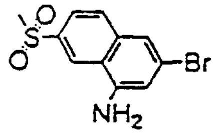 |
| 1 8 4 | 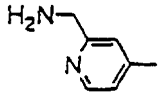 | 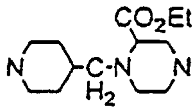 | 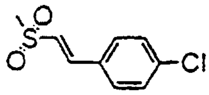 |
| 1 8 5 | 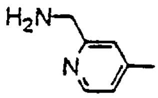 | 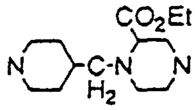 | 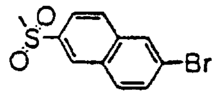 |
| 1 8 6 | 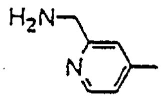 | 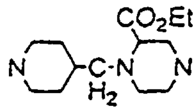 | 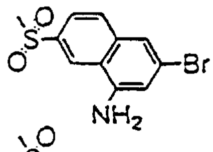 |
| 1 8 7 | 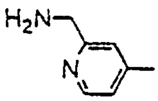 | 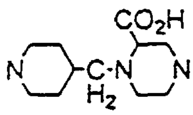 | 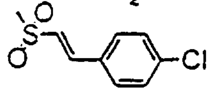 |
| 1 8 8 | 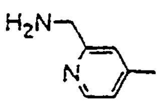 | 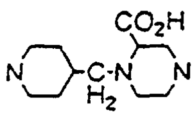 | 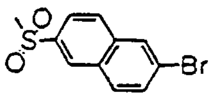 |
| 1 8 9 | 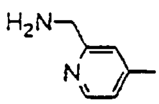 | 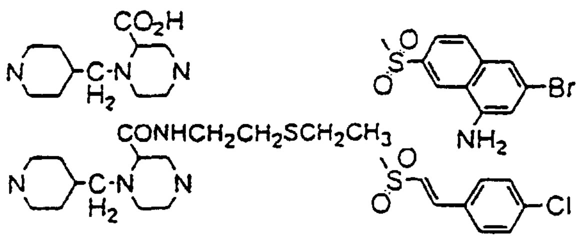 | |
| 1 9 0 | 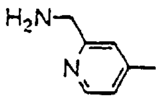 | | |

# FIG.17

| | S 1 | S 2 | S 3 |
|---|---|---|---|
| 1 9 1 | 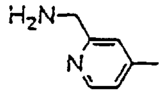 | 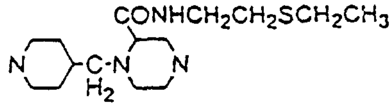 | 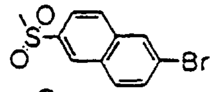 |
| 1 9 2 | 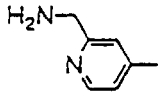 | 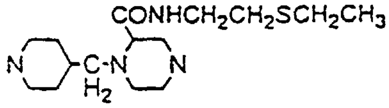 | 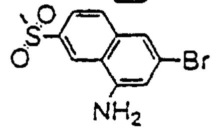 |
| 1 9 3 | 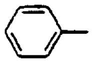 | 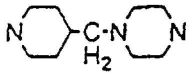 | 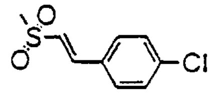 |
| 1 9 4 | 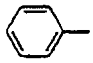 | 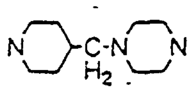 | 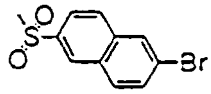 |
| 1 9 5 | 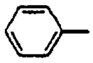 | 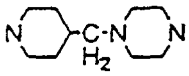 | 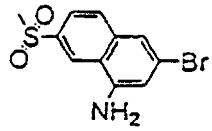 |
| 1 9 6 | 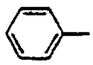 | 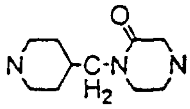 | 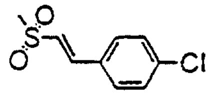 |
| 1 9 7 | 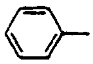 | 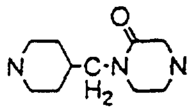 | 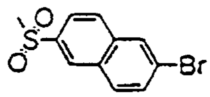 |
| 1 9 8 | 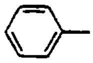 | 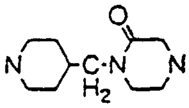 | 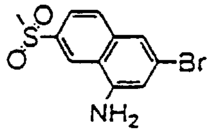 |
| 1 9 9 | 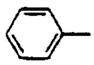 | 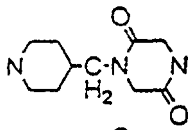 | 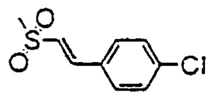 |
| 2 0 0 | 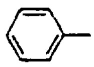 | 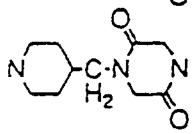 | 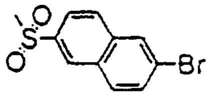 |
| 2 0 1 | 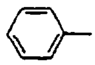 | 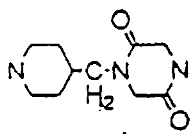 | 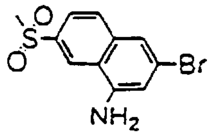 |
| 2 0 2 | 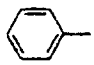 | 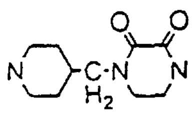 | 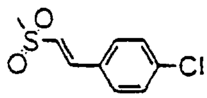 |

FIG.18

| | S 1 | S 2 | S 3 |
|---|---|---|---|
| 2 0 3 | 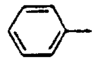 | 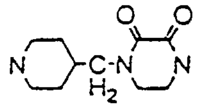 | 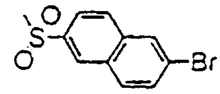 |
| 2 0 4 | 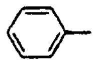 | 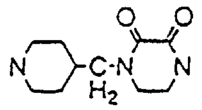 | 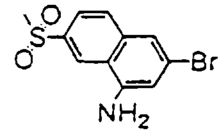 |
| 2 0 5 | 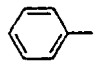 | 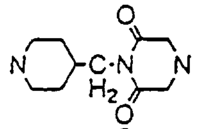 | 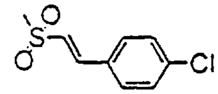 |
| 2 0 6 | 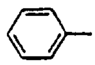 | 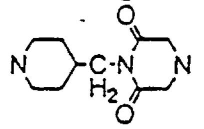 | 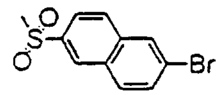 |
| 2 0 7 | 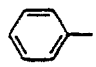 | 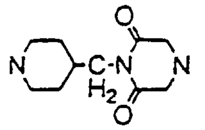 | 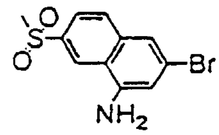 |
| 2 0 8 | 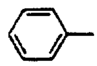 | 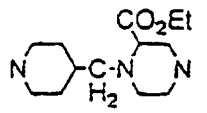 | 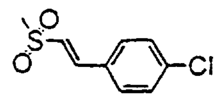 |
| 2 0 9 | 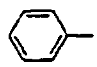 | 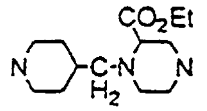 | 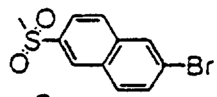 |
| 2 1 0 | 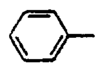 | 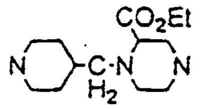 | 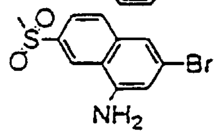 |
| 2 1 1 | 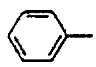 | 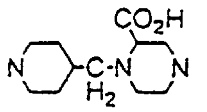 | 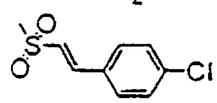 |
| 2 1 2 | 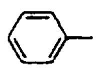 | 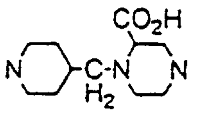 | 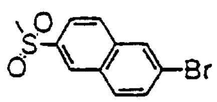 |
| 2 1 3 | 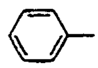 | 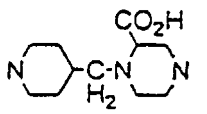 | 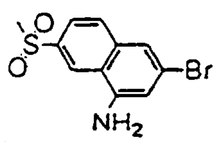 |
| 2 1 4 | 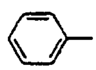 | 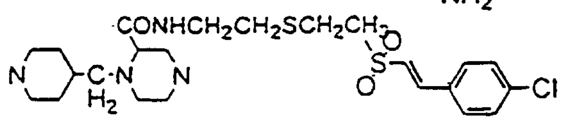 | |

# FIG.19

| | S1 | S2 | S3 |
|---|---|---|---|
| 215 | 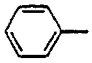 | 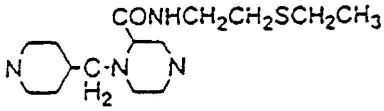 | 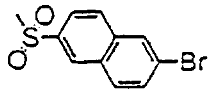 |
| 216 | 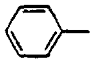 | 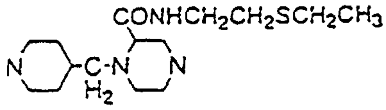 | 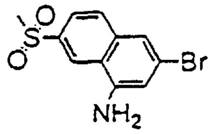 |
| 217 | 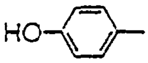 | 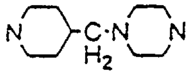 | 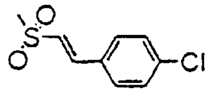 |
| 218 | 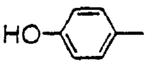 | 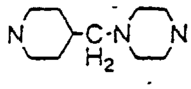 | 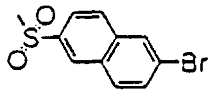 |
| 219 | 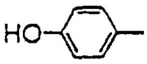 | 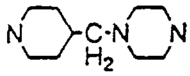 | 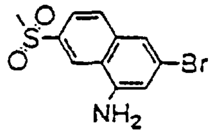 |
| 220 | 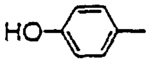 | 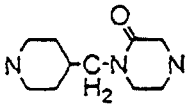 | 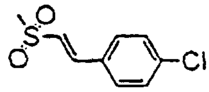 |
| 221 | 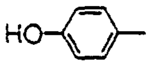 | 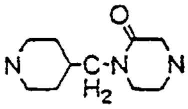 | 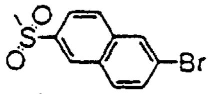 |
| 222 | 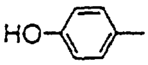 | 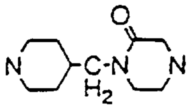 | 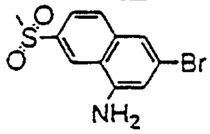 |
| 223 | 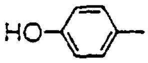 | 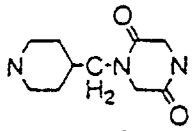 | 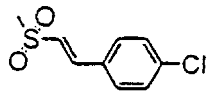 |
| 224 | 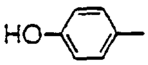 | 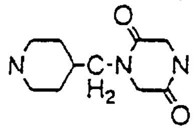 | 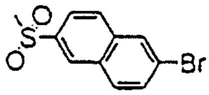 |
| 225 | 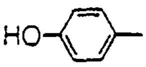 | 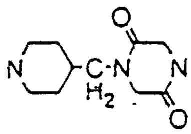 | 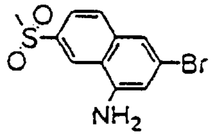 |
| 226 | 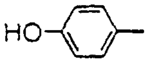 | 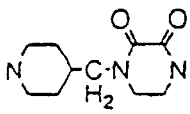 | 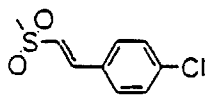 |

# FIG.20

| | S 1 | S 2 | S 3 |
|---|---|---|---|
| 227 | 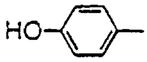 | 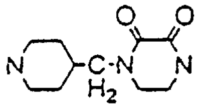 | 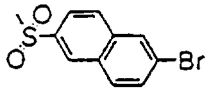 |
| 228 | 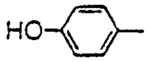 | 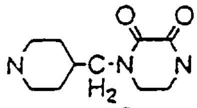 | 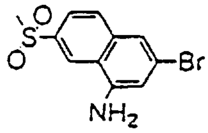 |
| 229 | 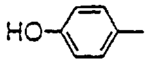 | 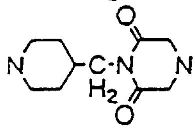 | 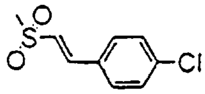 |
| 230 | 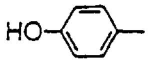 | 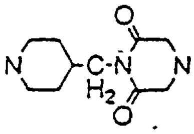 | 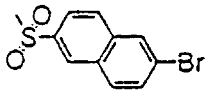 |
| 231 | 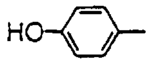 | 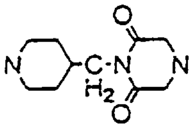 | 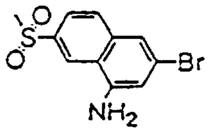 |
| 232 | 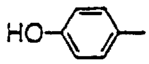 | 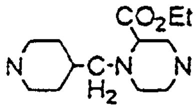 | 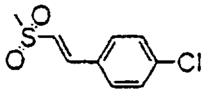 |
| 233 | 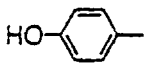 | 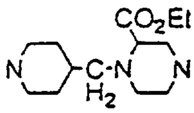 | 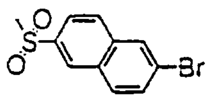 |
| 234 | 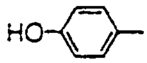 | 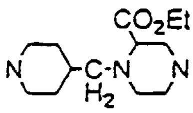 | 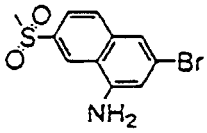 |
| 235 | 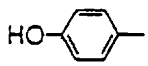 | 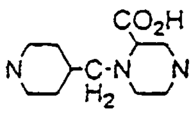 | 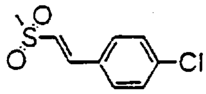 |
| 236 | 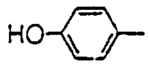 | 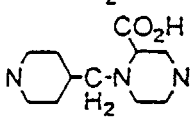 | 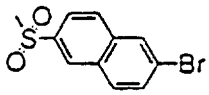 |
| 237 | 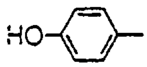 | 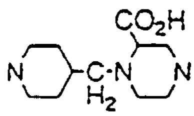 | 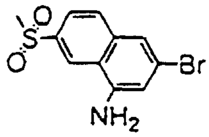 |
| 238 | 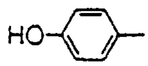 | 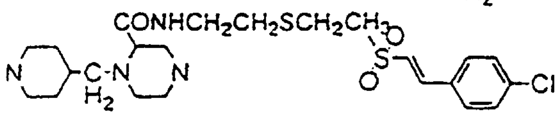 | |

# FIG.21

| | S 1 | S 2 | S 3 |
|---|---|---|---|
| 239 | 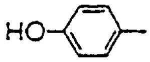 | 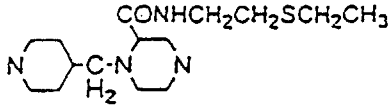 | 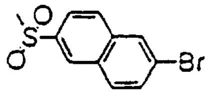 |
| 240 | 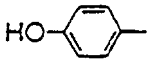 | 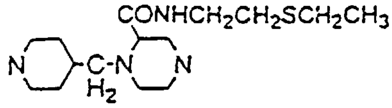 | 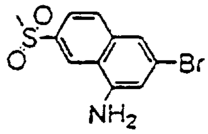 |
| 241 | 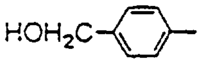 | 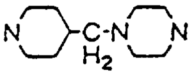 | 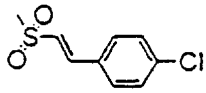 |
| 242 | 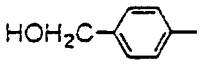 | 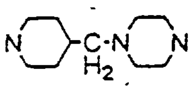 | 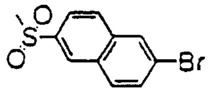 |
| 243 | 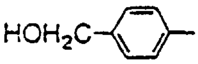 | 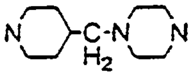 | 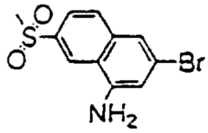 |
| 244 | 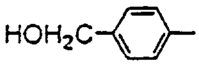 | 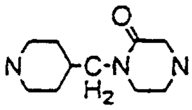 | 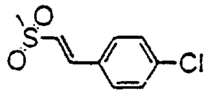 |
| 245 | 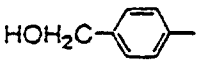 | 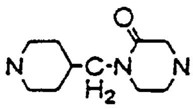 | 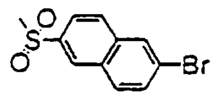 |
| 246 | 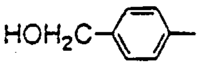 | 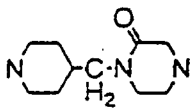 | 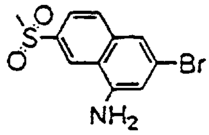 |
| 247 | 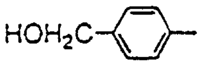 | 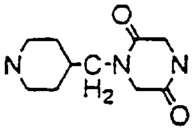 | 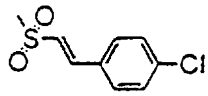 |
| 248 | 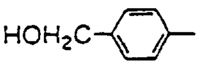 | 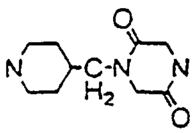 | 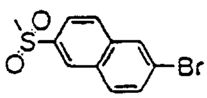 |
| 249 | 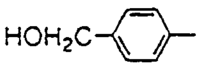 | 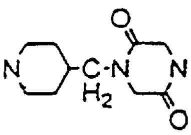 | 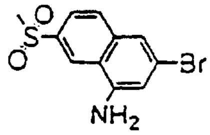 |
| 250 | 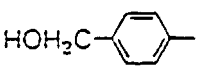 | 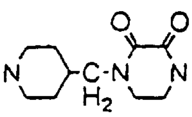 | 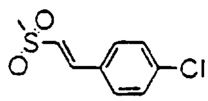 |

# FIG.22

| | S 1 | S 2 | S 3 |
|---|---|---|---|
| 251 | 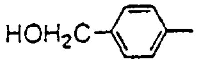 | 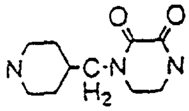 | 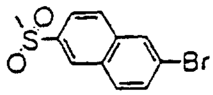 |
| 252 | 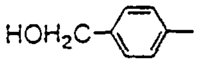 | 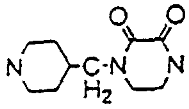 | 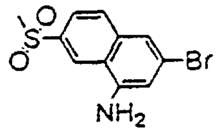 |
| 253 | 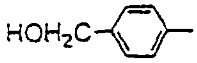 | 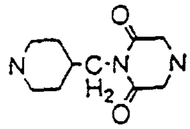 | 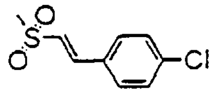 |
| 254 | 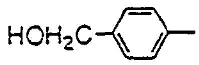 | 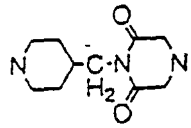 | 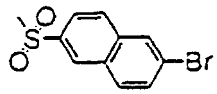 |
| 255 | 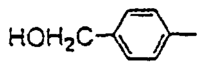 | 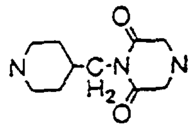 | 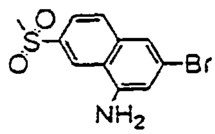 |
| 256 | 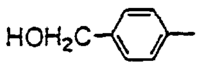 | 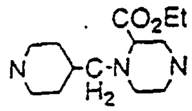 | 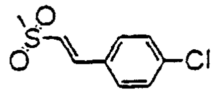 |
| 257 | 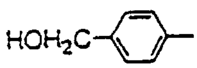 | 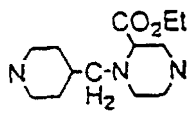 | 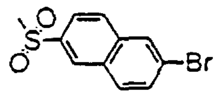 |
| 258 | 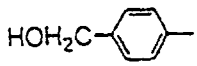 | 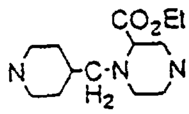 | 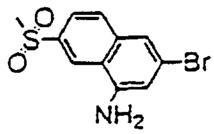 |
| 259 | 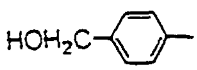 | 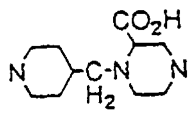 | 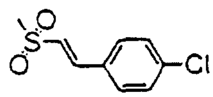 |
| 260 | 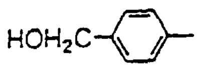 | 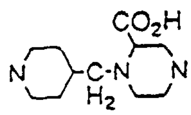 | 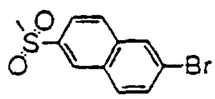 |
| 261 | 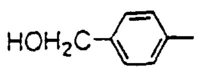 | 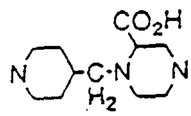 | 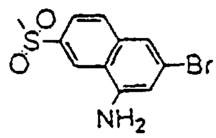 |
| 262 | 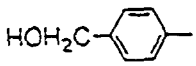 | 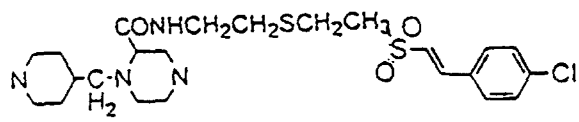 | |

FIG.23

| | S 1 | S 2 | S 3 |
|---|---|---|---|
| 263 | 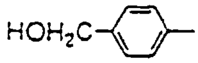 | 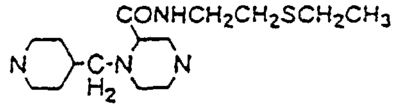 | 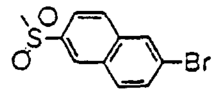 |
| 264 | 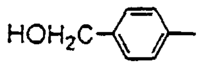 | 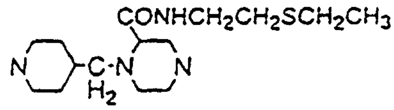 | 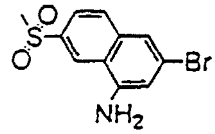 |
| 265 | 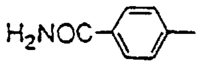 | 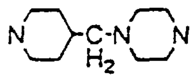 | 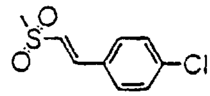 |
| 266 | 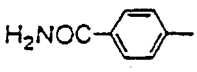 | 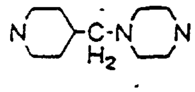 | 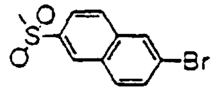 |
| 267 | 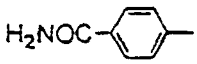 | 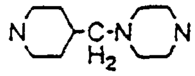 | 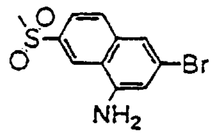 |
| 268 | 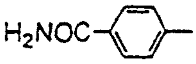 | 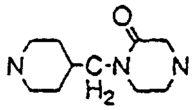 | 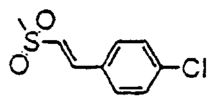 |
| 269 | 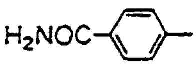 | 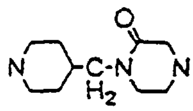 | 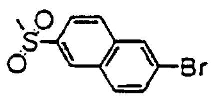 |
| 270 | 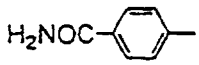 | 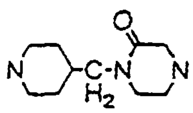 | 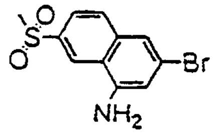 |
| 271 | 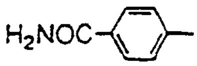 | 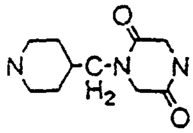 | 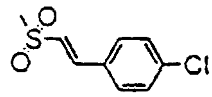 |
| 272 | 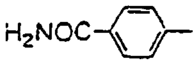 | 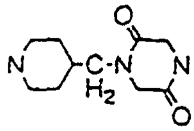 | 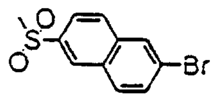 |
| 273 | 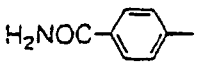 | 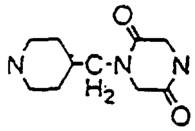 | 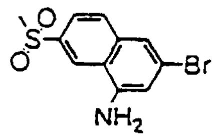 |
| 274 | 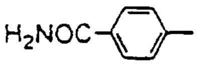 | 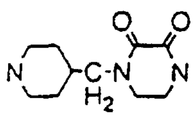 | 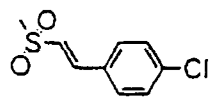 |

# FIG.24

| | S 1 | S 2 | S 3 |
|---|---|---|---|
| 2 7 5 | 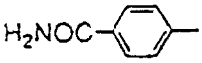 | 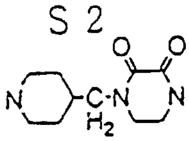 | 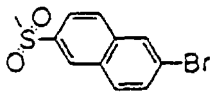 |
| 2 7 6 | 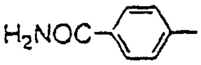 | 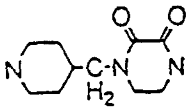 | 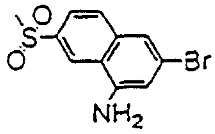 |
| 2 7 7 | 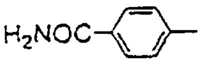 | 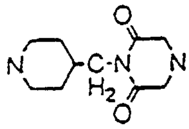 | 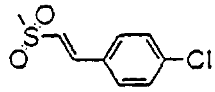 |
| 2 7 8 | 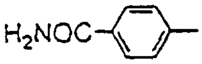 | 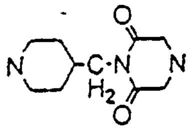 | 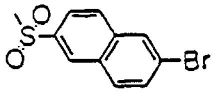 |
| 2 7 9 | 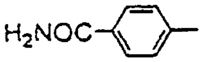 | 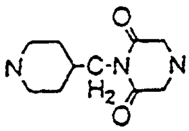 | 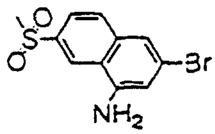 |
| 2 8 0 | 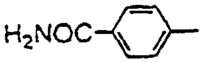 | 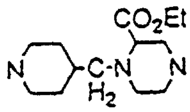 | 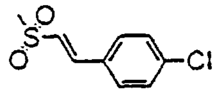 |
| 2 8 1 | 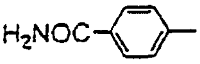 | 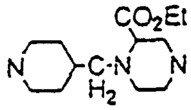 | 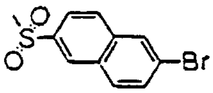 |
| 2 8 2 | 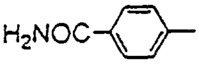 | 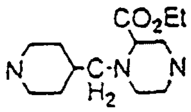 | 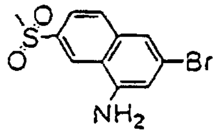 |
| 2 8 3 | 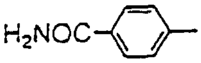 | 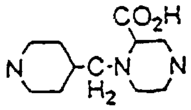 | 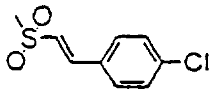 |
| 2 8 4 | 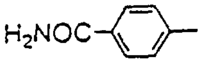 | 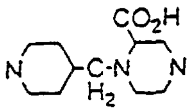 | 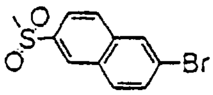 |
| 2 8 5 | 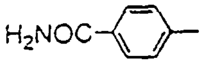 | 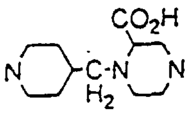 | 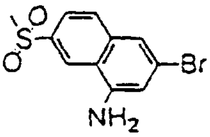 |
| 2 8 6 | 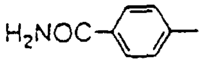 | 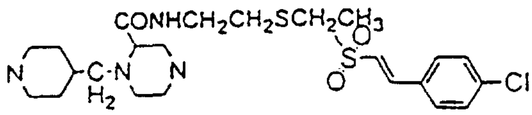 | |

## FIG.25

| | S 1 | S 2 | S 3 |
|---|---|---|---|
| 287 | 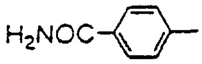 | 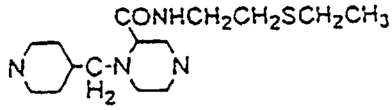 | 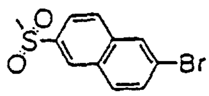 |
| 288 | 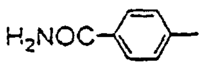 | 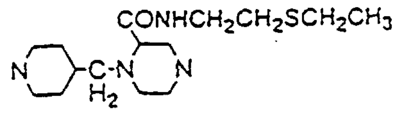 | 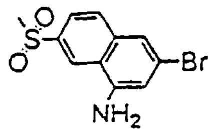 |
| 289 | 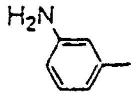 | 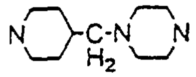 | 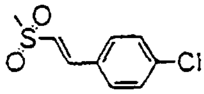 |
| 290 | 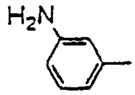 | 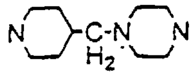 | 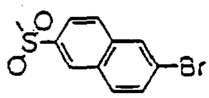 |
| 291 | 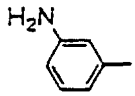 | 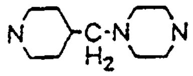 | 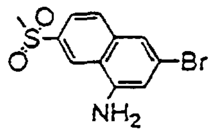 |
| 292 | 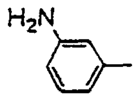 | 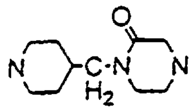 | 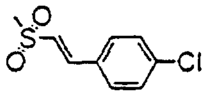 |
| 293 | 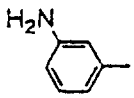 | 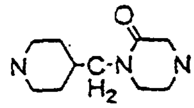 | 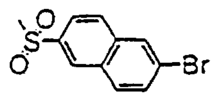 |
| 294 | 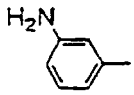 | 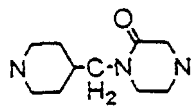 | 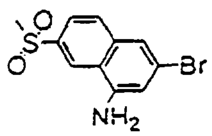 |
| 295 | 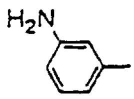 | 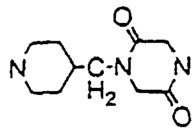 | 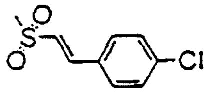 |
| 296 | 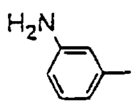 | 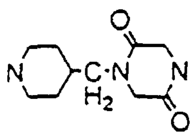 | 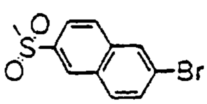 |
| 297 | 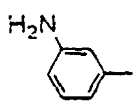 | 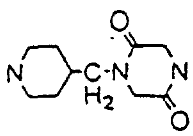 | 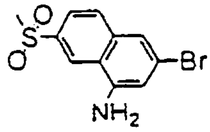 |
| 298 | 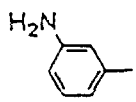 | 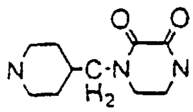 | 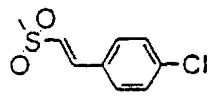 |

FIG.26

| | S 1 | S 2 | S 3 |
|---|---|---|---|
| 299 | 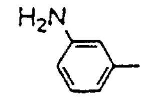 | 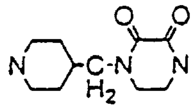 | 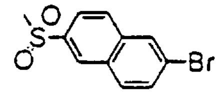 |
| 300 | 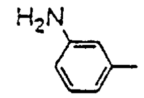 | 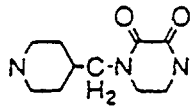 | 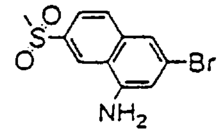 |
| 301 | 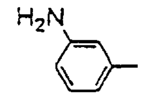 | 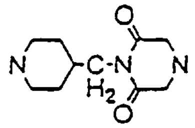 | 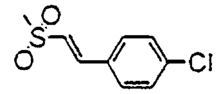 |
| 302 | 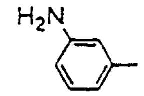 | 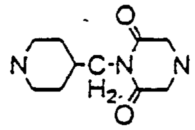 | 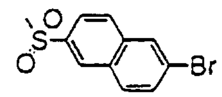 |
| 303 | 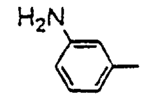 | 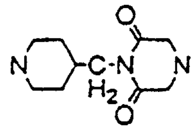 | 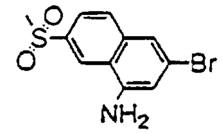 |
| 304 | 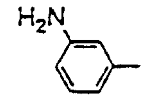 | 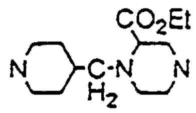 | 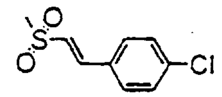 |
| 305 | 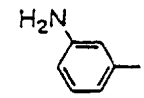 | 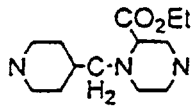 | 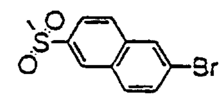 |
| 306 | 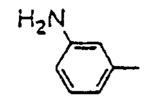 | 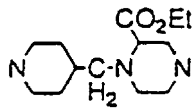 | 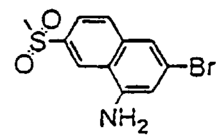 |
| 307 | 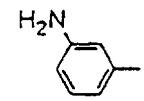 | 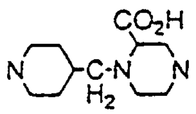 | 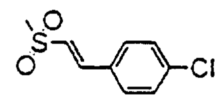 |
| 308 | 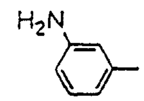 | 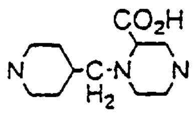 | 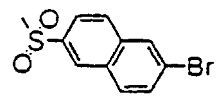 |
| 309 | 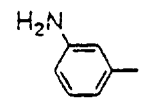 | 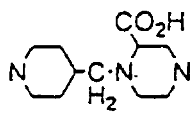 | 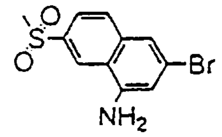 |
| 310 | 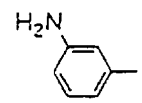 | 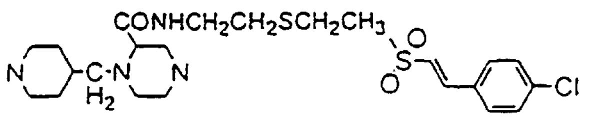 | |

# FIG.27

| | S 1 | S 2 | S 3 |
|---|---|---|---|
| 3 1 1 | 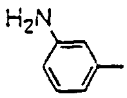 | 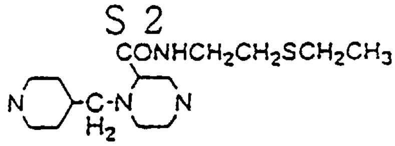 | 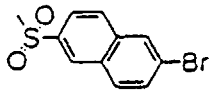 |
| 3 1 2 | 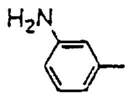 | 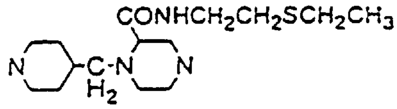 | 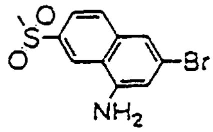 |
| 3 1 3 | 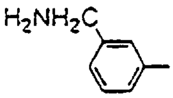 | 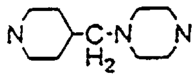 | 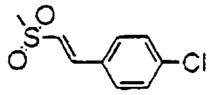 |
| 3 1 4 | 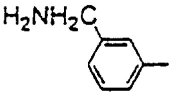 | 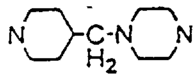 | 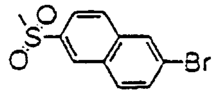 |
| 3 1 5 | 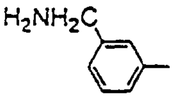 | 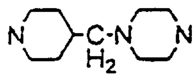 | 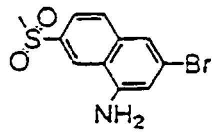 |
| 3 1 6 | 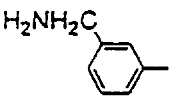 | 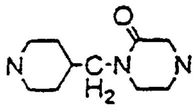 | 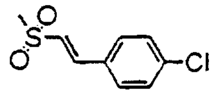 |
| 3 1 7 | 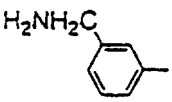 | 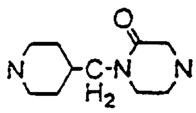 | 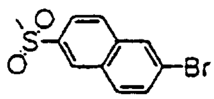 |
| 3 1 8 | 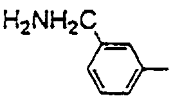 | 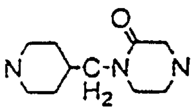 | 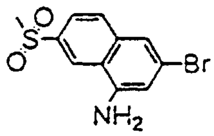 |
| 3 1 9 | 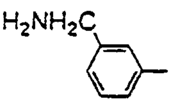 | 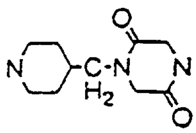 | 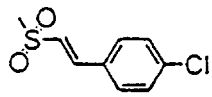 |
| 3 2 0 | 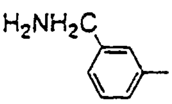 | 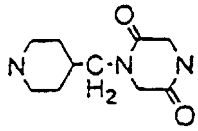 | 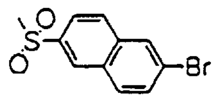 |
| 3 2 1 | 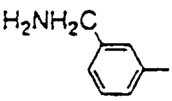 | 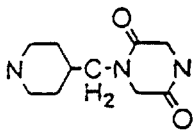 | 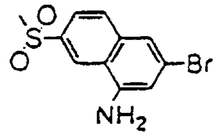 |
| 3 2 2 | 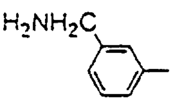 | 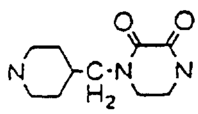 | 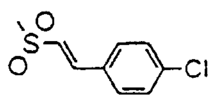 |

FIG.28

| | S 1 | S 2 | S 3 |
|---|---|---|---|
| 3 2 3 | 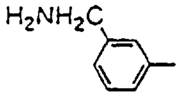 | 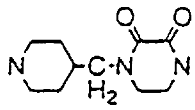 | 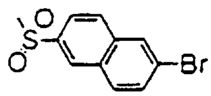 |
| 3 2 4 | 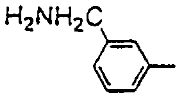 | 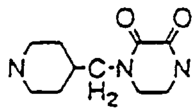 | 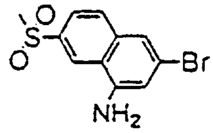 |
| 3 2 5 | 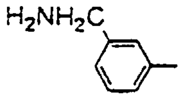 | 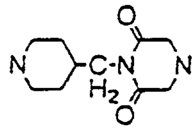 | 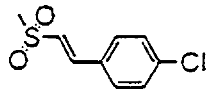 |
| 3 2 6 | 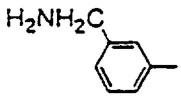 | 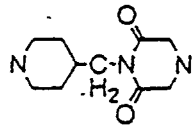 | 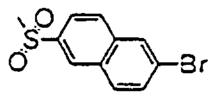 |
| 3 2 7 | 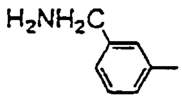 | 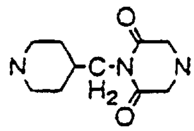 | 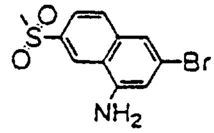 |
| 3 2 8 | 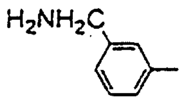 | 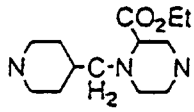 | 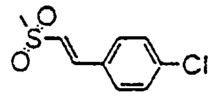 |
| 3 2 9 | 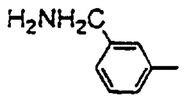 | 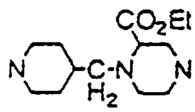 | 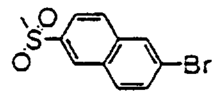 |
| 3 3 0 | 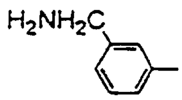 | 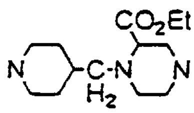 | 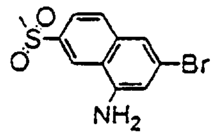 |
| 3 3 1 | 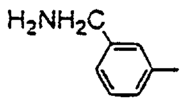 | 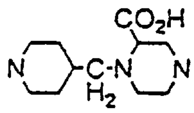 | 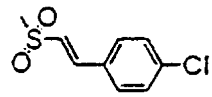 |
| 3 3 2 | 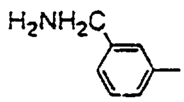 | 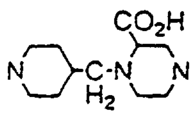 | 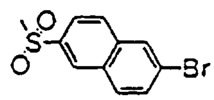 |
| 3 3 3 | 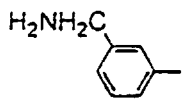 | 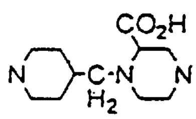 | 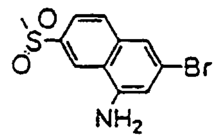 |
| 3 3 4 | 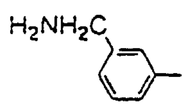 | 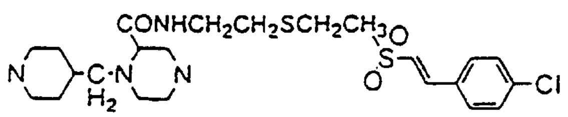 | |

FIG.29

| | S 1 | S 2 | S 3 |
|---|---|---|---|
| 335 | 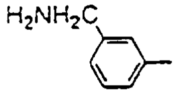 | 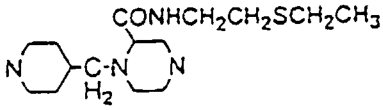 | 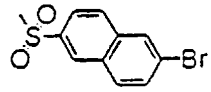 |
| 336 | 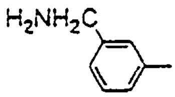 | 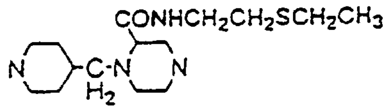 | 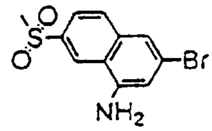 |
| 337 | 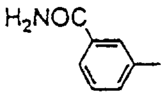 | 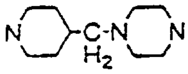 | 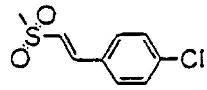 |
| 338 | 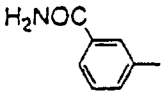 | 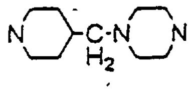 | 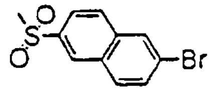 |
| 339 | 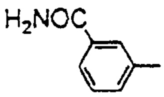 | 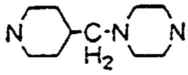 | 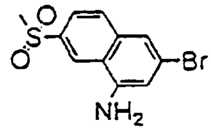 |
| 340 | 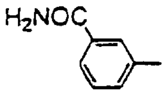 | 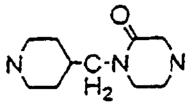 | 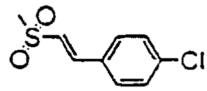 |
| 341 | 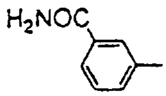 | 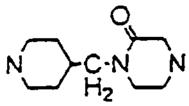 | 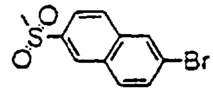 |
| 342 | 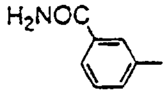 | 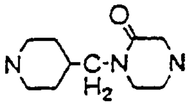 | 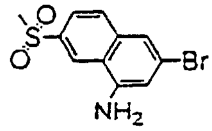 |
| 343 | 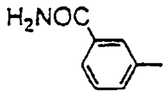 | 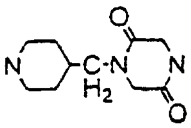 | 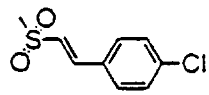 |
| 344 | 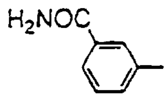 | 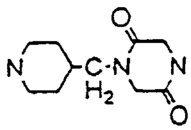 | 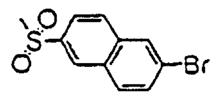 |
| 345 | 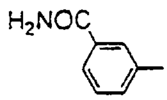 | 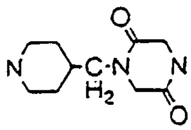 | 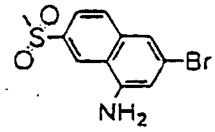 |
| 346 | 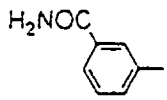 | 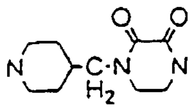 | 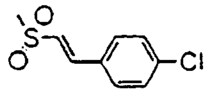 |

FIG.30

| | S 1 | S 2 | S 3 |
|---|---|---|---|
| 3 4 7 | 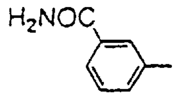 | 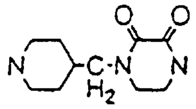 | 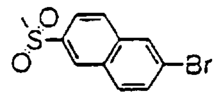 |
| 3 4 8 | 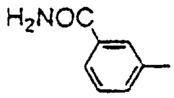 | 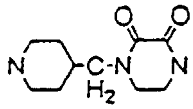 | 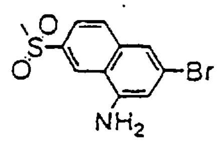 |
| 3 4 9 | 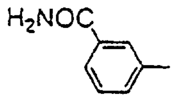 | 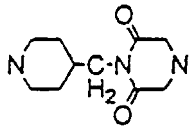 | 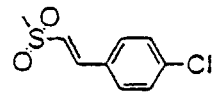 |
| 3 5 0 | 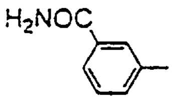 | 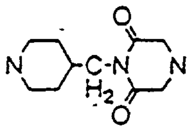 | 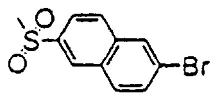 |
| 3 5 1 | 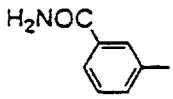 | 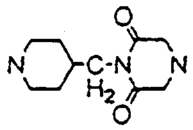 | 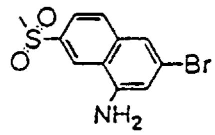 |
| 3 5 2 | 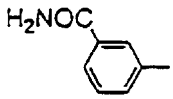 | 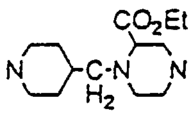 | 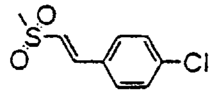 |
| 3 5 3 | 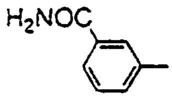 | 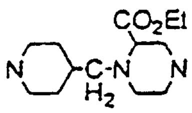 | 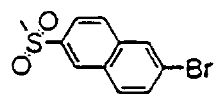 |
| 3 5 4 | 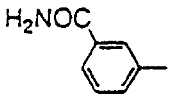 | 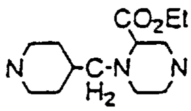 | 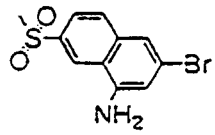 |
| 3 5 5 | 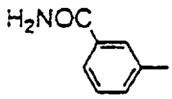 | 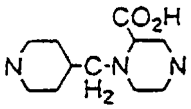 | 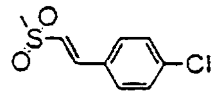 |
| 3 5 6 | 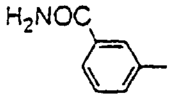 | 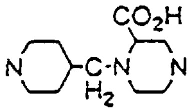 | 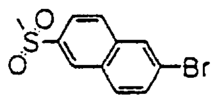 |
| 3 5 7 | 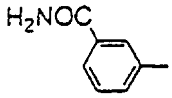 | 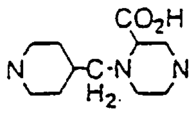 | 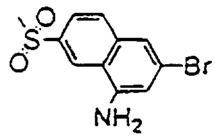 |
| 3 5 8 | 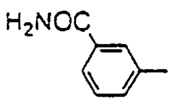 | 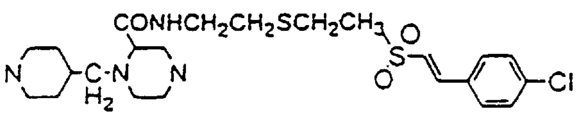 | |

# FIG.31

| | S 1 | S 2 | S 3 |
|---|---|---|---|
| 359 | 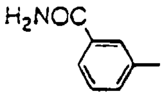 | 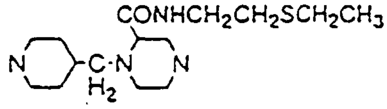 | 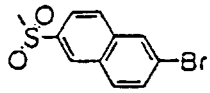 |
| 360 | 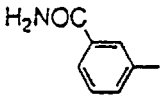 | 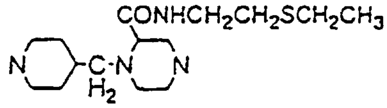 | 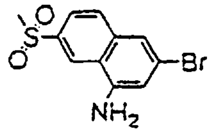 |
| 361 | 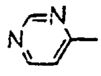 | 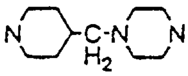 | 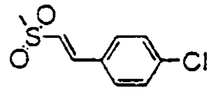 |
| 362 | 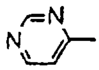 | 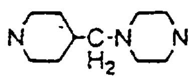 | 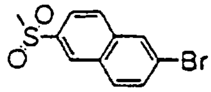 |
| 363 | 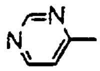 | 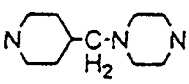 | 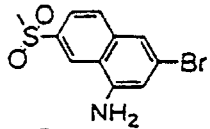 |
| 364 | 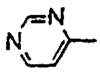 | 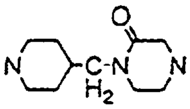 | 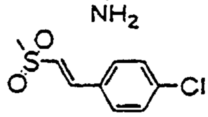 |
| 365 | 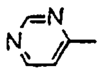 | 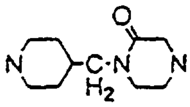 | 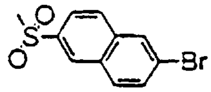 |
| 366 | 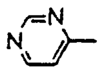 | 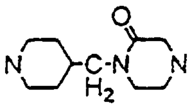 | 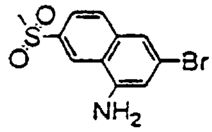 |
| 367 | 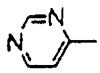 | 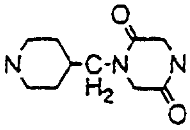 | 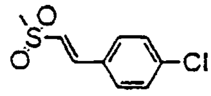 |
| 368 | 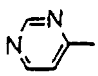 | 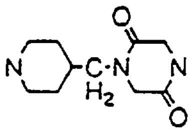 | 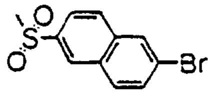 |
| 369 | 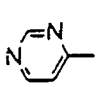 | 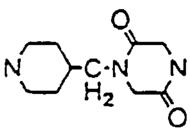 | 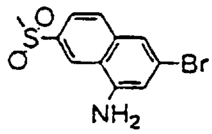 |
| 370 | 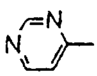 | 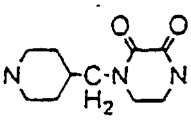 | 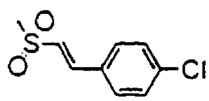 |

# FIG.32

| | S 1 | S 2 | S 3 |
|---|---|---|---|
| 371 | 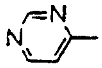 | 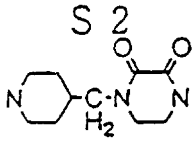 | 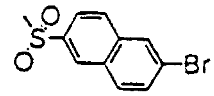 |
| 372 | 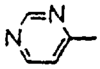 | 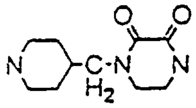 | 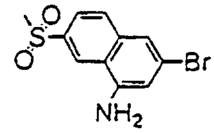 |
| 373 | 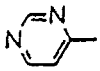 | 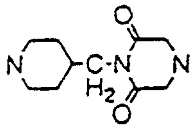 | 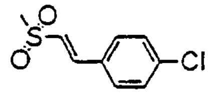 |
| 374 | 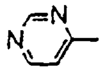 | 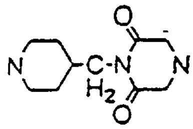 | 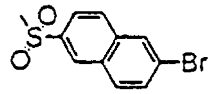 |
| 375 | 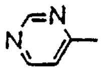 | 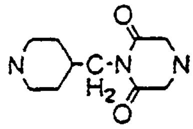 | 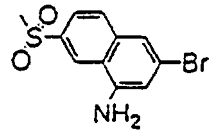 |
| 376 | 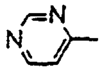 | 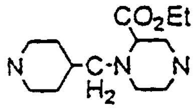 | 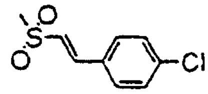 |
| 377 | 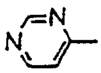 | 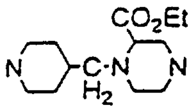 | 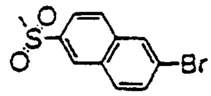 |
| 378 | 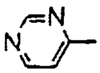 | 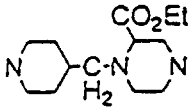 | 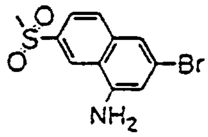 |
| 379 | 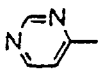 | 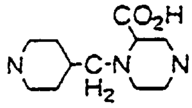 | 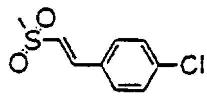 |
| 380 | 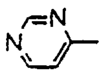 | 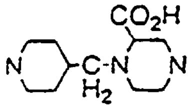 | 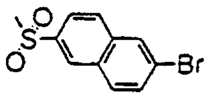 |
| 381 | 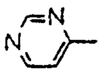 | 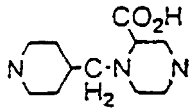 | 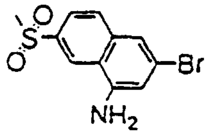 |
| 382 | 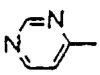 | 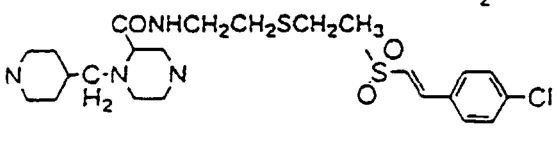 | |

# FIG.33

| | S 1 | S 2 | S 3 |
|---|---|---|---|
| 383 | 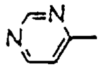 | 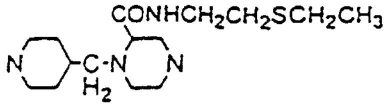 | 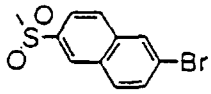 |
| 384 | 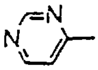 | 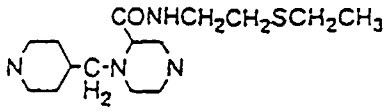 | 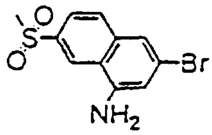 |
| 385 | 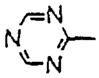 | 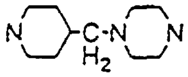 | 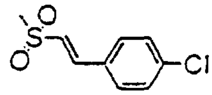 |
| 386 | 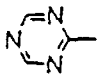 | 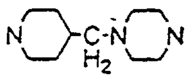 | 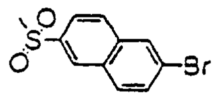 |
| 387 | 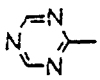 | 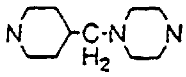 | 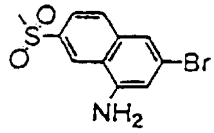 |
| 388 | 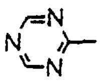 | 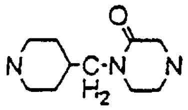 | 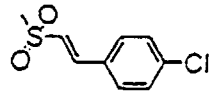 |
| 389 | 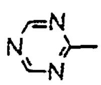 | 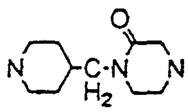 | 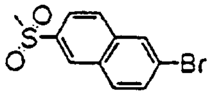 |
| 390 | 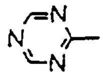 | 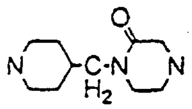 | 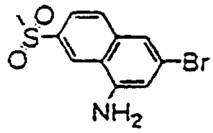 |
| 391 | 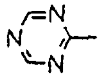 | 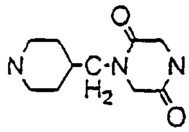 | 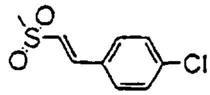 |
| 392 | 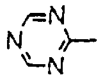 | 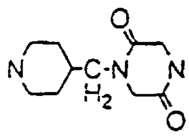 | 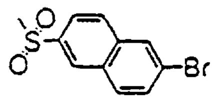 |
| 393 | 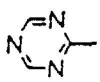 | 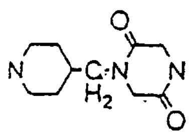 | 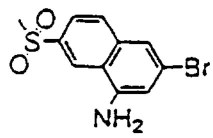 |
| 394 | 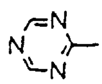 | 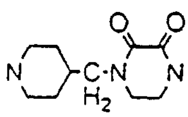 | 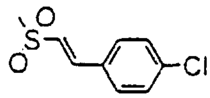 |

FIG.34

| | S 1 | S 2 | S 3 |
|---|---|---|---|
| 395 | 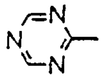 | 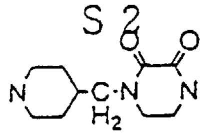 | 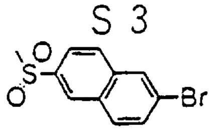 |
| 396 | 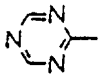 | 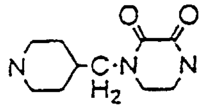 | 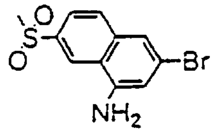 |
| 397 | 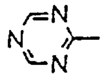 | 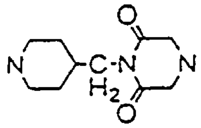 | 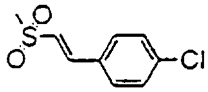 |
| 398 | 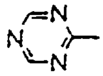 | 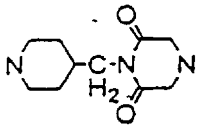 | 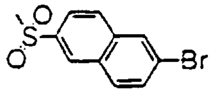 |
| 399 | 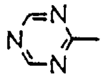 | 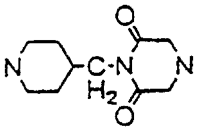 | 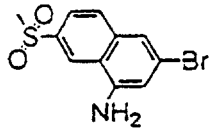 |
| 400 | 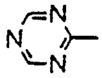 | 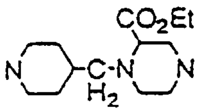 | 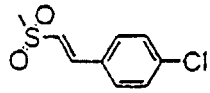 |
| 401 | 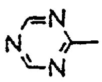 | 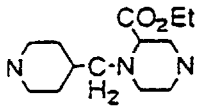 | 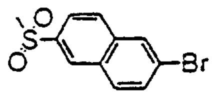 |
| 402 | 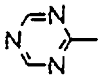 | 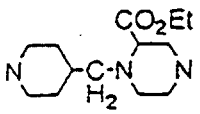 | 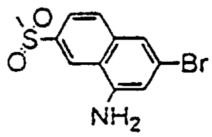 |
| 403 | 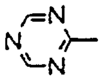 | 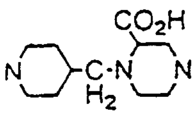 | 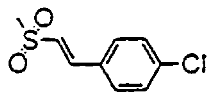 |
| 404 | 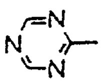 | 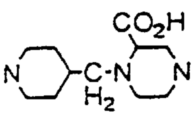 | 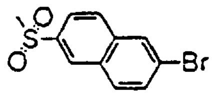 |
| 405 | 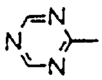 | 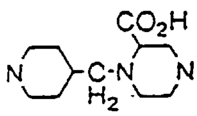 | 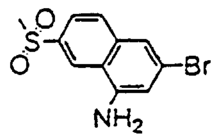 |
| 406 | 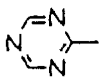 | 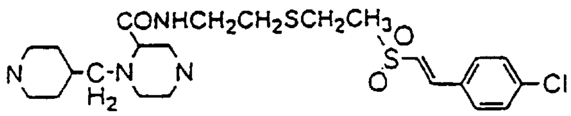 | |

# FIG.35

| | S 1 | S 2 | S 3 |
|---|---|---|---|
| 407 | 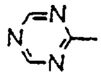 | 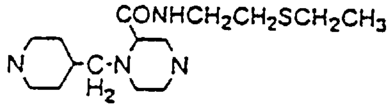 | 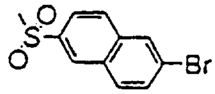 |
| 408 | 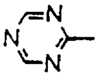 | 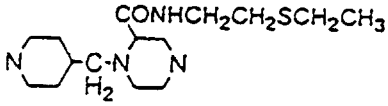 | 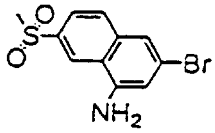 |
| 409 | 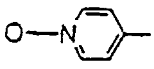 | 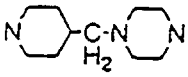 | 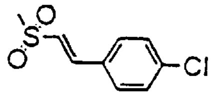 |
| 410 | 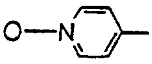 | 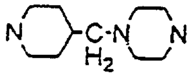 | 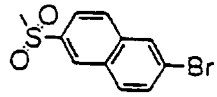 |
| 411 | 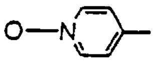 | 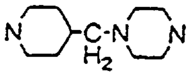 | 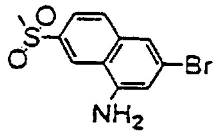 |
| 412 | 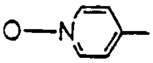 | 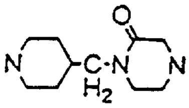 | 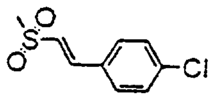 |
| 413 | 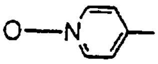 | 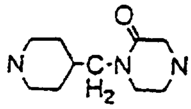 | 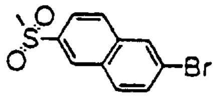 |
| 414 | 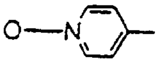 | 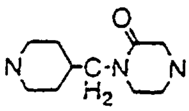 | 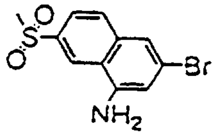 |
| 415 | 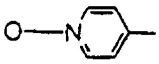 | 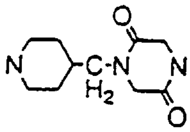 | 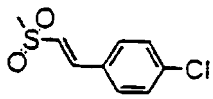 |
| 416 | 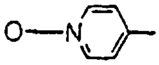 | 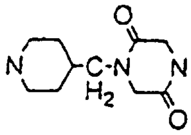 | 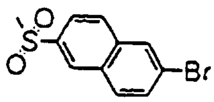 |
| 417 | 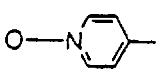 | 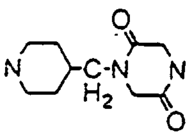 | 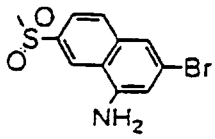 |
| 418 | 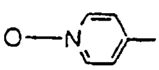 | 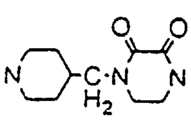 | 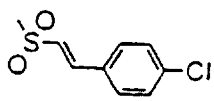 |

FIG.36

| | S 1 | S 2 | S 3 |
|---|---|---|---|
| 4 1 9 | 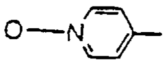 | 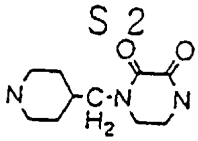 | 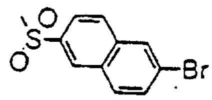 |
| 4 2 0 | 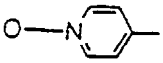 | 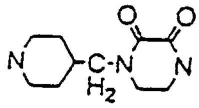 | 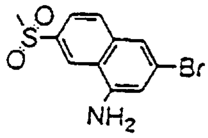 |
| 4 2 1 | 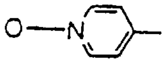 | 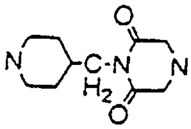 | 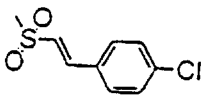 |
| 4 2 2 | 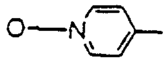 | 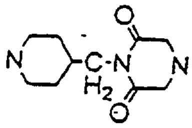 | 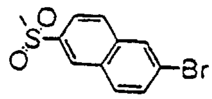 |
| 4 2 3 | 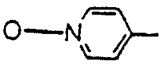 | 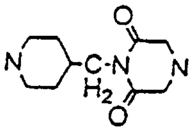 | 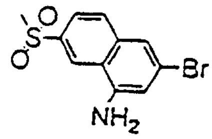 |
| 4 2 4 | 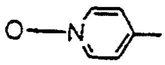 | 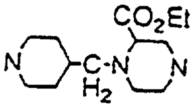 | 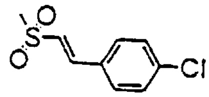 |
| 4 2 5 | 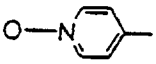 | 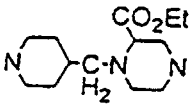 | 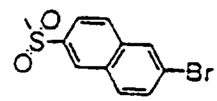 |
| 4 2 6 | 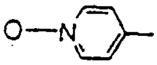 | 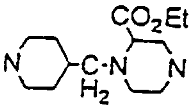 | 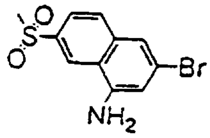 |
| 4 2 7 | 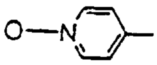 | 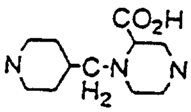 | 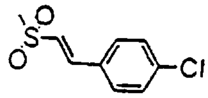 |
| 4 2 8 | 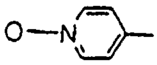 | 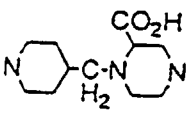 | 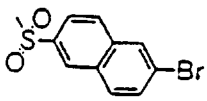 |
| 4 2 9 | 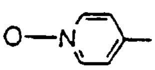 | 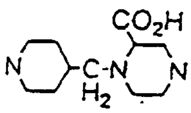 | 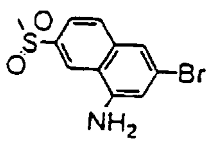 |
| 4 3 0 | 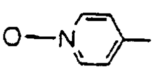 | 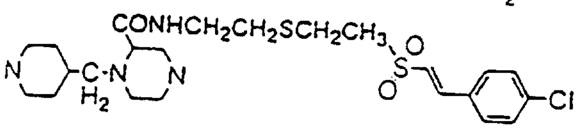 | |

# FIG.37

| | S 1 | S 2 | S 3 |
|---|---|---|---|
| 4 3 1 | 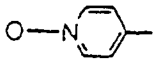 | 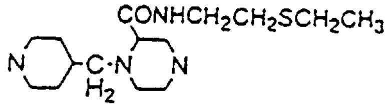 | 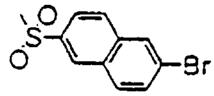 |
| 4 3 2 | 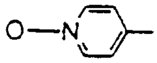 | 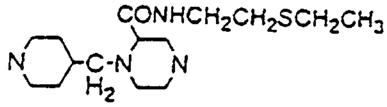 | 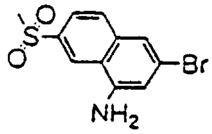 |
| 4 3 3 | 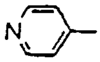 | 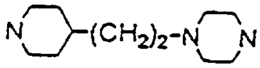 | 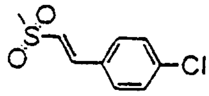 |
| 4 3 4 | 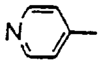 | 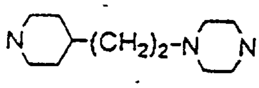 | 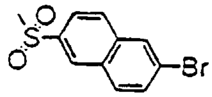 |
| 4 3 5 | 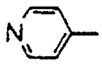 | 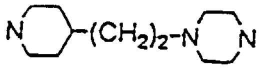 | 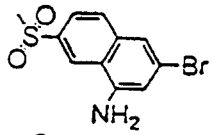 |
| 4 3 6 | 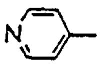 | 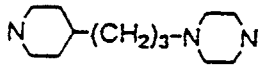 | 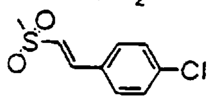 |
| 4 3 7 | 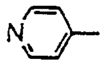 | 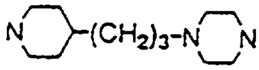 | 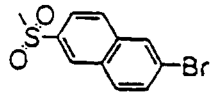 |
| 4 3 8 | 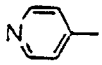 | 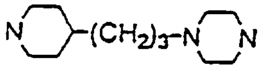 | 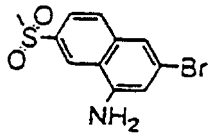 |
| 4 3 9 | 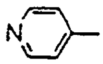 | 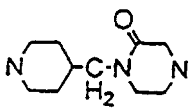 | 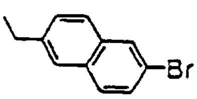 |
| 4 4 0 | 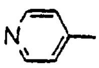 | 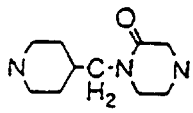 | 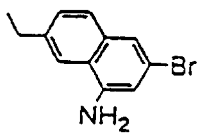 |
| 4 4 1 | 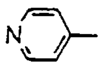 | 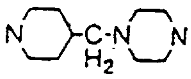 | 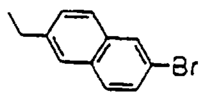 |
| 4 4 2 | 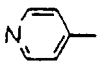 | 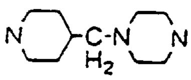 | 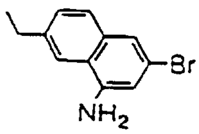 |

## FIG.38

| | S 1 | S 2 | S 3 |
|---|---|---|---|
| 443 | 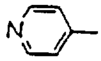 | 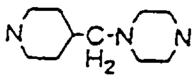 | 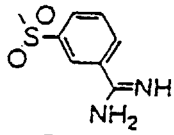 |
| 444 | 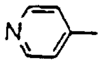 | 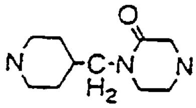 | 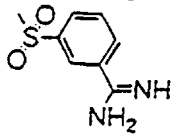 |
| 445 | 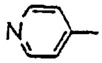 | 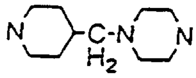 | 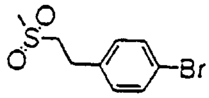 |
| 446 | 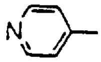 | 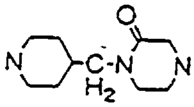 | 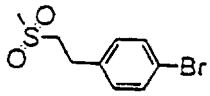 |
| 447 | 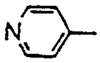 | 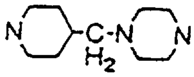 | 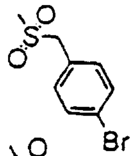 |
| 448 | 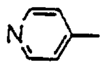 | 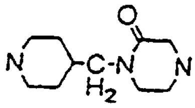 | 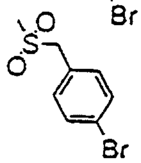 |
| 449 | 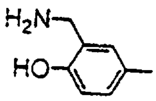 | 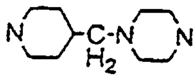 | 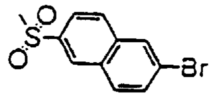 |
| 450 | 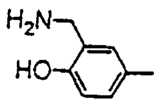 | 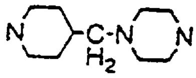 | 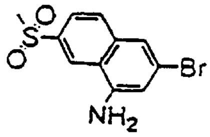 |
| 451 | 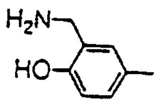 | 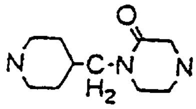 | 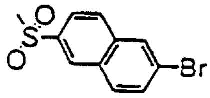 |
| 452 | 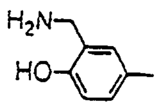 | 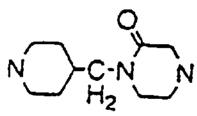 | 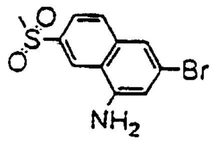 |
| 453 | 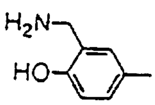 | 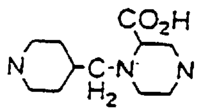 | 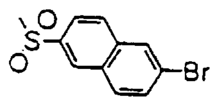 |
| 454 | 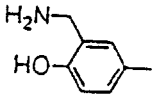 | 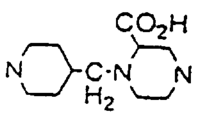 | 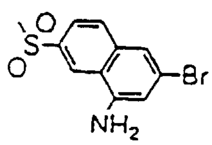 |

FIG.39

| | S 1 | S 2 | S 3 |
|---|---|---|---|
| 455 | 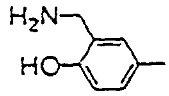 | 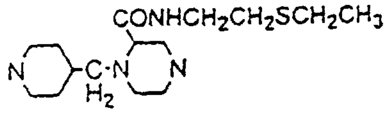 | 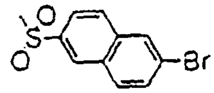 |
| 456 | 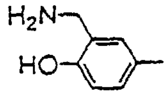 | 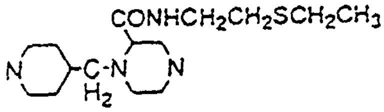 | 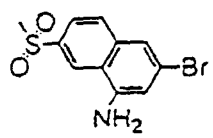 |
| 457 | 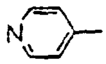 | 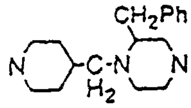 | 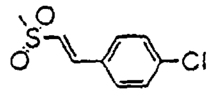 |
| 458 | 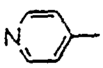 | 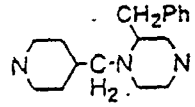 | 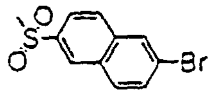 |
| 459 | 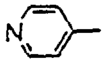 | 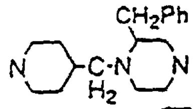 | 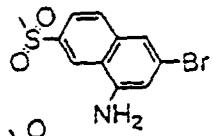 |
| 460 | 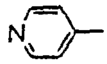 | 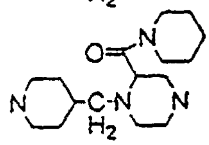 | 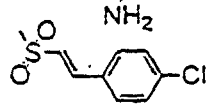 |
| 461 | 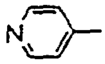 | 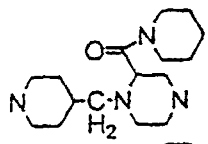 | 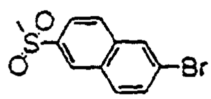 |
| 462 | 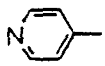 | 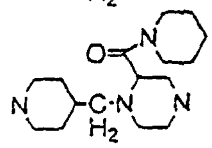 | 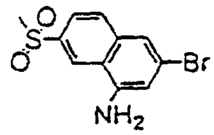 |
| 463 | 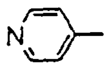 | 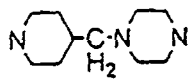 | 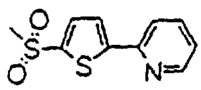 |
| 464 | 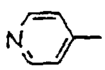 | 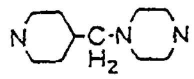 | 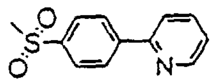 |
| 465 | 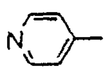 | 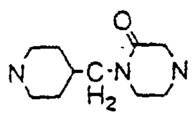 | 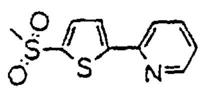 |
| 466 | 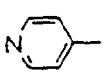 | 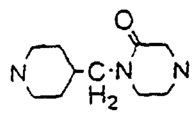 | 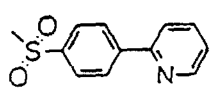 |

# FIG.40

| Ex.No. | NMR (ppm)<br>(*:300MHz,Without asterisk:270MHz) |
|---|---|
| 1 | DMSO-$d_6$*: 8. 20-8. 10 (2H, m), 7. 81 (2H, d, J=9Hz), 7. 52 (2H, d, J=9Hz), 7. 46-7. 30 (2H, m), 6. 90-6. 75 (2H, m), 3. 65-3. 55 (2H, m), 3. 43-3. 18 (4H, m), 2. 75-2. 62 (2H, m), 2. 61-2. 44 (4H, m), 2. 44-2. 31 (1H, m), 1. 90-1. 80 (2H, m), 1. 58-1. 40 (2H, m) |
| 2 | DMSO-$d_6$*: 8. 17-8. 11 (2H, m), 7. 81 (2H, d, J=9Hz), 7. 52 (2H, d, J=9Hz), 7. 44-7. 29 (2H, m), 6. 83-6. 77 (2H, m), 3. 63-3. 52 (2H, m), 3. 31-3. 20 (4H, m), 2. 72-2. 58 (2H, m), 2. 47-2. 37 (4H, m), 2. 17 (2H, d, J=7Hz), 1. 86-1. 75 (2H, m), 1. 70-1. 55 (1H, m), 1. 25-1. 05 (2H, m) |
| 3 | $CDCl_3$*: 8. 26-8. 17 (2H, m), 7. 47-7. 37 (5H, m), 6. 73-6. 62 (3H, m), 3. 93-3. 80 (2H, m), 3. 30-3. 16 (4H, m), 112. 89-2. 75 (2H, m), 2. 60-2. 47 (4H, m), 2. 24 (2H, d, J=7Hz), 1. 89-1. 65 (3H, m), 1. 30-1. 13 (2H, m) |
| 4 | $CDCl_3$: 8. 36-8. 32 (1H, m), 8. 20-8. 11 (2H, m), 8. 04-7. 92 (3H, m), 7. 76 (1H, dd, J=2, 9Hz), 7. 72-7. 60 (2H, m), 6. 69-6. 62 (2H, m), 3. 93-3. 82 (2H, m), 3. 16-3. 04 (4H, m), 2. 98-2. 78 (2H, m), 2. 55-2. 44 (4H, m), 2. 19 (2H, d, J=7Hz), 1. 86-1. 63 (3H, m), 1. 29-1. 04 (2H, m) |
| 5 | $CDCl_3$: 8. 32-8. 29 (1H, m), 8. 22-8. 16 (2H, m), 8. 01-7. 86 (3H, m), 7. 77 (1H, dd, J=2, 9Hz), 7. 58 (1H, dd, J=2, 9Hz), 6. 73-6. 65 (2H, m), 3. 97-3. 84 (2H, m), 3. 16-2. 88 (6H, m), 2. 56-2. 43 (4H, m), 2. 19 (2H, d, J=7Hz), 1. 90-1. 67 (3H, m), 1. 25-1. 06 (2H, m) |
| 6 | $CDCl_3$: 8. 32-8. 28 (1H, m), 8. 23-8. 18 (2H, m), 8. 11 (1H, d, J=2Hz), 7. 90 (1H, d, J=9Hz), 7. 85 (1H, d, J=9Hz), 7. 78 (1H, dd, J=2, 9Hz), 7. 71 (1H, dd, J=2, 9Hz), 6. 62-6. 57 (2H, m), 3. 88-3. 74 (2H, m), 3. 18-2. 99 (4H, m), 2. 88-2. 70 (2H, m), 2. 59-2. 42 (4H, m), 2. 18 (2H, d, J=7Hz), 1. 81-1. 57 (3H, m), 1. 35-1. 04 (2H, m) |
| 7 | $CDCl_3$*: 8. 22-8. 16 (2H, m), 7. 95-7. 86 (2H, m), 7. 80 (1H, s), 7. 56-7. 46 (2H, m), 6. 75-6. 68 (2H, m), 4. 00-3. 92 (2H, m), 3. 25-3. 13 (4H, m), 3. 06-2. 95 (2H, m), 2. 60-2. 50 (4H, m), 2. 23 (2H, d, J=7Hz), 1. 93-1. 72 (3H, m), 1. 28-1. 10 (2H, m) |
| 8 | DMSO-$d_6$: 8. 21 (1H, dd, J=5, 8Hz), 8. 14-8. 08 (2H, m), 8. 04 (1H, s), 7. 94-7. 87 (1H, m), 7. 51 (1H, dt, J=3, 9Hz), 6. 91-6. 83 (2H, m), 4. 03-3. 87 (2H, m), 3. 14-2. 97 (4H, m), 2. 95-2. 78 (2H, m), 2. 57-2. 49 (4H, m), 2. 15 (2H, d, J=7Hz), 1. 85-1. 62 (3H, m), 1. 12-0. 93 (2H, m) |
| 9 | $CDCl_3$*: 8. 25-8. 17 (2H, m), 7. 90-7. 80 (2H, m), 7. 76 (1H, s), 7. 48-7. 42 (1H, m), 6. 67-6. 59 (2H, m), 3. 90-3. 78 (2H, m), 3. 23-3. 11 (4H, m), 2. 85-2. 74 (2H, m), 2. 60-2. 48 (4H, m), 2. 21 (2H, d, J=7Hz), 1. 85-1. 53 (3H, m), 1. 30-1. 09 (2H, m) |
| 10 | $CDCl_3$*: 8. 24-8. 11 (2H, m), 7. 95 (1H, s), 7. 49-7. 36 (2H, m), 6. 87-6. 66 (3H, m), 4. 06-3. 90 (5H, m), 3. 25-2. 95 (6H, m), 2. 61-2. 40 (4H, m), 2. 22 (2H, d, J=7Hz), 1. 95-1. 72 (3H, m), 1. 33-1. 07 (2H, m) |

FIG.41

| Ex.No. | NMR (ppm) (*:300MHz,Without asterisk:270MHz) |
|---|---|
| 11 | $CDCl_3$*:8.24-8.18 (2H, m), 7.77 (1H, d, J=9Hz), 7.71 (1H, s), 7.29 (1H, d, J=2Hz), 7.09 (1H, dd, J=2, 9Hz), 6.65-6.59 (2H, m), 3.91 (3H, s), 3.89-3.78 (2H, m), 3.24-3.06 (4H, m), 2.85-2.72 (2H, m), 2.58-2.44 (4H, m), 2.21 (2H, d, J=7Hz), 1.85-1.56 (3H, m), 1.35-1.08 (2H, m) |
| 12 | $CDCl_3$:8.23-8.15 (2H, m), 7.90-7.76 (2H, m), 7.56-7.45 (2H, m), 6.67-6.60 (2H, m), 4.33 (2H, s), 4.18 (2H, q, J=7Hz), 3.90-3.80 (2H, m), 3.32-3.20 (4H, m), 2.90-2.76 (2H, m), 2.57-2.45 (4H, m), 2.20 (2H, d, J=7Hz), 1.85-1.62 (3H, m), 1.30-1.10 (2H, m), 1.26 (3H, t, J=7Hz) |
| 13 | $CDCl_3$*:8.25-8.17 (2H, m), 8.14-8.07 (1H, m), 7.93-7.86 (1H, m), 7.62-7.53 (2H, m), 6.71-6.63 (2H, m), 3.95-3.84 (2H, m), 3.47-3.35 (4H, m), 2.94-2.80 (2H, m), 2.60-2.49 (4H, m), 2.24 (2H, d, J=7Hz), 1.91-1.67 (3H, m), 1.35-1.12 (2H, m) |
| 14 | $CDCl_3$:8.48-8.40 (1H, m), 8.38-8.17 (2H, m), 7.60-7.22 (3H, m), 6.74-6.64 (2H, m), 4.00-3.85 (2H, m), 3.60-3.44 (4H, m), 2.98-2.82 (2H, m), 2.75-2.61 (4H, m), 2.31 (2H, d, J=7Hz), 1.97-1.50 (3H, m), 1.40-1.16 (2H, m) |
| 15 | $CDCl_3$:8.25-8.14 (2H, m), 7.75-7.66 (1H, m), 7.61-7.33 (4H, m), 6.77-6.66 (2H, m), 4.00-3.88 (2H, m), 3.38-3.23 (4H, m), 3.05-2.90 (2H, m), 2.59-2.42 (4H, m), 2.22 (2H, d, J=7Hz), 1.93-1.71 (3H, m), 1.31-1.10 (2H, m) |
| 16 | $CDCl_3$:8.21-8.16 (2H, m), 7.68 (1H, d, J=2Hz), 7.51 (1H, d, J=9Hz), 7.44 (1H, dd, J=2, 9Hz), 7.31 (1H, s), 6.69-6.62 (2H, m), 3.95-3.85 (2H, m), 3.35-3.25 (4H, m), 2.93-2.80 (2H, m), 2.55-2.47 (4H, m), 2.22 (2H, d, J=7Hz), 1.90-1.60 (3H, m), 1.35-1.10 (2H, m) |
| 17 | $CDCl_3$:8.31-8.13 (3H, m), 8.07 (1H, d, J=9Hz), 7.82 (1H, dd, J=2, 9Hz), 6.66-6.53 (2H, m), 3.88-3.71 (2H, m), 3.14-2.96 (4H, m), 2.92 (3H, s), 2.85-2.68 (2H, m), 2.61-2.39 (4H, m), 2.19 (2H, d, J=7Hz), 1.90-1.55 (3H, m), 1.31-1.03 (2H, m) |
| 18 | $CDCl_3$:8.25-8.18 (2H, m), 7.83 (2H, d, J=9Hz), 7.74 (2H, d, J=9Hz), 7.66-7.58 (2H, m), 7.56-7.40 (3H, m), 6.65-6.57 (2H, m), 3.88-3.76 (2H, m), 3.16-3.02 (4H, m), 2.86-2.71 (2H, m), 2.60-2.47 (4H, m), 2.21 (2H, d, J=7Hz), 1.85-1.59 (3H, m), 1.30-1.08 (2H, m) |
| 19 | $CDCl_3+CD_3OD$:8.64-8.55 (1H, m), 8.36-7.97 (3H, m), 7.47 (1H, d, J=8Hz), 6.85-6.59 (2H, m), 4.03-3.86 (2H, m), 3.38-3.13 (4H, m), 3.07-2.87 (2H, m), 2.58-2.06 (6H, m), 1.94-1.68 (3H, m), 1.37-1.07 (2H, m) |

FIG.42

| Ex.No. | NMR (ppm) (*:300MHz,Without asterisk:270MHz) |
|---|---|
| 20 | $CDCl_3$:8. 25-8. 18 (2H, m), 7. 87-7. 85 (1H, m), 7. 55-7. 44 (2H, m), 6. 70-6. 65 (2H, m), 3. 98-3. 86 (2H, m), 3. 67 (2H, s), 3. 25-3. 16 (4H, m), 3. 03-2. 90 (2H, m), 2. 50-2. 41 (4H, m), 2. 20-2. 15 (2H, m), 2. 05-1. 80 (3H, m), 1. 30-1. 10 (2H, m) |
| 21 | $CDCl_3$:8. 31 (1H, s), 8. 20-8. 10 (2H, m), 8. 03 (1H, d, J=9Hz), 7. 95-7. 82 (2H, m), 7. 78-7. 70 (1H, m), 7. 68-7. 58 (1H, m), 6. 62- 6. 57 (2H, m), 3. 89-3. 78 (2H, m), 3. 15-2. 98 (4H, m), 2. 88-2. 73 (2H, m), 2. 56-2. 44 (4H, m), 2. 37 (3H, s), 2. 18 (2H, d, J=7Hz), 1. 80-1. 60 (3H, m), 1. 28-1. 05 (2H, m) |
| 22 | $CDCl_3$*:8. 28-8. 13 (3H, m), 7. 96 (1H, d, J=9Hz), 7. 73-7. 64 (1H, m), 7. 47-7. 37 (2H, m), 6. 96-6. 90 (1H, m), 6. 63-6. 54 (2H, m), 4. 40-4. 10 (1H, m), 3. 90-3. 74 (2H, m), 3. 16-2. 97 (4H, m), 2. 83-2. 68 (2H, m), 2. 58-2. 40 (4H, m), 2. 17 (2H, d, J=7Hz), 1. 80-1. 60 (3H, m), 1. 22-1. 02 (2H, m) |
| 23 | $CDCl_3$:8. 28-8. 20 (2H, m), 7. 49 (1H, d, J=16Hz), 7. 48-7. 38 (4H, m), 6. 69-6. 58 (3H, m), 3. 89 (2H, s), 3. 94-3. 78 (2H, m), 3. 57-3. 44 (4H, m), 3. 32 (2H, d, J=7Hz), 2. 88-2. 72 (2H, m), 2. 05-1. 87 (1H, m), 1. 83-1. 64 (2H, m), 1. 40-1. 22 (2H, m) |
| 24 | $CDCl_3$*:8. 37-8. 34 (1H, m), 8. 26-8. 19 (2H, m), 8. 16-8. 11 (1H, m), 7. 93 (1H, d, J=9Hz), 7. 88 (1H, d, J=9Hz), 7. 80 (1H, dd, J=2, 9Hz), 7. 75 (1H, dd, J=2, 9Hz), 6. 66-6. 59 (2H, m), 3. 89-3. 77 (2H, m), 3. 80 (2H, s), 3. 49-3. 38 (4H, m), 3. 25 (2H, d, J=7Hz), 2. 84-2. 72 (2H, m), 1. 99-1. 83 (1H, m), 1. 68-1. 57 (2H, m), 1. 35-1. 17 (2H, m) |
| 25 | $CDCl_3$:8. 29 (1H, s), 8. 20-8. 11 (2H, m), 7. 91-7. 83 (3H, m), 7. 73 (1H, dd, J=2, 9Hz), 7. 55 (1H, dd, J=2, 9Hz), 6. 57-6. 50 (2H, m), 3. 78-3. 68 (4H, m), 3. 40-3. 30 (4H, m), 3. 17 (2H, d, J=8Hz), 2. 74-2. 60 (2H, m), 1. 90-1. 70 (1H, m), 1. 60-1. 48 (2H, m), 1. 28-1. 07 (2H, m) |
| 26 | $CDCl_3$*:8. 41-7. 93 (6H, m), 7. 86-7. 64 (3H, m), 6. 75-6. 52 (2H, m), 3. 88-3. 77 (4H, m), 3. 48-3. 38 (4H, m), 3. 24 (2H, d, J=8Hz), 2. 85-2. 71 (2H, m), 1. 99-1. 84 (1H, m), 1. 68-1. 58 (2H, m), 1. 34-1. 17 (2H, m) |
| 27 | $CDCl_3$*:8. 35-8. 32 (1H, m), 8. 26-8. 19 (2H, m), 7. 94-7. 88 (2H, m), 7. 77-7. 70 (2H, m), 7. 54-7. 48 (1H, m), 6. 65-6. 58 (2H, m), 3. 86-3. 76 (4H, m), 3. 48-3. 37 (4H, m), 3. 27-3. 21 (2H, m), 2. 83-2. 70 (2H, m), 2. 59 (3H, s), 1. 97-1. 81 (1H, m), 1. 66-1. 56 (2H, m), 1. 29-1. 16 (2H, m) |
| 28 | $CDCl_3$*:8. 45-8. 43 (1H, m), 8. 37-8. 34 (1H, m), 8. 26-8. 21 (2H, m), 8. 14-8. 09 (2H, m), 7. 94-7. 88 (1H, m), 7. 84-7. 79 (1H, m), 6. 65-6. 60 (2H, m), 3. 92-3. 79 (4H, m), 3. 54-3. 40 (4H, m), 3. 30-3. 24 (2H, m), 2. 88-2. 75 (2H, m), 2. 00-1. 50 (3H, m), 1. 37-1. 16 (2H, m) |

# FIG.43

| Ex.No. | NMR (ppm) (*:300MHz,Without asterisk:270MHz) |
|---|---|
| 29 | $CDCl_3$*:8.30-8.24 (1H, m), 8.13-8.05 (2H, m), 7.92-7.86 (1H, m), 7.84-7.78 (1H, m), 7.72-7.65 (1H, m), 7.32-7.20 (2H, m), 6.70-6.61 (2H, m), 3.93-3.74 (4H, m), 3.49-3.36 (4H, m), 3.28-3.19 (2H, m), 2.85-2.61 (2H, m), 1.96-1.82 (1H, m), 1.60-1.50 (2H, m), 1.35-1.10 (2H, m) |
| 30 | $CDCl_3$*:8.26-8.16 (3H, m), 7.98-7.92 (1H, m), 7.86-7.66 (4H, m), 6.69-6.64 (2H, m), 3.97-3.83 (4H, m), 3.67-3.58 (2H, m), 3.55-3.44 (2H, m), 3.34-3.26 (2H, m), 2.94-2.80 (2H, m), 2.08-1.88 (1H, m), 1.76-1.64 (2H, m), 1.38-1.19 (2H, m) |
| 31 | $CDCl_3$*:8.31 (1H, s), 8.26-8.15 (2H, m), 7.98-7.88 (3H, m), 7.80-7.74 (1H, m), 7.63-7.56 (1H, m), 6.63-6.56 (2H, m), 4.25-4.10 (2H,. m), 3.85-3.76 (2H, m), 3.63-3.52 (1H, m), 3.37-3.18 (3H, m), 3.07-2.99 (1H, m), 2.94-2.84 (1H, m), 2.82-2.70 (2H, m), 2.62-2.46 (2H, m), 2.37-2.27 (1H, m), 1.80-1.54 (3H, m), 1.34-1.23 (3H, m), 1.20-1.03 (2H, m) |
| 32 | $CDCl_3$:8.31 (1H, s), 8.20-8.16 (2H, m), 7.97-7.88 (3H, m), 7.80-7.74 (1H, m), 7.62-7.55 (1H, m), 6.68-6.62 (2H, m), 3.95-3.79 (3H, m), 3.66-3.55 (1H, m), 3.36-3.30 (2H, m), 3.06-2.78 (5H, m), 2.65-2.40 (3H, m), 2.25-2.14 (1H, m), 1.89-1.70 (3H, m), 1.30-1.10 (2H, m) |
| 33 | DMSO-$d_6$:8.51 (1H, s), 8.34-8.02 (5H, m), 7.87-7.68 (2H, m), 6.82-6.68 (2H, m), 3.95-3.74 (2H, m), 3.57-2.20 (11H, m), 1.78-1.52 (3H, m), 1.07-0.82 (2H, m) |
| 34 | $CDCl_3$*:8.37-8.27 (1H, m), 8.26-8.15 (2H, m), 8.00-7.92 (3H, m), 7.81-7.71 (1H, m), 7.64-7.53 (1H, m), 6.66-6.53 (2H, m), 4.26-4.10 (2H, m), 3.90-3.34 (5H, m), 3.33-1.94 (10H, m), 1.90-1.57 (3H, m), 1.35-0.94 (5H, m) |
| 35 | $CDCl_3$:8.32 (1H, s), 8.28-8.17 (2H, m), 8.00-7.89 (3H, m), 7.85-7.75 (1H, m), 7.67-7.57 (1H, m), 6.65-6.56 (2H, m), 6.33-6.25 (1H, m), 5.39-5.32 (1H, m), 3.91-3.75 (2H, m), 3.75-3.65 (1H, m), 3.65-3.52 (1H, m), 3.14-2.99 (2H, m), 2.87-2.62 (4H, m), 2.46-2.31 (2H, m), 2.29-2.16 (1H, m), 1.91-1.53 (3H, m), 1.32-1.04 (2H, m) |
| 36 | $CDCl_3$*:8.34-8.29 (1H, m), 8.24-8.18 (2H, m), 7.98-7.90 (3H, m), 7.81-7.75 (1H, m), 7.63-7.56 (1H, m), 6.73-6.66 (1H, m), 6.64-6.34 (2H, m), 3.94-3.79 (2H, m), 3.77-3.69 (1H, m), 3.66-3.56 (1H, m), 3.51-3.31 (2H, m), 3.14-2.98 (2H, m), 2.88-2.55 (6H, m), 2.47-2.29 (4H, m), 2.22-2.13 (1H, m), 1.92-1.61 (3H, m), 1.40-1.09 (5H, m) |
| 37 | $CDCl_3$*:8.38-8.14 (3H, m), 8.00-7.84 (3H, m), 7.81-7.69 (1H, m), 7.64-7.54 (1H, m), 6.66-6.51 (2H, m), 3.89-3.24 (2H, m), 3.38-2.92 (6H, m), 2.84-2.67 (2H, m), 2.50-2.11 (3H, m), 2.18 (3H, s), 1.89-1.56 (3H, m), 1.36-1.00 (2H, m) |

# FIG.44

| Ex.No. | NMR (ppm) (*:300MHz,Without asterisk:270MHz) |
|---|---|
| 38 | $CDCl_3$:8.29 (1H, s), 8.31-8.03 (2H, m), 8.03-7.82 (3H, m), 7.82-7.69 (1H, m), 7.57 (1H, dd, J=2, 9Hz), 6.74-6.50 (2H, m), 3.95-3.75 (2H, m), 3.65 (2H, d, J=11Hz), 3.60-3.43 (1H, m), 3.28-2.40 (6H, m), 3.15 (3H, s), 2.94 (3H, s), 2.26 (1H, dd, J=9, 12Hz), 2.12-1.97 (1H, m), 1.87 (1H, d, J=13Hz), 1.80-1.55 (2H, m), 1.35-1.02 (2H, m) |
| 39 | $CDCl_3$:8.34 (1H, s), 8.25-8.18 (2H, m), 7.98-7.90 (3H, m), 7.77 (1H, dd, J=2, 9Hz), 7.64-7.58 (1H, m), 6.64-6.57 (2H, m), 4.38-4.04 (5H, m), 3.96-3.75 (3H, m), 3.46 (1H, d, J=17Hz), 3.07-2.96 (1H, m), 2.88-2.68 (2H, m), 2.66-2.55 (1H, m), 1.93-1.75 (1H, m), 1.73-1.55 (2H, m), 1.32 (3H, t, J=7Hz), 1.32-1.14 (2H, m) |
| 40 | $CD_3OD$*:8.47 (1H, s), 8.16-7.99 (5H, m), 7.90-7.83 (1H, m), 7.64 (1H, dd, J=2, 9Hz), 7.02 (2H, d, J=8Hz), 4.18-3.91 (5H, m), 3.81 (1H, dd, J=8, 14Hz), 3.58 (1H, d, J=16Hz), 3.30-3.20 (1H, m), 3.12-2.93 (2H, m), 2.72 (1H, dd, J=7, 14Hz), 2.08-1.92 (1H, m), 1.82-1.71 (1H, m), 1.62-1.52 (1H, m), 1.31-1.04 (2H, m) |
| 41 | $CDCl_3$:8.36 (1H, s), 8.21 (2H, d, J=7Hz), 8.00-7.90 (3H, m), 7.84-7.76 (1H, m), 7.62 (1H, dd, J=2, 9Hz), 6.58 (2H, d, J=7Hz), 4.28-4.12 (2H, m), 3.95-3.72 (5H, m), 3.48-3.35 (2H, m), 2.84-2.63 (4H, m), 2.05-1.46 (3H, m), 1.34-1.13 (2H, m) |
| 42 | $CDCl_3$*:8.36 (1H, s), 8.26-8.19 (2H, m), 7.98-7.92 (3H, m), 7.80 (1H, dd, J=2, 9Hz), 7.61 (1H, dd, J=2, 9Hz), 6.62-6.55 (2H, m), 4.17 (1H, d, J=17Hz), 4.05 (1H, d, J=12Hz), 3.94-3.70 (3H, m), 3.70-3.43 (3H, m), 3.38 (3H, s), 3.38 (1H, d, J=17Hz), 2.88-2.66 (4H, m), 2.08-1.90 (1H, m), 1.71-1.54 (2H, m), 1.38-1.07 (2H, m) |
| 43 | $CDCl_3$:8.37 (1H, s), 8.23-8.19 (2H, m), 7.98-7.93 (3H, m), 7.80 (1H, dd, J=2, 9Hz), 7.62 (1H, dd, J=2, 9Hz), 6.60-6.56 (2H, m), 4.40-4.33 (1H, m), 4.25-4.11 (2H, m), 4.04 (1H, d, J=12Hz), 3.95-3.74 (3H, m), 3.61-3.50 (1H, m), 3.38 (1H, d, J=17Hz), 2.88-2.67 (4H, m), 2.10 (3H, s), 2.03-1.86 (1H, m), 1.70-1.55 (2H, m), 1.38-1.16 (2H, m) |
| 44 | $CDCl_3$*:8.27-8.19 (2H, m), 7.47 (1H, d, J=15Hz), 7.47-7.38 (4H, m), 6.67-6.60 (2H, m), 6.59 (1H, d, J=15Hz), 4.28-4.07 (5H, m), 4.00-3.82 (3H, m), 3.73 (1H, d, J=17Hz), 3.32 (1H, dd, J=4, 13Hz), 2.89-2.75 (2H, m), 2.67 (1H, dd, J=8, 14Hz), 2.00-1.63 (3H, m), 1.40-1.20 (2H, m), 1.30 (3H, t, J=7Hz) |
| 45 | $CD_3OD$*:8.08-8.02 (2H, m), 7.69-7.63 (1H, m), 7.52-7.37 (4H, m), 7.17-7.00 (3H, m), 4.29-4.10 (2H, m), 4.09-3.75 (4H, m), 3.62-3.02 (4H, m), 2.90-2.73 (1H, m), 2.21-2.05 (1H, m), 1.95-1.73 (2H, m), 1.43-1.12 (2H, m) |

# FIG.45

| Ex.No. | NMR (ppm) (*:300MHz.Without asterisk:270MHz) |
|---|---|
| 46 | $CDCl_3$+$CD_3OD$:8. 48-8. 44 (1H, m), 8. 14-8. 00 (5H, m), 7. 85 (1H, dd, J=2, 9Hz), 7. 65 (1H, dd, J=2, 9Hz), 6. 72 (2H, d, J=6Hz), 4. 19 (1H, t, J=3Hz), 4. 13-4. 04 (2H, m), 3. 96-3. 80 (3H, m), 3. 55 (1H, d, J=17Hz), 3. 24 (1H, dd, J=4, 12Hz), 2. 86-2. 69 (2H, m), 2. 62 (1H, dd, J=7, 14Hz), 1. 98-1. 80 (1H, m), 1. 75-1. 50 (2H, m), 1. 35-1. 10 (2H, m) |
| 47 | $CDCl_3$:8. 37-8. 35 (1H, m), 8. 17 (2H, d, J=6Hz), 7. 98-7. 92 (3H, m), 7. 83-7. 77 (1H, m), 7. 65-7. 58 (1H, m), 7. 46 (0. 7H, d, J=8Hz), 6. 84 (0. 3H, d, J=7Hz), 6. 59 (2H, d, J=6Hz), 4. 96-4. 87 (0. 3H, m), 4. 18-4. 00 (1. 7H, m), 3. 95-3. 48 (5H, m), 3. 19-3. 05 (1H, m), 2. 90-2. 64 (3H, m), 2. 15-1. 95 (1H, m), 1. 70-1. 45 (2H, m), 1. 35-1. 10 (2H, m) |
| 48 | $CDCl_3$*:8. 37-8. 33 (1H, m), 8. 22 (2H, d, J=6Hz), 7. 97-7. 90 (3H, m), 7. 75 (1H, dd, J=2, 9Hz), 7. 61 (1H, dd, J=2, 9Hz), 6. 60 (2H, d, J=7Hz), 4. 37 (1H, t, J=3Hz), 4. 09 (1H, d, J=17Hz), 3. 97-3. 63 (8H, m), 3. 63 (1H, d, J=17Hz), 3. 58-3. 48 (4H, m), 3. 28 (1H, dd, J=3, 12Hz), 2. 83-2. 70 (2H, m), 2. 42 (1H, dd, J=8, 14Hz), 1. 95-1. 60 (3H, m), 1. 35-1. 15 (2H, m) |
| 49 | $CDCl_3$*:8. 37-8. 34 (1H, m), 8. 21 (2H, d, J=7Hz), 7. 97-7. 89 (3H, m), 7. 75 (1H, dd, J=2, 9Hz), 7. 61 (1H, dd, J=2, 9Hz), 6. 61 (2H, d, J=7Hz), 4. 47 (1H, t, J=4Hz), 3. 97 (1H, d, J=17Hz), 3. 90-3. 73 (4H, m), 3. 76 (1H, d, J=17Hz), 3. 43 (1H, dd, J=4, 13Hz), 3. 11 (3H, s), 2. 96 (3H, s), 2. 86-2. 72 (2H, m), 2. 42 (1H, dd, J=8, 14Hz), 1. 97-1. 79 (1H, m), 1. 79-1. 58 (2H, m), 1. 30-1. 12 (2H, m) |
| 50 | $CDCl_3$*:8. 48-8. 43 (1H, m), 8. 23-8. 12 (2H, m), 7. 98-7. 90 (3H, m), 7. 82-7. 74 (1H, m), 7. 64-7. 57 (1H, m), 6. 64-6. 54 (2H, m), 4. 16-4. 08 (1H, m), 3. 98-3. 67 (6H, m), 3. 82 (3H, s), 3. 50-3. 38 (1H, m), 2. 70-2. 50 (4H, m), 1. 85-1. 70 (1H, m), 1. 65-1. 50 (2H, m), 1. 25-1. 05 (2H, m) |
| 51 | $CDCl_3$*:8. 37-8. 32 (1H, m), 8. 25-8. 17 (2H, m), 7. 97-7. 90 (3H, m), 7. 78-7. 73 (1H, m), 7. 64-7. 58 (1H, m), 6. 63-6. 57 (2H, m), 4. 45-4. 40 (1H, m), 4. 05 (1H, d, J=16Hz), 3. 95-3. 60 (10H, m), 3. 32-3. 24 (1H, m), 2. 85-2. 73 (2H, m), 2. 50-2. 30 (1H, m), 2. 10-1. 50 (5H, m), 1. 40-1. 20 (4H, m) |
| 52 | $CDCl_3$*:8. 36 (1H, d, J=1Hz), 8. 22-8. 19 (2H, m), 7. 97-7. 94 (3H, m), 7. 82-7. 78 (1H, m), 7. 61 (1H, dd, J=2, 9Hz), 6. 59-6. 57 (2H, m), 4. 25-4. 16 (2H, m), 3. 89-3. 76 (3H, m), 3. 36 (1H, d, J=17Hz), 3. 30-3. 22 (1H, m), 3. 07 (1H, dd, J=10, 13Hz), 2. 96 (1H, dd, J=4, 13Hz), 2. 79-2. 68 (3H, m), 2. 63 (1H, dd, J=8, 14Hz), 2. 05-1. 87 (1H, m), 1. 73-1. 57 (2H, m), 1. 35-1. 14 (2H, m) |

# FIG.46

| Ex.No. | NMR (ppm) (*:300MHz.Without asterisk:270MHz) |
|---|---|
| 53 | $CDCl_3$*:8. 36 (1H, d, J=2Hz), 8. 21 (2H, dd, J=2, 5Hz), 7. 97-7. 93 (3H, m), 7. 81 (1H, dd, J=2, 9Hz), 7. 64-7. 60 (1H, m), 6. 58 (2H, dd, J=2, 5Hz), 4. 26 (1H, d, J=10Hz), 4. 21 (1H, d, J=15Hz), 3. 94-3. 64 (7H, m), 3. 35-3. 24 (2H, m), 2. 91-2. 52 (9H, m), 2. 46-2. 36 (1H, m), 2. 12-1. 87 (1H, m), 1. 68-1. 57 (2H, m), 1. 37-1. 14 (2H, m) |
| 54 | $CDCl_3$*:8. 36 (1H, s), 8. 21 (2H, d, J=6Hz), 7. 96-7. 93 (3H, m), 7. 81 (1H, dd, J=2, 9Hz), 7. 63-7. 59 (1H, m), 6. 58 (2H, d, J=6Hz), 4. 23-4. 18 (2H, m), 3. 89-3. 76 (3H, m), 3. 29 (1H, d, J=17Hz), 3. 30-3. 21 (1H, m), 2. 87-2. 55 (5H, m), 2. 38-2. 24 (1H, m), 2. 31 (6H, s), 2. 04-1. 87 (1H, m), 1. 72-1. 57 (2H, m), 1. 34-1. 16 (2H, m) |
| 55 | $CDCl_3$:8. 37-8. 33 (1H, m), 8. 20 (2H, d, J=6Hz), 8. 01-7. 92 (3H, m), 7. 83-7. 75 (1H, m), 7. 67-7. 59 (1H, m), 6. 57 (2H, d, J=6Hz), 6. 29-6. 20 (1H, m), 4. 28 (1H, d, J=17Hz), 3. 98 (1H, d, J=13Hz), 3. 93-3. 62 (5H, m), 3. 48-3. 14 (2H, m), 2. 92 (1H, dd, J=7, 14Hz), 2. 82-2. 64 (3H, m), 2. 06 (3H, s), 2. 03-1. 87 (1H, m), 1. 75-1. 54 (2H, m), 1. 40-1. 17 (2H, m) |
| 56 | $CDCl_3$:8. 37 (1H, s), 8. 22-8. 16 (2H, m), 8. 02-7. 90 (3H, m), 7. 82-7. 24 (1H, m), 7. 65-7. 58 (1H, m), 6. 62-6. 52 (2H, m), 5. 60-5. 40 (1H, m), 4. 31 (1H, d, J=17Hz), 4. 17 (1H, d, J=13Hz), 3. 97-3. 74 (3H, m), 3. 68-3. 59 (1H, m), 3. 46-3. 31 (3H, m), 3. 05 (3H, s), 2. 81-2. 62 (4H, m), 2. 01-1. 85 (1H, m), 1. 74-1. 50 (2H, m), 1. 38-1. 16 (2H, m) |
| 57 | $CDCl_3$*:8. 36 (1H, s), 8. 25-8. 18 (2H, m), 7. 99-7. 92 (3H, m), 7. 86-7. 78 (1H, m), 7. 66-7. 58 (1H, m), 6. 62-6. 56 (2H, m), 4. 25-4. 08 (2H, m), 3. 93-3. 62 (4H, m), 3. 36-3. 22 (2H, m), 2. 90-2. 54 (7H, m), 2. 44-2. 20 (3H, m), 2. 10-1. 10 (9H, m) |
| 58 | $CDCl_3$*:8. 39 (1H, s), 8. 23-8. 12 (2H, m), 8. 06-7. 94 (3H, m), 7. 80-7. 64 (3H, m), 6. 65-6. 57 (2H, m), 4. 25-4. 12 (2H, m), 3. 95-3. 75 (5H, m), 3. 50-3. 40 (1H, m), 3. 38 (1H, d, J=17Hz), 2. 84-2. 68 (4H, m), 2. 05-1. 89 (1H, m), 1. 69-1. 57 (2H, m), 1. 36-1. 13 (2H, m) |
| 59 | $CDCl_3$:8. 40 (1H, s), 8. 25-8. 18 (2H, m), 8. 08-7. 43 (3H, m), 7. 33-7. 14 (3H, m), 6. 62-6. 55 (2H, m), 4. 42-4. 33 (1H, m), 4. 24-4. 00 (3H, m), 3. 94-3. 76 (3H, m), 3. 58-3. 50 (1H, m), 3. 38 (1H, d, J=17Hz), 2. 83-2. 67 (4H, m), 2. 07 (3H, s), 2. 01-1. 87 (1H, m), 1. 68-1. 53 (2H, m), 1. 35-1. 18 (2H, m) |
| 60 | $CDCl_3$:8. 34 (1H, s), 8. 25-8. 18 (2H, m), 7. 98-7. 90 (3H, m), 7. 77 (1H, dd, J=2, 9Hz), 7. 64-7. 58 (1H, m), 6. 64-6. 57 (2H, m), 4. 38-4. 04 (5H, m), 3. 96-3. 75 (3H, m), 3. 46 (1H, d, J=17Hz), 3. 07-2. 96 (1H, m), 2. 88-2. 68 (2H, m), 2. 66-2. 55 (1H, m), 1. 93-1. 75 (1H, m), 1. 73-1. 55 (2H, m), 1. 32 (3H, t, J=7Hz), 1. 32-1. 14 (2H, m) |

# FIG.47

| Ex.No. | NMR (ppm) (*:300MHz,Without asterisk:270MHz) |
|---|---|
| 61 | $CDCl_3$:8.34 (1H, s), 8.25-8.18 (2H, m), 7.98-7.90 (3H, m), 7.77 (1H, dd, J=2, 9Hz), 7.64-7.58 (1H, m), 6.64-6.57 (2H, m), 4.38-4.04 (5H, m), 3.96-3.75 (3H, m), 3.46 (1H, d, J=17Hz), 3.07-2.96 (1H, m), 2.88-2.68 (2H, m), 2.66-2.55 (1H, m), 1.93-1.75 (1H, m), 1.73-1.55 (2H, m), 1.32 (3H, t, J=7Hz), 1.32-1.14 (2H, m) |
| 62 | $CDCl_3$:8.36 (1H, s), 8.24-8.17 (2H, m), 7.98-7.91 (3H, m), 7.80 (1H, dd, J=2, 9Hz), 7.61 (1H, dd, J=2, 9Hz), 6.60-6.54 (2H, m), 4.17 (1H, d, J=16Hz), 4.06 (1H, d, J=12Hz), 3.92-3.75 (3H, m), 3.65-3.33 (4H, m), 3.39 (3H, s), 2.80-2.65 (4H, m), 2.03-1.85 (1H, m), 1.66-1.53 (2H, m), 1.35-1.13 (2H, m) |
| 63 | $CDCl_3$:8.36 (1H, s), 8.24-8.17 (2H, m), 7.98-7.91 (3H, m), 7.80 (1H, dd, J=2, 9Hz), 7.61 (1H, dd, J=2, 9Hz), 6.60-6.54 (2H, m), 4.17 (1H, d, J=16Hz), 4.06 (1H, d, J=12Hz), 3.92-3.75 (3H, m), 3.65-3.33 (4H, m), 3.39 (3H, s), 2.80-2.65 (4H, m), 2.03-1.85 (1H, m), 1.66-1.53 (2H, m), 1.35-1.13 (2H, m) |
| 64 | DMSO-$d_6$*:13.31 (1H, brs), 8.61 (1H, s), 8.35-8.26 (2H, m), 8.24-8.11 (3H, m), 7.92-7.84 (1H, m), 7.79-7.72 (1H, m), 7.12 (2H, d, J=7Hz), 4.30 (1H, s), 4.21-4.02 (3H, m), 3.87 (1H, d, J=16Hz), 3.76-3.66 (1H, m), 3.36 (1H, d, J=16Hz), 3.13-2.91 (3H, m), 2.66-2.54 (1H, m), 2.04-1.83 (1H, m), 1.76-1.52 (2H, m), 1.29-0.91 (2H, m) |
| 65 | $CDCl_3$*:8.35 (1H, s), 8.26-8.19 (2H, m), 7.98-7.91 (3H, m), 7.80-7.74 (1H, m), 7.65-7.59 (1H, m), 6.61-6.56 (2H, m), 4.35-4.27 (1H, m), 4.24-4.05 (4H, m), 3.98-3.89 (1H, m), 3.86-3.76 (2H, m), 3.47 (1H, d, J=17Hz), 3.02 (1H, dd, J=3, 12Hz), 2.79-2.68 (2H, m), 2.63-2.54 (1H, m), 1.93-1.54 (5H, m), 1.33-1.17 (2H, m), 0.97 (3H, t, J=7Hz) |
| 66 | $CDCl_3$*:8.35 (1H, s), 8.26-8.19 (2H, m), 7.98-7.91 (3H, m), 7.80-7.74 (1H, m), 7.65-7.59 (1H, m), 6.61-6.56 (2H, m), 4.35-4.27 (1H, m), 4.24-4.05 (4H, m), 3.98-3.89 (1H, m), 3.86-3.76 (2H, m), 3.47 (1H, d, J=17Hz), 3.02 (1H, dd, J=3, 12Hz), 2.79-2.68 (2H, m), 2.63-2.54 (1H, m), 1.93-1.54 (5H, m), 1.33-1.17 (2H, m), 0.97 (3H, t, J=7Hz) |
| 67 | $CDCl_3$:8.34 (1H, d, J=1Hz), 8.22 (2H, dd, J=1, 5Hz), 8.00-7.89 (3H, m), 7.81-7.74 (1H, m), 7.65-7.58 (1H, m), 6.58 (2H, dd, J=1, 5Hz), 5.20-5.08 (1H, m), 4.37-(1H, m), 4.20 (1H, d, J=17Hz), 4.03 (1H, t, J=3Hz), 3.98-3.74 (3H, m), 3.41 (1H, d, J=17Hz), 3.02-2.92 (1H, m), 2.80-2.66 (2H, m), 2.60 (1H, dd, J=8, 14Hz), 1.92-1.53 (3H, m), 1.34 (3H, d, J=7Hz), 1.32 (3H, d, J=6Hz), 1.37-1.17 (2H, m) |

FIG.48

| Ex.No. | NMR (ppm) (*:300MHz,Without asterisk:270MHz) |
|---|---|
| 68 | $CDCl_3$:8.34 (1H, d, J=1Hz), 8.22 (2H, dd, J=1, 5Hz), 8.00-7.89 (3H, m), 7.81-7.74 (1H, m), 7.65-7.58 (1H, m), 6.58 (2H, dd, J=1, 5Hz), 5.20-5.08 (1H, m), 4.37-4.26 (1H, m), 4.20 (1H, d, J=17Hz), 4.03 (1H, t, J=3Hz), 3.98-3.74 (3H, m), 3.41 (1H, d, J=17Hz), 3.02-2.92 (1H, m), 2.80-2.66 (2H, m), 2.60 (1H, dd, J=8, 14Hz), 1.92-1.53 (3H, m), 1.34 (3H, d, J=7Hz), 1.32 (3H, d, J=6Hz), 1.37-1.17 (2H, m) |
| 69 | $CDCl_3$:8.34 (1H, s), 8.25-8.17 (2H, m), 7.98-7.89 (3H, m), 7.81-7.75 (1H, m), 7.64-7.58 (1H, m), 6.62-6.54 (2H, m), 4.36-4.27 (1H, m), 4.24-4.15 (1H, m), 3.99-3.74 (4H, m), 3.37 (1H, d, J=17Hz), 2.91 (1H, dd, J=3, 12Hz), 2.80-2.57 (3H, m), 1.88-1.48 (3H, m), 1.56 (9H, s), 1.33-1.13 (2H, m) |
| 70 | $CDCl_3$*:8.35 (1H, d, J=2Hz), 8.46-8.18 (2H, m), 7.98-7.92 (3H, m), 7.79 (1H, dd, J=2, 9Hz), 7.64-7.58 (1H, m), 6.62-6.68 (2H, m), 3.89-3.80 (2H, m), 3.74 (2H, s), 3.17 (2H, d, J=7Hz), 3.12 (2H, s), 2.80-2.67 (2H, m), 2.07-1.87 (1H, m), 1.71-1.62 (2H, m), 1.37 (6H, s), 1.36-1.18 (2H, m) |
| 71 | $CDCl_3$*:8.24 (2H, d, J=6Hz), 7.53-7.38 (5H, m), 6.70-6.57 (3H, m), 4.11 (1H, d, J=17Hz), 4.03-3.80 (4H, m), 3.70-3.46 (4H, m), 3.39 (3H, s), 3.11-3.03 (1H, m), 2.88-2.68 (3H, m), 2.12-1.90 (1H, m), 1.77-1.63 (2H, m), 1.43-1.16 (2H, m) |
| 72 | DMSO-$d_6$*:8.22-8.14 (2H, m), 7.83 (2H, d, J=8Hz), 7.53 (2H, d, J=8Hz), 7.47-7.33 (2H, m), 7.19-7.12 (2H, m), 4.24-4.12 (2H, m), 3.55-3.25 (4H, m), 3.21-3.04 (6H, m), 2.33 (3H, s), 2.30-2.18 (2H, m), 2.10-1.78 (3H, m), 1.18-1.00 (2H, m) |
| 73 | DMSO-$d_6$*:8.22-8.13 (2H, m), 7.84-7.77 (2H, m), 7.57-7.38 (4H, m), 7.16-7.08 (2H, m), 4.18-4.06 (2H, m), 3.77 (2H, s), 3.55-3.27 (2H, m), 3.25-2.99 (4H, m), 2.56-2.43 (2H, m), 2.29 (3H, s), 2.01-1.95 (1H, m), 1.72-1.61 (2H, m), 1.19-1.03 (2H, m) |
| 74 | DMSO-$d_6$*:8.63-8.60 (1H, m), 8.33-8.27 (2H, m), 8.22-8.15 (3H, m), 7.90 (1H, dd, J=2, 9Hz), 7.76 (1H, dd, J=2, 9Hz), 7.12 (1H, d, J=8Hz), 4.14-4.03 (2H, m), 3.69 (2H, s), 3.46-3.28 (4H, m), 3.14 (2H, d, J=7Hz), 3.07-2.92 (2H, m), 2.29 (3H, s), 2.00-1.83 (1H, m), 1.60-1.48 (2H, m), 1.12-0.94 (2H, m) |
| 75 | $CD_3OD$*:8.50-8.45 (1H, m), 8.16-8.00 (5H, m), 7.87-7.81 (1H, m), 7.69-7.63 (1H, m), 7.07 (2H, d, J=8Hz), 4.44-4.06 (7H, m), 3.84-3.73 (1H, m), 3.45 (1H, d, J=17Hz), 3.17-3.02 (3H, m), 2.79 (1H, dd, J=7, 14Hz), 2.69 (3H, s), 2.08-1.93 (1H, m), 1.88-1.78 (1H, m), 1.74-1.64 (1H, m), 1.29 (3H, t, J=7Hz), 1.34-1.22 (2H, m) |

# FIG.49

| Ex.No. | NMR (ppm) (*:300MHz,Without asterisk:270MHz) |
|---|---|
| 76 | DMSO-d6*:13.16 (1H, brs), 8.61 (1H, s), 8.34-8.26 (2H, m), 8.23-8.11 (3H, m), 7.92-7.83 (1H, m), 7.80-7.72 (1H, m), 7.12 (2H, d, J=7Hz), 4.30 (1H, s), 4.22-4.03 (3H, m), 3.87 (1H, d, J=16Hz), 3.77-3.66 (1H, m), 3.37 (1H, d, J=16Hz), 3.12-2.92 (3H, m), 2.68-2.56 (1H, m), 2.31 (3H, s), 2.03-1.86 (1H, m), 1.77-1.54 (2H, m), 1.29-0.92 (2H, m) |
| 77 | CD3OD*:8.43-8.37 (1H, m), 8.07-7.91 (5H, m), 7.82-7.76 (1H, m), 7.60-7.53 (1H, m), 6.98 (2H, d, J=8Hz), 4.14-3.98 (2H, m), 3.98-3.86 (2H, m), 3.79-3.59 (3H, m), 3.47-3.32 (3H, m), 3.08-2.75 (4H, m), 2.60 (3H, s), 2.09-1.94 (1H, m), 1.73-1.52 (2H, m), 1.23-1.14 (2H, m) |
| 78 | CD3OD:8.51-8.47 (1H, m), 8.17-8.00 (5H, m), 7.91-7.84 (1H, m), 7.69-7.63 (1H, m), 7.07 (2H, d, J=8Hz), 4.25-3.92 (4H, m), 3.86-3.74 (1H, m), 3.68-3.41 (4H, m), 3.34 (3H, s), 3.18-2.84 (4H, m), 2.69 (3H, s), 2.20-2.01 (1H, m), 1.85-1.62 (2H, m), 1.37-1.10 (2H, m) |
| 80 | CD3OD*:7.97-7.93 (2H, m), 7.60-7.52 (2H, m), 7.45-7.32 (3H, m), 7.06-6.95 (3H, m), 4.38-4.32 (1H, m), 4.28-4.02 (4H, m), 3.96-3.88 (1H, m), 3.79-3.60 (2H, m), 3.34-3.17 (2H, m), 3.14-3.00 (2H, m), 2.81-2.70 (1H, m), 2.60 (3H, s), 2.06-1.90 (1H, m), 1.86-1.64 (2H, m), 1.31-1.13 (5H, m) |
| 81 | CD3OD*:8.10-8.02 (2H, m), 7.69-7.61 (1H, m), 7.49-7.35 (4H, m), 7.16-7.04 (3H, m), 4.44-4.36 (1H, m), 4.31-4.14 (2H, m), 4.06-3.45 (4H, m), 3.44-3.09 (3H, m), 2.92-2.77 (1H, m), 2.69 (3H, s), 2.23-2.03 (1H, m), 1.98-1.74 (2H, m), 1.44-1.20 (2H, m) |
| 83 | CD3OD:8.55-8.49 (1H, m), 8.19-8.00 (5H, m), 7.90 (1H, dd, J=2, 9Hz), 7.67 (1H, dd, J=2, 9Hz), 7.06 (2H, d, J=8Hz), 4.26-4.06 (4H, m), 3.96-3.84 (2H, m), 3.52-2.64 (7H, m), 2.72 (6H, s), 2.24-2.06 (1H, m), 1.88-1.62 (2H, m), 1.46-1.12 (2H, m) |
| 89 | CD3OD*:8.50-8.45 (1H, m), 8.16-8.00 (5H, m), 7.87-7.81 (1H, m), 7.69-7.63 (1H, m), 7.07 (2H, d, J=8Hz), 4.44-4.06 (7H, m), 3.84-3.73 (1H, m), 3.45 (1H, d, J=17Hz), 3.17-3.02 (3H, m), 2.79 (1H, dd, J=7, 14Hz), 2.69 (3H, s), 2.08-1.93 (1H, m), 1.88-1.78 (1H, m), 1.74-1.64 (1H, m), 1.29 (3H, t, J=7Hz), 1.34-1.22 (2H, m) |
| 90 | CD3OD*:8.50-8.45 (1H, m), 8.16-8.00 (5H, m), 7.87-7.81 (1H, m), 7.69-7.63 (1H, m), 7.07 (2H, d, J=8Hz), 4.44-4.06 (7H, m), 3.84-3.73 (1H, m), 3.45 (1H, d, J=17Hz), 3.17-3.02 (3H, m), 2.79 (1H, dd, J=7, 14Hz), 2.69 (3H, s), 2.08-1.93 (1H, m), 1.88-1.78 (1H, m), 1.74-1.64 (1H, m), 1.29 (3H, t, J=7Hz), 1.34-1.22 (2H, m) |

FIG.50

| Ex.No. | NMR (ppm) (*:300MHz,Without asterisk:270MHz) |
|---|---|
| 91 | CD3OD:8.51-8.47 (1H, m), 8.17-8.00 (5H, m), 7.91-7.84 (1H, m), 7.69-7.63 (1H, m), 7.07 (2H, d, J=8Hz), 4.25-3.92 (4H, m), 3.86-3.74 (1H, m), 3.68-3.41 (4H, m), 3.34 (3H, s), 3.18-2.84 (4H, m), 2.69 (3H, s), 2.20-2.01 (1H, m), 1.85-1.62 (2H, m), 1.37-1.10 (2H, m) |
| 92 | CD3OD:8.51-8.47 (1H, m), 8.17-8.00 (5H, m), 7.91-7.84 (1H, m), 7.69-7.63 (1H, m), 7.07 (2H, d, J=8Hz), 4.25-3.92 (4H, m), 3.86-3.74 (1H, m), 3.68-3.41 (4H, m), 3.34 (3H, s), 3.18-2.84 (4H, m), 2.69 (3H, s), 2.20-2.01 (1H, m), 1.85-1.62 (2H, m), 1.37-1.10 (2H, m) |
| 93 | DMSO-d6*:13.16 (1H, brs), 8.61 (1H, s), 8.34-8.26 (2H, m), 8.23-8.11 (3H, m), 7.92-7.83 (1H, m), 7.80-7.72 (1H, m), 7.12 (2H, d, J=7Hz), 4.30 (1H, s), 4.22-4.03 (3H, m), 3.87 (1H, d, J=16Hz), 3.77-3.66 (1H, m), 3.37 (1H, d, J=16Hz), 3.12-2.92 (3H, m), 2.68-2.56 (1H, m), 2.31 (3H, s), 2.03-1.86 (1H, m), 1.77-1.54 (2H, m), 1.29-0.92 (2H, m) |
| 94 | CD3OD*:8.48 (1H, s), 8.17-7.99 (5H, m), 7.88-7.82 (1H, m), 7.66 (1H, dd, J=2, 9Hz), 7.06 (2H, d, J=8Hz), 4.45-4.39 (1H, m), 4.30 (1H, d, J=13Hz), 4.23-4.05 (5H, m), 3.80 (1H, dd, J=8, 14Hz), 3.46 (1H, d, J=17Hz), 3.16-3.00 (3H, m), 2.82-2.72 (1H, m), 2.69 (3H, s), 2.08-1.92 (1H, m), 1.88-1.77 (1H, m), 1.69-1.61 (3H, m), 1.34-1.11 (2H, m), 0.97 (3H, t, J=7Hz) |
| 95 | CD3OD*:8.48 (1H, s), 8.17-8.00 (5H, m), 7.85 (1H, dd, J=2, 9Hz), 7.66 (1H, dd, J=2, 9Hz), 7.03 (2H, d, J=8Hz), 5.16-5.01 (1H, m), 4.37 (1H, t, J=3Hz), 4.31 (1H, d, J=13Hz), 4.22-4.05 (3H, m), 3.86-3.73 (1H, m), 3.39 (1H, d, J=17Hz), 3.12-2.97 (3H, m), 2.82-2.71 (1H, m), 2.69 (3H, s), 2.07-1.90 (1H, m), 1.87-1.76 (1H, m), 1.72-1.61 (1H, m), 1.35-1.09 (2H, m), 1.32 (3H, d, J=6Hz), 1.31 (3H, d, J=6Hz) |

# INTERNATIONAL SEARCH REPORT

International application No.
PCT/JP98/06002

A. CLASSIFICATION OF SUBJECT MATTER
Int.Cl$^6$ C07D211/26, 401/04, 401/06, 401/14, A61K31/495, 31/505

According to International Patent Classification (IPC) or to both national classification and IPC

B. FIELDS SEARCHED

Minimum documentation searched (classification system followed by classification symbols)
Int.Cl$^6$ C07D211/26, 401/04, 401/06, 401/14, A61K31/495, 31/505

Documentation searched other than minimum documentation to the extent that such documents are included in the fields searched

Electronic data base consulted during the international search (name of data base and, where practicable, search terms used)
CAPLUS (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO, 93/10091, A1 (GLAXO GROUP Ltd.), 27 May, 1993 (27. 05. 93), Chuukantai 33 & EP, 542363, A & EP, 612313, A & JP, 7-501063, A | 1-3, 10<br>4-9, 11-16 |
| X<br>A | WO, 88/8424, A1 (THE UPJOHN Co.), 3 November, 1988 (03. 11. 88), Example 24 & EP, 293078, A & EP, 358676, A & EP, 487510, A & JP, 2-503198, A & US, 5120843, A | 1-3<br>4-16 |
| A | JP, 63-23874, A (Adir et Co.), 1 February, 1988 (01. 02. 88) & FR, 2601364, A & US, 4820707, A & EP, 262993, A | 1-16 |
| EX | WO, 99/6395, A1 (ZENECA LTD.), 11 February, 1999 (11. 02. 99) (Family: none) | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 1 April, 1999 (01. 04. 99) | 13 April, 1999 (13. 04. 99) |
| Name and mailing address of the ISA/<br>Japanese Patent Office | Authorized officer |
| Facsimile No. | Telephone No. |

Form PCT/ISA/210 (second sheet) (July 1992)